# EUROPEAN PATENT APPLICATION

(11) **EP 1 774 977 A2**
(43) Date of publication of application: **18.04.2007**
(21) Application number: 06124088.3
(22) Date of filing: 31.07.2000
(51) Int. Cl.: A61K 39/095

(54) **Genetically engineered bleb vaccine**

(30) Priority: 03.08.1999 GB 9918319
(62) Divisional of application: 00956369.3
(71) Applicant: GlaxoSmithKline Biologicals S.A., 1330 Rixensart (BE)
(72) Inventor: Berthet, Francois-Xavier Jacques, rue de l'Institut 89, 1330 Rixensart (BE); Dalemans, Wilfred L. J., rue de l'Institut 89, 1330 Rixensart (BE); Denoel, Philippe, rue de l'Institut 89, 1330 Rixensart (BE); Dequesne, Guy, rue de l'Institut 89, 1330 Rixensart (BE); Feron, Christiane, rue d l'Institut 89, 1330 Rixensart (BE); Lobet, Yves, rue de l'Institut 89, 1330 Rixensart (BE); Poolman, Jan, rue de l'Institut 89, 1330 Rixensart (BE); Thiry, Georges, rue de l'Institut 89, 1330 Rixensart (BE); Thonnard, Joelle, rue de l'Institut 89, 1330 Rixensart (BE); Voet, Pierre, rue de l'Institut 89, 1330 Rixensart (BE)
(74) Representative: Lubienski, Michael John

(57) **Abstract**

The present invention relates to an immune-protective and non-toxic Gram-negative bleb vaccine suitable for paediatric use. Examples of the Gram-negative strains from which the blebs are made are *N. meningitidis, M. catarrhalis* and *H. influenzae.* The blebs of the invention are improved by one or more genetic changes to the chromosome of the bacterium, including up-regulation of protective antigens, down-regulation of immunodominant non-protective antigens, and detoxification of the Lipid A moiety of LPS.

## Description

### FIELD OF THE INVENTION

The present invention relates to the field of Gram-negative bacterial vaccine compositions, their manufacture, and the use of such compositions in medicine. More particularly it relates to the field of novel outer-membrane vesicle (or bleb) vaccines, and advantageous methods of rendering these vaccines more effective and safer.

### BACKGROUND OF THE INVENTION

Gram-negative bacteria are separated from the external medium by two successive layers of membrane structures. These structures, referred to as the cytoplasmic membrane and the outer membrane (OM), differ both structurally and functionally. The outer membrane plays an important role in the interaction of pathogenic bacteria with their respective hosts. Consequently, the surface exposed bacterial molecules represent important targets for the host immune response, making outer-membrane components attractive candidates in providing vaccine, diagnostic and therapeutics reagents.

Whole cell bacterial vaccines (killed or attenuated) have the advantage of supplying multiple antigens in their natural micro-environment. Drawbacks around this approach are the side effects induced by bacterial components such as endotoxin and peptidoglycan fragments. On the other hand, acellular subunit vaccines containing purified components from the outer membrane may supply only limited protection and may not present the antigens properly to the immune system of the host.

Proteins, phospholipids and lipopolysaccharides are the three major constituents found in the outer-membrane of all Gram-negative bacteria. These molecules are distributed asymmetrically: membrane phospholipids (mostly in the inner leaflet), lipooligosaccharides (exclusively in the outer leaflet) and proteins (inner and outer leaflet lipoproteins, integral or polytopic membrane proteins). For many bacterial pathogens which impact on human health, lipopolysaccharide and outer-membrane proteins have been shown to be immunogenic and amenable to confer protection against the corresponding disease by way of immunization.

The OM of Gram-negative bacteria is dynamic and, depending on the environmental conditions, can undergo drastic morphological transformations. Among these manifestations, the formation of outer-membrane vesicles or "blebs" has been studied and documented in many Gram-negative bacteria (Zhou, L et al. 1998. FEMS Microbiol. Lett. 163: 223-228). Among these, a non-exhaustive list of bacterial pathogens reported to produce blebs include: *Bordetella pertussis, Borrelia burgdorferi, Brucella melitensis, Brucella ovis, Chlamydia psittaci, Chlamydia trachomatis, Esherichia coli, Haemophilus influenzae, Legionella pneumophila, Neisseria gonorrhoeae, Neisseria meningitidis, Pseudomonas aeruginosa* and *Yersinia enterocolitica.* Although the biochemical mechanism responsible for the production of OM blebs is not fully understood, these outer membrane vesicles have been extensively studied as they represent a powerful methodology in order to isolate outer-membrane protein preparations in their native conformation. In that context, the use of outer-membrane preparations is of particular interest to develop vaccines against *Neisseria, Moraxella catarrhalis, Haemophilus influenzae, Pseudomonas aeruginosa* and *Chlamydia.* Moreover, outer membrane blebs combine multiple proteinaceaous and non-proteinaceous antigens that are likely to confer extended protection against intra-species variants.

In comparison with the other, more widely used, types of bacterial vaccine (whole cell bacterial and purified subunit vaccines), the inventors will show that outer membrane bleb vaccines (if modified in certain ways) may represent the ideal compromise.

The wide-spread use of bacterial subunit vaccines has been due to the intensive study of bacterial surface proteins that have been found to be useful in vaccine applications [for instance *B. pertussis* pertactin]. These proteins are loosely associated with the bacterial outer membrane and can be purified from culture supernatant or easily extracted from the bacterial cells. However it has also been shown that structural, integral outer membrane proteins are also protective antigens. Examples are PorA for *N. meningitidis* serogroup B; D15 for *H. influenzae;* OMP CD for *M. catarrhalis;* OMP F for *P. Aeruginosa.* Such proteins however have rather specific structural features, particularly multiple amphipathic β-sheets, which complicates their straightforward use as purified (recombinant) subunit vaccines.

In addition, it has become clear that multiple component vaccines are needed (in terms of bacterial surface proteins and integral membrane proteins) to supply a reasonable level of protection. For instance, in the case of *B. pertussis* subunit vaccines multicomponent vaccines are superior to mono or bicomponcnt products.

In order to incorporate integral outer-membrane proteins into such a subunit product, native (or near-native) conformational folding of the proteins must be present in the product in order to have a useful immunological effect. The use of excreted outer membrane vesicles or blebs may be an elegant solution to the problem of including protective integral membrane proteins into a subunit vaccine whilst still ensuring that they fold properly.

*N. meningitidis* serogroup B (menB) excretes outer membrane blebs in sufficient quantities to allow their manufacture on an industrial scale. Such multicomponent outer-membrane protein vaccines from naturally-occurring menB strains have been found to be efficacious in protecting teenagers from menB disease and have become registered in Latin America. An alternative method of preparing outer-membrane vesicles is via the process of detergent extraction of the bacterial cells (EP 11243).

Examples of bacterial species from which bleb vaccines can be made are the following.

### Neisseria meningitidis:

*Neisseria meningitidis* (meningococcus) is a Gram-negative bacterium frequently isolated from the human upper respiratory tract. It occasionally causes invasive bacterial diseases such as bacteremia and meningitis. The incidence of meningococcal disease shows geographical seasonal and annual differences (Schwartz, B., Moore, P.S., Broome, C.V.; Clin. Microbiol. Rev. 2 (Supplement), S18-S24, 1989). Most disease in temperate countries is due to strains of serogroup B and varies in incidence from 1-10/100,000/year total population sometimes reaching higher values (Kaczmarski, E.B. (1997), Commun. Dis. Rep. Rev. 7: R55-9, 1995; Scholten, R.J.P.M., Bijlmer, H.A., Poolman, J.T. et al. Clin. Infect. Dis. 16: 237-246, 1993; Cruz, C., Pavez, G., Aguilar, E., et al. Epidemiol. Infect. 105: 119-126, 1990). Age-specific incidences in the two high risk-groups, infants and teenagers, reach higher levels.

Epidemics dominated by serogroup A meningococci occur, mostly in central Africa, sometimes reaching levels up to 1000/100,000/year ( Schwartz, B., Moore, P.S., Broome, C.V. Clin. Microbiol. Rev. 2 (Supplement), S18-524, 1989). Nearly all cases of meningococcal disease as a whole are caused by serogroup A, B, C, W-135 and Y meningococci. A tetravalent A, C, W-135, Y capsular polysaccharide vaccine is available (Armand, J., Arminjon, F., Mynard, M.C., Lafaix, C., J. Biol. Stand. 10: 335-339, 1982).

The polysaccharide vaccines are currently being improved by way of chemically conjugating them to carrier proteins (Lieberman, J.M., Chiu, S.S., Wong, V.K., et al. JAMA 275 : 1499-1503, 1996). A serogroup B vaccine is not available, since the B capsular polysaccharide is non-immunogenic, most likely because it shares structural similarity to host components (Wyle, F.A., Artenstein, M.S., Brandt, M.L. et al. J. Infect. Dis. 126: 514-522, 1972; Finne, J.M., Leinonen, M., Mäkelä, P.M. Lancet ii.: 355-357, 1983).

For many years efforts have been focused on developing meningococcal outer membrane based vaccines (de Moraes, J.C., Perkins, B., Camargo, M.C. et al. Lancet 340: 1074-1078, 1992; Bjune, G., Hoiby, E.A. Gronnesby, J.K. et al. 338: 1093-1096, 1991). Such vaccines have demonstrated efficacies from 57% - 85% in older children (>4 years) and adolescents. Most of these efficacy trials were performed with OMV (outer membrane vesicles, derived by LPS depletion from blebs) vaccines derived from wild-type *N. meningitidis* B strains.

Many bacterial outer membrane components are present in these vaccines, such as PorA, PorB, Rmp, Opc, Opa, FrpB and the contribution of these components to the observed protection still needs further definition. Other bacterial outer membrane components have been defined (using animal or human antibodies) as potentially being relevant to the induction of protective immunity, such as TbpB, NspA (Martin, D., Cadieux, N., Hamel, J., Brodeux, B.R., J. Exp. Med. 185: 1173-1183, 1997; Lissolo, L., Maitre-Wilmotte, C., Dumas, p. et al., Inf. Immun. 63: 884-890, 1995). The mechanism of protective immunity will involve antibody mediated bactericidal activity and opsonophagocytosis.

The frequency of *Neisseria meningitidis* infections has risen dramatically in the past few decades. This has been attributed to the emergence of multiple antibiotic resistant strains, and increased exposure due to an increase in social activities (for instance swimming pools or theatres). It is no longer uncommon to isolate *Neisseria meningitidis* strains that are resistant to some or all of the standard antibiotics. This phenomenon has created an unmet medical need and demand for new anti-microbial agents, vaccines, drug screening methods, and diagnostic tests for this organism.

### Moraxella catarrhalis

*Moraxella catarrhalis* (also named *Branhamella catarrhalis)* is a Gram-negative bacterium frequently isolated from the human upper respiratory tract. It is responsible for several pathologies, the main ones being otitis media in infants and children, and pneumonia the elderly. It is also responsible for sinusitis, nosocomial infections and, less frequently, for invasive diseases.

Bactericidal antibodies have been identified in most adults tested (Chapman, AJ et al. (1985) J. lnfect.Dis. 151:878). Strains of *M. catarrhalis* present variations in their capacity to resist serum bactericidal activity: in general, isolates from diseased individuals are more resistant than those who are simply colonized (Hol, C et al. (1993) Lancet 341:1281, Jordan, KL et al. (1990) Am.J.Med. 88 (suppl. 5A):28S). Serum resistance could therfore be considered as a virulence factor of the bacteria. An opsonizing activity has been observed in the sera of children recovering from otitis media.

The antigens targetted by these different immune responses in humans have not been identified, with the exception of OMP B1, a 84 kDa protein, the expression of which is regulated by iron, and that is recognized by the sera of patients with pneumonia (Sethi, S, et al. (1995) Infect.Immun. 63:1516), and of UspA1 and UspA2 (Chen D. et al.(1999), Infect.Immun. 67:1310).

A few other membrane proteins present on the surface of *M. catarrhalis* have been characterized using biochemical methods for their potential implication in the induction of a protective immunity (for review, see Murphy, TF (1996) Microbiol.Rev. 60:267). In a mouse pneumonia model, the presence of antibodies raised against some of them (UspA, CopB) favors a faster clearance of the pulmonary infection. Another polypeptide (OMP CD) is highly conserved among *M. catarrhalis* strains, and presents homologies with a porin of *Pseudomonas aeruginosa,* which has been demonstrated to be efficacious against this bacterium in animal models.

*M. catarrhalis* produces outer membrane vesicles (Blebs). These Blebs have been isolated or extracted by using different methods (Murphy T.F., Loeb M.R. 1989. Microb. Pathog. 6: 159-174; Unhanand M., Maciver, I., Ramillo O., Arencibia-Mireles O., Argyle J.C., McCracken G.H. Jr., Hansen E.J. 1992. J. Infect. Dis. 165:644-650). The protective capacity of such Bleb preparations has been tested in a murine model for pulmonary clearance of *M. catarrhalis.* It has been shown that active immunization with Bleb vaccine or passive transfer of anti-Blebs antibody induces significant protection in this model (Maciver I., Unhanand M., McCracken G.H. Jr., Hansen, E.J. 1993. J. Infect. Dis. 168: 469-472).

### Haemophilus influenzae

*Haemophilus influenzae* is a non-motile Gram-negative bacterium. Man is its only natural host. *H. influenzae* isolates are usually classified according to their polysaccharide capsule. Six different capsular types designated 'a' through `f' have been identified. Isolates that fail to agglutinate with antisera raised against one of these six serotypes are classified as nontypeable, and do not express a capsule.

*H. influenzae* type b (Hib) is clearly different from the other types in that it is a major cause of bacterial meningitis and systemic diseases. Nontypeable strains of *H*. *influenzae* (NTHi) are only occasionally isolated from the blood of patients with systemic disease. NTHi is a common cause of pneumonia, exacerbation of chronic bronchitis, sinusitis and otitis media. NTHi strains demonstrate a large variability as identified by clonal analysis, whilst Hib strains as a whole are more homogeneous.

Various proteins of *H. influenzae* have been shown to be involved in pathogenesis or have been shown to confer protection upon vaccination in animal models.

Adherence of NTHi to human nasopharygeal epithelial cells has been reported (Read RC. et al. 1991. J. Infect. Dis. 163:549). Apart from fimbriae and pili (Brinton CC. et al. 1989. Pediatr. Infect. Dis. J. 8:S54; Kar S. et al. 1990. Infect. Immun. 58:903; Gildorf JR. et al. 1992. Infect. Immun. 60:374; St. Geme JW et al. 1991. Infect. Immun. 59:3366; St. Geme JW et al. 1993. Infect. Immun. 61: 2233), many adhesins have been identified in NTHi. Among them, two surface exposed high-molecular-weight proteins designated HMW1 and HMW2 have been shown to mediate adhesion of NTHi to epithelial cells (St. Geme JW. et al. 1993. Proc. Natl. Acad. Sci. USA 90:2875). Another family of high-molecular-weight proteins has been identified in NTHi strains that lack proteins belonging to HMW1/HMW2 family. The NTHi 115-kDa Hia protein (Barenkamp SJ., St Geme S. W. 1996. Mol. Microbiol. In press) is highly similar to the Hsf adhesin expressed by *H. influenzae* type b strains ( St. Geme JW. et al. 1996. J. Bact. 178:6281). Another protein, the Hap protein shows similarity to IgA1 serine proteases and has been shown to be involved in both adhesion and cell entry (St. Geme JW. et al. 1994. Mol. Microbiol. 14:217).

Five major outer membrane proteins (OMP) have been identified and numerically numbered. Original studies using *H.influenzae* type b strains showed that antibodies specific for P1 and P2 OMPs protected infant rats from subsequent challenge (Loeb MR. et al. 1987. Infect. Immun. 55:2612; Musson RS. Jr. et al. 1983. J. Clin. Invest. 72:677). P2 was found to be able to induce bactericidal and opsonic antibodies, which are directed against the variable regions present within surface exposed loop structures of this integral OMP (Haase EM. et al. 1994 Infect. Immun. 62:3712; Troelstra A. et al. 1994 Infect. Immun. 62:779). The lipoprotein P4 also may induce bactericidal antibodies (Green BA. et al. 1991. Infect.Immun.59:3191).

OMP P6 is a conserved peptidoglycan associated lipoprotein making up 1-5 % of the outer membrane (Nelson MB. et al. 1991. Infect. Immun. 59:2658). Later a lipoprotein of about the same molecular weight was recognized called PCP (P6 cross-reactive protein) (Deich RM. et al. 1990. Infect. Immun. 58:3388). A mixture of the conserved lipoproteins P4, P6 and PCP did not reveal protection as measured in a chinchilla otitis-media model (Green BA. et al. 1993. Infect.immun. 61:1950). P6 alone appears to induce protection in the chinchilla model (Demaria TF. et al. 1996. Infect. Immun. 64:5187).

A fimbrin protein (Miyamoto N., Bakaletz, LO. 1996. Microb. Pathog. 21:343) has also been described with homology to OMP P5, which itself has sequence homology to the integral *Escherichia coli* OmpA (Miyamoto N., Bakaletz, LO. 1996. Microb. Pathog. 21:343; Munson RS. Jr. et al. 1993. Infect. Immun. 61:1017). NTHi seem to adhere to mucus by way of fimbriae.

In line with the observations made with gonococci and meningococci, NTHi expresses a dual human transferrin receptor composed of TbpA and TbpB when grown under iron limitation. Anti-TbpB antibody protected infant rats (Loosmore SM. et al. 1996. Mol. Microbiol. 19:575). Hemoglobin / haptoglobin receptor also have been described for NTHi (Maciver I. et al. 1996. Infect. Immun. 64:3703). A receptor for Haem:Hemopexin has also been identified (Cope LD. et al. 1994. Mol.Microbiol. 13:868). A lactoferrin receptor is also present amongst NTHi, but is not yet characterized (Schryvers AB. et al. 1989. J. Med. Microbiol. 29:121). A protein similar to neisserial FrpB-protein has not been described amongst NTHi.

An 80kDa OMP, the D15 surface antigen, provides protection against NTHi in a mouse challenge model. (Flack FS. et al. 1995. Gene 156:97). A 42kDa outer membrane lipoprotein, LPD is conserved amongst *Haemophilus influenzae* and induces bactericidal antibodies (Akkoyunlu M. et al. 1996. Infect. Immun. 64:4586). A minor 98kDa OMP (Kimura A. et al. 1985. Infect. Immun. 47:253), was found to be a protective antigen, this OMP may very well be one of the Fe-limitation inducible OMPs or high molecular weight adhesins that have been characterized thereafter. *H*. *Influenzae* produces IgA1-protease activity (Mulks MH., Shoberg RJ. 1994. Meth. Enzymol. 235:543). IgA1-proteases of NTHi have a high degree of antigenic variability (Lomholt H., van Alphen L., Kilian, M. 1993. Infect. Immun. 61:4575).

Another OMP of NTHi, OMP26, a 26-kDa protein has been shown to enhance pulmonary clearance in a rat model (Kyd, J.M., Cripps, A.W. 1998. Infect. Immun. 66:2272). The NTHi HtrA protein has also been shown to be a protective antigen. Indeed, this protein protected Chinchilla against otitis media and protected infant rats against *H. influenzae* type b bacteremia (Loosmore S.M. et al. 1998. Infect. Immun. 66:899).

Outer membrane vesicles (or blebs) have been isolated from *H. influenzae* (Loeb M.R., Zachary A.L., Smith D.H. 1981. J. Bacteriol. 145:569-604; Stull T.L., Mack K., Haas J.E., Smit J., Smith A.L. 1985. Anal. Biochem. 150: 471-480). The vesicles have been associated with the induction of blood-brain barrier permeability (Wiwpelwey B., Hansen E.J., Scheld W.M. 1989 Infect. Immun. 57: 2559-2560), the induction of meningeal inflammation (Mustafa M.M., Ramilo O., Syrogiannopoulos G.A., Olsen K.D., McCraken G.H. Jr., Hansen, E.J. 1989. J. Infect. Dis. 159: 917-922) and to DNA uptake (Concino M.F., Goodgal S.H. 1982 J. Bacteriol. 152: 441-450). These vesicles are able to bind and be absorbed by the nasal mucosal epithelium (Harada T., Shimuzu T., Nishimoto K., Sakakura Y. 1989. Acta Otorhinolarygol. 246: 218-221) showing that adhesins and/or colonization factors could be present in Blebs. Immune response to proteins present in outer membrane vesicles has been observed in patients with various *H. influenzae* diseases (Sakakura Y., Harada T., Hamaguchi Y., Jin C.S. 1988. Acta Otorhinolarygol. Suppl. (Stockh.) 454: 222-226; Harada T., Sakakura Y., Miyoshi Y. 1986. Rhinology 24: 61-66).

### Pseudomonas aeruginosa:

The genus *Pseudomonas* consists of Gram-negative, polarly flagellated, straight and slightly curved rods that grow aerobically and do not forms spores. Because of their limited metabolic requirements, *Pseudomonas spp.* are ubiquitous and are widely distributed in the soil, the air, sewage water and in plants. Numerous species of *Pseudomonas* such as *P. aeruginosa, P. pseudomallei, P. mallei, P. maltophilia and P. cepacia* have also been shown to be pathogenic for humans. Among this list, *P. aeruginosa* is considered as an important human pathogen since it is associated with opportunistic infection of immuno-compromised host and is responsible for high morbidity in hospitalized patients. Nosocomial infection with *P. aeruginosa* afflicts primarily patients submitted for prolonged treatment and receiving immuno-suppressive agents, corticosteroids, antimetabolites antibiotics or radiation.

The *Pseudomonas,* and particularly *P. aeruginosa,* produces a variety of toxins (such as hemolysins, fibrinolysins, esterases, coagulases, phospholipases, endo- and exo-toxins) that contribute to the pathogenicity of these bacteria. Moreover, these organisms have high intrinsic resistance to antibiotics due to the presence of multiple drug efflux pumps. This latter characteristic often complicates the outcome of the disease.

Due to the uncontrolled use of antibacterial chemotherapeutics the frequency of nosocomial infection caused by *P. aeruginosa* has increased considerably over the last 30 years. In the US, for example, the economic burden of *P. aeruginosa* nosocomial infection is estimated to 4.5 billion US$ annually. Therefore, the development of a vaccine for active or passive immunization against *P. aeruginosa* is actively needed (for review see Stanislavsky et al. 1997. FEMS Microbiol. Lett. 21: 243-277).

Various cell-associated and secreted antigens of *P. aeruginosa* have been the subject of vaccine development. Among *Pseudomonas* antigens, the mucoid substance, which is an extracellular slime consisting predominantly of alginate, was found to be heterogenous in terms of size and immunogenicity. High molecular mass alginate components (30-300 kDa) appear to contain conserved epitopes while lower molecular mass alginate components (10-30 kDa) possess conserved epitopes in addition to unique epitopes. Among surface-associated proteins, PcrV, which is part of the type III secretion-translocation apparatus, has also been shown to be an interesting target for vaccination (Sawa et al. 1999. Nature Medicine 5:392-398).

Surface-exposed antigens including O-antigens (O-specific polysaccharide of LPS) or H-antigens (flagellar antigens) have been used for serotyping due to their highly immunogenic nature. Chemical structures of repeating units of O-specific polysaccharides have been elucidated and these data allowed the identification of 31 chemotypes of *P. aeruginosa.* Conserved epitopes among all serotypes *of P. aeruginosa* are located in the core oligosaccharide and the lipid A region of LPS and immunogens containing these epitopes induce cross-protective immunity in mice against different *P. aeruginosa* immunotypes. The outer core of LPS was implicated to be a ligand for binding of *P. aeruginosa* to airway and ocular epithelial cells of animals. However, heterogeneity exists in this outer core region among different serotypes. Epitopes in the inner core are highly conserved and have been demonstrated to be surface-accessible, and not masked by O-specific polysaccharide.

To examine the protective properties of OM proteins, a vaccine containing *P. aeruginosa* OM proteins of molecular masses ranging from 20 to 100 kDa has been used in pre-clinical and clinical trials. This vaccine was efficacious in animal models against *P. aeruginosa* challenge and induced high levels of specific antibodies in human volunteers. Plasma from human volunteers containing anti-P. *aeruginosa* antibodies provided passive protection and helped the recovery of 87% of patients with severe forms of *P. aeruginosa* infection. More recently, a hybrid protein containing parts of the outer membrane proteins OprF (amino acids 190-342) and OprI (amino acids 21-83) *from Pseudomonas aeruginosa* fused to the glutathione-S-transferase was shown to protect mice against a 975-fold 50% lethal dose of *P. aeruginosa* (Knapp et al. 1999. Vaccine. 17:1663-1669).

The present inventors have realised a number of drawbacks associated with the above wild-type bleb vaccines (either naturally occurring or chemically made).

Examples of such problems are the following:
- the presence of immunodominant but variable proteins on the bleb (PorA; TbpB, Opa [*N. meningitidis* B]; P2, P5 [non-typeable *H. influenzae*]) *-* such blebs being effective only against a restricted selection of bacterial species. Type-specificity of the bactericidal antibody response may preclude the use of such vaccines in infancy.
- the presence of unprotective (non relevant) antigens (Rmp, H8, ...) on the bleb - antigens that are decoys for the immune system
- the lack of presence of important molecules which are produced conditionally (for instance iron-regulated outer membrane proteins, IROMP's, in vivo regulated expression mechanisms) - such conditions are hard to control in bleb production in order to optimise the amount of antigen on the surface
- the low level of expression of protective, (particularly conserved) antigens (NspA, P6)
- the toxicity of the LPS remaining on the surface of the bleb
- the potential induction of an autoimmune response because of host-identical structures (for example the capsular polysaccharide in *Neisseria meningitidis* serogroup B, the lacto-N-neotetraose in *Neisseria* LPS, saccharide structure within ntHi LPS, saccharide structures within Pili).

Such problems may prevent the use of bleb vaccines as human vaccine reagents. This is particularly so for paediatric use (<4 years) where reactogenicity against bleb vaccines is particularly important, and where bleb vaccines (for instance the previously mentioned marketed MenB bleb vaccine) have been shown to be ineffective at immuno-protecting. Accordingly, the present invention provides methods of alleviating the above problems using genetically engineered bacterial strains, which result in improved bleb vaccines. Such methods will be especially useful in the generation of new vaccines against bacterial pathogens such as *Neisseiria meningitidis, Moraxella catarrhalis, Haemophilus influenzae, Pseudomonas aeruginosa,* and others.

The bleb vaccines of the invention are designed to focus the immune response on a few protective (preferably conserved) antigens or epitopes - formulated in a multiple component vaccine. Where such antigens are integral OMPs, the outer membrane vesicles of bleb vaccines will ensure their proper folding. This invention provides methods to optimize the OMP and LPS composition of OMV (bleb) vaccines by deleting immunodominant variable as well as non protective OMPs, by creating conserved OMPs by deletion of variable regions, by upregulating expression of protective OMPs, and by eliminating control mechanisms for expression (such as iron restriction) of protective OMPs. In addition the invention provides for the reduction in toxicity of lipid A by modification of the lipid portion or by changing the phosphoryl composition whilst retaining its adjuvant activity or by masking it. Each of these new methods of improvement individually improve the bleb vaccine, however a combination of one or more of these methods work in conjunction so as to produce an optimised engineered bleb vaccine which is immuno-protective and non-toxic - particularly suitable for paediatric use.

### SUMMARY OF THE INVENTION

The present invention provides a genetically-engineered bleb preparation from a Gram-negative bacterial strain characterized in that said preparation is obtainable by employing one or more processes selected from the following group:
a) a process of reducing immunodominant variable or non-protective antigens within the bleb preparation comprising the steps of determining the identity of such antigen, engineering a bacterial strain to produce less or none of said antigen, and making blebs from said strain;
b) a process of upregulating expression of protective, endogenous (and preferably conserved) OMP antigens within the bleb preparation comprising the steps of identifying such antigen, engineering a bacterial strain so as to introduce a stronger promoter sequence upstream of a gene encoding said antigen such that said gene is expressed at a level higher than in the non-modified bleb, and making blebs from said strain;
c) a process of upregulating expression of conditionally-expressed, protective (and preferably conserved) OMP antigens within the bleb preparation comprising the steps of identifying such an antigen, engineering a bacterial strain so as to remove the repressive control mechanisms of its expression (such as iron restriction), and making blebs from said strain;
d) a process of modifying lipid A portion of bacterial LPS within the bleb preparation, comprising the steps of identifying a gene involved in rendering the lipid A portion of LPS toxic, engineering a bacterial strain so as to reduce or switch off expression of said gene, and making blebs from said strain;
e) a process of modifying lipid A portion of bacterial LPS within the bleb preparation, comprising the steps of identifying a gene involved in rendering the lipid A portion of LPS less toxic, engineering a bacterial strain so as to introduce a stronger promoter sequence upstream of said gene such that said gene is expressed at a level higher than in the non-modified bleb, and making blebs from said strain;
f) a process of reducing lipid A toxicity within the bleb preparation and increasing the levels of protective antigens, comprising the steps of engineering the chromosome of a bacterial strain to incorporate a gene encoding a Polymyxin A peptide, or a derivative or analogue thereof, fused to a protective antigen, and making blebs from said strain;
g) a process of creating conserved OMP antigens on the bleb preparation comprising the steps of identifying such antigen, engineering a bacterial strain so as to delete variable regions of a gene encoding said antigen, and making blebs from said strain;
h) a process of reducing expression within the bleb preparation of an antigen which shares a structural similarity with a human structure and may be capable of inducing an auto-immune response in humans (such as the capsular polysaccharide of *N. meningitidis* B), comprising the steps of identifying a gene involved in the biosynthesis of the antigen, engineering a bacterial strain so as to reduce or switch off expression of said gene, and making blebs from said strain; or
i) a process of upregulating expression of protective, endogenous (and preferably conserved) OMP antigens within the bleb preparation comprising the steps of identifying such antigen, engineering a bacterial strain so as to introduce into the chromosome one or more further copies of a gene encoding said antigen controlled by a heterologous, stronger promoter sequence, and making blebs from said strain.

Further aspects of the invention include, preferential processes for obtaining the above bleb preparation, including optimal positioning of strong promoters for the upregulation of expression of antigens within blebs, preferential antigens for upregulation and downreguation for various bacterial strains in order to obtain bleb preparations particularly suitable for vaccine use. Preferential formulations comprising the blebs of the invention are also provided which are particularly suitable for global vaccines against certain disease states. Vectors for producing the blebs of the invention, and modified bacterial strains from which the blebs of the invention are produced are still further aspects of the invention.

The present invention provides for the first time a bleb vaccine which is immuno-protective and non-toxic when used with children under 4 years of age.

### BRIEF DESCRIPTION OF THE DRAWINGS

**Figure 1:** Reactivity of the 735 mAb on different colonies.
**Figure 2:** Reactivities of specific monoclonal antibodies by whole cell Elisa.
**Figure 3:** Schematic representation of the pCMK vectors used to deliver genes, operons and/or expression cassettes in the genome of *Neisseria meningitidis.*
**Figure 4:** Analysis of PorA expression in total protein extracts of recombinant *N*. *meningitidis* serogroupB (H44/76 derivatives). Total proteins were recovered from cps- (lanes 3 and 4), *cps- porA::* pCMK+ (lanes 2 and 5) and *cps- porA::nspA* (lanes 1 and 6) recombinant *N. meningitidis* serogroupB strains and were analyzed under SDS-PAGE conditions in a 12% polyacrylamide gel. Gels were stained with Coomassie blue (lanes 1 to 3) or transferred to a nitrocellulose membrane and immuno-stained with an anti-PorA monoclonal antibody.

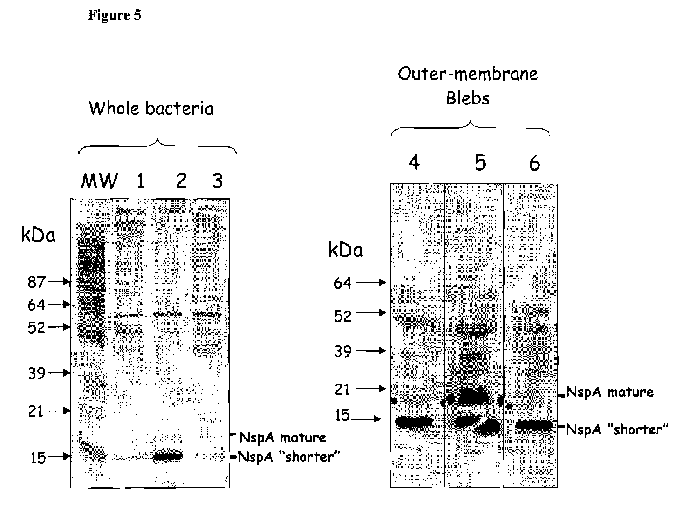

**Figure 5:** Analysis of NspA expression in protein extracts of recombinant *N*. *meningitidis* serogroupB strains (H44/76 derivatives). Proteins were extracted from whole bacteria (lanes 1 to 3) or outer-membrane blebs preparations (lanes 4 to 6) separated by SDS-PAGE on a 12% acrylamide gel and analyzed by immuno-blotting using an anti-NspA polyclonal serum. Samples corresponding to *cps-* (lanes 1 and 6), *cps- pora::pCMK+* (lanes 3 and 4) and *cps- porA::nspA* (lanes 2 and 5) were analyzed. Two forms of NspA were detected: a mature form (18kDa) co-migrating with the recombinant purified NspA, and a shorter form (15kDa).
**Figure 6**: Analysis of D15/omp85 expression in protein extracts of recombinant *N. meningitidis* serogroupB strains (H44/76 derivatives). Proteins were extracted from outer-membrane blebs preparations and were separated by SDS-PAGE on a 12% acrylamide gel and analyzed by immuno-blotting using an anti-omp85 polyclonal serum. Samples corresponding to *cps-* (lane 2), and *cps-, PorA*+*, pCMK*+O*mp85*/*D15* (lane 1) recombinant *N. meningitidis* serogroupB strains were analyzed.
**Figure 7:** General strategy for modulating gene expression by promoter delivery (RS stands for restriction site).
**Figure 8:** Analysis of outer-membrane blebs produced by recombinant *N. meningitidis* serogroupB *cps-* strains (H44/76 derivatives). Proteins were extracted from outer-membrane bleb preparations and were separated by SDS-PAGE under reducing conditions on a 4-20% gradient polyacrylamide gel. The gel was stained with Coomassie brilliant blue R250. Lanes 2, 4, 6 corresponded to 5µg of total proteins whereas lanes 3, 5 and 7 were loaded with 10µg proteins.
**Figure 9:** Construction of a promoter replacement plasmid used to up-regulate the expression/production ofOmp85/D15 in *Neisseria meningitidis* H44/76.
**Figure 10:** Analysis of OMP85 expression in total protein extracts of recombinant *NmB* (H44/76 derivatives). Gels were stained with Coomassie blue (A) or transferred to nitrocellulose membrane and immuno-stained with rabbit anti-OMP85 (*N.gono*) monoclonal antibody (B).

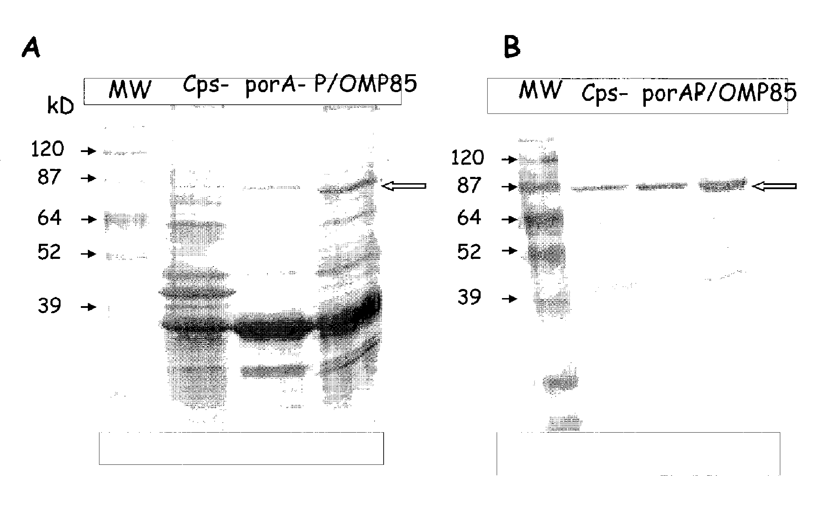

**Figure 11:** Analysis of OMP85 expression in OMV preparations from recombinant *NmB* (H44/76 derivatives). Gels were stained with Coomassie blue (A) or transferred to nitrocellulose membrane and immuno-stained with rabbit anti-OMP85 polyclonal antibody (B).
**Figure 12:** Schematic representation of the recombinant PCR strategy used to delete the *lacO* in the chimeric *porA*/*lacO* promoter.
**Figure 13:** Analysis of Hsf expression in total protein extracts of recombinant *N. meningitidis* serogroup B (H44/76 derivatives). Total proteins were recovered from Cps-PorA+(lanes 1), and Cps-PorA+/Hsf (lanes 2) recombinant *N. meningitidis* serogroup B strains and were analyzed under SDS-PAGE conditions in a 12% polyacrylamide gel. Gels were stained with Coomassie blue.
**Figure 14:** Analysis of GFP expression in total protein extracts of recombinant *N. meningitidis* (H44/76 derivative). Total protein were recovered from Cps-, PorA+ (lane1), Cps-, PorA- GFP+ (lane2 & 3) recombinant strains. Proteins were separated by PAGE-SDS in a 12% polyacrylamide gel and then stained with Coomassie blue.
**Figure 15:** Illustration of the pattern of major proteins on the surface of various recominant bleb preparations as analysed by SDS-PAGE (Coomassie staining).
**Figure 16:** Specific anti-Hsf response for various bleb and recombinant bleb preparations using purified recombinant Hsf protein.
**Figure 17:** Analysis of NspA expression in total protein extracts of recombinant *Nm*B (serogroup B derivatives). Gels were stained with Coomassie blue (A) or transferred to nitrocellulose membrane and immuno-stained with mouse anti-PorA monoclonal antibody (B) or mouse anti-NspA polyclonal antibody (C).

### DESCRIPTION OF THE INVENTION

The present invention relates to a general set of tools and methods capable of being used for manufacturing improved, genetically engineered blebs from Gram-negative bacterial strains. The invention includes methods used to make recombinant blebs more immunogenic, less toxic and safer for their use in a human and/or animal vaccine. Moreover, the present invention also describes specific methods necessary for constructing, producing, obtaining and using recombinant, engineered blebs from various Gram-negative bacteria, for vaccine, therapeutic and/or diagnostic purposes. By the methods of the invention, the biochemical composition of bacterial blebs can be manipulated by acting upon/altering the expression of bacterial genes encoding products present in or associated with bacterial outer-membrane blebs (outer membrane proteins or OMPs). The production of blebs using a method of genetic modification to increase, decrease or render conditional the expression of one or more genes encoding outer-membrane components are also included in the scope of this invention.

For clarity, the term "expression cassette" will refer herein to all the genetic elements necessary to express a gene or an operon and to produce and target the corresponding protein(s) of interest to outer-membrane blebs, derived from a given bacterial host. A non-exhaustive list of these features includes control elements (transcriptional and/or translational), protein coding regions and targeting signals, with appropriate spacing between them. Reference to the insertion of promoter sequences means, for the purposes of this invention, the insertion of a sequence with at least a promoter function, and preferably one or more other genetic regulatory elements comprised within an expression cassette. Moreover, the term "integrative cassette" will refer herein to all the genetic elements required to integrate a DNA segment in given bacterial host. A non-exhaustive list of these features includes a delivery vehicle (or vector), with recombinogenic regions, and selectable and counter selectable markers.

Again for the purpose of clarity, the terms 'engineering a bacterial strain to produce less of said antigen' refers to any means to reduce the expression of an antigen of interest, relative to that of the non-modified (i.e., naturally occurring) bleb such that expression is at least 10% lower than that of the non-modified bleb. Preferably it is at least 50% lower. "Stronger promoter sequence" refers to a regulatory control element that increases transcription for a gene encoding antigen of interest. "Upregulating expression" refers to any means to enhance the expression of an antigen of interest, relative to that of the non-modified (i.e., naturally occurring) bleb. It is understood that the amount of 'upregulation' will vary depending on the particular antigen of interest but will not exceed an amount that will disrupt the membrane integrity of the bleb. Upregulation of an antigen refers to expression that is at least 10% higher than that of the non-modified bleb. Preferably it is at least 50% higher. More preferably it is at least 100% (2 fold) higher.

Aspects of the invention relate to individual methods for making improved engineered blebs, to a combination of such methods, and to the bleb compositions made as a result of these methods. Another aspect of the invention relates to the genetic tools used in order to genetically modify a chosen bacterial strain in order to extract improved engineered blebs from said strain.

The engineering steps of the processes of the invention can be carried out in a variety of ways known to the skilled person. For instance, sequences (e.g. promoters or open reading frames) can be inserted, and promoters/genes can be disrupted by the technique of transposon insertion. For instance, for upregulating a gene's expression, a strong promoter could be inserted via a transposon up to 2 kb upstream of the gene's initiation codon (more preferably 200-600 bp upstream, most preferably approximately 400 bp upstream). Point mutation or deletion may also be used (particularly for down-regulating expression of a gene).

Such methods, however, may be quite unstable or uncertain, and therefore it is preferred that the engineering step [particularly for processes a), b), c), d), e), h) and i) as described below] is performed via a homologous recombination event. Preferably, the event takes place between a sequence (a recombinogenic region) of at least 30 nucleotides on the bacterial chromosome, and a sequence (a second recombinogenic region) of at least 30 nucleotides on a vector transformed within the strain. Preferably the regions are 40-1000 nucleotides, more preferably 100-800 nucleotides, most preferably 500 nucleotides). These recombinogenic regions should be sufficiently similar that they are capable of hybridising to one another under highly stringent conditions (as defined later).

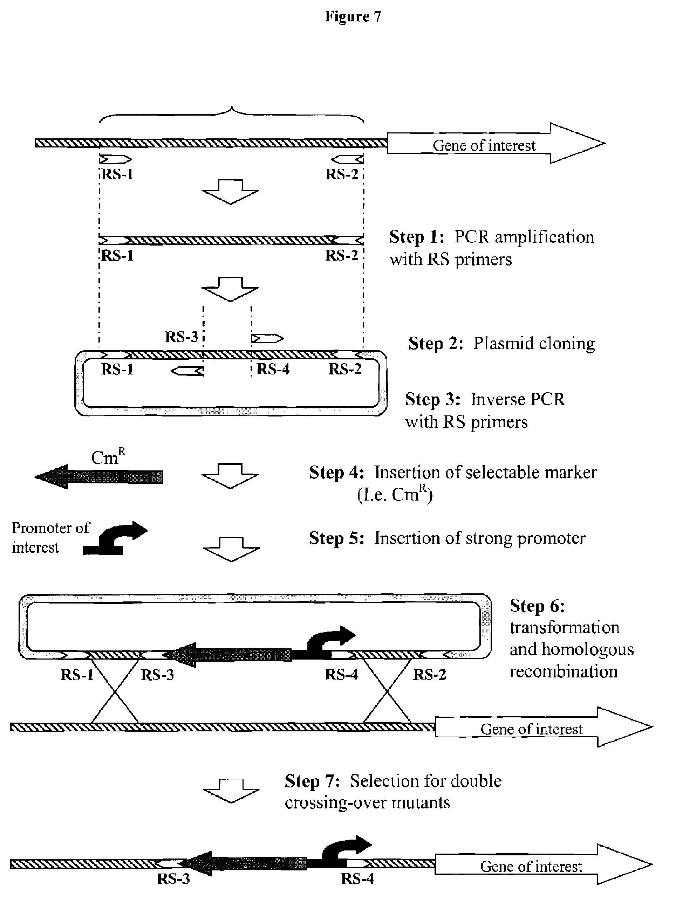

Recombination events may take place using a single recombinogenic region on chromosome and vector, or via a double cross-over event (with 2 regions on chromosome and vector). In order to perform a single recombination event, the vector should be a circular DNA molecule. In order to perform a double recombination event, the vector could be a circular or linear DNA molecule (see Figure 7). It is preferable that a double recombination event is employed and that the vector used is linear, as the modified bacterium so produced will be more stable in terms of reversion events. Preferably the two recombinogenic regions on the chromosome (and on the vector) are of similar (most preferably the same) length so as to promote double cross-overs. The double cross-over functions such that the two rccombinogenic regions on the chromosome (separated by nucleotide sequence 'X') and the two recombinogenic regions on the vector (separated by nucleotide sequence 'Y') recombine to leave a chromosome unaltered except that X and Y have interchanged. The position of the recombinogenic regions can both be positioned upstream or down stream of, or may flank, an open reading frame of interest. These regions can consist of coding, non-coding, or a mixture of coding and non-coding sequence. The identity of X and Y will depend on the effect desired. X may be all or part of an open reading frame, and Y no nucleotides at all, which would result in sequence X being deleted from the chromosome. Alternatively Y may be a strong promoter region for insertion upstream of an open reading frame, and therefore X may be no nucleotides at all.

Suitable vectors will vary in composition depending what type of recombination event is to be performed, and what the ultimate purpose of the recombination event is. Integrative vectors used to deliver region Y can be conditionally replicative or suicide plasmids, bacteriophages, transposons or linear DNA fragments obtained by restriction hydrolysis or PCR amplification. Selection of the recombination event is selected by means of selectable genetic marker such as genes conferring resistance to antibiotics (for instance kanamycin, erythromycin, chloramphenicol, or gentamycin), genes conferring resistance to heavy metals and/or toxic compounds or genes complementing auxotrophic mutations (for instance *pur*, *leu, met, aro*).

### Process a) and f) - Down regulation/Removal of Variable and non-protective immunodominant antigens

Many surface antigens are variable among bacterial strains and as a consequence are protective only against a limited set of closely related strains. An aspect of this invention covers the reduction in expression, or, preferably, the deletion of the gene(s) encoding variable surface protein(s) which results in a bacterial strain producing blebs which, when administered in a vaccine, have a stronger potential for cross-reactivity against various strains due to a higher influence exerted by conserved proteins (retained on the outer membranes) on the vaccinee's immune system. Examples of such variable antigens include: for *Neisseria* - pili (PilC) which undergoes antigenic variations, PorA, Opa, TbpB, FrpB; for *H. influenzae -* P2, P5, pilin, IgA1-protease; and for *Moraxella* - CopB, OMP106.

Other types of gene that could be down-regulated or switched off are genes which, *in vivo,* can easily be switched on (expressed) or off by the bacterium. As outer membrane proteins encoded by such genes are not always present on the bacteria, the presence of such proteins in the bleb preparations can also be detrimental to the effectiveness of the vaccine for the reasons stated above. A preferred example to down-regulate or delete is *Neisseria* Opc protein. Anti-Opc immunity induced by an Opc containing bleb vaccine would only have limited protective capacity as the infecting organism could easily become Opc⁻. *H. influenzae* HgpA and HgpB are other examples of such proteins.

In process a), these variable or non-protective genes are down-regulated in expression, or terminally switched off. This has the above-mentioned surprising advantage of concentrating the immune system on better antigens that are present in low amounts on the outer surface of blebs.

The strain can be engineered in this way by a number of strategies including transposon insertion to disrupt the coding region or promoter region of the gene, or point mutations or deletions to achieve a similar result. Homologous recombination may also be used to delete a gene from a chromosome (where sequence X comprises part (preferably all) of the coding sequence of the gene of interest). It may additionally be used to change its strong promoter for a weaker (or no) promoter (where nucleotide sequence X comprises part (preferably all) of the promoter region of the gene, and nucleotide sequence Y comprises either a weaker promoter region [resulting in a decreased expression of the gene(s)/operon(s) of interest], or no promoter region). In this case it is preferable for the recombination event to occur within the region of the chromosome 1000 bp upstream of the gene of interest.

Alternatively, Y may confer a conditional transcriptional activity, resulting in a conditional expression of the gene(s)/operon(s) of interest (down-regulation). This is useful in the expression of molecules that are toxic to or not well supported by the bacterial host.

Most of the above-exemplified proteins are integral OMPs and their variability may be limited only to one or few of their surface exposed loops. Another aspect of this invention [process g)] covers the deletion of DNA regions coding for these surface exposed loops which leads to the expression of an integral OMP containing conserved surface exposed loops. Surface exposed loops of *H. influenzae* P2 and P5 are preferred examples of proteins that could be transformed into cross-reactive antigens by using such a method. Again, homologous recombination is a preferred method of performing this engineering process.

### Process b) - Promoter delivery and modulation:

A further aspect of the invention relates to modifying the composition of blebs by altering *in situ* the regulatory region controlling the exprcssion of gene(s) and/or operon(s) of interest. This alteration may include partial or total replacement of the endogenous promoter controlling the expression of a gene of interest, with one conferring a distinct transcriptional activity. This distinct transcriptional activity may be conferred by variants (point mutations, deletions and/or insertions) of the endogenous control regions, by naturally occurring or modified heterologous promoters or by a combination of both. Such alterations will preferably confer a transcriptional activity stronger than the endogenous one (introduction of a strong promoter), resulting in an enhanced expression of the gene(s)/operon(s) of interest (up-regulation). Such a method is particularly useful for enhancing the production of immunologically relevant Bleb components such as outer-membrane proteins and lipoproteins (preferably conserved OMPs, usually present in blebs at low concentrations).

Typical strong promoters that may be integrated in *Neisseria* are *porA* [SEQ ID NO: 24], *porB* [SEQ ID NO:26], *lgtF,* Opa, *p110, lst,* and *hpuAB.* PorA and PorB are preferred as constitutive, strong promoters. It has been established (Example 9) that the PorB promoter activity is contained in a fragment corresponding to nucleotides -1 to -250 upstream of the initation codon of *porB.* In *Moraxella,* it is preferred to use the ompH, ompG, ompE, OmpB1, ompB2, ompA, OMPCD and Omp106 promoters, and in *H. influenzae,* it is preferred to integrate the P2, P4, P1, P5 and P6 promoters.

Using the preferred double cross-over homologous recombination technology to introduce the promoter in the 1000 bp upstream region, promoters can be placed anywhere from 30-970 bp upstream of the initiation codon of the gene to be up-regulated. Although conventionally it is thought the promoter region should be relatively close to the open reading frame in order to obtain optimal expression of the gene, the present inventors have surprisingly found that placement of the promoter further away from the initiation codon results in large increases in expression levels. Thus it is preferred if the promoter is inserted 200-600 bp from the initiation codon of the gene, more preferably 300-500 bp, and most preferably approximately 400 bp from the initiation ATG.

### Process c) - Bleb components produced conditionally

The expression of some genes coding for certain bleb components is carcfully regulated. The production of the components is conditionally modulated and depends upon various metabolic and/or environmental signals. Such signals include, for example, iron-limitation, modulation of the redox potential, pH and temperature variations, nutritional changes. Some examples of bleb components known to be produced conditionally include iron-regulated outer-membrane proteins from *Neisseiria* and *Moraxella* (for instance TbpB, LbpB), and substrate-inducible outer-membrane porins. The present invention covers the use of the genetic methods described previously (process a) or b)) to render constitutive the expression of such molecules. In this way, the influence of environmental signal upon the expression of gene(s) of interest can be overcome by modifying/replacing the gene's corresponding control region so that it becomes constitutively active (for instance by deleting part [preferably all] or the repressive control sequence - e.g. the operator region), or inserting a constitutive strong promoter. For iron regulated genes the *fur* operator may be removed. Alternatively, process i) may be used to deliver an additional copy of the gene/operon of interest in the chromosome which is placed artificially under the control of a constitutive promoter.

### Processes d), and e) - Detoxification of LPS

The toxicity of bleb vaccines presents one of the largest problems in the use of blebs in vaccines. A further aspect of the invention relates to methods of genetically detoxifying the LPS present in Blebs. Lipid A is the primary component of LPS responsible for cell activation. Many mutations in genes involved in this pathway lead to essential phenotypes. However, mutations in the genes responsible for the terminal modifications steps lead to temperature-sensitive (*htrB*) or permissive (*msbB*) phenotypes. Mutations resulting in a decreased (or no) expression of these genes result in altered toxic activity of lipid A. Indeed, the non-lauroylated (htrB mutant) or non-myristoylated (msbB mutant) lipid A are less toxic than the wild-type lipid A. Mutations in the lipid A 4'-kinase encoding gene (*lpxK*) also decreases the toxic activity of lipid A.

Process d) thus involves either the deletion of part (or preferably all) of one or more of the above open reading frames or promoters. Alternatively, the promoters could be replaced with weaker promoters. Preferably the homologous recombination techniques described above are used to carry out the process.

The sequences of the htrB and msbB genes from *Neisseria meningitidis* B, *Moraxella catarrhalis,* and *Haemophilus influenzae* are additionally provided for this purpose.

LPS toxic activity could also be altered by introducing mutations in genes/loci involved in polymyxin B resistance (such resistance has been correlated with addition of aminoarabinose on the 4' phosphate of lipid A). These genes/loci could be *pmrE* that encodes a UDP-glucose dehydrogenase, or a region of antimicrobial peptide-resistance genes common to many enterobacteriaciae which could be involved in aminoarabinose synthesis and transfer. The gene *pmrF* that is present in this region encodes a dolicol-phosphate manosyl transferase (Gunn J.S., Kheng, B.L., Krueger J., Kim K.,Guo L., Hackett M., Miller S.I. 1998. Mol. Microbiol. 27: 1171-1182).

Mutations in the PhoP-PhoQ regulatory system, which is a phospho-relay two component regulatory system (f. i. PhoP constitutive phenotype, PhoP^{c}), or low Mg⁺⁺ environmental or culture conditions (that activate the PhoP-PhoQ regulatory system) lead to the addition of aminoarabinose on the 4'-phosphate and 2-hydroxymyristate replacing myristate (hydroxylation of myristate). This modified lipid A displays reduced ability to stimulate E-selectin expression by human endothelial cells and TNF-α secretion from human monocytes.

Process e) involves the upregulation of these genes using a strategy as described above (strong promoters being incorporated, preferably using homologous recombination techniques to carry out the process).

Alternatively, rather than performing any such mutation, a polymyxin B resistant strain could be used as a vaccine production strain (in conjunction with one or more of the other processes of the invention), as blebs from such strains also have reduced LPS toxicity (for instance as shown for meningococcus - van der Ley, P, Hamstra, HJ, Kramer, M, Steeghs, L, Petrov, A and Poolman, JT. 1994. *In:* Proceedings of the ninth international pathogenic Neisseria conference. The Guildhall, Winchester, England).

As a further alternative (and further aspect of the invention) the inventors provide a method of detoxifying a Gram-negative bacterial strain comprising the step of culturing the strain in a growth medium containing 0.1mg-100g of aminoarabinose per litre medium.

As a further still alternative, synthetic peptides that mimic the binding activity of polymyxin B (described below) may be added to the Bleb preparation in order to reduce LPS toxic activity (Rustici, A, Velucchi, M, Faggioni, R, Sironi, M, Ghezzi, P, Quataert, S, Green, B and Porro M 1993. Science 259: 361-365; Velucchi, M, Rustici, A, Meazza, C, Villa, P, Ghezzi, P and Porro, M. 1997. J. Endotox. Res. 4:).

### Process f) - Anchoring homologous or heterologous proteins to outer-membrane blebs whilst reducing the toxicity of LPS

A further aspect of this invention covers the use of genetic sequences encoding polymyxin B peptides (or analogues thereof) as a means to target fusion proteins to the outer-membrane. Polymyxin B is a cyclic peptide composed of non tRNA-encoded amino acids (produced by Gram-positive actinomycetal organisms) that binds very strongly to the Lipid A part of LPS present in the outer-membrane. This binding decreases the intrinsic toxicity of LPS (endotoxin activity). Peptides mimicking the structure of Polymyxin B and composed of canonical (tRNA encoded) amino acids have been developed and also bind lipid A with a strong affinity. These peptides have been used for detoxifying LPS. One of these peptides known as SAEP-2 (Nterminus-Lys-Thr-Lys-Cys-Lys-Phe-Leu-Lys-Lys-Cys-Cterminus) was shown to be very promising in that respect (Molecular Mapping and detoxifying of the Lipid A binding site by synthetic peptides (1993). Rustici, A., Velucchi, M., Faggioni, R., Sironi, M., Ghczzi, P., Quataert, S., Green, B. and M. Porro. Science 259, 361-365).

The present process f) of the invention provides an improvement of this use. It has been found that the use of DNA sequences coding for the SEAP-2 peptide (or derivatives thereof), fused genetically to a gene of interest (encoding for instance a T cell antigen or a protective antigen that is usually secreted such as a toxin, or a cytosolic or periplasmic protein) is a means for targeting the corresponding recombinant protein to the outer-membrane of a preferred bacterial host (whilst at the same time reducing the toxicity of the LPS).

This system is suitable for labile proteins which would not be directly exposed to the outside of the bleb. The bleb would therefore act as a delivery vehicle which would expose the protein to the immune system once the blebs had been engulfed by T-cells. Alternatively, the genetic fusion should also comprise a signal peptide or transmembrane domain such that the recombinant protein may cross the outer membrane for exposure to the host's immune system.

This targeting strategy might be of particular interest in the case of genes encoding proteins that are not normally targeted to the outer-membrane. This methodology also allows the isolation of recombinant blebs enriched in the protein of interest. Preferably, such a peptide targeting signal allows the enrichment of outer membrane blebs in one or several proteins of interest, which are naturally not found in that given subcellular localization. A non exhaustive list of bacteria that can be used as a recipient host for such a production of recombinant blebs includes *Neisseria meningitidis, Neisseiria gonorrhoeae Moraxella catarrhalis, Haemophilus influenzae, Pseudomonas aeruginosa, Chlamydia trachomatis,* and *Chlamydia pneumoniae.*

Although it is preferred that the gene for the construct is engineered into the chromosome of the bacterium [using process i)], an alternative preferred embodiment is for SAEP-2-tagged recombinant proteins to be made independently, and attached at a later stage to a bleb preparation.

A further embodiment is the use of such constructs in a method of protein purification. The system could be used as part of an expression system for producing recombinant proteins in general. The SAEP-2 peptide tag can be used for affinity purification of the protein to which it is attached using a column containing immobilised lipid A molecules.

### Process h)- Cross-reactive polysaccharides

The isolation of bacterial outer-membrane blebs from encapsulated Gram-negative bacteria often results in the co-purification of capsular polysaccharide. In some cases, this "contaminant" material may prove useful since polysaccharide may enhance the immune response conferred by other bleb components. In other cases however, the presence of contaminating polysaccharide material in bacterial bleb preparations may prove detrimental to the use of the blebs in a vaccine. For instance, it has been shown at least in the case of *N. meningitidis* that the serogroup B capsular polysaccharide does not confer protective immunity and is susceptible to induce an adverse auto-immune response in humans. Consequently, process h) of the invention is the engineering of the bacterial strain for bleb production such that it is free of capsular polysaccharide. The blebs will then be suitable for use in humans. A particularly preferred example of such a bleb preparation is one from *N. meningitidis* serogroup B devoid of capsular polysaccharide.

This may be achieved by using modified bleb production strains in which the genes necessary for capsular biosynthesis and/or export have been impaired. Inactivation of the gene coding for capsular polysaccharide biosynthesis or export can be achieved by mutating (point mutation, deletion or insertion) either the control region, the coding region or both (preferably using the homologous recombination techniques described above). Moreover, inactivation of capsular biosynthesis genes may also be achieved by antisense over-expression or transposon mutagenesis. A preferred method is the deletion of some or all of the *Neisseria meningitidis cps* genes required for polysaccharide biosynthesis and export. For this purpose, the replacement plasmid pMF121 (described in Frosh et al.1990, Mol. Microbiol. 4:1215-1218) can be used to deliver a mutation deleting the *cpsCAD* (+ *galE*) gene cluster. Alternatively the *siaD* gene could be deleted, or down-regulated in expression (the meningococcal *siaD* gene encodes alpha-2,3-sialyltransferase, an enzyme required for capsular polysaccharide and LOS synthesis). Such mutations may also remove host-similar structures on the saccharide portion of the LPS of the bacteria.

### Process i) - Delivery of one or more further copies of a Gene and/or operon in a host chromosome, or delivery of a heterlogous gene and/or operon in a host chromosome.

An efficient strategy to modulate the composition of a Bleb preparation is to deliver one or more copies of a DNA segment containing an expression cassette into the genome of a Gram-negative bacterium. A non exhaustive list of preferred bacterial species that could be used as a recipient for such a cassette includes *Neisseria meningitidis, Netsseiria gonorrhoeae, Moraxella catarrhalis, Haemophilus influenzae, Pseudomonas aeruginosa, Chlamydia trachomatis, Chlamydia pneumoniae.* The gene(s) contained in the expression cassette may be homologous (or endogenous) (i.e. exist naturally in the genome of the manipulated bacterium) or heterologous (i.e. do not exist naturally in the genome of the manipulated bacterium). The reintroduced expression cassette may consist of unmodified, "natural'' promoter/gene/operon sequences or engineered expression cassettes in which the promoter region and/or the coding region or both have been altered. A non-exhaustive list of preferred promoters that could be used for expression includes the promoters *porA, porB, lbpB, tbpB, p110, lst, hpuAB* from *N. meningitidis* or *N. gonorroheae,* the promoters p2, p5, p4, ompF, p1, ompH, p6, hin47 from *H. influenzae,* the promoters ompH, ompG, ompCD, ompE, ompB1, ompB2, ompA of *M. catarrhalis,* the promoter λpL, *lac, tac, araB* of *Escherichia coli* or promoters recognized specifically by bacteriophage RNA polymerase such as the *E. coli* bacteriophage T7. A non-exhaustive list of preferred genes that could be expressed in such a system includes *Neisseria* NspA, Omp85, Pi1Q, TbpA/B complex, Hsf, P1dA, HasR; *Chlamydia* MOMP, HMWP; *Moraxella* OMP106, HasR, Pi1Q, OMP85, P1dA; *Bordetella pertussis* FHA, PRN, PT.

In a preferred embodiment of the invention the expression cassette is delivered and integrated in the bacterial chromosome by means of homologous and/or site specific recombination. Integrative vectors used to deliver such genes and/or operons can be conditionally replicative or suicide plasmids, bacteriophages, transposons or linear DNA fragments obtained by restriction hydrolysis or PCR amplification. Integration is preferably targeted to chromosomal regions dispensable for growth *in vitro.* A non exhaustive list of preferred loci that can be used to target DNA integration includes the *porA, porB, opa, opc, rmp, omp26, lecA, cps, lgtB* genes of *Neisseiria meningitidis* and *Neisseria gonorrhoeae,* the *P1, P5, hmw1*/*2, IgA-protease, fimE* genes of NTHi; the *lecA1, lecA2, omp106, uspA1, uspA2* genes of *Moraxella catarrhalis.* Alternatively, the expression cassette used to modulate the expression of bleb component(s) can be delivered into a bacterium of choice by means of episomal vectors such as circular/linear replicative plasmids, cosmids, phasmids, lysogenic bacteriophages or bacterial artificial chromosomes. Selection of the recombination event can be selected by means of selectable genetic marker such as genes conferring resistance to antibiotics (for instance kanamycin, erythromycin, chloramphenicol, or gentamycin), genes conferring resistance to heavy metals and/or toxic compounds or genes complementing auxotrophic mutations (for instance *pur, leu, met, aro).*

### Heterologous Genes - Expression of foreign proteins in outer-membrane blebs

Outer-membrane bacterial blebs represent a very attractive system to produce, isolate and deliver recombinant proteins for vaccine, therapeutic and/or diagnostic uses. A further aspect of this invention is in respect of the expression, production and targeting of foreign, heterologous proteins to the outer-membrane, and the use of the bacteria to produce recombinant blebs.

A preferred method of achieving this is via a process comprising the steps of: introducing a heterologous gene, optionally controlled by a strong promoter sequence, into the chromosome of a Gram-negative strain by homologous recombination. Blebs may be made from the resulting modified strain.

A non-exhaustive list of bacteria that can be used as a recipient host for production of recombinant blebs includes *Neisseria meningitidis, Neisseiria gonorrhoeae Moraxella catarrhalis, Haemophilus influenzae, Pseudomonas aeruginosa, Chlamydia trachomatis, Chlamydia pneumoniae.* The gene expressed in such a system can be of viral, bacterial, fungal, parasitic or higher eukaryotic origin.

A preferred application of the invention includes a process for the expression of *Moraxella, Haemophilus* and/or *Pseudomonas* outer-membrane proteins (integral, polytopic and/or lipoproteins) in *Neisseria meningitidis* recombinant blebs. The preferable integration loci are stated above, and genes that are preferably introduced are those that provide protection against the bacterium from which they were isolated. Preferred protective genes for each bacterium are described below.

Further preferred applications are: blebs produced from a modified *Haemophilus influenzae* strain where the heterologous gene is a protective OMP from *Moraxella catarrhalis;* and blebs produced from a modified *Moraxella catarrhalis* strain where the heterologous gene is a protective OMP from *Haemophilus influenzae* (preferred loci for gene insertion are given above, and preferred protective antigens are described below).

A particularly preferred application of this aspect is in the field of the prophylaxis or treatment of sexually-transmitted diseaseses (STDs). It is often difficult for practitioners to determine whether the principal cause of a STD is due to gonococcus or *Chlamydia trachomatis* infection. These two organisms are the main causes of salpingitis - a disease which can lead to sterility in the host. It would therefore be useful if a STD could be vaccinated against or treated with a combined vaccine effective against disease caused by both organisms. The Major Outer Membrane Protein (MOMP) of *C. trachomatis* has been shown to be the target of protective antibodies. However, the structural integrity of this integral membrane protein is important for inducing such antibodies. In addition, the epitopes recognised by these antibodies are variable and define more than 10 serovars. The previously described aspect of this invention allows the proper folding of one or more membrane proteins within a bleb outer membrane preparation. The engineering of a gonococcal strain expressing multiple *C. trachomatis* MOMP serovars in the outer membrane, and the production of blebs therefrom, produces a single solution to the multiple problems of correctly folded membrane proteins, the presentation of sufficient MOMP serovars to protect against a wide spectrum of serovars, and the simultaneous prophylaxis/treatment of gonococcal infection (and consequently the non-requirement of practitioners to initially decide which organism is causing particular clinical symptoms - both organisms can be vaccinated against simultaneously thus allowing the treatment of the STD at a very early stage). Preferred loci for gene insertion in the gonoccocal chromosome are give above. Other preferred, protective *C. trachomatis* genes that could be incorporated are HMWP, PmpG and those OMPs disclosed in WO 99/28475.

### Targeting of heterologous proteins to outer-membrane blebs:

The expression of some heterologous proteins in bacterial blebs may require the addition of outer-membrane targeting signal(s). The preferred method to solve this problem is by creating a genetic fusion between a heterologous gene and a gene coding for a resident OMP as a specific approach to target recombinant proteins to blebs. Most preferably, the heterologous gene is fused to the signal peptides sequences of such an OMP.

### Neisserial bleb preparations

One or more of the following genes (encoding protective antigens) are preferred for upregulation via processes b) and/or i) when carried out on a Neisserial strain, including gonococcus, and meningococcus (particularly *N. meningitidis* B): NspA (WO 96/29412), Hsf-like (WO 99/31132), Hap (PCT/EP99/02766), PorA, PorB, OMP85 (WO 00/23595), PilQ (PCT/EP99/03603), P1dA (PCT/EP99/06718), FrpB (WO 96/31618), TbpA (US 5,912,336), TbpB, FrpA/FrpC (WO 92/01460), LbpA/LbpB (PCT/EP98/05117), FhaB (WO 98/02547), HasR (PCT/EP99/05989), lipo02 (PCT/EP99/08315), Tbp2 (WO 99/57280), M1tA (WO 99/57280), and ctrA (PCT/EP00/00135). They are also preferred as genes which may be heterologously introduced into other Gram-negative bacteria.

One or more of the following genes are preferred for downregulation via process a): PorA, PorB, Pi1C, TbpA, TbpB, LbpA, LbpB, Opa, and Opc.

One or more of the following genes are preferred for downregulation via process d): htrB, msbB and lpxK.

One or more of the following genes are preferred for upregulation via process e): pmrA, pmrB, pmrE, and pmrF.

Preferred repressive control sequences for process c) are: the *fur* operator region (particularly for either or both of the TbpB or LbpB genes); and the DtxR operator region.

One or more of the following genes are preferred for downregulation via process h): galE, siaA, siaB, siaC, siaD, ctrA, ctrB, ctrC, and ctrD.

### Pseudomonas aeruginosa bleb preparations

One or more of the following genes (encoding protective antigens) are preferred for upregulation via processes b) and/or i): PcrV, OprF, OprI. They are also preferred as genes which may be heterologously introduced into other Gram-negative bacteria.

### Moraxella catarrhalis bleb preparations

One or more of the following genes (encoding protective antigens) are preferred for upregulation via processes b) and/or i): OMP106 (WO 97/41731 & WO 96/34960), HasR (PCT/EP99/03824), PilQ (PCT/EP99/03823), OMP85 (PCT/EP00/01468), lipo06 (GB 9917977.2), lipo10 (GB 9918208.1), lipo11 (GB 9918302.2), lipo18 (GB 9918038.2), P6 (PCT/EP99/03038), ompCD, CopB (Helminen ME, et al (1993) Infect. Immun. 61;2003-2010), D15 (PCT/EP99/03822), OmplA1 (PCT/EP99/06781), H1y3 (PCT/EP99/03257), LbpA and LbpB (WO 98/55606), TbpA and TbpB (WO 97/13785 & WO 97/32980), OmpE, UspA1 and UspA2 (WO 93/03761), and Omp21. They are also preferred as genes which may be heterologously introduced into other Gram-negative bacteria.

One or more of the following genes are preferred for downregulation via process a): CopB, OMP106, OmpB1, TbpA, TbpB, LbpA, and LbpB.

One or more of the following genes are preferred for downregulation via process d): htrB, msbB and 1pxK.

One or more of the following genes are preferred for upregulation via process e): pmrA, pmrB, pmrE, and pmrF.

### Haemophilus influenzae bleb preparations

One or more of the following genes (encoding protective antigens) are preferred for upregulation via processes b) and/or i): D15 (WO 94/12641), P6 (EP 281673), TbpA, TbpB, P2, P5 (WO 94/26304), OMP26 (WO 97/01638), HMW1, HMW2, HMW3, HMW4, Hia, Hsf, Hap, Hin47, and Hif (all genes in this operon should be upregulated in order to upregulate pilin). They are also preferred as genes which may be heterologously introduced into other Gram-negative bacteria.

One or more of the following genes are preferred for downregulation via process a): P2, P5, Hif, IgA1-protease, HgpA, HgpB, HMW1, HMW2, Hxu, TbpA, and TbpB.

One or more of the following genes are preferred for downregulation via process d): htrB, msbB and lpxK.

One or more of the following genes are preferred for upregulation via process e): pmrA, pmrB, pmrE, and pmrF.

### Vaccine Formulations

A preferred embodiment of the invention is the formulation of the bleb preparations of the invention in a vaccine which may also comprise a pharmaceutically acceptable excipient.

The manufacture of bleb preparations from any of the aforementioned modified strains may be achieved by any of the methods well known to a skilled person. Preferably the methods disclosed in EP 301992, US 5,597,572, EP 11243 or US 4,271,147 are used. Most preferably, the method described in Example 8 is used.

Vaccine preparation is generally described in Vaccine Design ("The subunit and adjuvant approach" (eds Powell M.F. & Newman M.J.) (1995) Plenum Press New York).

The bleb preparations of the present invention may be adjuvanted in the vaccine formulation of the invention. Suitable adjuvants include an aluminium salt such as aluminum hydroxide gel (alum) or aluminium phosphate, but may also be a salt of calcium (particularly calcium carbonate), iron or zinc, or may be an insoluble suspension of acylated tyrosine, or acylated sugars, cationically or anionically derivatised polysaccharides, or polyphosphazenes.

Suitable Th1 adjuvant systems that may be used include, Monophosphoryl lipid A, particularly 3-de-O-acylated monophosphoryl lipid A, and a combination of monophosphoryl lipid A, preferably 3-de-O-acylated monophosphoryl lipid A (3D-MPL) together with an aluminium salt. An enhanced system involves the combination of a monophosphoryl lipid A and a saponin derivative particularly the combination of QS21 and 3D-MPL as disclosed in WO 94/00153, or a less reactogenic composition where the QS21 is quenched with cholesterol as disclosed in WO96/33739. A particularly potent adjuvant formulation involving QS21 3D-MPL and tocopherol in an oil in water emulsion is described in WO95/17210 and is a preferred formulation.

The vaccine may comprise a saponin, more preferably QS21. It may also comprise an oil in water emulsion and tocopherol. Unmethylated CpG containing oligo nucleotides (WO 96/02555) are also preferential inducers of a TH1 response and are suitable for use in the present invention.

The vaccine preparation of the present invention may be used to protect or treat a mammal susceptible to infection, by means of administering said vaccine via systemic or mucosal route. These administrations may include injection via the intramuscular, intraperitoneal, intradermal or subcutaneous routes; or *via* mucosal administration to the oral/alimentary, respiratory, genitourinary tracts. Thus one aspect of the present invention is a method of immunizing a human host against a disease caused by infection of a gram-negative bacteria, which method comprises administering to the host an immunoprotective dose of the bleb preparation of the present invention.

The amount of antigen in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-100µg of protein antigen, preferably 5-50µg, and most typically in the range 5-25µg.

An optimal amount for a particular vaccine can be ascertained by standard studies involving observation of appropriate immune responses in subjects. Following an initial vaccination, subjects may receive one or sevcral booster immunisations adequately spaced.

### Ghost or Killed Whole cell vaccines

The inventors envisage that the above improvements to bleb preparations and vaccines can be easily extended to ghost or killed whole cell preparations and vaccines (with identical advantages). The modified Gram-negative strains of the invention from which the bleb preparations are made can also be used to made ghost and killed whole cell preparations. Methods of making ghost preparations (empty cells with intact envelopes) from Gram-negative strains are well known in the art (see for example WO 92/01791). Methods of killing whole cells to make inactivated cell preparations for use in vaccines are also well known. The terms 'bleb preparations' and 'bleb vaccines' as well as the processes described throughout this document are therefore applicable to the terms 'ghost preparation' and 'ghost vaccine', and 'killed whole cell preparation' and 'killed whole cell vaccine', respectively, for the purposes of this invention.

### Combinations of methods a) - i)

It may be appreciated that one or more of the above processes may be used to produce a modified strain from which to make improved bleb preparations of the invention. Preferably one such process is used, more preferably two or more (2, 3, 4, 5, 6, 7, 8 or 9) of the processes are used in order to manufacture the bleb vaccine. As each additional method is used in the manufacture of the bleb vaccine, each improvement works in conjunction with the other methods used in order to make an optimised engineered bleb preparation.

A preferred meningococcal (particularly *N. meningitidis* B) bleb preparation comprises the use of processes a), b), d) and/or e), and h). Such bleb preparations are safe (no structures similar to host structures), non-toxic, and structured such that the host immune response will be focused on high levels of protective (and preferably conserved) antigens. All the above elements work together in order to provide an optimised bleb vaccine.

Similarly for M. catarrhalis and non-typeable H. influenzae, preferred bleb preparations comprise the use of processes a), b), and d) and/or e).

A further aspect of the invention is thus an immuno-protective and non-toxic Gram-negative bleb, ghost, or killed whole cell vaccine suitable for paediatric use.

By paediatric use it is meant use in infants less than 4 years old.

By immunoprotective it is meant that at least 40% (and preferably 50, 60, 70, 80, 90 and 100%) of infants seroconvert (4-fold increase in bactericidal activity [the dilution of antisera at which 50% of bacteria die - see for example PCT/EP98/05117]) against a set of heterologous strains to be selected from the major clonal groups known. For meningococcus B these stains should have a different PorA type from the bleb production strain, and should preferably be 2, 3, 4 or, most preferably, all 5 of strains H44/76, M97/252078, BZ10, NGP165 and CU385. For non-typeable *H. influenzae,* the strains should preferably be 2, 3, 4 or, most preferably, all 5 of strains 3224A, 3219C, 3241A, 640645, and A840177. For *M*. *catarrhalis,* the strains should preferably be 2, 3, 4 or, most preferably, all 5 of strains ATCC 43617, 14, 358, 216 and 2926.

By non-toxic it is meant that there is a significant (2-4 fold, preferably 10 fold) decrease of endotoxin activity as measured by the well-known LAL and pyrogenicity assays.

### Vaccine Combinations

A further aspect of the invention are vaccine combinations comprising the bleb preparations of the invention with other antigens which are advantageously used against certain disease states. It has been found that blebs are particularly suitable for formulating with other antigens, as they advantageously have an adjuvant effect on the antigens they are mixed with.

In one preferred combination, the meningoccocus B bleb preparations of the invention are formulated with 1, 2, 3 or preferably all 4 of the following meningococcal capsular polysaccharides which may be plain or conjugated to a protein carrier: A, C, Y or W. Such a vaccine may be advantageously used as a global meningococcus vaccine. Rather than use the meningoccocus B bleb preparations of the invention, it is also envisaged that the formulation could alternatively contain wild-type meningococcus B bleb preparations from 2 or more (preferably several) strains belonging to several subtype/serotypes (for instance chosen from P1.15, P1.7,16, P1.4, and P1.2).

In a further preferred embodiment, the meningoccocus B bleb preparations of the invention [or the aforementioned mix of 2 or more wild-type meningococcus B bleb preparations], preferably formulated with 1, 2, 3 or all 4 of the plain or conjugated meningococcal capsular polysaccharides A, C, Y or W, are formulated with a conjugated *H. influenzae* b capsular polysaccharide, and one or more plain or conjugated pneumococcal capsular polysaccharides. Optionally, the vaccine may also comprises one or more protein antigens that can protect a host against *Streptococcus pneumoniae* infection. Such a vaccine may be advantageously used as a global meningitis vaccine.

The pneumococcal capsular polysaccharide antigens are preferably selected from serotypes 1, 2, 3, 4, 5, 6B, 7F, 8, 9N, 9V, 10A, 11A, 12F, 14, 15B, 17F, 18C, 19A, 19F, 20, 22F, 23F and 33F (most preferably from serotypes 1, 3, 4, 5, 6B, 7F, 9V, 14, 18C, 19F and 23F).

Preferred pneumococcal proteins antigens are those pneumococcal proteins which are exposed on the outer surface of the pneumococcus (capable of being recognised by a host's immune system during at least part of the life cycle of the pneumococcus), or are proteins which are secreted or released by the pneumococcus. Most preferably, the protein is a toxin, adhesin, 2-component signal tranducer, or lipoprotein of *Streptococcus pneumoniae,* or fragments thereof. Particularly preferred proteins include, but are not limited to: pneumolysin (preferably detoxified by chemical treatment or mutation) [Mitchell et al. Nucleic Acids Res. 1990 Jul 11; 18(13): 4010 "Comparison of pneumolysin genes and proteins from Streptococcus pneumoniae types 1 and 2." , Mitchell et al. Biochim Biophys Acta 1989 Jan 23; 1007(1): 67-72 "Expression of the pneumolysin gene in Escherichia coli: rapid purification and biological properties.", WO 96/05859 (A. Cyanamid), WO 90/06951 (Paton et al), WO 99/03884 (NAVA)]; PspA and transmembrane deletion variants thereof (US 5804193 - Briles et al.); PspC and transmembrane deletion variants thereof (WO 97/09994 - Briles et al); PsaA and transmembrane deletion variants thereof (Berry & Paton, Infect Immun 1996 Dec;64(12):5255-62 "Sequence heterogeneity of PsaA, a 37-kilodalton putative adhesin essential for virulence of Streptococcus pneumoniae*"*)*;* pneumococcal choline binding proteins and transmembrane deletion variants thereof; CbpA and transmembrane deletion variants thereof (WO 97/41151; WO 99/51266); Glyceraldehyde-3-phosphate - dehydrogenase (Infect. Immun. 1996 64:3544); HSP70 (WO 96/40928); PcpA (Sanchez-Beato et al. FEMS Microbiol Lett 1998, 164:207-14); M like protein, SB patent application No. EP 0837130; and adhesin 18627, SB Patent application No. EP 0834568. Further preferred pneumococcal protein antigens are those disclosed in WO 98/18931, particularly those selected in WO 98/18930 and PCT/US99/30390.

In a further preferred embodiment, the *Moraxella catarrhalis* bleb preparations of the invention are formulated with one or more plain or conjugated pneumococcal capsular polysaccharides, and one or more antigens that can protect a host against non-typeable *H. influenzae* infection. Optionally, the vaccine may also comprise one or more protein antigens that can protect a host against *Streptococcus pneumoniae* infection. The vaccine may also optionally comprise one or more antigens that can protect a host against RSV and/or one or more antigens that can protect a host against influenza virus. Such a vaccine may be advantageously used as a global otitis media vaccine.

Preferred non-typeable *H. influenzae* protein antigens include Fimbrin protein (US 5766608) and fusions comprising peptides therefrom (eg LB1 Fusion) (US 5843464 - Ohio State Research Foundation), OMP26, P6, protein D, TbpA, TbpB, Hia, Hmw1, Hmw2, Hap, and D15.

Preferred influenza virus antigens include whole, live or inactivated virus, split influenza virus, grown in eggs or MDCK cells, or Vero cells or whole flu virosomes (as described by R. Gluck, Vaccine, 1992, 10, 915-920) or purified or recombinant proteins thereof, such as HA, NP, NA, or M proteins, or combinations thereof.

Preferred RSV (Respiratory Syncytial Virus) antigens include the F glycoprotein, the G glycoprotein, the HN protein, or derivatives thereof.

In a still further preferred embodiment, the non-typeable *H. influenzae* bleb preparations of the invention are formulated with one or more plain or conjugated pneumococcal capsular polysaccharides, and one or more antigens that can protect a host against *M. catarrhalis* infection. Optionally, the vaccine may also comprise one or more protein antigens that can protect a host against *Streptococcus pneumoniae* infection. The vaccine may also optionally comprise one or more antigens that can protect a host against RSV and/or one or more antigens that can protect a host against influenza virus. Such a vaccine may be advantageously used as a global otitis media vaccine.

### Nucleotide sequences of the invention

A further aspect of the invention relates to the provision of new nucleotide sequences which may be used in the processes of the invention. Specific upstream regions from various genes from various strains are provided which can be used in, for instance, processes a), b), d) and h). In addition, coding regions are provided for performing process d).

### General method for the analysis of the non-coding flanking region of a bacterial gene, and its exploitation for modulated expression of the gene in blebs

The non-coding flanking regions of a specific gene contain regulatory elements important in the expression of the gene. This regulation takes place both at the transcriptional and translational level. The sequence of these regions, either upstream or downstream of the open reading frame of the gene, can be obtained by DNA sequencing. This sequence information allows the determination of potential regulatory motifs such as the different promoter elements, terminator sequences, inducible sequence elements, repressors, elements responsible for phase variation, the Shine-Dalgarno sequence, regions with potential secondary structure involved in regulation, as well as other types of regulatory motifs or sequences.

This sequence information allows the modulation of the natural expression of the gene in question. The upregulation of the gene expression may be accomplished by altering the promoter, the Shine-Dalgarno sequence, potential repressor or operator elements, or any other elements involved. Likewise, downregulation of expression can be achieved by similar types of modifications. Alternatively, by changing phase variation sequences, the expression of the gene can be put under phase variation control, or may be uncoupled from this regulation. In another approach, the expression of the gene can be put under the control of one or more inducible elements allowing regulated expression. Examples of such regulation includes, but is not limited to, induction by temperature shift, addition of inductor substrates like selected carbohydrates or their derivatives, trace elements, vitamins, co-factors, metal ions, etc.

Such modifications as described above can be introduced by several different means. The modification of sequences involved in gene expression can be done *in vivo* by random mutagenesis followed by selection for the desired phenotype. Another approach consists in isolating the region of interest and modifying it by random mutagenesis, or site-directed replacement, insertion or deletion mutagenesis. The modified region can then be reintroduced into the bacterial genome by homologous recombination, and the effect on gene expression can be assessed. In another approach, the sequence knowledge of the region of interest can be used to replace or delete all or part of the natural regulatory sequences. In this case, the regulatory region targeted is isolated and modified so as to contain the regulatory elements from another gene, a combination of regulatory elements from different genes, a synthetic regulatory region, or any other regulatory region, or to delete selected parts of the wild-type regulatory sequences. These modified sequences can then be reintroduced into the bacterium via homologous recombination into the genome.

In process b), for example, the expression of a gene can be modulated by exchanging its promoter with a stronger promoter (through isolating the upstream sequence of the gene, in vitro modification of this sequence, and reintroduction into the genome by homologous recombination). Upregulated expression can be obtained in both the bacterium as well as in the outer membrane vesicles shed (or made) from the bacterium.

In other preferred examples, the described approaches can be used to generate recombinant bacterial strains with improved characteristics for vaccine applications, as described above. These can be, but are not limited to, attenuated strains, strains with increased expression of selected antigens, strains with knock-outs (or decreased expression) of genes interfering with the immune response, and strains with modulated expression of immunodominant proteins.

SEQ ID NO:2-23, 25, 27-38 are all Neisserial upstream sequences (upstream of the initiation codon of various preferred genes) comprising approximately 1000 bp each. SEQ ID NO: 39-62 are all *M. catarrhalis* upstream sequences (upstream of the initiation codon of various preferred genes) comprising approximately 1000 bp each. SEQ ID NO: 63-75 are all *H. influenzae* upstream sequences (upstream of the initiation codon of various preferred genes) comprising approximately 1000 bp each. All of these can be used in genetic methods (particularly homologous recombination) for up-regulating, or down-regulating the open reading frames to which they are associated (as described before). SEQ ID NO: 76-81 are the coding regions for the HtrB and MsbB genes from Neisseria, *M. catarrhalis,* and *Haemophilus influenzae.* These can be used in genetic methods (particularly homologous recombination) for down-regulating (in particular deleting) part (preferably all) of these genes [process d)].

Another aspect of the invention is thus an isolated polynucleotide sequence which hybridises under highly stringent conditions to at least a 30 nucleotide portion of the nucleotides in SEQ ID NO: 2-23, 25, 27-81 or a complementary strand thereof. Preferably the isolated sequence should be long enough to perform homologous recombination with the chromosomal sequence if it is part of a suitable vector - namely at least 30 nucleotides (preferably at least 40, 50, 60, 70, 80, 90, 100, 200, 300, 400, or 500 nucleotides). More preferably the isolated polynucleotide should comprise at least 30 nucleotides (preferably at least 40, 50, 60, 70, 80, 90, 100, 200, 300, 400, or 500 nucleotides) of SEQ ID NO: 2-23, 25, 27-81 or a complementary strand thereof.

As used herein, highly stringent hybridization conditions include, for example, 6X SSC, 5X Denhardt, 0.5% SDS, and 100 µg/mL fragmented and denatured salmon sperm DNA hybridized overnight at 65 °C and washed in 2X SSC, 0.1% SDS one time at room temperature for about 10 minutes followed by one time at 65 °C for about 15 minutes followed by at least one wash in 0.2X SCC, 0.1% SDS at room temperature for at least 3-5 minutes.

A further aspect is the use of the isolated polynucleotide sequences of the invention in performing a genetic engineering event (such as transposon insertion, or site specific mutation or deletion, but preferably a homologous recombination event) within 1000 bp upstream of a Gram-negative bacterial chromosomal gene in order to either increase or decrease expression of the gene. Preferably the strain in which the recombination event is to take place is the same as the strain from which the upstream sequences of the invention were obtained. However, the meningococcus A, B, C, Y and W and gonococcus genomes are sufficiently similar that upstream sequence from any of these strains may be suitable for designing vectors for performing such events in the other strains. This is may also be the case for *Haemophilus influenzae* and non-typeable *Haemophilus influenzae.*

### EXAMPLES

The examples below are carried out using standard techniques, which are well known and routine to those of skill in the art, except where otherwise described in detail. The examples are illustrative, but do not limit the invention.

### Example 1: Construction of a Neisseiria meningitidis serogroup B strain lacking capsular polysaccharides.

The plasmid pMF121 (Frosch et al., 1990) has been used to construct a *Neisseria meningitidis B* strain lacking the capsular polysaccharide. This plasmid contains the flanking regions of the gene locus coding for the biosynthesis pathway of the group B polysaccharide (B PS), and the erythromycin resistance gene. Deletion of the B PS resulted in loss of expression of the group B capsular polysaccharide as well as a deletion in the active copy of *galE* leading to the synthesis of galactose deficient LPS.

### Strain transformation:

*Neisseria meningitidis* B H44/76 strain (B:15:P1.7, 16;Los 3,7,9) was selected for transformation. After an overnight CO₂ incubation on MH plate (without erythromycin), cells were collected in liquid MH containing 10 mM MgCl₂ (2 ml were used per MH plate) and diluted up to an OD of 0.1 (550 nm). To this 2 ml solution, 4 µl of the plasmid pMF121 stock solution (0.5 µg/ml) were added for a 6 hours incubation period at 37°C (with shaking). A control group was done with the same amount of *Neisseria meningitidis* B bacteria, but without addition of plasmid. After the incubation period, 100 µl of culture, as such, at 1/10, 1/100 and 1/1000 dilutions, were put in MH plates containing 5, 10, 20, 40 or 80 µg erythromycin /ml before incubation for 48 hours at 37°C.

### Colony blotting:

After plate incubation, 20 colonies were grown and selected from the 10 and 20 µg erythromycin/ml MH plates, while there was no colony growth in the control group without plasmid transformation. The H44/76 wild type strain was unable to grow in the selected erythromycin plates (10 to 80 µg erythromycin/ml). The day after, all the visible colonies were placed on new MH plates without erythromycin in order to let them grow. Afterwards, they were transferred onto nitrocellulose sheets (colony blotting) for presence of B polysaccharide. Briefly, colonies were blotted onto a nitrocellulose sheet and rinsed directly in PBS-0.05 % Tween 20 before cell inactivation for 1 hour at 56°C in PBS-0.05% Tween 20 (diluant buffer). Afterwards, the membrane was overlaid for one hour in the diluant buffer at room temperature (RT). Then, sheets were washed again for three times 5 minutes in the diluant buffer before incubation with the anti-B PS 735 Mab (Boerhinger) diluted at 1/3000 in the diluant buffer for 2 hours at RT. After a new washing step (3 times 5 minutes), the monoclonal antibody was detected with a biotinylated anti-mouse Ig from Amersham (RPN 1001) diluted 500 times in the diluant buffer (one hour at RT) before the next washing step (as described above). Afterwards, sheets were incubated for one hour at RT with a solution of streptavidin-peroxidase complex diluted 1/1000 in the diluant buffer. After the last three washing steps using the same method, nitrocellulose sheets were incubated for 15 min in the dark using the revelation solution (30 mg of 4-chloro-1-naphtol solution in 10 ml methanol plus 40 ml PBS and 30 mcl of H₂O₂ 37% from Merck). The reaction was stopped with a distillated water-washing step.

### Whole cell Elisas:

Whole cell Elisas were also done using the two transformed colonies ("D" and "R") and the wild type strain (H44/76) as coated bacteria (20 µg protein/ml), and a set of different monoclonal antibodies used to characterize *Neisseria meningitidis* strains. The following Mabs were tested: anti-B PS (735 from Dr Frosch), and the other Mabs from NIBSC: anti-B PS (Ref 95/750) anti-P1.7 (A-PorA, Ref 4025), anti-P1.16 (A-PorA, Ref 95/720), anti-Los 3,7,9 (A-LPS, Ref 4047), anti-Los 8 (A-LPS, Ref 4048), and anti-P1.2 (A-PorA Ref 95/696).

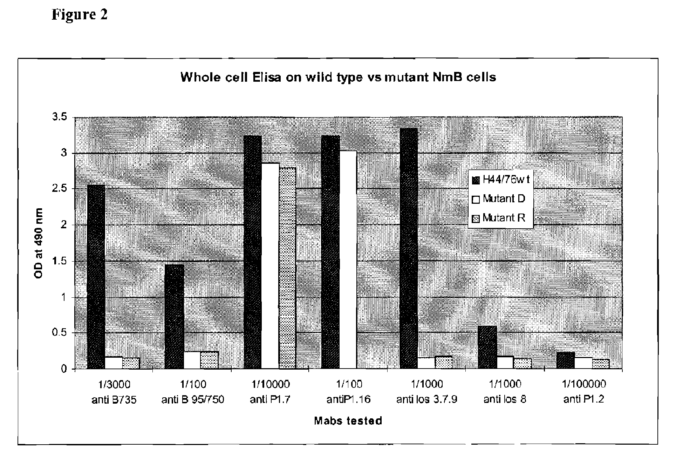

Microtiter plates (Maxisorp, Nunc) were coated with 100 µl of the recombinant meningococcal B cells solution overnight (ON) at 37°C at around 20 µg/ml in PBS. Afterwards, plates are washed three times with 300 µl of 150 mM NaCl - 0.05 % Tween 20, and were overlaid with 100 µl of PBS-0.3 % Casein and incubated for 30 min at room temperature with shaking. Plates were washed again using the same procedure before incubation with antibodies. Monoclonal antibodies (100 µl) were used at different dilutions (as shown in Figure 2) in PBS-0.3 % Casein 0.05 % Tween 20 and put onto the microplates before incubation at room temperature for 30 min with shaking, before the next identical washing step. 100 µl of the anti-mouse Ig (from rabbit, Dakopatts E0413) conjugated to biotin and diluted at 1/2000 in PBS-0.3 % Casein - 0.05 % Tween 20 were added to the wells to detect bound monoclonal antibodies. After the washing step (as before), plates were incubated with a streptavidin-peroxidase complex solution (100 µl of the Amersham RPN 1051) diluted at 1/4000 in the same working solution for 30 min at room temperature under shaking conditions. After this incubation and the last washing step, plates are incubated with 100 µl of the chromogen solution (4 mg orthophenylenediamine (OPD) in 10 ml 0.1 M citrate buffer pH4.5 with 5 µl H₂O₂) for 15 min in the dark. Plates are then read at 490/620 nm using a spectrophotometer.

### Results:

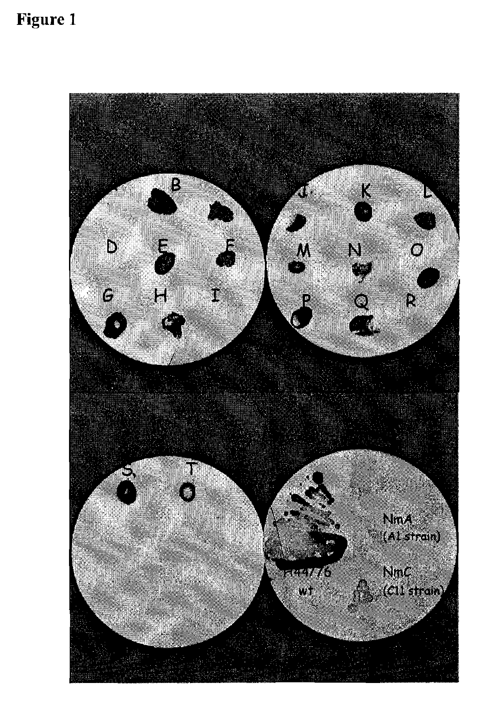

Figure 1 shows that from the 20 isolated colonies, which were able to growth on the selected medium with erythromycin, only two (the "D" and the "R") colonies were shown negative for presence of B polysaccharide. Among the others, 16 were clearly positive for B PS and still resistant to erythromycin. This indicated that they integrated the plasmid into their genome, but in the wrong orientation, and keeping intact the B PS and LPS gene (no double crossing-over). Positive and negative controls were also tested on the plates, and showed that the H44/76 wild type NmB strain was clearly positive for the B polysaccharide, while meningococcus A (A1) and meningococcus C (C11) strains were clearly negative with this anti-B PS 735 Mab. These results indicate that around 10 % of the selected colonies correctly integrated the plasmid in their genome by making a double crossing-over, while the other strains/colonies were obtained after a simple crossing-over, keeping the B PS and LPS genes intact and expressed.

Using whole cell Elisa, results (Figure 2 and the Table below) clearly indicate that the two "D" and "R" transformants (derived from D and R colonies) can not be recognized anymore by the anti-B PS Mabs (735 and 95/750), nor by the anti-Los 3,7,9 and anti-Los 8 Mabs. However, when using specific anti-PorA Mabs, there is a clear reaction with the anti-P1.7 and anti-P1.16 Mabs on the cells, as also observed in the wild-type strain. No reaction was observed with a non-specific anti-PorA Mab (anti-P1.2 mab). These results confirm that the PorA protein, and specifically P1.7 and P1.16 epitopes are still present after transformation, while B polysaccharide and Los 3.7,9 and Los 8 epitopes (LPS) were not.

**Table : Specificities of the monoclonal antibodies tested**

| **Mabs Tested** | **Directed against** | **Result** |
|---|---|---|
| Anti-B PS 735 | B polysaccharide | ++ on the wild type strain |
| | | (-) on the "D" and "R" mutants |
| Anti-B PS 95/750 from NIBSC | B PS | ++ on the wild type strain |
| | | (-) on the "D" and "R" mutants |
| Anti-P1.7 (NIBSC) | Loop of Porin A | ++ on all wild type and mutants strains |
| Anti-P1.16 (NIBSC) | Loop 4 of Porin A | ++ on all wild type and mutants strains |
| Anti-Los 3,7,9 | LPS | ++ on the wild type strain |
| | | (-) on the "D" and "R" mutants |
| Anti-Los 8 (NIBSC) | LPS | +/- on the wild type strain |
| | | (-) on the "D" and "R" mutants |
| Anti-P 1.2 (NIBSC) | Anti-Porin A Sero-subtype 1.2 | (-) on all wild type and mutants strains |

### Example 2: Construction of versatile gene delivery vectors (the pCMK series) targeting integration in the porA locus of Neisseiria meningitidis.

A plasmid allowing homologous recombination and stable integration of foreign DNA in the *porA* locus of *Neisseiria meningitidis* was constructed. This delivery vector (genes, operons and/or expression cassettes) is useful for constructing *Neisseiria meningitidis* strains producing recombinant, improved blebs. Typically, such a vector contains at least: (1) a plasmid backbone replicative in *E. coli* but not in *Neisseria meningitidis* (a suicide plasmid), (2) at least one, but preferably two regions of homology for targeting the integration in a chromosomal locus such as porA, (3) Efficient transcriptional (promoter, regulatory region and terminator) and translational (optimised ribosome binding site and initiation codon) signals functional in *Neisseria meningitidis,* (4) a multiple cloning site and (5) selectable gene(s) allowing the maintenance of the plasmid in *E. coli* and the selection of integrants in *Neisseria meningitidis.* Additional elements include, for example, uptake sequences to facilitate the entry of foreign DNA in *Neisseiria meningitidis,* and counter selectable markers such as *sacB, rpsL, gltS* to enhance the frequency of double cross-over events.

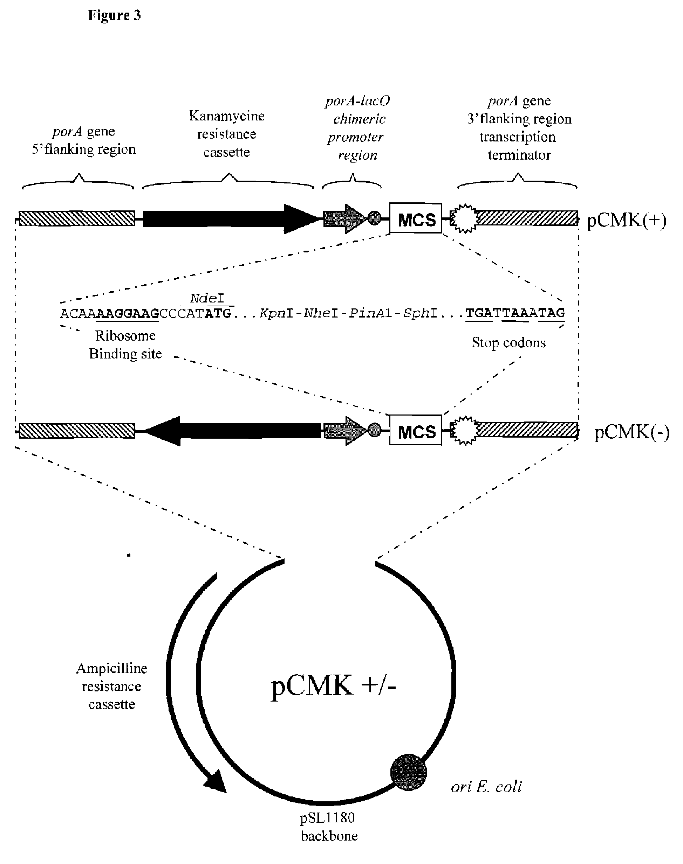

A schematic drawing of the vector constructed in this example and designated pCMK is represented in Figure 3. Its corresponding complete nucleotide sequence is shown in SEQ. ID NO:1. pCMK derives from a pSL1180 backbone (PharmaciaBiotech, Sweeden), a high copy-number plasmid replicative in *E. coli,* harbouring the *bla* gene (and thereby conferring resistance to ampicillin). In addition to this, pCMK functionally contains two *porA* flanking regions (porA5' and porA3' containing a transcription terminator) necessary for homologous recombination, a selectable marker conferring resistance to kanamycin, two uptake sequences, a porA/lacO chimeric promoter repressed in the *E.coli* host expressing *lacI^{q}* but transcriptionally active in *Neisseria meningitidis,* and a multiple cloning site (5 sites present: *Nde*I *, Kpn*I, *Nhe*I*, PinA*I and *Sph*I) necessary for the insertion of foreign DNA in pCMK.

pCMK was constructed as follows. The *porA5'* and *porA3'* recombinogenic regions, the *porA*/*lacO* promoter were PCR amplified using the oligonucleotides listed in the table below, cloned in pTOPO and sequenced. These DNA fragments were successively excised from pTOPO and recloned in pSL1180. The kanamycin resistance cassette was excised from pUC4K (PharmaciaBiotech, Sweeden) and was introduced between the porA5' flanking region and the *porA*/*lacO* promoter region.

**Table: Oligonucleotides used in this work**

| **Oligonucleotides** | **Sequence** | **Remark(s)** |
|---|---|---|
| PorA5' Fwd | 5'-CCC AAG CTT GCC GTC TGA ATA CAT CCC GTC ATT CCT CA-3' | *Hin*dIII **cloning site** Uplake sequence (_) |
| PorA5'Rev | 5'-CGA TGC TCG CGA CTC CAG AGA CCT CGT GCG GGC C-3' | *Nru* I **cloning site** |
| PorA3' Fwd | 5'-GGA AGA TCT GAT TAA ATA GGC GAA AAT ACC AGC TAC GA-3' | *Bgl* **cloning site** Stop codons (_) |
| PorA3'Rev | 5'-GCC GAA TTC TTC AGA CGG C GC AGC AGG AAT TTA TCG G-3' | *EcoR*I **cloning site** Uptake sequence (_) |
| PoLa Rev1 | 5'- GAA TTG TTA TCC GCT CAC AAT TCC GGG CAA ACA CCC GAT AC-3' | |
| PoLa Rev2 | 5'-GAA TTC CAT ATG ATC GGC TTC CTT TTG TAA ATT TGA TAA AAA CCT AAA AAC ATC GAA TTG TTA TCC GCT C-3' | *Nde*I **cloning site** |
| PorAlacO Fwd | 5'-AAG CTC TGC AGG AGG TCT GCG CTT GAA TTG-3' | *Pst*I **cloning site** |
| PorAlacO Rev | 5'-CTT AAG GCA TAT GGG CTT CCT TTT GTA A-3' | *Nde*I **cloning site** |
| PPA1 | 5'- GCG GCC GTT GCC GAT GTC AGC C-3' | |
| PPA2 | 5'-GGC ATA GCT GAT GCG TGG AAC TGC-3' | |
| N-full-01: | 5'-GGG AAT TCC ATA TGA AAA AAG CAC TTG CCA CAC-3' | *Nde*I **cloning site** |
| Nde-NspA-3: | 5'- GGA ATT CCA TAT GTC AGA ATT TGA CGC GCA C -3' | *Nde*I **cloning site** |
| PNS1 | 5'- CCG CGA ATT CGG AAC CGA ACA CGC CGT TCG-3' | *Eco*RI **cloning site** |
| YNS1 | 5'- CGT CTA GAC GTA GCG GTA TCC GGC TGC -3' | *Xba*I **cloning site** |
| PromD15-51X | 5'- GGG CGA ATT CGC GGC CGC CGT CAA CGG CAC ACC CGT TG-3' | *Eco*RI and *Not*I **cloning sites** |
| PromD15-S2 | 5'- GCT CTA GAG CGG AAT GCG GTT TCA GAC G- 3' | *Xba*I **cloning site** |
| PNS4 | 5'- AGC TTT ATT TAA ATC CTT AAT TAA CGC GTC CGG AAA ATA TGC TTA TC_34 | *SwaI* and *PacI* **cloning sites** |
| PNS5 | 5'- AGC TTT GTT TAA ACC CTG TTC CGC TGC TTC GGC-3' | *PmeI* **cloning site** |
| D15-S4 | 5'- GTC CGC ATT TAA ATC CTT AAT TAA GCA GCC GGA CAG GGC GTG G-3' | *SwaI* and *PacI* **cloning sites** |
| D15-S5 | 5'- AGC TTT GTT TAA AGG ATC AGG GTG TGG TCG GGC-3' | *PmeI* **cloning site** |

### Example 3: Construction of a Neisseiria meningitidis serogroup B strain lacking both capsular polysaccharides and the major immunodominant antigen PorA.

Modulating the antigenic content of outer membrane blebs may be advantageous in improving their safety and efficacy in their use in vaccines, or diagnostic or therapeutic uses. Components such as the *Neisseiria meningitidis* serogroup B capsular polysaccharides should be removed to exclude the risk of inducing autoimmunity (see example 1). Similarly, it is beneficial to suppress the immunodominance of major outer-membrane antigens such as PorA, which induce strain-specific bactericidal antibodies but fail to confer cross-protection. To illustrate such an approach, we used the pCMK(+) vector to construct a *Neisseiria meningitidis* serogroup B strain lacking both capsular polysaccharides and the immunodominant PorA outer membrane protein antigen. For this purpose, a deletion of the *porA* gene was introduced in the H44/76 *cps-* strain, described in example 1 by means of homologous recombination.

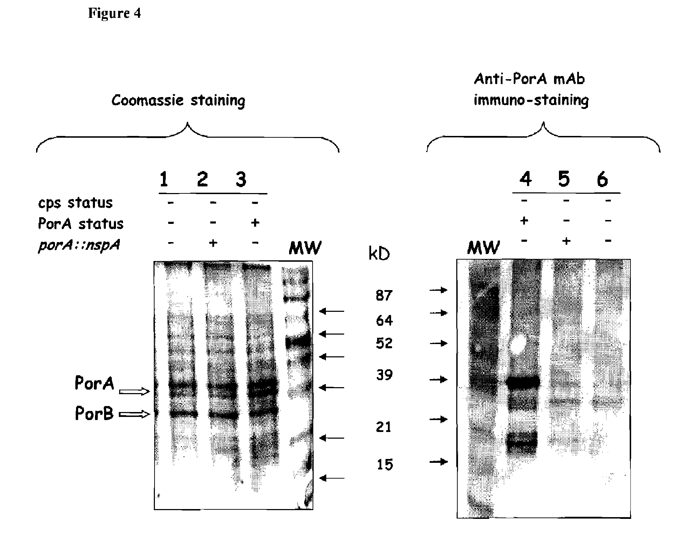

The H44/76 *cps-* strain was prepared competent and transformed with two 2µg of supercoiled pCMK(+) plasmid DNA as described previously. Aliquot fractions of the transformation mixture (100µl) were plated on Mueller-Hinton plates supplemented with Kanamycin (200µg/ml) and incubated at 37°C for 24 to 48 hours. Kanamycin-resistant colonies were selected, restreaked on MH-Kn and grown for an additional 24 hours at 37°C. At that stage half of the bacterial culture was used to prepare glycerol stocks (15 % vol./vol.) and was kept frozen at -70°C. Another fraction (estimated to be 10⁸ bacteria) was resuspended in 15 µl of distilled water, boiled ten minutes and used as a template for PCR screening. Two *porA* internal primers named , PPA1 and PPA2, were synthesized and used to perform PCR amplification on boiled bacterial lysates in the conditions described by the supplier (HiFi DNA polymerase, Boehringer Mannheim, GmbH). The thermal cycling used was the following: 25 times (94°C 1min., 52°C 1min., 72°C 3min.) and 1 time (72°C 10min., 4°C up to recovery). Since a double crossing-over between pCMK DNA and the chromosomal *porA* locus deletes the region required for #1 and #2 annealing, clones lacking a 1170bp PCR amplification fragment were selected as *porA* deletion mutants. These PCR results were further confirmed by analyzing in parallel, the presence of PorA in the corresponding bacterial protein extracts. For that purpose, another aliquot of bacteria (estimated to be 5.10⁸ bacteria) was re-suspended in 50 µl of PAGE-SDS buffer (SDS 5%, Glycerol 30%, Beta-mercaptoethanol 15%, Bromophenol blue 0.3mg/ml, Tris-HCl 250 mM pH6.8), boiled (100°C)frozen(-20°C) / boiled (100°C) three times and was separated by PAGE-SDS electrophoresis on a 12.5 % gel. Gels were then stained by Coomassie Brilliant blue R250 or transferred to a nitrocellulose membrane and probed with an anti-PorA monoclonal antibody as described in Maniatis *et al.* As represented in Figure 4, both Coomassie and immunoblot staining confirmed that *porA* PCR negative clones do not produce detectable levels of PorA. This result confirm that the pCMK vector is functional and can be used successfully to target DNA insertion in the *porA* gene, abolishing concomitantly the production of the PorA outer membrane protein antigen.

### Example 4: Up-regulation of the NspA outer membrane protein production in blebs derived from a recombinant Neisseiria meningitidis serogroup B strain lacking functional porA and cps genes.

Enriching bleb vesicles with protective antigens is advantageous for improving the efficiency and the coverage of outer membrane protein-based vaccines. In that context, recombinant *Neisseria meningitidis* strains lacking functional *cps* and *porA* genes were engineered so that the expressions level of the outer-membrane protein NspA was up-regulated. For that purpose, the gene coding for NspA was PCR amplified using the N01-full-*Nde*I and *Nde*I*-*3'oligonucleotide primers (see table in example 2). The conditions used for PCR amplification were those described by the supplier (HiFi DNA polymerase, Boehringer Mannheim, GmbH). Thermal cycling done was the following: 25 times (94°C 1min., 52°C 1min., 72°C 3min.) and 1 time (72°C 10min., 4°C up to recovery). The corresponding amplicon was digested with Ndel and inserted in the *Nde*I restriction site of the pCMK(+) delivery vector. Insert orientation was checked and recombinant plasmids, designed pCMK(+)-NspA, were purified at a large scale using the QIAGEN maxiprep kit and 2 µg of this material was used to transform a *Neisseiria meningitidis* serogroup B strain lacking functional *cps* genes (strain described in example 1). Integration resulting from a double crossing-over between the pCMK(+)-NspA vector and the chromosomal *porA* locus were selected using a combination of PCR and Western blot screening procedures presented in example 3.

Bacteria (corresponding to about 5.10⁸ bacteria) were re-suspended in 50 µl of PAGE-SDS buffer, frozen(-20°C) / boiled (100°C) three times and then were separated by PAGE-SDS electrophoresis on a 12.5 % gel. Gels were then stained by Coomassie Brilliant blue R250 or transferred to a nitrocellulose membrane and probed with an anti-NspA polyclonal serum. Both Coomassie (data not shown) and immunoblot staining (see figure 4) confirmed that *porA* PCR negative clones do not produce detectable levels of PorA. The expression ofNspA was examined in Whole-cell bacterial lysates (WCBL) or outer-membrane bleb preparations derived from NmB [*cps-, porA-*] or NmB [*cps-, porA-, Nspa*+]*.* Although no difference was observable by Coomassie staining, immunoblotting with the anti-NspA polyclonal serum detected a 3-5 fold increased in the expression of NspA (with respect to the endogenous NspA level), both in WCBL and outer-membrane bleb preparations (see figure 5). This result confirm that the pCMK(+)-NspA vector is functional and can be used successfully to up-regulate the expression of outer membrane proteins such as NspA, abolishing concomitantly the production of the PorA outer membrane protein antigen.

### Example 5: Up-regulation of the Dt5/Omp85 outer membrane protein antigen in blebs derived from a recombinant Neisseiria meningitidis serogroup B strain lacking functional cps genes but expressing PorA.

Certain geographically isolated human populations (such as Cuba) are infected by a limited number of *Neisseiria meningitidis* isolates belonging largely to one or few outer membrane protein serotypes. Since PorA is a major outer-membrane protein antigen inducing protective and strain-specific bactericidal antibodies, it is then possible to confer vaccine protection using a limited number of porA serotypes in a vaccine. In such a context, the presence of PorA in outer membrane vesicles may be advantageous, strengthening the vaccine efficacy of such recombinant improved blebs. Such PorA containing vaccines, however, can be improved still further by increasing the level of other cross-reactive OMPs such as omp85/D15.

In the following example, the pCMK(+) vector was used to up-regulate the expression of the Omp85/D15 outer membrane protein antigen in a strain lacking functional *cps* genes but expressing *porA.* For that purpose, the gene coding for Omp85/D15 was PCR amplified using the D15-*Nde*I and D15-*Not*I oligonucleotide primers. The conditions used for PCR amplification were those described by the supplier (HiFi DNA polymerase, Boehringer Mannheim, GmbH). Thermal cycling done was the following: 25 times (94°C 1min., 52°C 1min., 72°C 3min.) and 1 time (72°C 10min., 4°C up to recovery). The corresponding amplicon was inserted in the pTOPO cloning vector according to the manufacturer's specifications and confirmatory sequencing was performed. This Omp85/D15 DNA fragment was excised from pTOPO by restriction hydrolysis using *Nde*I*lNsi*I and subsequently cloned in the corresponding restriction sites of the pCMK(+) delivery vector. Recombinant plasmids, designed pCMK(+)-D15 were purified on a large scale using the QIAGEN maxiprep kit and 2 µg of this material was used to transform a *Neisseiria meningitidis* serogroup B strain lacking functional *cps* genes (strain described in example 1). In order to preserve the expression of *porA,* integration resulting from a single crossing-over (either in Omp85/D15 or in porA) were selected by a combination of PCR and Western blot screening procedures. Kanamycin resistant clones testing positive by *porA*-specific PCR and western blot were stored at -70°C as glycerol stocks and used for further studies.

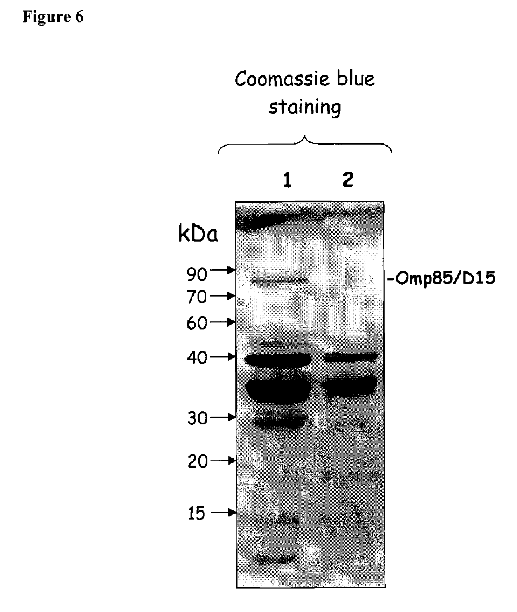

Bacteria (corresponding to about 5.10⁸ bacteria) were re-suspended in 50 µl of PAGE-SDS buffer, frozen(-20°C) / boiled (100°C) three times and then were separated by PAGE-SDS electrophoresis on a 12.5 % gel. Gels were then stained by Coomassie Brilliant blue R250 or transferred to a nitrocellulose membrane and probed with an anti-porA monoclonal antibody. As represented in Figure 6, both Coomassie and immunoblot staining confirmed that *porA* PCR positive clones produce PorA.

The expression of D15 was examined using outer-membrane bleb preparations derived from NmB [cps-, porA-] or NmB [cps-, porA+, D15+]. Coomassie detected a significant increase in the expression of D15 (with respect to the endogenous D15 level), preparations (see Figure 6). This result confirmed that the pCMK(+)-D15 vector is functional and can be used successfully to up-regulate the expression of outer membrane proteins such as D15, without abolishing the production of the major PorA outer membrane protein antigen.

### Example 6: Construction of versatile promoter delivery vectors

Rational: The rational of this approach is represented in Figure 7 and can be summarized in 7 essential steps. Some of these steps are illustrated below with the construction of Vector for up-regulating the expression of NspA and D15/Omp85.

### Vector for up-regulating the expression of the NspA gene.

**Step 1.** A DNA region (997bp) located upstream from the NspA coding gene was discovered (SEQ. ID NO:2) in the private Incyte PathoSeq data base containing unfinished genomic DNA sequences of the *Neisseria meningitidis* strain ATCC 13090. Two oligonucleotide primers referred to as PNS1 and PNS2 (see table in example 2) were designed using this sequence and synthesized. These primers were used for PCR amplification using genomic DNA extracted from the H44/76 strain. **Step 2.** The corresponding amplicons were cleaned-up using the Wizard PCR kit (Promega, USA) and submitted to digestion with the *Eco*RI*lXba*I restriction enzymes for 24 hours using the conditions described by the supplier (Boehringer Mannheim, Germany). The corresponding DNA fragments were gel purified and inserted in the corresponding sites of the pUC18 cloning vector. **Step 3.** Recombinant plasmids were prepared on a large scale and an aliquot fraction was used as a template for inverse PCR amplification. Inverse PCR was performed using the PNS4 and PNS5 oligonucleotides using the following thermal cycling conditions: 25 times (94°C 1min., 50°C 1min., 72°C 3min.) and 1 time (72°C 10min., 4°C up to recovery). Linearized pUC 18 vectors harbouring a deletion in the NspA upstream region insert were obtained.

### Vector for up-regulating the expression of the D15/omp85 gene.

**Step 1.** A DNA region (1000 bp) located upstream from the D15/omp85 coding gene was discovered (SEQ. ID NO:3) in the private Incyte PathoSeq database containing unfinished genomic DNA sequences of the *Neisseria meningitidis* strain ATCC 13090. Two oligonucleotide primers refererred to as PromD15-51X and PromD15-S2 (see table in example 2) were designed using this sequence and synthesized. These primers were used for PCR amplification using genomic DNA extracted from the H44/76 strain. **Step 2.** The corresponding amplicons were cleaned-up using the Wizard PCR kit (Promega, USA) and submitted to digestion with the *Eco*RI/*Xba*I restriction enzymes for 24 hours in the conditions described by the supplier (Boehringer Mannheim, Germany). The corresponding DNA fragments were gel purified and inserted in the corresponding sites of the pUC 18 cloning vector. Step 3. Recombinant plasmids were prepared on a large scale and an aliquot fraction was used as a template for inverse PCR amplification. Inverse PCR was performed using the D15-S4 and D15-S5 oligonucleotides using the following thermal cycling conditions: 25 times (94°C 1min., 50°C 1min., 72°C 3min.) and 1 time (72°C 10min., 4°C up to recovery). Linearized pUC 18 vectors harbouring a deletion in the D15/omp85 upstream region insert were obtained.

### Example 7: Fermentation processes for producing recombinant blebs .

The examples listed below describe methods for producing recombinant blebs lacking either capsular polysaccharides or capsular polysaccharides and PorA. Such a procedure may be used for a wide range of *Neisseiria meningitidis* recombinant strains and may be adapted over an extended scale range.

Culture media:*Neisseiria meningitidis* serogroup B strains were propagated in solid (FNE 004 AA, FNE 010 AA) or liquid (FNE 008 AA) culture media. These new media for growing meningococcus are advantageiously free of animal products, and are considered a further aspect of the invention.

| **Components** | **FNE 004 AA** | **FNE 008 AA** | **FNE 010 AA** |
|---|---|---|---|
| Agar | 18 g/L | - | 18 g/L |
| NaCl | 6 g/L | 6 g/L | 6 g/L |
| Na-Glutamate | - | 1.52 g/L | |
| NaH₂PO₄.2H₂O | 2.2 g/L | 2.2 g/L | 2.2 g/L |
| KCl | 0.09 g/L | 0.09 g/L | 0.09 g/L |
| NH₄Cl | 1.25 g/L | 1.25 g/L | 1.25 g/L. |
| Glucose | 5 g/L | 20 g/L | 5 g/L |
| Yeast Extract UF | - | 2.5 g/L | - |
| Soy Pepton | 5 g/L | 30 g/L | 5 g/L |
| CaCl₂.2H₂O | 0.015 g/L | - | 0.015 g/L |
| MgSO₄.7H₂O | 0.6 g/L | 0.6 g/L | 0.6 g/L |
| Erythromycine : | 0.015 g/L | - | - |
| Kanamycine | - | - | 0.2 g/L |

Flask cultivation of *Neisseiria meningitidis* serogroup B cps- recombinant blebs: This was performed in two steps comprising preculture on solid medium followed by liquid cultivation. **Solid pre-culture** A vial of seed was removed from freezer (-80°C), thawed to room temperature and 0.1 mL was streaked into a Petri dish containing 15 mL of FNE004AA (see above).The Petri dish was incubated at 37°C for 18 ± 2 hours. The surface growth was resuspended in 8 mL of FNE008AA (see above) supplemented with 15 mg/L of erythromycin. **Flask culture.** 2 mL of resuspended solid pre-culture were added to a 2 litre flask containing 400 mL of FNE008AA supplemented with 15 mg/L of erythromycin. The flask was placed on a shaking table (200 rpm) and incubated at 37°C for 16 ± 2 hours. The cells were separated from the culture broth by centrifugation at 5000g at 4°C for 15 minutes.

Batch mode cultivation of *Neisseiria meningitidis* serogroup B *cps-* recombinant blebs: This was performed in three steps comprising preculture on solid medium, liquid cultivation and batch mode cultivation. **Solid pre-culture**._A vial of seed was removed from freezer (-80°C), thawed to room temperature and 0.1 mL was streaked into a Petri dish containing 15 mL of FNE004AA (see above). The Petri dish was incubated at 37°C for 18 ± 2 hours. The surface growth was resuspended in 8 mL of FNE008AA (see above) supplemented with 15 mg/L of erythromycin. **Liquid pre-culture**._2 mL of resuspended solid pre-culture were added to one 2 liters flask containing 400 mL of FNE008AA supplemented with 15 mg/L of erythromycin. The flask was placed on a shaking table (200 rpm) and incubated at 37°C for 16 ± 2 hours. The content of the flask was used to inoculate the 20 liters fermenter. **Batch mode culture in fermenter**. The inoculum (400 mL) was added to a pre-sterilized 20 liters (total volume) fermenter containing 10 L of FNE008AA supplemented with 15 mg/L of erythromycin. The pH was adjusted to and maintained at 7.0 by the automated addition of NaOH (25% w/v) and H₃PO₄ (25% v/v). The temperature was regulated at 37°C. The aeration rate was maintained at 20 L of air / min and the dissolved oxygen concentration was maintained at 20% of saturation by the agitation speed control. The overpressure in the fermenter was maintained at 300 g/cm². After 9 ± 1 hours, the culture was in stationary phase. The cells were separated from the culture broth by centrifugation at 5000g at 4°C for 15 minutes.

Flask cultivation of *Neisseiria meningitidis* serogroup B *cps-,* PorA- recombinant blebs: This was performed in two steps comprising preculture on solid medium followed by liquid cultivation._**Solid pre-culture.** A vial of seed was removed from freezer (-80°C), thawed to room temperature and 0.1 mL was streaked into a Petri dish containing 15 mL of FNE010AA (see above). The Petri dish was incubated at 37°C for 18 ± 2 hours. The surface growth was resuspended in 8 mL of FNE008AA (see above) supplemented with 200 mg/L of kanamycin. **Flask culture**. 2 mL of resuspended solid pre-culture were added to a 2 litre flask containing 400 mL of FNE008AA supplemented with 200 mg/L of kanamycin. The flask was placed on a shaking table (200 rpm) and incubated at 37°C for 16 ± 2 hours. The cells were separated from the culture broth by centrifugation at 5000g at 4°C for 15 minutes.

### Example 8: Isolation and purification of blebs from meningococci devoid of capsular polysaccharide

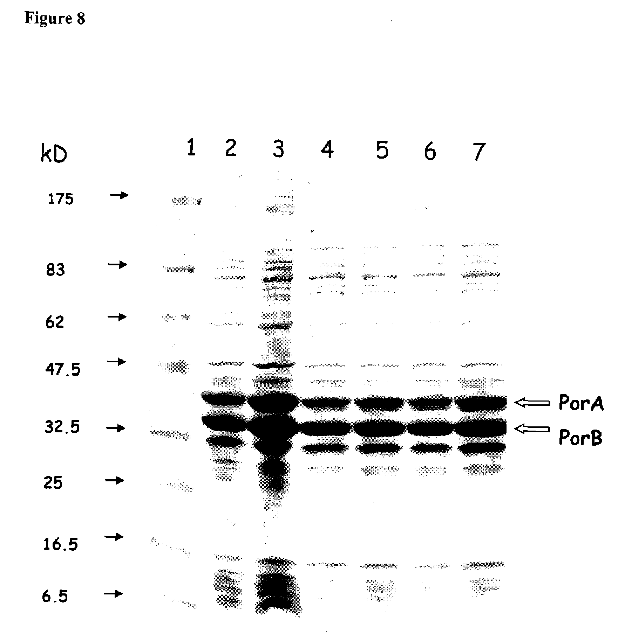

Recombinant blebs were purified as described below. The cell paste (42gr) was suspended in 211 ml of 0.1M Tris-Cl buffer pH 8.6 containing 10 mM EDTA and 0.5% Sodium Deoxycholate (DOC). The ratio of buffer to biomass was 5/1 (V/W). The biomass was extracted by magnetic stirring for 30 minutes at room temperature. Total extract was then centrifuged at 20,000g for 30 minutes at 4°C (13,000 rpm in a JA-20 rotor, Beckman J2-HS centrifuge). The pellet was discarded. The supernatant was ultracentrifuged at 125,000g for 2 hours at 4°C (40,000 rpm in a 50.2Ti rotor, Beckman L8-70M ultracentrifuge) in order to concentrate vesicles. The supernatant was discarded. The pellet was gently suspended in 25 ml of 50 mM Tris-Cl buffer pH 8.6 containing 2 mM EDTA, 1.2% DOC and 20% sucrose. After a second ultracentrifugation step at 125,000g for 2 hours at 4°C, vesicles were gently suspended in 44 ml of 3% sucrose and stored at 4°C. All solutions used for bleb extraction and purification contained 0.01% thiomersalate. As illustrated in Figure 8, this procedure yields protein preparations highly enriched in outer-membrane proteins such as PorA and PorB.

### Example 9: Identification of bacterial promoters suitable for up-regulation antigens-coding genes

The use of strong bacterial promoter elements is essential to obtain up-regulation of genes coding for outer membrane proteins. In that context, we have shown previously that up-regulating the *Neisseria meningitidis nspA, hsf,* and *omp85* genes using the *porA* promoter has allowed us to isolate recombinant blebs enriched in the corresponding NspA, Hsf and Omp85 proteins. Alternatives to the *porA* promoter may be useful to obtain different levels of up-regulation, to overcome potential *porA* phase variation and/or to achieve conditional gene expression (iron-regulated promoters). Here we describe a method allowing the identification of a precise transcriptional start site of strong promoter elements likely to confer high level of expression in bacteria. Since promoter regulatory elements are classically encompassed within 200 bp upstream and 50bp dowtream from the +1 site (Collado-Vides J, Magasanik B, Gralla JD, 1991, Microbiol Rev 55(3):371-94), the result of such an experiment allows us to identify DNA fragments of about 250 bp carrying strong promoter activities. Major outer membrane proteins such as *Neisseria meningitidis* PorA, PorB & Rmp, *Haemophilus influenzae* P1, P2, P5 & P6, *Moraxella catarrhalis* OmpCD, OmpE, as well as some cyoplasmic and/or iron regulated proteins of these bacteria possess strong promoter elements. As a validation of this general methodology, we mapped the transcriptional start site of the strong *Neisseria meningitidis porA* and *porB* promoters using rapid amplification of cDNA elements (5' RACE).

The principles of 5' RACE are the following: 1) Total RNA extraction using QIAGEN "RNeasy" Kit. Genomic DNA removing by DNase treatment followed by QIAGEN purification; 2) mRNA reverse transcription with a *porA* specific 3' end primer (named porA3). Expected cDNA size: 307 nt. RNA removing by alkaline hydrolysis; 3) Ligation of a single-stranded DNA oligo anchor (named DT88) to the 3' end of the cDNA using T4 RNA ligase. Expected product size: 335 nt. Amplification of the anchor-ligated cDNA using a combination of hemi-nested PCR; 4) PCR amplification of the anchor-ligated cDNA using a complementary-sequence anchor primer as the 5' end primer (named DT89) and a 3'end primer (named p1-2) which is internal to the 3'end RT primer porA3. Expected product size: 292 bp; 5) PCR amplification of previous PCR products using DT89 as 5'end primer and p1-1 as 3'end primer (internal to p1-2). Expected product size: 211bp; and 6) Sequencing with p1-1 primer (expected products size can be calculated because *porA* transcription start site is known: 59 nt before the "ATG" translation start site).

### Experimental procedure

Total RNA was extracted from approximately 10⁹ cells of *Neisseria meningitidis* serogroup B *cps- porA*+ strain. Extraction of 1 ml of a liquid culture at appropriate optical density (OD₆₀₀ = 1) was performed by the QIAGEN "RNAeasy" kit according to the manufacturer's instructions. Chromosomal DNA was removed by addition of 10U of RNase-free DNase (Roche Diagnostics, Mannheim, Germany) to the 30 µl of eluted RNA and was incubated at 37°C for 15 min. The DNA-free RNA was purified with the same QIAGEN kit according to instructions.

Reverse transcription reactions were performed using primer porA3 and 200U of SUPERSCRIPT II reverse transcriptase (Life Technologies). The RT reactions were performed in a 50µl volume containing: 5µl of 2mM dNTP, 20 pmol of porA3 pimer, 5µl of 10X SUPERSCRIPT II buffer, 9µl of 25mM MgCl2, 4µl of 0.1M DTT, 40U of recombinant ribonuclease inhibitor and 1 µg of total RNA. The porA3 primer was annealed stepwise (70°C for 2 min, 65°C for 1 min, 60°C for 1 min, 55°C for 1 min, 50°C for 1 min, and 45°C for 1 min) before the SUPERSCRIPT II was added. The RT reaction was performed at 42°C for 30 min, followed by 5 cycles (50°C for 1 min, 53°C for 1 min and 56°C for 1 min) to destabilize RNA secondary structure. Two parallel reactions were performed with the reverse transcriptase omitted from one reaction as negative control.

The RNA was removed by alkaline hydrolysis cleavage with the addition of I µl of 0.5M EDTA followed by 12.5 µl of 0.2 M NaOH before incubation at 68°C for 5 min. The reactions were neutralized by adding 12.5 µl of 1 M Tris-HCl (pH7.4) and precipitated by the addition of 20 µg of glycogen (Roche Molecular Biochemicals, Mannheim, Germany), 5 µl of 3 M sodium acetate and 60 µl of isopropanol. Both samples were resuspended in 20 µl of 10:1 TE (10 mM Tris-HCl, pH 7.4; 1 mM EDTA, pH8).

T4 RNA ligase was used to anchor a 5'-phosphorylated, 3'end ddCTP-blocked anchor oligonucleotide DT88 (see table below). Two parallel ligations were performed overnight at room temperature with each containing: 1.3 µl of 10X RNA ligase buffer (Roche Molecular Biochemicals), 0.4 µM DT88, 10 µl of either cDNA or RT control sample and 3 U of T4 RNA ligase. As negative controls, a second set of ligations reactions was performed, omitting the T4 RNA ligase. The resulting ligation-reaction mixtures were used directly without purification in the subsequent PCR.

The anchor-ligated cDNA was amplified using a combination of hemi-ncsted and hot-started PCR approaches to increase specificity and product yield. Four separate first-round PCR were performed on the RT/ligase reaction and controls in a 30 µl volume, each containing: 3 µl of 10X Taq Platinium buffer, 3µl of 25 mM MgCl₂, 1 µl of 10mM dNTP, 10 pmol of each primers and 1 µl of corresponding RNA ligation reaction. The PCR were hot started by the use of Taq Platinium (Life Technologies) DNA polymerase (2U added). The first ligation-anchored PCR (LA-PCR) was performed using 10 pmol of both the anchor-specific primer DT89 and the transcript-specific primer p1-2 (see table below) which is internal to the 3' end RT primer porA3. The PCR was performed using an initial 95°C for a 5 min step (for DNA polymerase activation) followed by 10 cycles at 95°C for 10 s and 70°C for 1 min (reducing one degree per cycle), 15 cycles at 95°C for 10 s and 60°C for 1 min. The second hemi-nested LA-PCR was performed under the same conditions using primer DT89 and the pl-2 internal primer, together with 10 pmol of pl-1 (see table below) and 1 µl of first-round PCR. Amplification products were purified using the QIAGEN "QIAquick PCR purification" kit according to manufacturer instructions before submitted to sequencing.

The CEQ^{™} Dye Terminator Cycle Sequencing kit (Beckman, France) was used to sequence the RACE PCR products using 10 pmol of primer p1-1. Sequencing reactions were performed according to the provided instructions and sequencing products were analyzed by the Ceq2000 DNA Analysis System (Beckman-Coulter).

| | |
|---|---|
| DT88 | 5' GAAGAGAAGGTGGAAATGGCGTTTTGGC 3' |
| DT89 | 5' CCAAAACGCCATTTCCACCTTCTCTTC 3' |
| porA3 | 5' CCAAATCCTCGCTCCCCTTAAAGCC 3' |
| pl-2 | 5' CGCTGATTTTCGTCCTGATGCGGC 3' |
| pl-1 | 5' GGTCAATTGCGCCTGGATGTTCCTG 3' |

### Results for the Neisseria meningitidis porA promoter

The start of transcription for *Neisseria meningitidis* serogroup B (strain H44/76) *porA-*mRNA was mapped 59 bp upstream of the ATG start codon using the described 5'-RACE procedure. This result confirms the mapping performed by primer extension and published by van der Ende *et al* (1995). This result supports that a DNA fragment containing nucleotides -9 to -259 with regard to the *porA* ATG is suitable for driving strong gene expression in *Neisseria meningitidis* and possibly in other bacterial species such as *Haemophilus, Moraxella, Pseudomonas.*

### Results for the Neisseria meningitidis porB promoter

The same experimental strategy has been applied for *Neisseria meningitidis* serogroup B (strain H44/76) *porB* transcription start site mapping. Primers listed in the table below correspond to 3' end RT primer (porB3), transcript-specific primer that is internal to the porB3 (porB2) and internal to the porB2 (porB1). porB3, porB2 and porB1 arc respectively located 265 bp, 195 bp and 150 bp downstream the ATG start codon.

| | |
|---|---|
| porB1 | 5' GGTAGCGGTTGTAACTTCAGTAACTT 3' |
| porB2 | 5' GTCTTCTTGGCCTTTGAAGCCGATT 3' |
| porB3 | 5' GGAGTCAGTACCGGCGATAGATGCT 3' |

Using porB1 and DT89 primers a ~200 bp PCR amplicon was obtained by performing 5' - RACE mapping. Since porB1 is located 150 bp from the *porB* ATG start codon, this result supports that the *porB* transcriptional start site is located about 50 bp (+/-30 bp) upstream of the porB ATG.

The exact nucleotide corresponding to transcription initiation is presently being determined by DNA sequencing. The above PCR result supports that a DNA fragment containing nucleotides -1 to -250 with regard to the *porB* ATG start codon is suitable for driving strong gene expression in *Neisseria meningitidis* and possibly in other bacterial species such as *Haemophilus, Moraxella, Pseudomonas.*

### Example 10: Up-regulation of the N. meningitidis serogroup B Omp85 gene by promoter replacement.

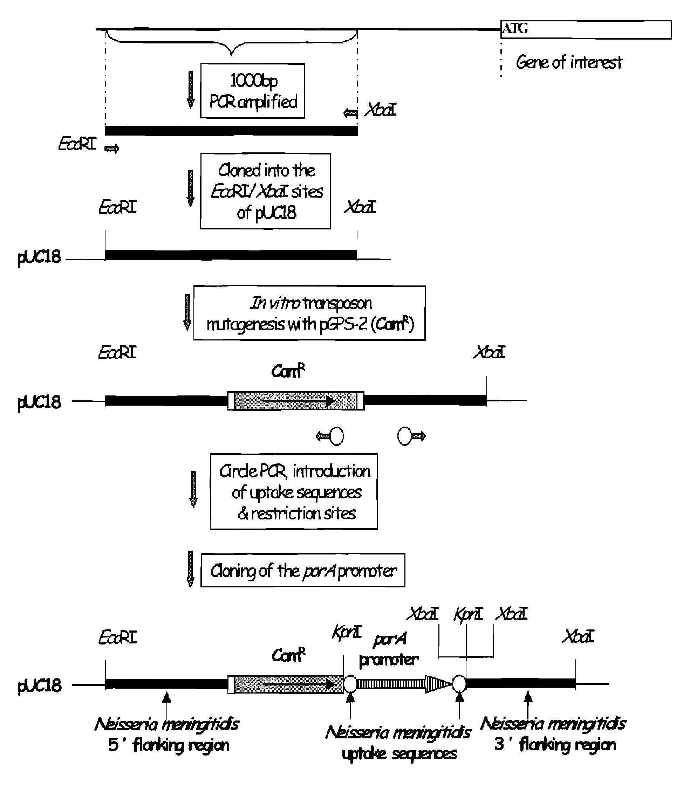

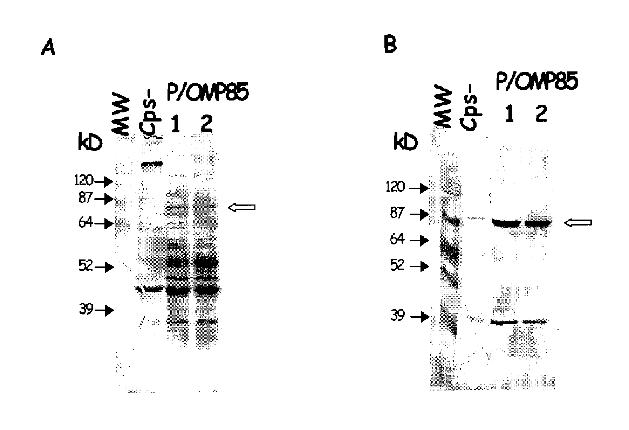

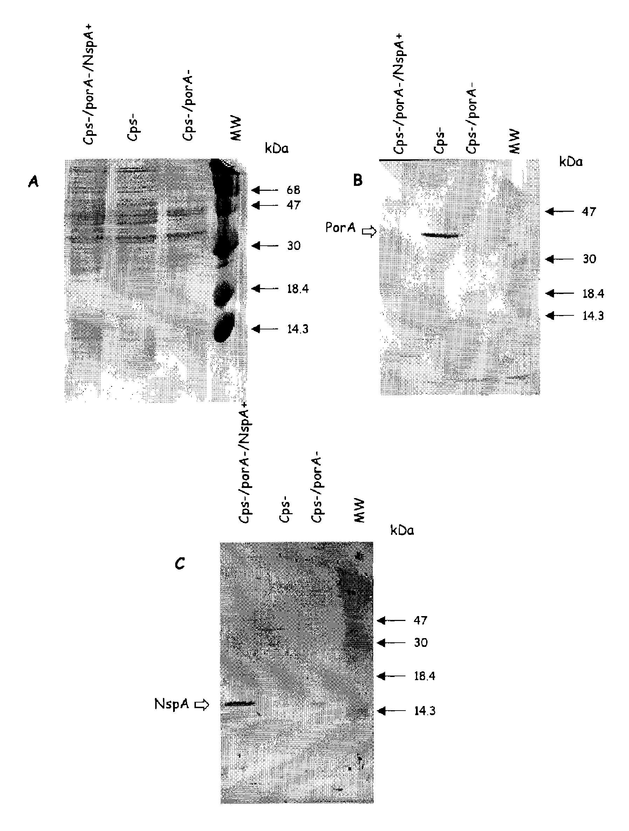

The aim of the experiment was to replace the endogenous promoter region of the D15/Omp85 gene by the strong *porA* promoter in order to up-regulate the production of the D15/Omp85 antigen. For that purpose, a promoter replacement plasmid was constructed using *E. coli* cloning methodologies. A DNA region (1000 bp) located upstream from the *D15*/*omp85* coding gene was discovered (SEQ ID NO:3) in the private Incyte PathoSeq data base containing unfinished genomic DNA sequences of the *Neisseria meningitidis* strain ATCC 13090. The main steps of this procedure are represented in Figure 9. Briefly, a DNA fragment (1000bp) covering nucleotides -48 to -983 with respect to the *D15*/*Omp85* gene start codon (ATG) was PCR amplified using oligonucleotides **ProD15-51X** (5'-GGG CGA ATT CGC GGC CGC CGT CAA CGG CAC ACC GTT G-3') and **ProD15-52** (5'-GCT CTA GAG CGG AAT GCG GTT TCA GAC G-3') containing *Eco*RI and *Xba*I restriction sites (underlined) respectively. This fragment was submitted to restriction and inserted in pUC18 plasmid restricted with the same enzymes. The construct obtained was submitted to *in vitro* mutagenesis using the Genome Priming system (using the pGPS2 donor plasmid) commercialized by New England Biolabs (MA, USA). Clones having inserted a mini-transposon (derived from Tn7 and harboring a chloramphenicol resistance gene) were selected. One clone containing a mini-transposon insertion located in the *D15*/*Omp85* 5' flanking region, 401 bp downstream from the *Eco*RI site was isolated and used for further studies. This plasmid was submitted to circle PCR mutagenesis (Jones & Winistofer (1992), Biotechniques12: 528-534) in order to (i) delete a repeated DNA sequence (Tn7R) generated by the transposition process, (ii) insert meningococcal uptake sequences required for transformation, and (iii) insert suitable restriction sites allowing cloning of foreign DNA material such as promoters. The circle PCR was performed using the **TnRD15-KpnI/XbaI** + US (5'-CGC C GG TAC CTC TAG AGC CGT CTG AAC CAC TCG TGG ACA ACC C-3') & **TnR03Cam(KpnI)** (5'-CGC CGG TAC CGC CGC TAA CTA TAA CGG TC-3') oligonucleotides containing uptake sequences and suitable restriction sites (*Kpn*I and *Xba*I) underlined. The resulting PCR fragment was gel-purified, digested with *Asp*718 (isoschizomer of *Kpn*I) and ligated to a 184bp DNA fragment containing the *porA* promoter and generated by PCR using the
**PorA-01** (5'-CGC CGG TAC CGA GGT CTG CGC TTG AAT TGT G-3') and
**PorA02** (5'-CGC CGG TAC CTC TAG ACA TCG GGC AAA CAC CCG-3') oligonucleotides containing *Kpn*I restriction sites. Recombinant clones carrying a *porA* promoter inserted in the correct orientation (transcription proceeding in the *Eco*RI to *Xba*I direction) were selected and used to transform a strain of *Neisseria meningitidis* serogroup B lacking capsular polysaccharide *(cps-)* and one of the major outer membrane proteins - PorA (*porA-*). Recombinant *Neisseria meningitidis* clones resulting from a double crossing over event (PCR screening using oligonucleotides
**Cam-05** (5'-GTA CTG CGA TGA GTG GCA GG-3') & **proD15-52**) were selected on GC medium containing 5µg/ml chloramphenicol and analyzed for D15/Omp85 expression. As represented in Figure 10, the production of D15/Omp85 was significantly increased in the total protein extracts of *Nm* strains resulting from promoter replacement, when compared to parental strain *(cps-).* This result was also observed when analyzing outer-membrane blebs prepared from the same strains (see Figure 17). These results are attributable to the replacement of the endogenous *D15* promoter by the strong *porA* promoter. In addition, it was surprisingly found that expression, where the porA promoter was introduced approximately 400 bp upstream of the initiator codon, was approximately 50 times greater than when the promoter was placed approximately 100 bp upstream. Altogether, these experiments support that the promoter replacement strategy works and allows the up-regulation of the synthesis of integral outer-membrane proteins in outer-membrane blebs.

Certain geographically isolated human populations (such as Cuba) are infected by a limited number of *Neisseiria meningitidis* isolates belonging largely to one or few outer membrane protein serotypes. Since PorA is a major outer-membrane protein antigen which can induce protective and strain-specific bactericidal antibodies, it may be possible to confer vaccine protection in such a population using a limited number of porA serotypes. Moreover, PorA may interact with or stabilize some other outer membrane proteins. In this context, the presence of PorA in outer membrane vesicles may be advantageous, strengthening the vaccine efficacy of such recombinant improved blebs.

For such a reason, it may be desirable to up-regulate the expression of D15/Omp85 outer membrane protein in a *Neisseria meningitidis* serogroup B strain lacking functional *cps* genes but expressing PorA. Genomic DNA was extracted from the recombinant *Neisseria meningitidis* serogroup B *cps-, porA-,* D15/Omp85+ strain using the QIAGEN Genomic Tips 100-G kit. 10µgr of this material was linearized and used to transform *Neisseria meningitidis* serogroup B *cps-* following a classical transformation protocol. Recombinant *Neisseria* were obtained on GC agar plates containing 5µgr/ml chloramphenicol.

Integrations resulting from a double crossing-over upstream of the *D15* gene were screened by PCR as described previously. As homologous recombinations can occur everywhere in the chromosome, a second PCR screening was performed to control the integrity of the *porA* locus in the recombinant strain. For this purpose, internal *porA* primers **PPA1** (5- GCG GCC GTT GCC GAT GTC AGC C -3') and **PpA2** ( 5- GGC ATA GCT GAT GCG TGG AAC TGC -3' ) were used in a PCR screening experiment. The amplification of an 1170bp fragment confirms the presence of the *porA* gene in the recombinant bacteria.

Recombinant bacteria (corresponding to about 5.10⁸ bacteria) can be re-suspended in 50 µl of PAGE-SDS buffer, frozen(-20°C) / boiled (100°C) three times and then separated by PAGE-SDS electrophoresis on a 12.5 % gel. Gels can then be stained by Coomassie Brilliant blue R250 or transferred to a nitrocellulose membrane and probed either with an anti-porA monoclonal antibody or with an anti-D15/Omp85 rabbit polyclonal antibody. Analysis of outer-membrane blebs prepared from the same strains can also be performed.

### Example 11: Up-regulation of the Hsf protein antigen in a recombinant Neisseiria meningitidis serogroup B strain lacking functional cps genes but expressing PorA.

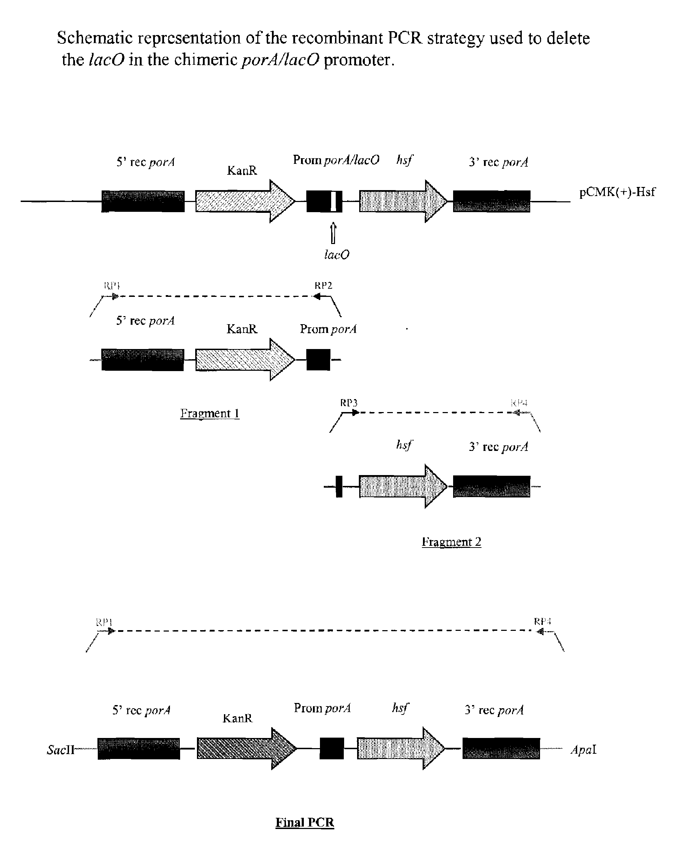

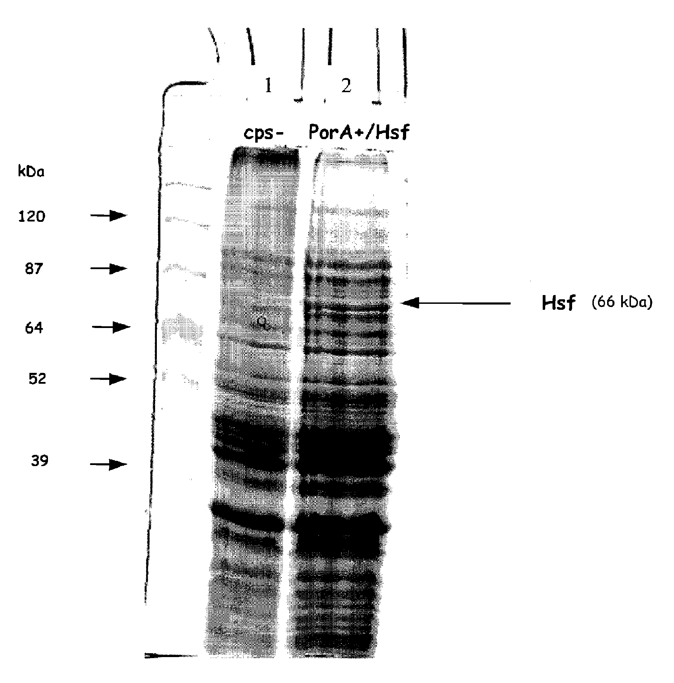

As described above, in certain countries, the presence of PorA in outer membrane vesicles may be advantageous, and can strengthen the vaccine efficacy of recombinant improved blebs. In the following example, we have used a modified pCMK(+) vector to up-regulate the expression of the Hsf protein antigen in a strain lacking functional *cps* genes but expressing PorA. The original pCMK(+) vector contains a chimeric *porA*/*lacO* promoter repressed in *E. coli* host expressing *lacI^{q}* but transcriptionally active in *Neisseria meningitidis.* In the modified pCMK(+), the native *porA* promoter was used to drive the transcription of the *hsf* gene. The gene coding for Hsf was PCR amplified using the HSF 01-*Nde*I and HSF 02*-Nhe*I oligonucleotide primers, presented in the table below. Because of the sequence of the HSF 01*-Nde*I primer the Hsf protein expressed will contain two methionine residues at the 5' end. The conditions used for PCR amplification were those described by the supplier (HiFi DNA polymerase, Boehringer Mannheim, GmbH). Thermal cycling was the following: 25 times (94°C 1min., 48°C 1min., 72°C 3min.) and 1 time (72°C 10min., 4°C up to recovery). The corresponding amplicon was subsequently cloned in the corresponding restriction sites of pCMK(+) delivery vector. In this recombinant plasmid, designed pCMK(+)-Hsf, we deleted the *lacO* present in the chimeric *porA*/*lacO* promoter by a recombinant PCR strategy (See Figure 12). The pCMK(+)-Hsf plasmid was used as a template to PCR amplify 2 separate DNA fragments:
- fragment 1 contains the *porA* 5' recombinogenic region, the Kanamycin resistance gene and the *porA* promoter. Oligonucleotide primers used, RP1(*Sac*II) and RP2, are presented in the table below. RP1 primer is homologous to the sequence just upstream of the *lac* operator.
- fragment 2 contains the Shine-Dalgarno sequence from the *porA* gene, the *hsf* gene and the *porA* 3' recombinogenic region. Oligonucleotide primers used, RP3 and RP4(*Apa*I*),* are presented in the table below. RP3 primer is homologous to the sequence just downstream of the *lac* operator. The 3' end of fragment 1 and the 5'end of fragment 2 have 48 bases overlapping. 500ng of each PCR (1 and 2) were used for a final PCR reaction using primers RP1 and RP4. The final amplicon obtained was subcloned in pSL1180 vector restricted with *Sac*II and *Apa*I*.* The modified plasmid
pCMK(+)-Hsf was purified at a large scale using the QIAGEN maxiprep kit and 2 µg of this material was used to transform a *Neisseiria meningitidis* serogroup B strain lacking functional *cps* genes (the strain described in example 1). In order to preserve the expression of *porA,* integration resulting from a single crossing-over was selected by a combination of PCR and Western blot screening procedures. Kanamycin resistant clones testing positive by *porA*-specific PCR and western blot were stored at -70°C as glycerol stocks and used for further studies. Bacteria (corresponding to about 5.10⁸ bacteria) were re-suspended in 50 µl of PAGE-SDS buffer, frozen (-20°C) / boiled (100°C) three times and then were separated by PAGE-SDS electrophoresis on a 12.5 % gel. The expression of Hsf was examined in Whole-cell bacterial lysates (WCBL) derived from *NmB* [Cps-, PorA+] or *NmB* [Cps-, PorA+, Hsf+]. Coomassie staining detected a significant increase in the expression of Hsf (with respect to the endogenous Hsf level) (See in Figure 13). This result confirms that the modified pCMK(+)-Hsf vector is functional and can be used successfully to up-regulate the expression of outer membrane proteins, without abolishing the production of the major PorA outer membrane protein antigen.

**Oligonucleotides used in this work**

| **Oligonucleotides** | **Sequence** | **Remark(s)** |
|---|---|---|
| Hsf 01-Nde | 5'- GGA ATT CCA TAT GAT GAA CAA AAT ATA CCG C-3' | *Nde*I **cloning site** |
| Hsf 02-Nhe | 5'-GTA GCT AGC TAG CTT ACC ACT GAT AAC CGA C -3' | *Nhe* **I cloning site** |
| GFP-mut-Asn | 5'-AAC TGC AGA ATT AAT ATG AAA GGA GAA GAA CTT TTC-3' | *Asn*I **cloning site** Compatible with *Nde*I |
| GFP-Spe | 5'-GAC ATA CTA GTT TAT TTG TAG AGC TCA TCC ATG -3' | *Spe*I **cloning site** Compatible with *Nhe*I |
| RP 1 (SacII) | 5'- TCC CCG CGG GCC GTC TGA ATA CAT CCC GTC-3' | *Sac*II **cloning site** |
| RP2 | 5'-CAT ATG GGC TTC CTT TTG TAA ATT TGA GGG CAA ACA CCC GAT ACG TCT TCA-3' | |
| RP3 | 5'-AGA CGT ATC GGG TGT TTG CCC TCA AAT TTA CAA AAG GAA GCC CAT ATG -3' | |
| RP4(ApaI) | 5'-GGG TAT TCC GGG CCC TTC AGA CGG CGC AGC AGG -3' | *Apa*I **cloning site** |

### Example 12: Expression of the Green Fluorescent Protein in a recombinant Neisseria meningitidis serogroup B strain lacking functional cps genes but expressing PorA.

In the following example, the pCMK vector was used to test the expression of a cytoplasmic heterologous protein in *Neisseria meningitidis.* The Green Fluorescent Protein was amplified from the pKen-Gfpmut2 plasmid with the primers GFP-Asn-mut2 and GFP-Spe (see table in Example 11). *Asn*I gives cohesive ends compatible with *Nde*I, *Spe*I gives cohesive ends compatible with *Nhe*I*.* The conditions used for PCR amplification were those described by the supplier (HiFi DNA polymerase, Boehringer Mannheim, GmbH). Thermal cycling was the following: 25 times (94°C 1min., 48°C 1min., 72°C 3min.) and 1 time (72°C 10min., 4°C up to recovery).The corresponding amplicon was subsequently cloned in the pCMK(+) delivery vector digested with *Nde*I and *Nhe*I restriction enzymes. In this recombinant plasmid, designed pCMK(+)-GrFP, we deleted the *lacO* present in the chimeric *porA*/*lacO* promoter by a recombinant PCR strategy. The pCMK(+)-GFP plasmid was used as template to PCR amplify 2 separate DNA fragments:
- fragment 1 contained the *porA* 5' recombinogenic region, the Kanamycin resistance gene and the *porA* promoter. Oligonucleotide primers used, RP1(*Sac*II) and RP2 (see table in example 11). RP1 primer is homologous to the sequence just upstream of the *lac* operator.
- fragment 2 contains the PorA Shine-Dalgarno sequence, the *gfp* gene and the *porA* 3' recombinogenic region. Oligonucleotide primers used, RP3 and RP4(*Apa*I)*,* are presented in the table in example 11. RP3 primer is homologous to the sequence just downstream of the *lac* operator.

The 3'end of fragment 1 and the 5'end of fragment 2 have 48 bases overlapping. 500ng of each PCR (1 and 2) were used for a final PCR reaction using primers RP1 and RP4. Twenty µg of this PCR fragment were used to transform a *Neisseiria meningitidis* serogroup B strain lacking functional *cps* genes.

Transformation with linear DNA is less efficient than with circular plasmid DNA but all the recombinants obtained performed a double crossing-over (confirmed by a combination of PCR and Western blot screening procedures). Kanamycin resistant clones were stored at -70°C as glycerol stocks and used for further studies. Bacteria (corresponding to about 5.10⁸ bacteria) were re-suspended in 50 µl of PAGE-SDS buffer, frozen (-20°C) / boiled (100°C) three times and then were separated by PAGE-SDS electrophoresis on a 12.5 % gel.

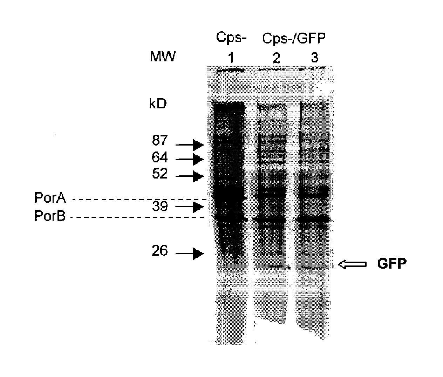

The expression of GFP was examined in Whole-cell bacterial lysates (WCBL) derived from *NmB* [Cps-, PorA+] or *NmB* [Cps-, PorA-, GFP+]. Coomassie staining detected an expression of GFP absent in the recipient *Neisseria meningitidis* strain (see figure 14).

### Example 13: Up-regulation of the N. meningitidis serogroup B NspA gene by promoter replacement

The aim of the experiment was to replace the endogenous promoter region of the *NspA* gene by the strong *porA* promoter, in order to up-regulate the production of the NspA antigen. For that purpose, a promoter replacement plasmid was constructed using *E. coli* cloning methodologies. A DNA region (924bp) located upstream from the *NspA* coding gene was discovered (SEQ ID NO: 7) in the private Incyte PathoSeq data base containing unfinished genomic DNA sequences of the *Neisseria meningitidis* strain ATCC 13090. A DNA fragment (675bp) covering nucleotides -115 to -790 with respect to the *NspA* gene start codon (ATG) was PCR amplified using oligonucleotides **PNS1'** (5'-CCG CGA ATT CGA CGA AGC CGC CCT CGA C-3') and **PNS2** (5'-CGT CTA GAC GTA GCG GTA TCC GGC TGC -3') containing *Eco*RI and *Xba*I restriction sites (underlined) respectively. The PCR fragment was submitted to restriction with *EcoR*I and *Xba*I and inserted in pUC18. This plasmid was submitted to circle PCR mutagenesis (Jones & Winistofer (1992), Biotechniques12: 528-534) in order to insert meningococcal uptake sequences required for transformation, and suitable restriction sites allowing cloning of a *CmR*/PorA promoter cassette. The circle PCR was performed using the
**BAD01-2** (5'- GGC GCC CGG GCT CGA GCT TAT CGA TGG AAA ACG CAG C-3') & **BAD02-2** (5'-GGC GCC CGG GCT CGA GTT CAG ACG GCG CGC TTA TAT AGT GGA TTA AC -3') oligonucleotides containing uptake sequences and suitable restriction sites (*Xma*I and *Xho*I) underlined. The resulting PCR fragment was gel-purified and digested with *Xho*I*.* The *Cm*R/PorA promoter cassette was amplified from the pUC D15/Omp85 plasmid previously described, using primers
**BAD 15-2** (5'-GGC GCC CGG GCT CGA GTC TAG ACA TCG GGC AAA CAC CCG-3') & **BAD 03-2** (5'- GGC GCC CGG GCT CGA GCA CTA GTA TTA CCC TGT TAT CCC-3') oligonucleotides containing suitable restriction sites (*Xma*I*, Xba*I*, Spe*I and *Xho*I*)* underlined. The PCR fragment obtained was submitted to digestion and inserted in the circle PCR plasmid restricted with the corresponding enzymes. 10 µg of the recombinant plasmid were linearized and used to transform a strain of *Neisseria meningitidis* serogroup B lacking capsular polysaccharide *(cps-)* and one of the major outer membrane proteins - PorA (*porA-*). Recombinant *Neisseria meningitidis* clones resulting from a double crossing over event (PCR screening using oligonucleotides **BAD 25** (5'-GAG CGA AGC CGT CGA ACG C -3') & **BAD08** (5'-CTT AAG CGT CGG ACA TTT CC-3')) were selected on GC agar plates containing 5µg/ml chloramphenicol and analyzed for NspA expression. Recombinant bacteria (corresponding to about 5.10⁸ bacteria) were re-suspended in 50 µl of PAGE-SDS buffer, frozen (-20°C) /boiled (100°C) three times and then were separated by PAGE-SDS electrophoresis on a 12.5 % gel. Gels were then stained by Coomassie Brilliant blue R250 or transferred to a nitrocellulose membrane and probed either with an anti-PorA monoclonal antibody or with anti-NspA polyclonal antibody (figure 17). As for Omp85, there is a surprising indication that insertion of the promoter approximately 400 bp upstream of the NspA initiation codon expresses more protein than if placed approximately 100 bp upstream.

The same recombinant pUC plasmid can be used to up-regulate the expression of NspA in a *Neisseria meningitidis* serogroup B strain lacking functional *cps* gene but still expressing PorA.

### Example 14: Up-regulation of the N. meningitidis serogroup B pldA (omplA) gene by promoter replacement.

The aim of the experiment was to replace the endogenous promoter region of the *pldA* (*omplA*) gene by the strong *porA* promoter in order to up-regulate the production of the P1dA (Omp1A1) antigen. For that purpose, a promoter replacement plasmid was constructed using *E. coli* cloning methodologies. A DNA region (373bp) located upstream from the *pldA* coding sequence was discovered (SEQ ID NO: 18) in the private Incyte PathoSeq data base of the *Neisseria meningitidis* strain ATCC 13090. This DNA contains the sequence coding for a putative *rpsT* gene. The stop codon of *rpsT* is located 169bp upstream the *pldA* ATG. To avoid the disruption of this potentially important gene, we decided to insert the *CmR*/PorA promoter cassette just upstream of the ATG of *pldA.* For that purpose, a DNA fragment of 992 bp corresponding to the the *rpsT* gene, the 169 bp intergenic sequence and the 499 first nucleotides of *pldA* gene was PCR amplified from *Neisseria meningitidis* serogroup B genomic DNA using oligonucleotides **PLA1 Amo5** (5'-GCC GTC TGA ATT TAA AAT TGC GCG TTT ACA G-3') and **PLA1 Amo3** (5'-GTA GTC TAG ATT CAG ACG GCG CAA TTT GGT TTC CGC AC -3') containing uptake sequences (underlined). PLA1 Amo3 contains also a *Xba*I restriction site. This PCR fragment was cleaned with a High Pure Kit (Roche, Mannheim, Germany) and directly cloned in a pGemT vector (Promega, USA). This plasmid was submitted to circle PCR mutagenesis (Jones & Winistofer (1992)) in order to insert suitable restriction sites allowing cloning of a *CmR*/PorA promoter cassette. The circle PCR was performed using the **CIRC1-Bg1** (5'CCT AGA TCT CTC CGC CCC CCA TTG TCG -3') & either **CIRC1-XH-RBS/2** (5'-CCG CTC GAG TAC AAA AGG AAG CCG ATA TGA ATA TAC GGA ATA TGC G-3') or **C1RC2-XHO/2** (5'-CCG CTC GAG ATG AAT ATA CGG AAT -3') oligonucleotides containing suitable restriction sites (*Bgl*II and *Xho*I*)* underlined. The *CmR*/PorA promoter cassette was amplified from the pUC D15/Omp85 plasmid previously described, using primers **BAD20** (5'- TCC CCC GGG AGA TCT CAC TAG TAT TAC CCT GTT ATC CC-3') and **CM-PORA-3** (5'- CCG CTC GAG ATA AAA ACC TAA AAA CAT CGG GC-3') containing suitable restriction sites (*Bgl*II and *Xho*I) underlined. This PCR fragment was cloned in the circle PCR plasmid obtained with primers CIRC1-Bgl and CIRC1-XH-RBS/2. This plasmid can be used to transform *Neisseria meningitidis* serogroup B *(cps-)* and (cps- porA-) strains. Integration by double crossing-over in the upstream region of *pldA* will direct the insertion of the *porA* promoter directly upstream of the *pldA* ATG. Another cassette was amplified from the genomic DNA of the recombinant *Neisseria meningitidis* serogroup B (*cps-, porA-,* D15/Omp85+) over-expressing D15/Omp85 by promoter replacement. This cassette contains the *cmR* gene, the *porA* promoter and 400bp corresponding to the 5' flanking sequence of the *D15*/*Omp85* gene. This sequence has been proven to be efficacious for up-regulation of the expression of D15/Omp85 in *Neisseria* and will be tested for the up-regulation of the expression of other *Neisseria* antigens. Primers used for the amplification were **BAD 20** and **CM-PORA-D15/3** (5'- CGG CTC GAG TGT CAG TTC CTT GTG GTG C-3') containing *Xho*I restriction sites (underlined). This PCR fragment was cloned in the circle PCR plasmid obtained with primers CIRC1-Bgl and CIRC2-XHO/2. This plasmid will be used to transform *Neisseria meningitidis* serogroup B *(cps-)* and (*cps-, porA-*) strains. Integration by double crossing-over in the upstream region of *pldA* will direct the insertion of the *porA* promoter 400bp upstream the *pldA* ATG.

### Example 15: Up-regulation of the N. meningitidis serogroup B tbpA gene by promoter replacement.

The aim of the experiment was to replace the endogenous promoter region of the *tbpA* gene by the strong *porA* promoter, in order to up-regulate the production of the TbpA antigen. For that purpose, a promoter replacement plasmid was constructed using *E. coli* cloning methodologies. A DNA region (731bp) located upstream from the *tbpA* coding sequence was discovered (SEQ ID NO: 17) in the private Incyte PathoSeq data base of the *Neisseria meningitidis* strain ATCC 13090. This DNA contains the sequence coding for TbpB antigen. The genes are organized in an operon. The *tbpB* gene will be deleted and replaced by the *CmR*/porA promoter cassette. For that purpose, a DNA fragment of 3218bp corresponding to the 509bp 5' flanking region of *tbpB* gene, the 2139bp *tbpB* coding sequence, the 87bp intergenic sequence and the 483 first nucleotides of *tbpA* coding sequence was PCR amplified from *Neisseria meningitidis* serogroup B genomic DNA using oligonucleotides **BAD16** (5'-GGC CTA GCT AGC CGT CTG AAG CGA TTA GAG TTT CAA AAT TTA TTC-3') and **BAD17** (5'-GGC CAA GCT TCA GAC GGC GTT CGA CCG AGT TTG AGC CTT TGC-3') containing uptake sequences and *Nhe*I and *Hind*III restriction sites (underlined). This PCR fragment was cleaned with a High Pure Kit ( Boerhinger Mannheim, Germany) and directly cloned in a pGemT vector (Promega, USA). This plasmid was submitted to circle PCR mutagenesis (Jones & Winistofer (1992)) in order to (i) insert suitable restriction sites allowing cloning of a *CmR*/PorA promoter cassette and (ii) to delete 209bp of the 5' flanking sequence of *tbpB* and the *tbpB* coding sequence. The circle PCR was performed using the **BAD 18** (5'-TCC CCC GGG AAG ATC TGG ACG AAA AAT CTC AAG AAA CCG-3') & the **BAD 19** (5'-GGA AGA TCT CCG CTC GAG CAA ATT TAC AAA AGG AAG CCG ATA TGC AAC AGC AAC ATT TGT TCC G -3') oligonucleotides containing suitable restriction sites *Xma*I*, Bgl*II and *Xho*I (underlined). The *Cm*R/PorA promoter cassette was amplified from the pUC D15/Omp85 plasmid previously described, using primers **BAD21** (5'- GGA AGA TCT CCG CTC GAG ACA TCG GGC AAA CAC CCG-3') & **BAD20** (5'- TCC CCC GGG AGA TCT CAC TAG TAT TAC CCT GTT ATC CC-3') containing suitable restriction sites *Xma*I*, Spe*I*, Bgl*II and *Xho*I (underlined). This PCR fragment was cloned in the circle PCR plasmid. This plasmid will be used to transform *Neisseria meningitidis* serogroup B *(cps-)* and (*cps- porA-*) strains. Integration by double crossing-over in the upstream region of *tbpA* will direct the insertion of the *porA* promoter directly upstream of the *tbpA* ATG.

### Example 16: Up-regulation of the N. meningitidis serogroup B pilQ gene by promoter replacement.

The aim of the experiment was to replace the endogenous promoter region of the *pilQ* gene by the strong *porA* promoter, in order to up-regulate the production of the PilQ antigen. For that purpose, a promoter replacement plasmid was constructed using *E. coli* cloning methodologies. A DNA region (772bp) located upstream from the *pilQ* coding gene was discovered (SEQ ID NO: 12) in the private Incyte PathoSeq data base of the *Neisseria meningitidis* strain ATCC 13090. This DNA contains the sequence coding for PilP antigen. The *pilQ* gene is part of an operon we do not want to disturb, pilins being essential elements of the bacteria. The *CmR*/porA promoter cassette was introduced upstream the *pilQ* gene following the same strategy described for the up-regulation of the expression of the *pldA* gene. For that purpose, a DNA fragment of 866 bp corresponding to the 3' part of the *pilP coding sequence,* the 18bp intergenic sequence and the 392 first nucleotides of *pilQ* gene was PCR amplified from *Neisseria* serogroup B genomic DNA using **PQ-rec5-Nhe** (5'-CTA GCT AGC GCC GTC TGA ACG ACG CGA AGC CAA AGC-3') and **PQ-rec3-Hin** (GCC AAG CTT TTC AGA CGG CAC GGT ATC GTC CGA TTC G-3') oligonucleotides containing uptake sequences and *Nhe*I and *HindIII* restriction sites (underlined). This PCR fragment was directly cloned in a pGemT vector (Promega, USA). This plasmid was submitted to circle PCR mutagenesis (Jones & Winistofer (1992)) in order to insert suitable restriction sites allowing cloning of a *CmR*/PorA promoter cassette. The circle PCR was performed using the **CIRC1-PQ-Bgl** (5'-GGA AGA TCT AAT GGA GTA ATC CTC TTC TTA-3') & either **CIRC1-PQ-XHO** (5'-CCG CTC GAG TAC AAA AGG AAG CCG ATA TGA TTA CCA AAC TGA CAA AAA TC-3') or **CIRC2-PQ-X** (5'-CCG CTC GAG ATG AAT ACC AAA CTG ACA AAA ATC -3') oligonucleotides containing suitable restriction sites *Bgl*II and *Xho*I (underlined). The *Cm*R/PorA promoter cassette was amplified from the pUC D15/Omp85 plasmid previously described, using primers **BAD20** (5'- TCC CCC GGG AGA TCT CAC TAG TAT TAC CCT GTT ATC CC-3') and **CM-PORA-3** (5'- CCG CTC GAG ATA AAA ACC TAA AAA CAT CGG GCA AAC ACC C-3') containing suitable restriction sites *Bgl*II and *Xho*I (underlined). This PCR fragment was cloned in the circle PCR plasmid obtained with primers CIRC1-PQ-Bgl and CIRC1-PQ-XHO. This plasmid can be used to transform *Neisseria meningitidis* serogroup B *(cps-)* and (cps-, porA-) strains. Integration by double crossing-over in the upstream region of *pilQ* will direct the insertion of the *porA* promoter directly upstream of the *pilQ* ATG.

Another cassette was amplified from the genomic DNA of the recombinant *Neisseria meningitidis* serogroup B (*cps-, porA-, D15*/*Omp85*+) over-expressing D15/Omp85 by promoter replacement. This cassette contains the *cmR* gene, the *porA* promoter and 400bp corresponding to the 5' flanking sequence of the *D15*/*Omp85* gene. This sequence has been proven to be efficacious for up-regulation of the expression of D15/Omp85 in *Neisseria meningitidis* and will be tested for the up-regulation of the expression of other *Neisseria* antigens. Primers used for the amplification were **BAD 20** and **CM-PORA-D153** (5'- GGG CTC GAG TGT CAG TTC CTT GTG GTG C-3') containing *Xho*I restriction sites (underlined). This PCR fragment was cloned in the circle PCR plasmid obtained with primers CIRC1-PQ-Bg1 and CIRC2-PQ-X. This plasmid can be used to transform *Neisseria meningitidis* serogroup B (*cps-*) and (*cps-, porA-*) strains. Integration by double crossing-over in the upstream region of *pilQ* will direct the insertion of the *porA* promoter 400bp upstream the *pilQ* ATG.

### Example 17: Construction of a kanR/sacB cassette for introducing "clean", unmarked mutations in the N. meningitidis chromosome.

The aim of the experiment is to construct a versatile DNA cassette containing a selectable marker for the positive screening of recombination in the chromosome of *Neisseria meningitidis (ie: kanR* gene), and a counter selectable marker to delete the cassette from the chromosome after recombination (ie: *sacB* gene). By this method, any heterologous DNA introduced during homologous recombination will be removed from the *Neisseria* chromosome.

A DNA fragment containing the *neoR* gene and the *sacB* gene expressed under the control of its own promoter was obtained by restriction of the pIB 279 plasmid (Blomfield IC, Vaughn V, Rest RF, Eisenstein BI (1991), Mol Microbiol 5:1447-57) with *BamH*I restriction enzyme. The recipient vector was derived from plasmid pCMK, previously described. The *kanR* gene of the pCMK was deleted by restriction with enzymes *Nru*I and *EcoRV.* This plasmid was named pCMKs. The *neoR*/*sacB* cassette was inserted in the pCMKs at a *Bgl*II restriction site compatible with *BamH*I ends.

*E. coli* harboring the plasmid is unable to grow in the presence of 2% sucrose in the culture medium, confirming the functionality of the *sacB* promoter. This plasmid contains recombinogenic sequences allowing the insertion of the cassette at the *porA* locus in the chromosome of *Neisseria meningitidis* serogroup B. Recombinant *Neisseria* were obtained on GC agar plates containing 200µg/ml of kanamycin. Unfortunately, the *sacB* promoter was not functional in *Neisseria meningitidis:* no growth difference was observed on GC agar plates containing 2% sucrose.

A new cassette was constructed containing the *sacB* gene under the control of the *kanR* promoter. A circle PCR was performed using the plasmid pUC4K ((Amersham Pharmacia Biotech, USA)) as a template with **CIRC-Kan-Nco** (5'-CAT GCC ATG GTT AGA AAA ACT CAT CGA GCA TC-3') & **CIRC-Kan-Xba** (5'-CTA GTC TAG ATC AGA ATT GGT TAA TTG GTT G-3') oligonucleotides containing *Nco*I and *XBa*I restriction sites (underlined). The resulting PCR fragment was gel-purified, digested with *Nco*I and ligated to the *sacB* gene generated by PCR from the pIB279 plasmid with **SAC/NCO/NEW5**
(5'-CAT GCC ATG GG**A GGA** TGA ACG ATG AAC ATC AAA AAG TTT GCA A-3') oligonucleotide containing a *Nco*I restriction site (underlined) and a RBS (bold) & **SAC/NCO/NEW3** (5'-GAT CCC ATG GTT ATT TGT TAA CTG TTA ATT GTC-3') oligonucleotide containing a *Nco*I restriction site (underlined). The recombinant *E. coli* clones can be tested for their sensitivity on agar plates containing 2% sucrose. The new *kanR*/*sacB* cassette can be subcloned in the pCMKs and used to transform a *Neisseria meningitidis* serogroup B *cps-* strain. The acquired sucrose sensitivity will be confirmed in *Neisseria.* The pCMKs plasmid will be used to transform the recombinant *kanR*/*SacB Neisseria* to delete the entire cassette inserted in the chromosome at the *porA* locus. Clean recombinant *Neisseria* will be obtained on GC agar plates containing 2% sucrose.

### Example 18: Use of small recombinogenic sequences (43bp) to allow homologous recombination in the chromosome of Neisseria meningitidis.

The aim of the experiment is to use small recombinogenic sequences (43bp) to drive insertions, modifications or deletions in the chromosome of *Neisseria.* The achievement of this experiment will greatly facilitate future work, in terms of avoiding subcloning steps of homologous sequences in *E. coli* (recombinogenic sequences of 43bp can easily be added in the PCR amplification primer). The *kanR* gene was PCR amplified from plasmid pUC4K with oligonucleotides **Kan-PorA-5** (5'-GCC GTC TGA A**CC CGT CAT TCC CGC GCA GGC GGG AAT CCA GTC CGT TCA GTT T**CG GGA AAG CCA CGT TGT GTC-3') containing 43bp homologous to the 5' flanking sequence of *Nm*B *porA* gene (bold) and an uptake sequence (underlined) & **Kan-PorA-3** (5'-TTC AGA CGG **CGC AGC AGG AAT TTA TCG GAA ATA ACT GAA ACC GAA CAG ACT AG**G CTG AGG TCT GCC TCG-3') containing 43bp homologous to the 3' flanking sequence of *NmB porA* gene (bold) and an uptake sequence (underlined). The 1300bp DNA fragment obtained was cloned in pGemT vector (Promega, USA). This plasmid can be used to transform a *Neisseria meningitidis* serogroupB *cps-* strain. Recombinant *Neisseria* will be obtained on GC plates containing 200µg/ml kanamycin. Integrations resulting from a double crossing-over at the *porA* locus will be screened by PCR with primers PPA1 & PPA2 as described previously.

### Example 19: Active protection of mice immunized with WT and recombinant Neisseria meningitidis blebs

Animals were immunised three times (IP route) with 5 µg of the different OMVs adsorbed on Al(OH)3 on days 0, 14 and 28. Bleedings were done on days 28 (day 14 Post II) and 35 (day 7 post III), and they were challenged on day 35 (IP route). The challenge dose was 20 x LD50 (~10⁷ CFU/mouse). Mortality rate was monitored for 7 days after challenge.

OMVs injected were:
Group 1: Cps-, PorA+ blebs
Group2: Cps-, PorA- blebs
Group3: Cps-, PorA-, NspA+ blebs
Group4: Cps-, PorA-, Omp85+ blebs
Group5: Cps-, PorA-, Hsf+ blebs

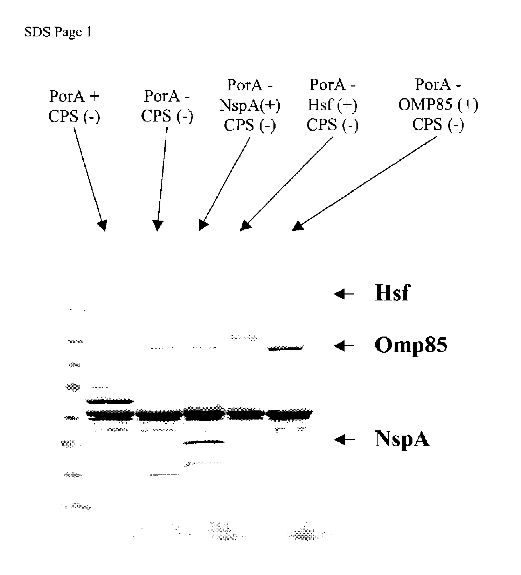

Figure 15 illustrates the pattern of these OMVs by analyzed SDS Page (Coomassie staining).

24 hours after the challenge, there was 100% mortality (8/8) in the negative control group (immunised with Al(OH)₃ alone) while mice immunised with the 5 different OMVs preparations were still alive (7 to 8/8 mice survived). Sickness was also monitored during the 7 days and the mice immunised with the NSPA over-expressed blebs appeared to be less sick than the other groups. PorA present in PorA+ blebs is likely to confer extensive protection against infection by the homologous strain. However, protection induced by PorA- up-regulated blebs is likely to be due at least to some extent, to the presence of increased amount of NspA, Omp85 or Hsf.

### Example 20: Immunogenicity of recombinant blebs measured by whole cell & specific ELISA methods

To measure the ability of the antibodies to recognize the antigens present on the MenB cell surface, the pooled mice sera (from Example 19) were tested by whole cell ELISA (using tetracyclin inactivated cells), and titers were expressed as mid-point titers. All types of bleb antibodies induce a high whole cell Ab titer while the negative control group was clearly negative.

| **Bleb** | **WCE**(H44/76) mid-point titer | |
|---|---|---|
| | **14P2** | **14P3** |
| **CPS(-)** | | |
| **PorA(+)** | **23849** | **65539** |
| **CPS(-)** | | |
| **ForA(-)** | **20130** | **40150** |
| **CPS(-)** | | |
| **PorA(-)** | | |
| **NSPA(+)** | **8435** | **23846** |
| **CPS(-)** | | |
| **PorA(-)** | | |
| **OMP85(+)** | **4747** | **16116** |
| **CPS(-)** | | |
| **PorA(-)** | | |
| **HSF(+)** | **6964** | **22504** |
| **(-)** | **51** | **82** |

The specific Ab response to available recombinant HSF protein was carried out. Microplates were coated with 1 µg/ml full length HSF molecule.

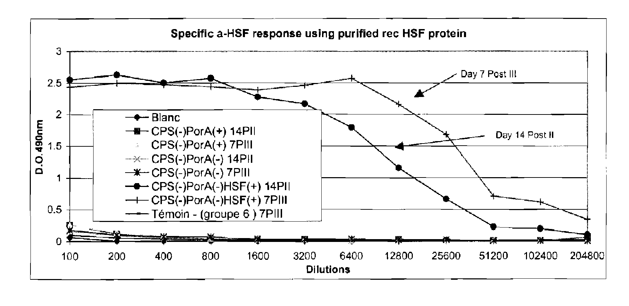

The results illustrated in Figure 16 show that there was a good specific HSF response when HSF over-expressed OMVs were used to immunize mice (using purified recombinant HSF on the plates). The HSF over-expressed blebs induce a good level of specific antibodies.

SEQ. ID NO:1
Nucleotide sequence of the pCMK(+) vector
TCTTCCGCTTCCTCGCTCACTGACTCGCTGCGCTCGGTCGTTCGGCTGCGGCGAGCGGTATCAGCTCACTCAAAGGCGGT
AATACGGTTATCCACAGAATCAGGGGATAACGCAGGAAAGAACATGTGAGCAAAAGGCCAGCAAAAGGCCAGGAACCGTA
AAAAGGCCGCGTTGCTGGCGTTTTTCCATAGGCTCCGCCCCCCTGACGAGCATCACAAAAATCGACGCTCAAGTCAGAGG
TGGCGAAACCCGACAGGACTATAAAGATACCAGGCGTTTCCCCCTGGAAGCTCCCTCGTGCGCTCTCCTGTTCCGACCCT
GCCGCTTACCGGATACCTGTCCGCCTTTCTCCCTTCGGGAAGCGTGGCGCTTTCTCATAGCTCACGCTGTAGGTATCTCA
GTTCGGTGTAGGTCGTTCGCTCCAAGCTGGGCTGTGTGCACGAACCCCCCGTTCAGCCCGACCGCTGCGCCTTATCCGGT
AACTATCGTCTTGAGTCCAACCCGGTAAGACACGACTTATCGCCACTGGCAGCAGCCACTGGTAACAGGATTAGCAGAGC
GAGGTATGTAGGCGGTGCTACAGAGTTCTTGAAGTGGTGGCCTAACTACGGCTACACTAGAAGAACAGTATTTGGTATCT
GCGCTCTGCTGAAGCCAGTTACCTTCGGAAAAAGAGTTGGTAGCTCTTGATCCGGCAAACAAACCACCGCTGGTAGCGGT
GGTTTTTTTGTTTGCAAGCAGCAGATTACGCGCAGAAAAAAAGGATCTCAAGAAGATCCTTTGATCTTTTCTACGGGGTC
TGACGCTCAGTGGAACGAAAACTCACGTTAAGGGATTTTGGTCATGAGATTATCAAAAAGGATCTTCACCTAGATCCTTT
TAAATTAAAAATGAAGTTTTAAATCAATCTAAAGTATATATGAGTAAACTTGGTCTGACAGTTACCAATGCTTAATCAGT
GAGGCACCTATCTCAGCGATCTGTCTATTTCGTTCATCCATAGTTGCCTGACTCCCCGTCGTGTAGATAACTACGATACG
GGAGGGCTTACCATCTGGCCCCAGTGCTGCAATGATACCGCGAGACCCACGCTCACCGGCTCCAGATTTATCAGCAATAA
ACCAGCCAGCCGGAAGGGCCGAGCGCAGAAGTGGTCCTGCAACTTTATCCGCCTCCATCCAGTCTATTAATTGTTGCCGG
GAAGCTAGAGTAAGTAGTTCGCCAGTTAATAGTTTGCGCAACGTTGTTGCCATTGCTACAGGCATCGTGGTGTCACGCTC
GTCGTTTGGTATGGCTTCATTCAGCTCCGGTTCCCAACGATCAAGGCGAGTTACATGATCCCCCATGTTGTGCAAAAAAG
CGGTTAGCTCCTTCGGTCCTCCGATCGTTGTCAGAAGTAAGTTGGCCGCAGTGTTATCACTCATGGTTATGGCAGCACTG
CATAATTCTCTTACTGTCATGCCATCCGTAAGATGCTTTTCTGTGACTGGTGAGTACTCAACCAAGTCATTCTGAGAATA
GTGTATGCGGCGACCGAGTTGCTCTTGCCCGGCGTCAATACGGGATAATACCGCGCCACATAGCAGAACTTTAAAAGTGC
TCATCATTGGAAAACGTTCTTCGGGGCGAAAACTCTCAAGGATCTTACCGCTGTTGAGATCCAGTTCGATGTAACCCACT
CGTGCACCCAACTGATCTTCAGCATCTTTTACTTTCACCAGCGTTTCTGGGTGAGCAAAAACAGGAAGGCAAAATGCCGC
AAAAAAGGGAATAAGGGCGACACGGAAATGTTGAATACTCATACTCTTCCTTTTTCAATATTATTGAAGCATTTATCAGG
GTTATTGTCTCATGAGCGGATACATATTTGAATGTATTTAGAAAAATAAACAAATAGGGGTTCCGCGCACATTTCCCCGA
AAAGTGCCACCTGACGTCTAAGAAACCATTATTATCATGACATTAACCTATAAAAATAGGCGTATCACGAGGCCCTTTCG
TCTCGCGCGTTTCGGTGATGACGGTGAAAACCTCTGACACATGCAGCTCCCGGAGACGGTCACAGCTTGTCTGTAAGCGG
ATGCCGGGAGCAGACAAGCCCGTCAGGGCGCGTCAGCGGGTGTTGGCGGGTGTCGGGGCTGGCTTAACTATGCGGCATCA
GAGCAGATTGTACTGAGAGTGCACCATAAAATTGTAAACGTTAATATTTTGTTAAAATTCGCGTTAAATTTTTGTTAAAT
CAGCTCATTTTTTAACCAATAGGCCGAAATCGGCAAAATCCCTTATAAATCAAAAGAATAGCCCGAGATAGGGTTGAGTG
TTGTTCCAGTTTGGAACAAGAGTCCACTATTAAAGAACGTGGACTCCAACGTCAAAGGGCGAAAAACCGTCTATCAGGGC
GATGGCCCACTACGTGAACCATCACCCAAATCAAGTTTTTTGGGGTCGAGGTGCCGTAAAGCACTAAATCGGAACCCTAA
AGGGAGCCCCCGATTTAGAGCTTGACGGGGAAAGCCGGCGAACGTGGCGAGAAAGGAAGGGAAGAAAGCGAAAGGAGCGG
GCGCTAGGGCGCTGGCAAGTGTAGCGGTCACGCTGCGCGTAACCACCACACCCGCCGCGCTTAATGCGCCGCTACAGGGC
GCGTACTATGGTTGCTTTGACGTATGCGGTGTGAAATACCGCACAGATGCGTAAGGAGAAAATACCGCATCAGGCGCCAT
TCGCCATTCAGGCTGCGCAACTGTTGGGAAGGGCGATCGGTGCGGGCCTCTTCGCTATTACGCCAGCTGGCGAAAGGGGG
ATGTGCTGCAAGGCGATTAAGTTGGGTAACGCCAGGGTTTTCCCAGTCACGACGTTGTAAAACGACGGCCAGTGCCAAGC
TTGCCGTCTGAATACATCCCGTCATTCCTCAAAAACAGAAAACCAAAATCAGAAACCTAAAATCCCGTCATTCCCGCGCA
GGCGGGAATCCAGTCCGTTCAGTTTCGGTCATTTCCGATAAATTCCTGCTGCTTTTCATTTCTAGATTCCCACTTTCGTG
GGAATGACGGCGGAAGGGTTTTGCTTTTTTCCGATAAATTCTTGAGGCATTGAAATTCTAGATTCCCGCCTGCGCGGGAA
TGACGGCTGTAGATGCCCGATGGTCTTTATAGCGGATTAACAAAAATCAGGACAAGGCGACGAAGCCGCAGACAGTACAG
ATAGTACGGAACCGATTCACTTGGTGCTTCAGCACCTTAGAGAATCGTTCTCTTTGAGCTAAGGCGAGGCAACGCCGTAC
TTGTTTTTGTTAATCCACTATAAAGTGCCGCGTGTGTTTTTTTATGGCGTTTTAAAAAGCCGAGACTGCATCCGGGCAGC
AGCGCATCGGCCCGCACGAGGTCTCTGGAGTCGCGAGCATCAAGGGCGAATTCTGCACGGGGGGCGGGGAAAGCCACGTT
GTGTCTCAAAATCTCTGATGTTACATTGCACAAGATAAAAATATATCATCATGAACAATAAAACTGTCTGCTTACATAAA
CAGTAATACAAGGGGTGTTATGAGCCATATTCAACGGGAAACGTCTTGCTCGAGGCCGCGATTAAATTCCAACATGGATG
CTGATTTATATGGGTATAAATGGGCTCGCGATAATGTCGGGCAATCAGGTGCGACAATCTATCGATTGTATGGGAAGCCC
GATGCGCCAGAGTTGTTTCTGAAACATGGCAAAGGTAGCGTTGCCAATGATGTTACAGATGAGATGGTCAGACTAAACTG
GCTGACGGAATTTATGCCTCTTCCGACCATCAAGCATTTTATCCGTACTCCTGATGATGCATGGTTACTCACCACTGCGA
TCCCCGGGAAAACAGCATTCCAGGTATTAGAAGAATATCCTGATTCAGGTGAAAATATTGTTGATGCGCTGGCAGTGTTC
CTGCGCCGGTTGCATTCGATTCCTGTTTGTAATTGTCCTTTTAACAGCGATCGCGTATTTCGTCTCGCTCAGGCGCAATC
ACGAATGAATAACGGTTTGGTTGATGCGAGTGATTTTGATGACGAGCGTAATGGCTGGCCTGTTGAACAAGTCTGGAAAG

AAATGCATAAGCTTTTGCCATTCTCACCGGATTCAGTCGTCACTCATGGTGATTTCTCACTTGATAACCTTATTTTTGAC
GAGGGGAAATTAATAGGTTGTATTGATGTTGGACGAGTCGGAATCGCAGACCGATACCAGGATCTTGCCATCCTATGGAA
CTGCCTCGGTGAGTTTTCTCCTTCATTACAGAAACGGCTTTTTCAAAAATATGGTATTGATAATCCTGATATGAATAAAT
TGCAGTTTCATTTGATGCTCGATGAGTTTTTCTAATCAGAATTGGTTAATTGGTTGTAACACTGGCAGAGCATTACGCTG
ACTTGACGGGACGGCGGCTTTGTTGAATAAATCGAACTTTTGCTGAGTTGAAGGATCAGATCACGCATCTTCCCGACAAC
GCAGACCGTTCCGTGGCAAAGCAAAAGTTCAAAATCACCAACTGGTCCACCTACAACAAAGCTCTCATCAACCGTGGCTC
CCTCACTTTCTGGCTGGATGATGGGGCGATTCAGGCCTGGTATGAGTCAGCAACACCTTCTTCACGAGGCAGACCTCAGC
GCCCCCCCCCCCCTGCAGGAGGTCTGCGCTTGAATTGTGTTGTAGAAACACAACGTTTTTGAAAAAATAAGCTATTGTTT
TATATCAAAATATAATCATTTTTAAAATAAAGGTTGCGGCATTTATCAGATATTTGTTCTGAAAAATGGTTTTTTGCGGG
GGGGGGGTATAATTGAAGACGTATCGGGTGTTTGCCCGGAATTGTGAGCGGATAACAATTCGATGTTTTTAGGTTTTTA
TCAAATTTACAAAAGGAAGCCCATATGCATCCTAGGCCTATTAATATTCCGGAGTATACGTAGCCGGCTAACGTTAACAA
CCGGTACCTCTAGAACTATAGCTAGCATGCGCAAATTTAAAGCGCTGATATCGATCGCGCGCAGATCTGATTAAATAGGC
GAAAATACCAGCTACGATCAAATCATCGCCGGCGTTGATTATGATTTTTCCAAACGCACTTCCGCCATCGTGTCTGGCGC
TTGGCTGAAACGCAATACCGGCATCGGCAACTACACTCAAATTAATGCCGCCTCCGTCGGTTTGCGCCACAAATTCTAAA
TATCGGGGCGGTGAAGCGGATAGCTTTGTTTTTGACGGCTTCGCCTTCATTCTTTGATTGCAATCTGACTGCCAATCTGC
TTCAGCCCCAAACAAAAACCCGGATACGGAAGAAAAACGGCAATAAAGACAGCAAATACCGTCTGAAAGATTTTCAGACG
GTATTTCGCATTTTTGGCTTGGTTTGCACATATAGTGAGACCTTGGCAAAAATAGTCTGTTAACGAAATTTGACGCATAA
AAATGCGCCAAAAAATTTTCAATTGCCTAAAACCTTCCTAATATTGAGCAAAAAGTAGGAAAAATCAGAAAAGTTTTGCA
TTTTGAAAATGAGATTGAGCATAAAATTTTAGTAACCTATGTTATTGCAAAGGTCTCGAATTGTCATTCCCACGCAGGCG
GGAATCTAGTCTGTTCGGTTTCAGTTATTTCCGATAAATTCCTGCTGCGCCGTCTGAAGAATTCGTAATCATGGTCATAG
CTGTTTCCTGTGTGAAATTGTTATCCGCTCACAATTCCACACAACATACGAGCCGGAAGCATAAAGTGTAAAGCCTGGGG
TGCCTAATGAGTGAGCTAACTCACATTAATTGCGTTGCGCTCACTGCCCGCTTTCCAGTCGGGAAACCTGTCGTGCCAGC
TGCATTAATGAATCGGCCAACGCGCGGGGAGAGGCGGTTTGCGTATTGGGCGC

SEQ. ID NO:2
Nucleotide sequence of DNA region (997 bp) up-stream from the NspA gene in
the Neisseria meningitidis serogroup A strain Z2491.
GGAACCGAACACGCCGTTCGGTCATACGCCGCCGAAAGGTTTGCCGCAAGACGAAGCCGCCCTCGACATCGAAGACGCGG
TACACGGCGCGCTGGAAAGCGCGGGTTTTGTCCACTACGAAACATCGGCTTTTGCGAAACCAGCCATGCAGTGCCGCCAC
AATTTGAACTACTGGCAGTTCGGCGATTATTTAGGCATAGGCGCGGGCGCGCACGGCAAAATTTCCTATCCCGACCGCAT
CGAGCGCACCGTCCGCCGCCGCCACCCCAACGACTACCTCGCCTTAATGCAAAACCGACCGAGCGAAGCCGTCGAACGCA
AAACCGTCGCCGCCGAAGATTTGCCGTTCGAATTCATGATGAACGCCCTGCGCCTGACCGACGGCGTACCCACCGCGATG
TTGCAGGAGCGCACGGGCGTACCGAGTGCCAAAATCATGGCGCAAATCGAAACGGCAAGGCAAAAAGGCCTGCTGGAAAC
CGACCCCGCCGTATTCCGCCCGACCGAAAAAGGACGCTTGTTTTTAAACGATTTGCTGCAGTGTTTTTTATAGTGGATTA
ACAAAAACCAGTACGGCGTTGCCTCGCCTTAGCTCAAAGAGAACGATTCTCTAAGGTGCTGAAGCACCAAGTGAATCGGT
TCCGTACTATCTGTACTGTCTGCGGCTTCGTCGCCTTGTCCTGATTTTTGTTAATCCACTATATAAGCGCAAACAAATCG
GCGGCCGCCCGGGAAAACCCCCCCGAACGCGTCCGGAAAATATGCTTATCGATGGAAAACGCAGCCGCATCCCCCGCCGG
GCGTTTCAGACGGCACAGCCGCCGCCGGAAATGTCCGACGCTTAAGGCACAGACGCACACAAAAAACCGTATGCCTGCAC
CTGCAACAATCCGACAGATACCGCTGTTTTTTCCAAACCGTTTGCAAGTTTCACCCATCCGCCGCGTGATGCCGCCACCA
CCATTTAAAGGCAACGCGCGGGTTAACGGCTTTGCCG

SEQ. ID NO:3

Nucleotide sequence of DNA region (1000 bp ) up-stream from the D15/Omp85

gene in the Neisseria meningitidis serogroup B strain ATCC13090.

ACCATTGCCGCCCGCGCCGGCTTCCAAAGCGGCGACAAAATACAATCCGTCAACGGCACACCCGTTGCAGATTGGGGCAG

CGCGCAAACCGAAATCGTCCTCAACCTCGAAGCCGGCAAAGTCGCCGTCGGGTTCAGACGGCATCAGGCGCGCAAACCGT

CCGCACCATCGATGCCGCAGGCACGCCGGAAGCCGGTAAAATCGCAAAAAACCAAGGCTACATCGGACTGATGCCCTTTA

AAATCACAACCGTTGCCGGTGGCGTGGAAAAAGGCAGCCCCGCCGAAAAAGCAGGCCTGAAACCGGGCGACAGGCTGACT

GCCGCCGACGGCAAACCCATTACCTCATGGCAAGAATGGGCAAACCTGACCCGCCAAAGCCCCGGCAAAAAAATCACCCT

GAACTACGAACGCGCCGGACAAACCCATACCGCCGACATCCGCCCCGATACTGTCGAACAGCCCGACCACACCCTGATCG

GGCGCGTCGGCCTCCGTCCGCAGCCGGACAGGGCGTGGGACGCGCAAATCCGCCGCAGCTACCGTCCGTCTGTTATCCGC
GCATTCGGCATGGGCTGGGAAAAAACCGTTTCCCACTCGTGGACAACCCTCAAATTTTTCGGCAAACTAATCAGCGGCAA
CGCCTCCGTCAGCCATATTTCCGGGCCGCTGACCATTGCCGACATTGCCGGACAGTCCGCCGAACTCGGCTTGCAAAGTT
ATTTGGAATTTTTGGCACTGGTCAGCATCAGCCTCGGCGTGCTGAACCTGCTGCCCGTCCCCGTTTTGGACGGCGGCCAC
CTCGTGTTTTATACTGCCGAATGGATACGCGGCAAACCTTTGGGCGAACGCGTCCAAAACATCGGTTTGCGCTTCGGGCT
TGCCCTCATGATGCTGATGATGGCGGTCGCCTTCTTCAACGACGTTACCCGGCTGCTCGGTTAGATTTTACGTTTCGGAA
TGCCGTCTGAAACCGCATTCCGCACCACAAGGAACTGACA

SEQ. ID NO:4
Nucleotide sequence of DNA region (1000 bp ) up-stream from the Hsf-like gene
from Neisseria meningitidis
ATTCCCGCGCAGGCGGGAATCCAGAAACGCAACGCAACAGGAATTTATCGGAAAAAACAGAAACCTCACCGCCGTCATTC
CCGCAAAAGCGGGAATCTAGAAACACAACGCGGCAGGACTTTATCAGAAAAAACAGAAACCCCACCGCCGTCATTCCCGC
AAAAGCGGGAATCCAGACCCGTCGGCACGGAAACTTACCGGATAAAACAGTTTCCTTAGATTCCACGTCCTAGATTCCCG
CTTTCGCGGGAATGACGAGATTTTAGATTATGGGAATTTATCAGGAATGATTGAATCCATAGAAAAACCACAGGAATCTA
TCAGAAAAAACAGAAACCCCCACCGCGTCATTCCCGCGCAGGCGGGAATCCAGAAACACAACGCGGCAGGACTTTATCGG
AAAAAACCGAAACCCCACCGACCGTCATTCCCGCAAAAGTTGGAATCCAAAAACGCAACGCAACAGGAATTTATCGGAAA
AAACAGAAACCCCCACCGCGTCATTCCCGCGCAGGCGGGAATCCAGAAACACAACGCAACAGGAATTTATCGGAAAAAAC
AGAAACCCCACCGACCGTCATTCCCGCAAAAGCGGGAATCCAGCAACCGAAAAACCACAGGAATCTATCAGCAAAAACAG
AAACCCCCACCGACCGTCATTCCCGCGCAGGCGGGAATCCAGAAACACAACGCGGCAGGACTTTATCGGAAAAAACAGAA
ACCCCACCGACCGTCATTCCCGCAAAAGCTGGAATCCAAAAACGCAACGCAACAGGAATTTATCGGAAAAAACAGAAACC
CCACCGCCGTCATTCCCGCAAAAGCGGGAATCCAGACCCGTCGGCACGGAAACTTACCGGATAAAACAGTTTCCTTAGAT
TCCACGTCCCAGATTCCCGCCTTCGCGGGAATGACGAGATTTTAAGTTGGGGGAATTTATCAGAAAACCCCCAACCCCCA
AAAACCGGGCGGATGCCGCACCATCCGCCCCCAAACCCCGATTTAACCATTCAAACAAACCAAAAGAAAAAACAAA

SEQ. ID NO:5
Nucleotide sequence of DNA region (772 bp ) up-stream from the PilQ gene from
Neisseria meningitidis
GCGATGTCGGGAAGCCTTCTCCCGAATCATTACCCCTTGAGTCGCTGAAAATCGCCCAATCTCCGGAAAACGGCGGCAAT
CATGACGGCAAGAGCAGCATCCTGAACCTCAGTGCCATTGCCACCACCTACCAAGCAAAATCCGTAGAAGAGCTTGCCGC
AGAAGCGGCACAAAATGCCGAGCAAAAATAACTTACGTTAGGGAAACCATGAAACACTATGCCTTACTCATCAGCTTTCT
GGCTCTCTCCGCGTGTTCCCAAGGTTCTGAGGACCTAAACGAATGGATGGCACAAACGCGACGCGAAGCCAAAGCAGAAA
TCATACCTTTCCAAGCACCTACCCTGCCGGTTGCGCCGGTATACAGCCCGCCGCAGCTTACAGGGCCGAACGCATTCGAC
TTCCGCCGCATGGAAACCGACAAAAAAGGGGAAAATGCCCCCGACACCAAGCGTATTAAAGAAACGCTGGAAAAATTCAG
TTTGGAAAATATGCGTTATGTCGGCATTTTGAAGTCTGGACAGAAAGTCTCCGGCTTCATCGAGGCTGAAGGTTATGTCT
ACACTGTCGGTGTCGGCAACTATTTGGGACAAAACTACGGTAGAATCGAAAGCATTACCGACGACAGCATCGTCCTGAAC
GAGCTGATAGAAGACAGCACGGGCAACTGGGTTTCCCGTAAAGCAGAACTGCTGTTGAATTCTTCCGACAAAAACACCGA
ACAAGCGGCAGCACCTGCCGCAGAACAAAATTAAGAAGAGGATTACTCCATT

SEQ. ID NO:6
Nucleotide sequence of DNA region (1000 bp ) up-stream from the Hap gene
from Neisseria meningitidis
GTGCGGCAAAAAACAGCAAAAGCCCGCTGTCGATTGCCTGACCGTCCGCGTCCGTAAAATCAGCATAGGTTGCCACGCGC
GGCTTGGGCGTTTTCCCACACAAAGCCTCTGCCATCGGCAGCAGGTTTTTCCCCGATATGCGTATCACGCCCACGCCGCC
GCGCCCGGGTGCGGTAGCGACTGCCGCAATCGTTGGAACGTTATCCGACATAAAACCCCCGAAAATTCAAAACAGCCGCG
ATTATAGCAAATGCCGTCTGAAGTCCGACGGTTTGGCTTTCAGACGGCATAAAACCGCAAAAATGCTTGATAAATCCGTC
CGCCTGACCTAATATAACCATATGGAAAAACGAAACACATACGCCTTCCTGCTCGGTATAGGCTCGCTGCTGGGTCTGTT
CCATCCCGCAAAAACCGCCATCCGCCCCAATCCCGCCGACGATCTCAAAAACATCGGCGGCGATTTTCAACGCGCCATAG
AGAAAGCGCGAAAATGACCGAAAACGCACAGGACAAGGCGCGGCAGGCTGTCGAAACCGTCGTCAAATCCCCGGAGCTTG
TCGAGCAAATCCTGTCCGACGAGTACGTGCAAATAATGATAGCCCGGCGTTTCCATTCGGGATCGTTGCCGCCGCCGTCC
GACTTGGCGCAATACAACGACATTATCAGCAACGGGGCAGACCGCATTATGGCAATGGCGGAAAAAGAACAAGCCGTCCG
GCACGAAACCATACGGCAAGACCAAACCTTCAACAGGCGCGGGCAACTGTACGGCTTCATCAGCGTCATCCTGATACTGC
TTTTTGCCGTCTTCCTCGTATGGAGCGGCTACCCCGCAACCGCCGCCTCCCTTGCCGGCGGCACAGTGGTTGCCTTGGCG
GGTGCTTTCGTGATTGGAAGAAGCCGAGACCAAGGCAAAAATTAATTGCAAATCCTAGGGCGTGCTTCATATCCGCCCGA
ACGCCGAACCGCACATATAGGCACATCCCGCGCGCCGCCGGAAGCGGAAGCCGCGCCCTCCCAAACAAACCCGAATCCCG
TCAGATAAGGAAAAATA

SEQ. ID NO:7
Nucleotide sequence of DNA region (924 bp) up-stream from the NspA gene from
Neisseria meningitidis (serogroup B) (ATCC13090)
GGAACCGAACACGCCGTTCGGTCATACGCCGCCGAAAGGTTTGCCGCAAGACGAAGCCGCCCTCGACATCGAAGACGCGG
TACACGGCGCGCTGGAAGGCGCGGGTTTTGTCCACTACGAAACATCGGCTTTTGCGAAACCAGCCATGCAGTGCCGCCAC
AATTTGAACTACTGGCAGTTCGGCGATTATTTAGGCATAGGCGCGGGCGCTCACGGCAAAATTTCCTATCCCGACCGCAT
CGAGCGCACCGTCCGCCGCCGCCACCCCAACGACTACCTCGCCTTAATGCAAAGCCAACCGAGTGAAGCCGTCGAACGCA
AAACCGTTGCCGCCGAAGATTTGCCGTTTGAGTTCATGATGAACGCCCTGCGCCTGACCGACGCGTACCCGCCGCGATGT
TGCAGGAGCGCACGGGCGTACCGAGTGCCAAAATCATGGCGCAAATCGAAACGGCAAGGCAAAAAGGCCTGCTGGAAACC
GACCCCGCCGTATTCCGCCCGACCGAAAAAGGACGCTTGTTTTTAAACGATTTGCTGCAGTGTTTTTTATAGTGGATTAA
CAAAAACCAGTACGGCGTTGCCTCGCCTTAGCTCAAAGAGAACGATTCTCTAAGGTGCTGAAGCACCAAGTGAATCGGTT
CCGTACTATTTGTACTGTCTGCGGCTTCGTCGCCTTGTCCTGATTTTTGTTAATCCACTATATAAGCGCAAACAAATCGG
CGGCCGCCCGGGAAAACCCGCCCCGAACGCGTCCGGAAAATATGCTTATCGATGGAAAACGCAGCCGCATCCCCCGCCGG
GCGTTTCAGACGGCACAGCCGCCGCCGGAAATGTCCGACGCTTAAGGCACAGACGCACACAAAACCGTATGCCTGCACCT
GCAACAATCCGACAGATACCGCTGTTTTTCCAAACCGTTTGCA

SEQ. ID NO:8
Nucleotide sequence of DNA region (1000 bp ) up-stream from the FrpB gene
from Neisseria meningitidis (serogroup B)
AAGTGGGAATCTAAAAATGAAAAGCAACAGGAATTTATCGGAAATGACCGAAACTGAACGGACTGGATTCCCGCTTTCGC
GGGAATGACGGCGACAGGGTTGCTGTTATAGTGGATGAACAAAAACCAGTACGTCGTTGCCTCGCCTTAGCTCAAAGAGA
ACGATTCTCTAAGGTGCTGAAGCACCAAGTGAATCGGTTCCGTCCTATTTGTACTGTCTGCGGCTTCGTCGCCTTGTCCT
GATTTCTGTTCGTTTTCGGTTATTCCCGATAAATTACCGCCGTTTCTCGTCATTTCTTTAACCCTTCGTCATTCCCGCGC
AGGCGGGAATCTAGTTTTTTTGAGTTCCAGTTGTTTCTGATAAATTCTTGCAGCTTTGAGTTCCTAGATTCCCACTTTCG
TGGGAATGACGGTGGAAAAGTTGCCGTGATTTCGGATAAATTTTCGTAACGCATAATTTCCGTTTTACCCGATAAATGCC
CGCAATCTCAAATCCCGTCATTCCCCAAAAACAAAAAATCAAAAACAGAAATATCGTCATTCCCGCGCAGGCGGGAATCT
AGACCTTAGAACAACAGCAATATTCAAAGATTATCTGAAAGTCCGAGATTCTAGATTCCCACTTTCGTGGGAATGACGAA
TTTTAGGTTTCTGTTTTTGGTTTTCTGTCCTTGCGGGAATGATGAAATTTTAAGTTTTAGGAATTTATCGGAAAAAAACAG
AAACCGCTCCGCCGTCATTCCCGCACAGGCTTCGTCATTCCCGCGCAGGCTTCGTCATTCCCGCATTTGTTAATCCACTA
TATTCCCGCCGTTTTTTACATTTCCGACAAAACCTGTCAACAAAAAACAACACTTCGCAAATAAAAACGATAATCAGCTT
TGCAAAAATCCCCCCCCCCTGTTAATATAAATAAAAATAATTAATTAATTATTTTTCTTATCCTGCCAAATCTTAACGGT
TTGGATTTACTTCCCTTCATACACTCAAGAGGACGATTGA

SEQ. ID NO:9
Nucleotide sequence of DNA region (1000 bp ) up-stream from the FrpA gene
from Neisseria meningitidis (serogroup B)

CTATAAAGATGTAAATAAAAATCTCGGTAACGGTAACACTTTGGCTCAGCAAGGCAGCTACACCAAAACAGACGGTACAA
CCGCAAAAAATGGGGGATTTACTTTTAGCAGCCGACAATCTGCACAGCCGCTTCACGAACAAAATGCTATCCATTAGCCAT
GTTCGGGAAAACACGATTTCCCCGTTTGTTTTAGGCTGTCTAAACAAATAACCATAAATGTATATCATTATTTAAAATAA
ATAAAAGTATTTAACTATTATTGACGAAATTTTAGAGAAAGAGTAGACTGTCGATTAAATGACAAACAATAGTGAGAAAG
GAAATATTTACTATCCGAGCACAGAGCATATTTTAGGTAGCCTGTAACTGTTCCTGCTGGCGGAAGAGGATGAAGGTGGA
CTTACCCGAGAATAAATGTCCTGTTGTGTGATATGGATGCCATGCCGCGAAGCAATTGATGCAATCACGGCAGTCCTACT
TGAATGAAACCTGTCGTTGCAGAATTTGAAAACGCTATTTTTAAGAAAGGATAAAGGGAGAAAGAATTTTTGGTTTTTAA
GCTGCATGAAACCGTGTTGGAATAAATGCACACCTACGATAATTAATAATTTTCGTTTTTTATTCTACAAGCTATTTATA
TATGATTGCTAAAAGTTTATTTTTTAGATGCCAAAAAAATATATTTATATACTTCATATTGTTTATATGTCTTTATTTGA
ATATATCTTACGATGGGGAAATATTTATATATTTTATAATAAATTTTACTCATTTGCTAATATGTCATGGAATATTACTT
GTATTTTGTAGAATTTTTCCATATGAAAATATTCCATTTACTATTTTTCTGAACTTTATTAGTTTATTTTTAATATTTTT
ACCTCTTATATTTACCATAAGAGAGCTAATTGATTCATATTATATTGAGTCGATAATTAATTTATTCTTAATTTTAATTC
CTCACGTTATTTTTTTAATTTACTTGAAAGGAAAGCAGAT

SEQ. ID NO:10
Nucleotide sequence of DNA region (1000 bp ) up-stream from the FrpC gene
from Neisseria meningitidis (serogroup B)
GGAAACAGAGAAAAAAGTTTCTCTTCTATCTTGGATAAATATATTTACCCTCAGTTTAGTTAAGTATTGGAATTTATACC
TAAGTAGTAAAAGTTAGTAAATTATTTTTAACTAAAGAGTTAGTATCTACCATAATATATTCTTTAACTAATTTCTAGGC
TTGAAATTATGAGACCATATGCTACTACCATTTATCAACTTTTTATTTTGTTTATTGGGAGTGTTTTTACTATGACCTCA
TGTGAACCTGTGAATGAAAAGACAGATCAAAAAGCAGTAAGTGCGCAACAGGCTAAAGAACAAACCAGTTTCAACAATCC
CGAGCCAATGACAGGATTTGAACATACGGTTACATTTGATTTTCAGGGCACCAAAATGGTTATCCCCTATGGCTATCTTG
CACGGTATACGCAAGACAATGCCACAAAATGGCTTTCCGACACGCCCGGGCAGGATGCTTACTCCATTAATTTGATAGAG
ATTAGCGTCTATTACAAAAAAACCGACCAAGGCTGGGTTCTTGAGCCATACAACCAGCAAAACAAAGCACACTTTATCCA
ATTTCTACGCGACGGTTTGGATAGCGTGGACGATATTGTTATCCGAAAAGATGCGTGTAGTTTAAGTACGACTATGGGAG
AAAGATTGCTTACTTACGGGGTTAAAAAAATGCCATCTGCCTATCCTGAATACGAGGCTTATGAAGATAAAAGACATATT
CCTGAAAATCCATATTTTCATGAATTTTACTATATTAAAAAAGGAGAAAATCCGGCGATTATTACTCATCGGAATAATCG
AATAAACCAAACTGAAGAAGATAGTTATAGCACTAGCGTAGGTTCCTGTATTAACGGTTTCACGGTACAGTATTACCCGT
TTATTCGGGAAAAGCAGCAGCTCACACAGCAGGAGTTGGTAGGTTATCACCAACAAGTAGAGCAATTGGTACAGAGTTTT
GTAAACAATTCAAATAAAAAATAATTTAAAGGATCTTATT

SEQ. ID NO:11

Nucleotide sequence of DNA region (1000 bp ) up-stream from the Omp85 gene
from Neisseria meningitidis (serogroup B)
ACGTCCGAACCGTGATTCCGCAACGCCGCGCCCAAAACCAAAGCCCAAGCCAAAATGCCGATATAGTTGGCATTGGCAAT
CGCGTTAATCGGGTTGGCGACCAGGTTCATCAGCAGCGATTTCAACACTTCCACAATGCCGGAAGGCGGCGCGGCGGACA
CATCGCCCGCGCCCGCCAAAACAATGTGCGTCGGGAAAACCATACCGGCGATGACGGCGGTCAGGGCTGCGGAAAACGTA
CCAATGAGGTAAAGGATGATAATCGGCCTGATATGCGCCTTGTTGCCTTTTTGGTGCTGCGCGATTGTGGCCGCCACCAA
AATAAATACCAAAACCGGCGCGACCGCTTTGAGCGCGCCGACAAACAGGCTGCCGAACAAGCCTGCCGCCAAGCCCAGTT
GCGGGGAAACCGAACCGATTACGATGCCCAACGCCAAACCGGCGGCAATCTGCCTGACCAGGCTGACGCGGCCGATCGCA
TGAAATAAGGATTTGCCGAACGCCATAATTCTTCCTTATGTTGTGATATGTTAAAAAATGTTGTATTTTAAAAGAAAACT
CATTCTCTGTGTTTTTTTTATTTTTCGGCTGTGTTTTAAGGTTGCGTTGATTTGCCCTATGCAGTGCCGGACAGGCTTTG
CTTTATCATTCGGCGCAACGGTTTAATTTATTGAACGAAAATAAATTTATTTAATCCTGCCTATTTTCCGGCACTATTCC
GAAACGCAGCCTGTTTTCCATATGCGGATTGGAAACAAAATACCTTAAAACAAGCAGATACATTTCCGGCGGGCCGCAAC
CTCCGAAATACCGGCGGCAGTATGCCGTCTGAAGTGTCCCGCCCCGTCCGAACAACACAAAAACAGCCGTTCGAAACCCT
GTCCGAACAGTGTTAGAATCGAAATCTGCCACACCGATGCACGACACCCGTACCATGATGATCAAACCGACCGCCCTGCT
CCTGCCGGCTTTATTTTTCTTTCCGCACGCATACGCGCCT

SEQ. ID NO:12
Nucleotide sequence of DNA region (772 bp ) up-stream from the PilQ gene from
Neisseria meningitidis (serogroup B) (ATCC13090)
GCGATGTCGGGAAGCCTTCTCCCGAATCATTACCCCTTGAGTCGCTGAAAATCGCCCAATCTCCGGAAAACGGCGGCAAT
CATGACGGCAAGAGCAGCATCCTGAACCTCAGTGCCATTGCCACCACCTACCAAGCAAAATCCGTAGAAGAGCTTGCCGC
AGAAGCGGCACAAAATGCCGAGCAAAAATAACTTACGTTAGGGAAACCATGAAACACTATGCCTTACTCATCAGCTTTCT
GGCTCTCTCCGCGTGTTCCCAAGGTTCTGAGGACCTAAACGAATGGATGGCACAAACGCGACGCGAAGCCAAAGCAGAAA
TCATACCTTTCCAAGCACCTACCCTGCCGGTTGCGCCGGTATACAGCCCGCCGCAGCTTACAGGGCCGAACGCATTCGAC
TTCCGCCGCATGGAAACCGACAAAAAAGGGGAAAATGCCCCCGACACCAAGCGTATTAAAGAAACGCTGGAAAAATTCAG
TTTGGAAAATATGCGTTATGTCGGCATTTTGAAGTCTGGACAGAAAGTCTCCGGCTTCATCGAGGCTGAAGGTTATGTCT
ACACTGTCGGTGTCGGCAACTATTTGGGACAAAACTACGGTAGAATCGAAAGCATTACCGACGACAGCATCGTCCTGAAC
GAGCTGATAGAAGACAGCACGGGCAACTGGGTTTCCCGTAAAGCAGAACTGCTGTTGAATTCTTCCGACAAAAACACCGA
ACAAGCGGCAGCACCTGCCGCAGAACAAAATTAAGAAGAGGATTACTCCATT

SEQ. ID NO:13
Nucleotide sequence of DNA region (1000 bp ) up-stream from the Hsf-like gene
from Neisseria meningitidis (serogroup B)
TTTGTTTTTCTTTTGGTTTGTTTGAATGGTTAAATCGGGGTTTGGGGGCGGATGGTGCGGCATCCGCCCGGTTTTTGGG
GGTTGGGGGTTTTCTGATAAATTCCCCCAACTTAAAATCTCGTCATTCCCGCGAAGGCGGGAATCTGGGACGTGGAATCT
AAGGAAACTGTTTTATCCGGTAAGTTTCCGTGCCGACGGGTCTGGATTCCCGCTTTTGCGGGAATGACGGCGGTGGGGTT
TCTGTTTTTTCCGATAAATTCCTGTTGCGTTGCGTTTTTGGATTCCAGCTTTTGCGGGAATGACGGTCGGTGGGGTTTCT
GTTTTTTCCGATAAAGTCCTGCCGCGTTGTGTTTCTGGATTCCCGCCTGCGCGGGAATGACGGTCGGTGGGGGTTTCTGT
TTTTGCTGATAGATTCCTGTGGTTTTTCGGTTGCTGGATTCCCGCTTTTGCGGGAATGACGGTCGGTGGGGTTTCTGTTT
TTTCCGATAAATTCCTGTTGCGTTGTGTTTCTGGATTCCCGCCTGCGCGGGAATGACGCGGTGGGGGTTTCTGTTTTTTC
CGATAAATTCCTGTTGCGTTGCGTTTTTGGATTCCAACTTTTGCGGGAATGACGGTCGGTGGGGTTTCGGTTTTTTCCGA
TAAAGTCCTGCCGCGTTGTGTTTCTGGATTCCCGCCTGCGCGGGAATGACGCGGTGGGGGTTTCTGTTTTTTCTGATAGA
TTCCTGTGGTTTTTCTATGGATTCAATCATTCCTGATAAATTCCCATAATCTAAAATCTCGTCATTCCCGCGAAAGCGGG
AATCTAGGACGTGGAATCTAAGGAAACTGTTTTATCCGGTAAGTTTCCGTGCCGACGGGTCTGGATTCCCGCTTTTGCGG
GAATGACGGCGGTGGGGTTTCTGTTTTTTCTGATAAAGTCCTGCCGCGTTGTGTTTCTAGATTCCCGCTTTTGCGGGAAT
GACGGCGGTGAGGTTTCTGTTTTTTCCGATAAATTCCTGT

SEQ. ID NO:14
Nucleotide sequence of DNA region (1000 bp ) up-stream from the Hap gene
from Neisseria meningitidis (serogroup B)
AATCAGCATAGGTTGCCACGCGCGGCTTGGGCGTTTTCCCACACAAAGCCTCTGCCATCGGCAGCAGGTTTTTCCCCGAT
ATGCGTATCACGCCCACGCCGCCGCGCCCGGGTGCGGTAGCGACTGCCGCAATCGTTGGAACGTTATCCGACATAAAACC
CCCGAAAATTCAAAACAGCCGCGATTATAGCAAATGCCGTCTGAAGTCCGACGGTTTGGCTTTCAGACGGCATAAAACCG
CAAAAATGCTTGATAAATCCGTCCGCCTGACCTAATATAACCATATGGAAAAACGAAACACATACGCCTTCCTGCTCGGT
ATAGGCTCGCTGCTGGGTCTGTTCCATCCCGCAAAAACCGCCATCCGCCCCAATCCCGCCGACGATCTCAAAAACATCGG
CGGCGATTTTCAACGCGCCATAGAGAAAGCGCGAAAATGACCGAAAACGCACAGGACAAGGCGCGGCAGGCTGTCGAAAC
CGTCGTCAAATCCCCGGAGCTTGTCGAGCAAATCCTGTCCGACGAGTACGTGCAAATAATGATAGCCCGGCGTTTCCATT
CGGGATCGTTGCCGCCGCCGTCCGACTTGGCGCAATACAACGACATTATCAGCAACGGGGCAGACCGCATTATGGCAATG
GCGGAAAAAGAACAAGCCGTCCGGCACGAAACCATACGGCAAGACCAAACCTTCAACAGGCGCGGGCAACTGTACGGCTT
CATCAGCGTCATCCTGATACTGCTTTTTGCCGTCTTCCTCGTATGGAGCGGCTACCCCGCAACCGCCGCCTCCCTTGCCG
GCGGCACAGTGGTTGCCTTGGCGGGTGCTTTCGTGATTGGAAGAAGCCGAGACCAAGGCAAAAATTAATTGCAAATCCTA
GGGCGTGCTTCATATCCGCCCGAACGCCGAACCGCACATATAGGCACATCCCGCGCGCCGCCGGAAGCGGAAGCCGCGCC
CTCCCAAACAAACCCGAATCCCGTCAGATAAGGAAAAATA

SEQ. ID NO:15
Nucleotide sequence of DNA region (1000 bp ) up-stream from the LbpA gene
from Neisseria meningitidis (serogroup B)
GATTTTGGTCATCCCGACAAGCTTCTTGTCGAAGGGCGTGAAATTCCTTTGGTTAGCCAAGAGAAAACCATCAAGCTTGC
CGATGGCAGGGAAATGACCGTCCGTGCTTGTTGCGACTTTTTGACCTATGTGAAACTCGGACGGATAAAAACCGAACGCC
CGGCAAGTAAACCAAAGGCGGAAGATAAAAGGGAGGATGAAGAGAGTGCAGGCGTTGGTAACGTCGAAGAAGGCGAAGGC
GAAGTTTCCGAAGATGAAGGCGAAGAAGCCGAAGAAATCGTCGAAGAAGAACCCGAAGAAGAAGCTGAAGAGGAAGAAGC
TGAACCCAAAGAAGTTGAAGAAACCGAAGAAAAAATCGCCGACAGAAGAAAGCGGCAGCGGTTCAAACGCCATCCTGCCTG

CCTCGGAAGCCTCTAAAGGCAGGGACATCGACCTTTTCCTGAAAGGTATCCGCACGGCGGAAGCCGACATTCCAAGAACC
GGAAAAGCACACTATACCGGCACTTGGGAAGCGCGTATCGGCACACCCATTCAATGGGACAATCAGGCGGATAAAGAAGC
GGCAAAAGCAGAATTTACCGTTAATTTCGGCGAGAAATCGATTTCCGGAACGCTGACGGAGAAAAACGGTGTACAACCTG
CTTTCTATATTGAAAACGGCAAGATTGAGGGCAACGGTTTCCACGCAACAGCACGCACTCGTGAGAACGGCATCAATCTT
TCGGGAAATGGTTCGACCAACCCCAGAACCTTCCAAGCTAGTGATCTTCGTGTAGAAGGAGGATTTTACGGCCCGCAGCG
GAGGAATTGGGCGGTATTATTTTCAATAAGGATGGGAAATCTCTTGGTATAACTGAAGGTACTGAAAATAAAGTTGAAGT
TGAAGCTGAAGTTGAAGTTGAAGCTGAAACTGGTGTTGTCGAACAGTTAGAACCTGATGAAGTTAAACCCCAATTCGGCG
TGGTATTCGGTGCGAAGAAAGATAATAAAGAGGTGGAAAA

SEQ. ID NO:16
Nucleotide sequence of DNA region (1000 bp ) up-stream from the LbpB gene
from Neisseria meningitidis (serogroup A)
CGGCGTTAGAGTTTAGGGCAGTAAGGGCGCGTCCGCCCTTAGATCTGTAAGTTACGATTCCGTTAAATAACTTTTACTGA
CTTTGAGTTTTTTGACCTAAGGGTGAAAGCACCCTTACTGCTTAAAGTCCAACGACAAAAACCAAAAGACAAAAACACTT
TTATTACCCTAAAATCGAACACCCATAAATGACCTTTTTTGTCTTTGGCGAGGCGGCAGTAAGGGCGCGTCCGCCCTTAG
ATCTGTAAGTTATGATTCCGTTAAATAGCCTTTACTGACTTTGAGTTTTTTGACCTAAGGGCGGACGCGCCCTTACTGCT
TCACCTTCAATGGGCTTTGAATTTTGTTCGCTTTGGCTTGCTTGACCTAAGGGTGAAAGCACCCTTACTGCCGCCTCGCC
AAAGACGAAAAGGGTTATTTACGGGGGTTGGATTTTAGGCAGTAAGGGCGCGTCCGCCCTTAGATCTGTAAGTTATGATT
CCGTTAAATAGCCTTTACTGACTTTGAGTTTTTTGACCTAAGGGTGAAAGCACCCTTACTGCTTCACCTTCAATGGGCTT
TGAATTTTGTTCGCTTTGGCTTGCTTGATCTAAGGGTGAAAGCACCCTTACTGCCGTCTCGCCGAAGACAACGAGGGCTA
TTTACGGCGTTAGAGTTTAGGGCAGTAAGGGCGCGTCCGCCCTTAGATCCAGACAGTCACGCCTTTGAATAGTCCATTTT
GCCAAAGAACTCTAAAACGCAGGACCTAAGGGTGAAAGCACCCTTACTGCCTTACATCCAAGCACCCTTACTGCACCACG
TCCACGCACCCTTACTGCCCTACGTCCACGCACCCTTACTGCCCTACATCCAAGCACCCTTACTGCCTTACATAGACATG
ACAGACGCCGAGCAGCGGAACAGGACTAAAAACAATTAAGTGATATTTTTGCCCAACTATAATAGACATGTATAATTATA
TTACTATTAATAATAATTAGTTTATCCTCCTTTTCATCCC

SEQ. ID NO:17
Nucleotide sequence of DNA region (731 bp ) up-stream from the TbpA gene
from Neisseria meningitidis (serogroup B) (ATCC13090)
TATGAAGTCGAAGTCTGCTGTTCCACCTTCAATTATCTGAATTACGGAATGTTGACGCGC
AAAAACAGCAAGTCCGCGATGCAGGCAGGAGAAAGCAGTAGTCAAGCTGATGCTAAAACG
GAACAAGTTGGACAAAGTATGTTCCTCCAAGGCGAGCGCACCGATGAAAAAGAGATTCCA
AACGACCAAAACGTCGTTTATCGGGGGTCTTGGTACGGGCATATTGCCAACGGCACAAGC
TGGAGCGGCAATGCTTCCGATAAAGAGGGCGGCAACAGGGCGGACTTTACTGTGAATTTC
GGTACGAAAAAAATTAACGGCACGTTAACCGCTGACAACAGGCAGGCGGCAACCTTTACC
ATTGTGGGCGATATTGAGGGCAACGGTTTTTCCGGTACGGCGAAAACTGCTGACTCAGGT
TTTGATCTCGATCAAAGCAATAACACCCGCACGCCTAAGGCATATATCACAAACGCCAAG
GTGCAGGGCGGTTTTTACGGGCCCAAAGCCGAAGAGTTGGGCGGATGGTTTGCCTATTCG
GACGATAAACAAACGAAAAATGCAACAGATGCATCCGGCAATGGAAATTCAGCAAGCAGT
GCAACTGTCGTATTCGGTGCGAAACGCCAAAAGCCTGTGCAATAAGCACGGTTGCCGAAC
AATCAAGAATAAGGCCTCAGACGGCACCGCTCCTTCCGATACCGTCTGAAAGCGAAGAGT
AGGGAAACACT

SEQ. ID NO:18
Nucleotide sequence of DNA region (373 bp) up-stream from the OmplA gene
from Neisseria meningitidis (serogroup B) (ATCC13090)
CGTACCGCATTCCGCACTGCAGTGAAAAAAGTATTGAAAGCAGTCGAAGCAGGCGATAAAGCTGCCGCACAAGCGGTTTA
CCAAGAGTCCGTCAAAGTCATCGACCGCATCGCCGACAAGGGCGTGTTCCATAAAAACAAAGCGGCTCGCCACAAAACCC
GTTTGTCTCAAAAAGTAAAACCTTGGCTTGATTTTTGCAAAACCTGCAATCCGGTTTTCATCGTCGATTCCGAAAACCCC
TGAAGCCCGACGGTTTCGGGGTTTTCTGTATTGCGGGGACAAAATCCCGAAATGGCGGAAAGGGTGCGGTTTTTTATCCG
AATCCGCTATAAAATGCCGTCTGAAAACCAATATGCCGACAATGGGGGTGGAG

SEQ. ID NO:19
Nucleotide sequence of DNA region (1000 bp ) up-stream from the Pla1 gene
from Neisseria meningitidis (serogroup B)
TTTTGGCTTCCAGCGTTTCATTGTTTTCGTACAAGTCGTAAGTCAGCTTCAGATTGTTGG
CTTTTTTAAAGTCTTCGACCGTACTCTCATCAACATAGTTCGACCAGTTGTAGATGTTCA
GAGTATCGGTGGCAGCGGCTTCGGCATTGGCAGCAGACGCAGCGTCTGCTTGAGGTTGCA
CGGCGTTTTTTTCGCTGCCGCCGCAGGCTGCCAGAGACAGCGCGGCCAAAACGGCTAATA
CGGATTTTTTCATACGGGCAGATTCCTGATGAAAGAGGTTGGAAAAAAAGAAATCCCCGC
GCCCCATCGTTACCCCGGCGCAAGGTTTGGGCATTGTAAAGTAAATTTGTGCAAACTCAA
AGCGATATTGGACTGATTTTCCTAAAAAATTATCCTGTTTCCAAAAGGGGAGAAAAACGT
CCGCCCGATTTTGCCGTTTTTTTGCGCTGTCAGGGTGTCCGACGGGCGGATAGAGAGAAA
AGGCTTGCATATAATGTAAACCCCCTTTAAAATTGCGCGTTTACAGAATTTATTTTTCTT
CCAGGAGATTCCAATATGGCAAACAGCGCACAAGCACGCAAACGTGCCCGCCAGTCCGTC
AAACAACGCGCCCACAATGCTAGCCTGCGTACCGCATTCCGCACCGCAGTGAAAAAAGTA
TTGAAAGCAGTCGAAGCAGGCGATAAAGCTGCCGCACAAGCGGTTTACCAAGAGTCCGTC
AAAGTCATCGACCGCATCGCCGACAAGGGCGTGTTCCACAAAAACAAAGCGGCACGCCAC
AAAAGCCGTCTGTCTGCAAAAGTAAAAGCCTTGGCTTGATTTTTGCAAAACCGCCAAGGC
GGTTGATACGCGATAAGCGGAAAACCCTGAAGCCCGACGGTTTCGGGGTTTTCTGTATTG
CGGGGGCAAAATCCCGAAATGGCGGAAAGGGTGCGATTTTTTATCCGAATCCGCTATAAA
ATGCCGTTTGAAAACCAATATGCCGACAATGGGGGCGGAG

SEQ. ID NO:20
Nucleotide sequence of DNA region (1000 bp) up-stream from the FhaB gene
from Neisseria meningitidis (serogroup B)
TACGGAAACTGCAAGCGGATCCAGAAGTTACAGCGTGCATTATTCGGTGCCCGTAAAAAAATGGCTGTTTTCTTTTAATC
ACAATGGACATCGTTACCACGAAGCAACCGAAGGCTATTCCGTCAATTACGATTACAACGGCAAACAATATCAGAGCAGC
CTGGCCGCCGAGCGCATGCTTTGGCGTAACAGACTTCATAAAACTTCAGTCGGAATGAAATTATGGACACGCCAAACCTA
TAAATACATCGACGATGCCGAAATCGAAGTGCAACGCCGCCGCTCTGCAGGCTGGGAAGCCGAATTGCGCCACCGTGCTT
ACCTCAACCGTTGGCAGCTTGACGGCAAGTTGTCTTACAAACGCGGGACCGGCATGCGCCAAAGTATGCCTGCACCGGAA
GAAAACGGCGGCGATATTCTTCCAGGTACATCTCGTATGAAAATCATTACTGCCGGTTTGGACGCAGCCGCCCCATTTAT
TTTAGGCAAACAGCAGTTTTTCTACGCAACCGCCATTCAAGCTCAATGGAACAAAACGCCGTTGGTTGCCCAAGATAAAT
TGTCAATCGGCAGCCGCTACACCGTTCGCGGATTTGATGGGGAGCAGAGTCTTTTCGGAGAGCGAGGTTTCTACTGGCAG
AATACTTTAACTTGGTATTTTCATCCGAACCATCAGTTCTATCTCGGTGCGGACTATGGCCGCGTATTTGGCGAAAGTGC
ACAATATGTATCGGGCAAGCAGCTGATGGGTGCAGTGGTCGGCTTCAGAGGAGGGCATAAAGTAGGCGGTATGTTTGCTT
ATGATCTGTTTGCCGGCAAGCCGCTTCATAAACCCAAAGGCTTTCAGACGACCAACACCGTTTACGGCTTCAACTTGAAT
TACAGTTTCTAACCTCTGAATTTTTTACTGATATTTAGACGGTCTTTCCTTATCCTCAGACCGTCAAACTTTACCTACGT
ACTTGGCGCGCAGTACGTTCATCTTCAAAATGGAATAGAC

SEQ. ID NO:21
Nucleotide sequence of DNA region (1000 bp ) up-stream from the Lipo02 gene
from Neisseria meningitidis (serogroup B)
TTATCTTGGTGCAAAACTTTGTCGGGGTCGGACTGGCTACGGCTTTGGGTTTGGACCCGCTCATCGGTCTGATTACCGGT
TCGGTGTCGCTGACGGGCGGACACGGTACGTCAGGTGCGTGGGGACCTAATTTTGAAACGCAATACGGCTTGGTCGGCGC
AACCGGTTTGGGTATTGCATCGGCTACTTTCGGGCTGGTGTTCGGCGGCCTGATCGGCGGGCCGGTTGCGCGCCGCCTGA
TCAACAAAATGGGCCGCAAACCGGTTGAAAACAAAAAACAGGATCAGGACGACAACGCGGACGACGTGTTCGAGCAGGCA
AAACGCACCCGCCTGATTACGGCGGAATCTGCCGTTGAAACGCTTGCCATGTTTGCCGCGTGTTTGGCGTTTGCCGAGAT
TATGGACGGCTTCGACAAAGAATATCTGTTCGACCTGCCCAAATTCGTGTGGTGTCTGTTTGGCGGCGTGGTCATCCGCA
ACATCCTCACTGCCGCATTCAAGGTCAATATGTTCGACCGCGCCATCGATGTGTTCGGCAATGCTTCGCTTTCGCTTTTC
TTGGCAATGGCGTTGCTGAATTTGAAACTGTGGGAGCTGACCGGTTTGGCGGGGCCTGTAACCGTGATTCTTGCCGTACA
AACCGTGGTGATGGTTTTGTACGCGACTTTTGTTACCTATGTCTTTATGGGGCGCGACTATGATGCGGCAGTATTGGCTG
CCGGCCATTGCGGTTTCGGCTTGGGTGCAACGCCGACGGCGGTGGCAAATATGCAGTCCGTCACGCATACTTTCGGCGCG
TCGCATAAGGCGTTTTTGATTGTGCCTATGGTCGGCGCGTTCTTCGTCGATTTGATTAATGCCGCGATTCTCACCGGTTT
TGTGAATTTCTTTAAAGGCTGATTTTCCGCCTTTCCGACAAAGCACCTGCAAGGTTTACCGCCTGCAGGTGCTTTTGCTA
TGATAGCCGCTATCGGTCTGCACCGTTTGGAAGGAACATC

SEQ. ID NO:22
Nucleotide sequence of DNA region (1000 bp ) up-stream from the Tbp2 gene
from Neisseria meningitidis (serogroup B)
CCTACTCCACCGATTCCAATATGCTCGGCGCGACCCACGAAGCCAAAGACTTGGAATTTTTGAACTCGGGCATCAAAATC
GTCAAACCCATTATGGGCGTTGCCTTTTGGGACGAAAACGTTGAAGTCAGCCCCGAAGAAGTCAGCGTGCGCTTTGAAGA
AGGCGTGCCGGTTGCACTGAACGGCAAAGAATACGCCGACCCCGTCGAACTCTTCCTCGAAGCCAACCGCATCGGCGGCC
GCCACGGCTTGGGTATGAGCGACCAAATCGAAAACCGCATCATCGAAGCCAAATCGCGCGGCATCTACGAAGCCCCGGGT
ATGGCGTTGTTCCACATCGCCTACGAACGCTTGGTGACCGGCATCCACAACGAAGACACCATCGAACAATACCGCATCAA
CGGCCTGCGCCTCGGCCGTTTGCTCTACCAAGGCCGCTGGTTCGACAGCCAAGCCTTGATGTTGCGCGAAACCGCCCAAC
GCTGGGTCGCCAAAGCCGTTACCGGCGAAGTTACCCTCGAACTGCGGCGCGGCAACGACTACTCGATTCTGAACACCGAA
TCGCCCAACCTGACCTACCAACCCGAACGCCTGAGTATGGAAAAAGTCGAAGGTGCGGCGTTTACCCCGCTCGACCGCAT
CGGACAGCTCACGATGCGCAACCTCGACATCACCGACACCCGCGCCAAACTGGGCATCTACTCGCAAAGCGGTTTGCTGT
CGCTGGGCGAAGGCTCGGTATTACCGCAGTTGGGCAATAAGAAATAAGGTTTGCTGTTTTGCATCATTAGCAACTTAAGG
GGTCGTCTGAAAAGATGATCCCTTATGTTAAAAGGAATCCTATGAAAGAATACAAAGTCGTCATTTATCAGGAAAGCCAG
TTGTCCAGCCTGTTTTTCGGCGCGGCAAAGGTCAACCCCGTCAATTTCAGCGCGTTCCTCAACAAACAAACCCCCCGAAG
GCTGGCGGGTCGAGACCTTTGCAATAACATAGGTTACTAA

SEQ. ID NO:23
Nucleotide sequence of DNA region (1000 bp ) up-stream from the PorA gene
from Neisseria meningitidis (serogroup B)
GAATGACAATTCATAAGTTTCCCGAAATTCCAACATAACCGAAACCTGACAATAACCGTAGCAACTGAACCGTCATTCCC
GCAAAAGCGGGAATCCAGTCCGTTCAGTTTCGGTCATTTCCGATAAATGCCTGTTGCTTTTCATTTCTAGATTCCCACTT
TCGTGGGAATGACGGCGGAAGGGTTTTGGTTTTTTCCGATAAATTCTTGAGGCATTGAAATTCCAAATTCCCGCCTGCGC
GGGAATGACGGCTGCAGATGCCCGACGGTCTTTATAGTGGATTAACAAAAATCAGGACAAGGCGACGAGCTGCAGACAGT
ACAGATAGTACGGAACCGATTCACTTAGTGCTTCAGTATCTTAGAGAATCGTTCTCTTTGAGCTAAGGCGAGGCAACGTC
GTACTGGTTTTTGTTCATCCACTATATATGACACGGAAAACGCCGCCGTCCAAACCATGCCGTCTGAAGAAAACTACACA
GATACCGCCGCTTATATTACAATCGCCGCCCCGTGGTTCGAAAACCTCCCACACTAAAAAACTAAGGAAACCCTATGTCC
CGCAACAACGAAGAGCTGCAAGGTATCTCGCTTTTGGGTAATCAAAAAACCCAATATCCGGCCGAATACGCGCCCGAAAT
TTTGGAAGCGTTCGACAACAAACATCCCGACAACGACTATTTCGTCAAATTCGTCTGCCCAGAGTTCACCAGCCTCTGCC
CCATGACCGGGCAGCCCGACTTCGCCACCATCGTCATCCGCTACATTCCGCACATCAAAATGGTGGAAAGCAAATCCCTG
AAACTCTACCTCTTCAGCTTCCGCAACCACGGCGATTTTCATGAAGACTGCGTCAACATCATCATGAAAGACCTCATTGC
CCTGATGGATCCGAAATACATCGAAGTATTCGGCGAGTTCACACCGCGCGGCGGCATCGCCATTCATCCTTTCGCCAATT
ACGGCAAAGCAGGCACCGAGTTTGAAGCATTGGCGCGTAA

SEQ. ID NO:24
Neisseria meningitidis (serogroup B) PorA Promoter Region

GATATCGAGGTCTGCGCTTGAATTGTGTTGTAGAAACACAACGTTTTTGAAAAAATAAGCTATTGTTTTATATCAAAATA
TAATCATTTTTAAAATAAAGGTTGCGGCATTTATCAGATATTTGTTCTGAAAAATGGTTTTTTGCGGGGGCGGGGGTATA
ATTGAAGACGTATCGGGTGTTTGCCCGATGTTTTTAGGTTTTTATCAAATTTACAAAAGGAAGCCCAT

SEQ. ID NO:25
Nucleotide sequence of DNA region (1000 bp ) up-stream from the PorB gene
from Neisseria meningitidis (serogroup A)
gttttctgtttttgagggaatgacgggatgtaggttcgtaagaatgacgggatataggtttccgtgcggatggattcgtc
attcccgcgcaggcgggaatctagaacgtggaatctaagaaaccgttttatccgataagtttccgtgcggacaagtttgg
attcccgcctgcgcgggaatgacgggattttaggtttctaattttggttttctgtttttgagggaatgacgggatgtagg
ttcgtaggaatgacgggatataggtttccgtgcggatggattcgtcattcccgcgcaggcgggaatctagaccttagaac
aacagcaatattcaaagattatctgaaagtccgagattctagattcccgcctgagcgggaatgacgaaaagtggcgggaa
tgacggttagcgttgcctcgccttagctcaaagagaacgattctctaaggtgctgaagcaccaagtgaatcggttccgta
ctatttgtactgtctgcggcttcgtcgccttgtcctgattttgttaatccactatctcctgccgcagggcgggttttg
catccgcccgttccgaaagaaaccgcgtgtgcgtttttgccgtctttataaccccggtttgcaatgccctccaatacc
ctcccgagtaagtgttgtaaaaatgcaaatcttaaaaatttaaataaccatatgttataaaacaaaaaatacccataat
atctctatccgtccttcaaaatgcacatcgaattccacacaaaaacaggcagaagtttgtttttcagacaggaacatct
atagtttcagacatgtaatcgccgagcccctcggcggtaaatgcaaagctaagcggcttggaaagcccggcctgcttaaa
tttcttaaccaaaaaggaatacagcaatgaaaaatccctgattgccctgactttggcagcccttcctgttgcagcaat
ggctgacgttaccctgtacggcaccatcaaaaccggcgta

SEQ. ID NO:26
Neisseria meningitidis (serogroup B) PorB Promoter Region
GTTTTCTGTTTTTGAGGGAATGACGGGATGTAGGTTCGTAAGAATGACGGGATATAGGTTTCCGTGCGGATGGATTCGTC
ATTCCCGCGCAGGCGGGAATCTAGAACGTGGAATCTAAGAAACCGTTTTATCCGATAAGTTTTCCGTGCGGACAAGTTTG
GATTCCCGCCTGCGCGGGAATGACGGGATTTTAGGTTTCTAATTTTGGTTTTCTGTTTTTGAGGGAATGACGGGATGTAG
GTTCGTAGGAATGACGGGATATAGGTTTCCGTGCGGATGGATTCGTCATTCCCGCGCAGGCGGGAATCCAGACCTTAGAA
CAACAGCAATATTCAAAGATTATCTGAAAGTCCGAGATTCTAGATTCCCGCCTGAGCGGGAATGACGAAAAGTGGCGGGA
ATGACGGTTAGCGTTGCCTCGCCTTAGCTCAAAGAGAACGATTCTCTAAGGTGCTGAAGCACTAAGTGAATCGGTTCCGT
ACTATTTGTACTGTCTGCGGCTTCGTCGCCTTGTCCTGATTTTTGTTAATCCACTAT

SEQ. ID NO:27
Nucleotide sequence of DNA region (1000 bp ) up-stream from the siaABC gene
from Neisseria meningitidis (serogroup B)
ATACGGCCAATGGCTTCAGAAAGCGATAAGCCTCTGGCTGAAAAACCGATTTCTTGTGTTCTCCCCACCGCACCCATAGA
CGTAAAGGTATAGGGATTGGTAATCATGGTAACCACATCACCGCGACGCAGCAAAATATTTTGTCGCGGATTTGCAACTA
AATCTTCCAAGGCAACAGTTCGTACTACATTGCCACGTGTCAGCTGCACATTCGTATCCTGCACATTTGCCGTTGAACCA
CCTACCGCAGCCACCGCATCCAACACACGCTCACCGGCTGCCGTCAGCGGCATACGCACACTATTCCCAGCACGAATCAC
CGACACATTCGCCGCATTATTCTGCACCAAACGCACCATCACTTGTGGCTGATTGGCCATTTTTTTCAGGCGGCCTTTAA
TAATTTCCTGAACCTGACCAGGCGTTTTACCGACCACCGAAATATCGCCAACAAACGGCACAGAAACCGTACCACGTGCC
GTGACCAACTGCTCTGGCAACTTAGTTTGATGCGCACTACCCGAGCCCATCGAAGAAAGGCCACCACCAAACAATACTGC
CGGCGGCGCTTCCCAAATCATAATATCCAATACATCACCAATATTTAGCGTACCAGCCGAAGCATAACCATCGCCAAACT
GAGTGAATGACTGATTTATCTGAGCCTTATATAATAACTGAGCAACCGTATGATTCACATCAATCAGCTCCACTTCAGGA
ATTTGAACTTCAGATTGTTGCCCTAAAGAGACAATTTTTTTTGCGCTGGGGCCTGATGAAGGAATCGCAGAGCATCCTAC
AATTAAACTTCCACACAATAATAATACTGCGTGACGAATATAAAATTTCACTTTAAACACAAGCCAAATCCTAATATAAT
TATAAATGGCCTAATTATAGCACTTAATCGAAATAAATTTATGAGTACGTAGAGTATAATTAGTATTCTTCTTTCCAACT
TCCTTATACTTATATATATATACTTATAGATTCTAAAATC

SEQ. ID NO:28
Nucleotide sequence of DNA region (1000 bp ) up-stream from the lgt gene from
Neisseria meningitidis (serogroup B)
GCCAAAGCATTGGGCGCGGATGCCGCCGCTGCCGAACGCGCCGCGCGTCTTGCCAAAGCCGACTTGGTAACCGAAATGGT
CGGCGAGTTCCCCGAACTGCAAGGCACGATGGGCAAATACTATGCCTGTTTGGACGGCGAAACCGAAGAAATTGCCGAAG
CCGTCGAGCAGCACTATCAGCCGCGTTTTGCCGGCGACAAGCTGCCCGAAAGCAAAATTGCCGCCGCCGTGGCACTGGCC
GACAAACTAGAAACCTTGGTCGGCATTTGGGGCATCGGTCTGATTCCGACCGGCGACAAAGACCCCTACGCCCTGCGCCG
CGCTGCCTTGGGTATTTTGCGTATGCTGATGCAGTATGGTTTGGACGTGAACGAACTGATTCAGACGGCATTCGACAGCT
TCCCCAAAGGTTTGCTCAACGAAAAAACGCCGTCTGAAACCGCCGACTTTATGCAGGCGCGCCTTGCCGTGTTGCTGCAA
AACGATTATCCGCAAGACATCGTTGCCGCCGTACTCGCCAAACAGCCGCGCCGTTTGGACGATTTGACCGCCAAACTGCA
GGCCGTTGCCGCGTTCAAACAACTGCCCGAAGCCGCCGCGCTCGCCGCCGCCAACAAACGCGTGCAAAACCTGCTGAAAA
AAGCCGATGCCGAGTTGGGCGCGGTTAACGAAAGCCTGTTGCAACAGGACGAAGAAAAAGCCCTCTTTGCCGCCGCGCAA
GGCTTGCAGCCGAAAATCGCCGCCGCCGTCGCCGAAGGCAATTTCCAAACCGCCTTGTCCGAACTGGCTTCCGTCAAACC
GCAAGTCGATGCATTCTTTGACGGCGTGATGGTAATGGCGGAAGATGCCGCCGTAAAACAAAACCGCCTGAACCTGCTGA
ACCGCTTGGCAGAGCAAATGAACGCGGTAGCCGACATCGCGCTTTTGGGCGAGTAACCGTTGTACAGTCCAAATGCCGTC
TGAAGCCTTCAGACGGCATCGTGCCTATCGGGAGAATAAA

SEQ. ID NO:29
Nucleotide sequence of DNA region (1000 bp) up-stream from the TbpB gene
from Neisseria meningitidis (strain MC58)
GAACGAACCGGATTCCCACTTTCGTGGGAATGACGAATTTCAGGTTACTGTTTTTGGTTTTCTGTTTTTGTGAAAATAAT
GGGATTTCAGCTTGTGGGTATTTACCGGAAAAAACAGAAACCGCTCCGCCGTCATTCCCGCGCAGGCGGGAATCTAGGTC
TGTCGGTGCGGAAACTTATCGGATAAAACGGTTTCTTGAGATTTTTCGTCCTGGATTCCCACTTTCGTGGGAATGACGCG
AACAGAAACCGCTCCGCCGTCATTCCCGCGCAGGCGGGAATCTAGACATTCAATGCTAAGGCAATTTATCGGGAATGACT

GAAACTCAAAAAACTGGATTCCCACTTTCGTGGGAATGACGTGGTGCAGGTTTCCGTATGGATGGATTCGTCATTCCCGC
GCAGGCGGGAATCTAGACCTTCAATACTAAGGCAATTTATCGGAAATGACTGAAACTCGAAAAACTGGATTCCCACTTTT
GTGGGAATGACGCGATTAGAGTTTCAAAATTTATTCTAAATAGCTGAAACTCAACACACTGGATTCCCGCCTGCGCGGGA
ATGACGAAGTGGAAGTTACCCGAAACTTAAAACAAGCGAAACCGAACGAACTGGATTCCCACTTTCGTGGGAATGACGGA
ATGTAGGTTCGTGGGAATGACGGCGGAGCGGTTTCTGCTTTTTCCAATAAATGACCCCAACTTAAAATCCCGTCATTCCC
GCCCAGGCGGGAATCTAGGTCTGTCGGTGCGGAAACTTATCGGGTAAAACGGTTTCTTGAGATTTTGCGTCCTGGATTCC
CACTTTCGTGGGAATGACGGAATGTAGGTTCGTGGGAATGACGGGATATAGGTTTCCGTGCGGACGCGTTCGGATTCATG
ACTGCGCGGGAATGACGGGATTTTGGTGTATTCCCTAAAAAAATAAAAAAGTATTTGCAAATTTGTTAAAAATAAATAAA
ATAATAATCCTTATCATTCTTTAATTGAATTGGATTTATT

SEQ. ID NO:30
Nucleotide sequence of DNA region (1000 bp ) up-stream from the opc gene from
Neisseria meningitidis (serogroup A)
CAAAGGCTACGACAGTGCGGAAAACCGGCAACATCTGGAAGAACATCAGTTGTTGGACGGCATTATGCGCAAAGCCTGCC
GCAACCGTCCGCTGTCGGAAACGCAAACCAAACGCAACCGGTATTTGTCGAAGACCCGTTATAGTGGATTAAATTTAAAT
CAGGACAAGGCGACGAAGCCGCAGACAGTACAAATAGTACGGCAAGGCGAGGCAACGCCGTACTGGTTTAAATTTAATCC
ACTATATGTGGTCGAACAGAGCTTCGGTACGCTGCACCGTAAATTCCGCTATGCGCGGGCAGCCTATTTCGGACTGATTA
AAGTGAGTGCGCAAAGCCATCTGAAGGCGATGTGTTTGAACCTGTTGAAAGCCGCCAACAAGCTAAGTGCGCCCGCTGCC
GCCTAAAAGGAGACCGGATGCCTGATTATCGGGTATCCGGGGAGGGTTAAGGGGGTATTTGGTAAAATTAGGAGGTATT
TGGGGCGAAAATAGACGAAAACCTGTGTTTGGGTTTCGGCTGTCGGGAGGGAAAGGAATTTTGCAAAGATCTCATCCTGT
TATTTTCACAAAAACAGAAAACCAAAAACAGCAACCTGAAATTCGTCATTCCCGCGCAGGCGGGAATCCAGACCCCCAAC
GCGGCAGGAATCTATCGGAAATAACCGAAACCGGACGAACCTAGATTCCCGCTTTCGCGGGAATGACGGCAGAGTGGTTT
CAGTTGCTCCCGATAAATGCCGCCATCTCAAGTCTCGTCATTCCCTTAAAACAGAAAACCGAAATCAGAAACCTAAAATT
TCGTCATTCCCATAAAAAACAGAAAACCAAGTGAGAATAACAATTCGTTGTAAACAAATAACTATTTGTTAATTTTTATT
AATATATGTAAAATCCCCCCCCCCCCCCCCGAAAGCTTAAGAATATAATTGTAAGCGTAACGATTATTTACGTTATGTT
ACCATATCCGACTACAATCCAAATTTTGGAGATTTTAACT

SEQ. ID NO:31
Nucleotide sequence of DNA region (1000 bp ) up-stream from the siaD gene
from Neisseria meningitidis (serogroup B)
ATAATGCAGGCGCTGAAGTTGTTAAACATCAAACACACATCGTTGAAGACGAAATGTCTGATGAGGCCAAACAAGTCATT
CCAGGCAATGCAGATGTCTCTATTTATGAAATTATGGAACGTTGCGCCCTGAATGAAGAAGATGAGATTAAATTAAAAGA
ATACGTAGAGAGTAAGGGTATGATTTTTATCAGTACTCCTTTCTCTCGTGCAGCTGCTTTACGATTACAACGTATGGATA
TTCCAGCATATAAAATCGGCTCTGGCGAATGTAATAACTACCCATTAATTAAACTGGTGGCCTCTTTTGGTAAGCCTATT
ATTCTCTCTACCGGCATGAATTCTATTGAAAGCATCAAAAAGTCGGTAGAAATTATTCGAGAAGCAGGGGTACCTTATGC
TTTGCTTCACTGTACCAACATCTACCCAACCCCTTACGAAGATGTTCGATTGGGTGGTATGAACGATTTATCTGAAGCCT
TTCCAGACGCAATCATTGGCCTGTCTGACCATACCTTAGATAACTATGCTTGCTTAGGAGCAGTAGCTTTAGGCGGTTCG
ATTTTAGAGCGTCACTTTACTGACCGCATGGATCGCCCAGGTCCGGATATTGTATGCTCTATGAATCCGGATACTTTTAA
AGAGCTCAAGCAAGGCGCTCATGCTTTAAAATTGGCACGCGGCGGCAAAAAAGACACGATTATCGCGGGAGAAAAGCCAA
CTAAAGATTTCGCCTTTGCATCTGTCGTAGCAGATAAAGACATTAAAAAAGGAGAACTGTTGTCCGGAGATAACCTATGG
GTTAAACGCCCAGGCAATGGAGACTTCAGCGTCAACGAATATGAAACATTATTTGGTAAGGTCGCTGCTTGCAATATTCG
CAAAGGTGCTCAAATCAAAAAAACTGATATTGAATAATGCTTATTAACTTAGTTACTTTATTAACAGAGGATTGGCTATT
ACATATAGCTAATTCTCATTAATTTTTAAGAGATACAATA

SEQ. ID NO:32
Nucleotide sequence of DNA region (1000 bp ) up-stream from the ctrA gene
from Neisseria meningitidis (serogroup B)
ATACCTGCACTTGAGTTGCCGACCATAAATTTAGCATGTTTCAATAAGACTAAAAAATATTCAAATCGAATGGAAGGAAA
TGCAATAAATTTATCAGATTGATATTTTAATAATTCTTGCAGAATACTTTCAGTGCCAGTGTCATTATTAGGGTAGATGC
TAATGATATTTTGGCCACTTAATTCTAATGCTTTGAAATATTGGGCCGCATATTGTGGCATTAAATGTGCTTCTGTAGTC
ACGGGGTGAAACATAGAAATACCATAATTTTCGTATGGTAAACCGTAATATTCTTTGACTTCTTCTAAGGATGGGAGGGT
GGAAGAGGCCATAACATCTAAATCGGGGGAGCCGATGATGTGAATATGCTTTCTTTTTTCTCCCATTTGCACTAGGCGAG
TGACAGCTTGTTCATTTGCTACCAAGTGGATATGAGAAAGTTTACTAATAGAATGACGAATGGAGTCATCTACTGTACCA
GATAGTTCACCACCTTCGATATGGCAAACTAAACGGCTGCTTAATGCACCTACAGCTGCGCCTGCTAGTGCTTCTAAACG
GTCGCCGTGAATCATGACCATATCAGGTTCAATTTCATCAGATAGACGAGAGATAAACGTAATGGTATTGCCTAAAACGG
CACCCATTGGTTCACCTTGGATTTGATTTGAAAACAGATATGTATGTTGATAGTTTTCTCGAGTTACTTCCTTGTAGGTT
CTGCCATATGTTTTCATCATATGCATACCAGTTACAATCAAATGCAATTCAAGGTCTGGGTGATTTTCAATATAGGCTAA
TAAAGGTTTTAGCTTGCCGAAGTCGGCTCTGGTACCTGTAATGCAAAGAATTCTTTTCATGATTTTAGAATCTATAAGTA
TATATATATAAGTATAAGGAAGTTGGAAAGAAGAATACTAATTATACTCTACGTACTCATAAATTTATTTCGATTAAGTG
CTATAATTAGGCCATTTATAATTATATTAGGATTTGGCTT

SEQ. ID NO:33
Nucleotide sequence of DNA region (1000 bp ) up-stream from the lgtF gene
from Neisseria meningitidis (serogroup A)
TCTTTTTCGGACTGAAAGGACGCATCATCCCGACATCGAGCGCGTGTTCGTCCGGCAGCCAAGGCATAGGTTATGCCTAC
GAAGCCATCAAATACGGTCTGACCGATATGATGCTGGCGGGCGGAGGCGAAGAATTTTTCCCGTCCGAAGTGTATGTTTT
CGACTCGCTTTATGCCGCCAGCCGCCGCAACGGCGAACCGGAAAAAACCCCGCGCCCATACGACGCGAACCGCGACGGGC
TGGTCATCGGCGAAGGCGCGGGGATTTTCGTGCTGGAAGAATTGGAACACGCCAAACGGCGCGGTGCGATAATTTACGCC
GAACTCGTCGGCTACGGAGCCAACAGCGATGCCTACCATATTTCCACGCCCCCGCCCCGACGCGCAAGGCGCAATCCTTGC
CTTTCAGACGGCATTGCAACACGCAGACCTTGCGCCCGAAGACATCGGCTGGATTAATCTGCACGGCACCGGGACGCACC
ACAACGACAGTATGGAAAGCCGCGCCGTTGCAGCGGTTTTCGGCAACAATACGCCCTGCACGTCCACCAAGCCGCAAACC
GGACACACGCTGGGCGCGGCGGGCGCAATCGAAGCCGCGTTCGCGTGGGGCATTGCTGACCGGAAAAGCAATCCCGAAGG
GAAACTTCCGCCCCAGCTTTGGGACGGGCAGAACGATCCCGACCTTCCCGCCATCAACCTGACCGGCAGCGGCAGCCGCT

GGGAAACCGAAAAACGCATTGCCGCCAGCTCGTCGTTTGCCTTCGGAGGAAGCAACTGCGTTTTACTCATCGGATGAAAT
AAGTTTGTCAATCCCACCGCTATGCTATACAATACGCGCCTACTCTTGATGGGTCTGTAGCTCAGGGGTTAGAGCAGGGG
ACTCATAATCCCTTGGTCGTGGGTTCGAGCCCCACCGGACCCACCAATTCCCAAGCCCGGACGTATGTTTGGGCTTTTTT
GCCGCCCTGTGAAACCAAAATGCTTTGAGAAACCTTGATA

SEQ. ID NO:34
Nucleotide sequence of DNA region (1000 bp ) up-stream from the lgtB gene
from Neisseria meningitidis (serogroup B)
TAGAAAAATATTTCGCCCAATCATTAGCCGCCGTCGTGAATCAGACTTGGCGCAACTTGGAGATTTTGATTGTCGATGAC
GGCTCGACAGACGGTACGCTTGCCATTGCCAAGGATTTTCAAAAGCGGACAGCCGTATCAAAATCCTTGCACAAGCTCA
AAATTCCGGCCTGATTCCCTCTTTAAACATCGGGCTGGACGAATTGGCAAAGTCAGGAATGGGGGAATATATTGCACGCA
CCGATGCCGACGATATTGCCGCCCCCGACTGGATTGAGAAAATCGTGGGCGAGATGGAAAAAGACCGCAGCATCATCGCG
ATGGGCGCGTGGCTGGAAGTTTTGTCGGAAGAAAAGGACGGCAACCGGCTGGCGCGGCATCACAGGCACGGCAAAATTTG
GAAAAAGCCGACCCGGCACGAAGATATTGCCGACTTTTTCCCTTTCGGCAACCCCATACACAACAACACGATGATTATGA
GGCGCAGCGTCATTGACGGCGGTTTGCGTTACAACACCGAGCGGGATTGGGCGGAAGATTACCAATTTTGGTACGATGTC
AGCAAATTGGGCAGGCTGGCTTATTATCCCGAAGCCTTGGTCAAATACCGCCTTCACGCCAATCAGGTTTCATCCAAATA
CAGCATCCGCCAACACGAAATCGCGCAAGGCATCCAAAAAACCGCCAGAAACGATTTTTTGCAGTCTATGGGTTTTAAAA
CCCGGTTCGACAGCCTTGAATACCGCCAAATAAAAGCAGTAGCGTATGAATTGCTGGAGAAACATTTGCCGGAAGAAGAT
TTTGAACGCGCCCGCCGGTTTTTGTACCAATGCTTCAAACGGACGGACACGCTGCCCGCCGGCGCGTGGCTGGATTTTGC
GGCAGACGGCAGGATGCGGCGGCTGTTTACCTTGAGGCAATACTTCGGCATTTTGCACCGATTGCTGAAAAACCGTTGAA
AAACGCCGCTTTATCCAACAGACAAAAAACAGGATAAATT

SEQ. ID NO:35
Nucleotide sequence of DNA region (1000 bp ) up-stream from the lst gene from
Neisseria meningitidis (serogroup B)
GCGCACGGCTTTTTCTTCATCGGTTTGAGGGTCGGCAGGATAATCGGGGACGGCAAAGCCTTTAGACTGCAATTCTTTAA
TCGCGGCGGTCAGTTGAGGTACGGATGCGCTGATGTTCGGCAGTTTGATTACGTTTGCATCGGGCTGTTTCACCAGTTCG
CCCAATTCGGCAAGCGCGTCGGGTACGCGCTGCGCTTCGGTCAGATATTCGGGGAATGCCGCCAAAATACGGCCGGACAG
GGAAATGTCGGCAGTTTTGACATCAATATCGGCGTGGCGGGCAAACGCCTGCACAATCGGCAGCAGCGATTGGGTCGCCA
GCGCGGGGGCTTCGTCGGTATGGGTATAAACAATGGTGGATTTTTGAGTCATAGGATTATTCTCTTGTAGGTTGGTTTTT
TCTTTTGGAACACATTGCGCGGGGAATGTGCGCGGCTATTATGGCATATTTTGGCGGCTTTGTTCGCGCTTTGTTCGATC
TTGGCGTGTTTGAACGCGGCAGCGTGAAAGGAAGGGGGAAATGGTTTTCCCGCGTTTGGCGGCGGTGTCGGAGGTGCTGT
GCCTGATGTGCGGCGGCATATTTTCGGTGAAATTGATTTTATAGTGGTTTAAATTTAAACCAGTACAGCGTTGCCTCGCC
TTGTCGTACTATCTGTACTGTCTGCGGCTTCGTTGCCTTGTCCTGATTTAAATTTAAACCACTATAATATTCGGTAACTG
TCGGAATATCTGCTAAAATTCCGCATTTTTCCGCCTCGGGACACTCGGGGCGTATGTTTAATTTGTCGGAATGGAGTTTT
AGGGAT

SEQ. ID NO:36
Nucleotide sequence of DNA region (1000 bp ) up-stream from the msbB gene
from Neisseria meningitidis (serogroup B)
GCCCGACGGCGAACAGACACGTCGTGAAATCAACCGCTTGGACAGTACGGCGGCGCAATACGACATGCTTGCAGGTTATC
TTGAAAGACTTGCCGGAAAAACCGACCGTTGGGCGTGCGCCTACCGCCAAAATGCCGTCTGAACACCCGATTATCCTTTT
GAAAGCGCGATTATGCCCCATACCCTTCCCGATATTTCCCAATGTATCAGACAAAATTTGGAACAATATTTCAAAGACCT
GAACGGTACCGAACCTTGCGGCGTGTACGATATGGTCTTGCATCAGGTGGAAAAACCGCTGCTGGTGTGCGTGATGGAAC
AATGCGGCGGCAACCAGTCCAAAGCCTCCGTCATGTTGGGACTGAACCGCAATACTTTGCGTAAAAAACTGATTCAACAC
GGTTTGCTGTGAATATGTCGGCAACCGTCCGTATCTTGGGTATTGACCCGGGCAGTCGCGTAACGGGTTTCGGTGTCATC
GATGTCAGGGGGCGCGATCATTTTTACGTCGCCTCCGGCTGCATCAAAACGCCTGCCGATGCGCCTCTGGCAGACAGGAT
TGCCGTGATTGTGCGGCATATCGGCGAAGTCGTTACCGTTTACAAGCCTCAACAGGCGGCAGTGGAACAGGTGTTCGTCA
ACGTCAATCCGGCATCGACGCTGATGCTCGGTCAGGCTAGGGGCGCGGCATTGGCGGCATTGGTCAGCCATAAGCTGCCC
GTTTCGGAATACACGGCCTTGCAGGTCAAACAGGCGGTAGTCGGCAAGGGCAAGGCGGCAAAAGAACAGGTGCAGCATAT
GGTGGTGCAGATGCTGGGGCTTTCGGGAACGCCGCAGGANTGGCGGCGGACGGTCTTGCCGTCGCGCTGACCCACGCCTT
ACGCAACCACGGGCTTGCCGCCAAACTCAATCCTTCGGGGATGCAGGTCAAGCGCGGCAGGTTTCAATAGTTTCAGACGG
CATTTGTATTTTGCCGTCTGAAAAGAAAATGTGTATCGAG

SEQ. ID NO:37
Nucleotide sequence of DNA region (1000 bp ) up-stream from the htrB gene
from Neisseria meningitidis (serogroup B)
CCGCCAAGCGTTTCCCCCTTTGTCGGGCTTAACATTTGCTTTGTACGGCAGACTTTTTCCCTTCATAACGCCGCCTTTCC
GAAAAGACGATGGTAGGCGCGACGTAATTCTCAACCCTTAAGGTACGGTTGGACGAAAAGTTTTCCTTTTCATTCCACCT
GCCAACTTTTCGGCTACACCGAGTGGTCTCGTTAGGTTTGGGCGAACTACGCCCTTAAAAAAACGGACATTCTTTGCATG
CCCGTCTCTAAGGTTTCACGGTAAGTTTACCCTTATAAAGAGTTGACTTACCATACTTATCCCTTTAAAACGATATAAAG
GGCGACAGCTGTAATACAAGTATGTTGTACGGCAGACTTCTTCTACCAAACAAAAAGTTCCTTTTAGAGTTACTCGCTTA
TAGACAAATGAAGGCTTAGCCATAGGCTTCCGGTAGGCCTATTTCAACGGCTGGTTCACAGGCTACGCTAAAACCTACGG
TAGAACCGCGTTCTGGGGTTTCGCGCACAGCGGCGTCTTTGGAACCAGTTGTGTCCGAACACGCATAACCGCCCGCTTTA
ATGGTGGTGGCGGGTTCACCTGATGTAGTTTCAGCGTGCGCTTTGGTAGTTTGCGTAGCCGATGTTGAGGAGGCTCGACC
CGAAACTACGGTTGCCGACGCGCCAGCCGCACATGATGCTGGTCGTTAGAGGCCTGTAGCGGGTTCCGCACTTGCTTCCG
CTTCCGTAACTGAACTTGGTTCCGCGACCGCTGGTTCCAAACTACAAGCCGATACGGACGCTGCTTTGGGGCTGGGACTA
CGGCAAACGGTAGATAATGTCGGTGGCGGACTACGTCGCAGTTTCGCTTAATGCGTTTCTGCCGGAGGACGGAACCGACG
CAGGGCTGCGTTTTCGGGTTGACTGGCACCAAATGCTATCGCTTAGGCCGTTTCATTTTGCGTAACTATGGCAGCAGGAG
AGATACGTTGTGCTGGGCCTTTAGCCAATACTTCTCAACT

SEQ. ID NO:38

Nucleotide sequence of DNA region (1000 bp ) up-stream from the MltA gene
from Neisseria meningitidis (serogroup B)
CACAAAAACCAAGTTATGACGGGAATAAGGTACAGCAGCCAAACCAAGGCCTCGCCCTGC
GTCGGATGGTCGGTATAGCCGAAAAATCCGCCGAGCAGCACGCCCAACGGGCTGTCTTCG
TGCAAATATTTTGATGAGTCGAACACAATGTCCTGAAGCGCGTTCCAAATGCCTGCTTCG
TGCAGCGCACGCAGCGAACCGGCAAGCAGACCAGCGGCAACGATAATCAGAAACGCCCCT
GTCCAACGGAAAAACTTCGCCAGATTCAGGCGCATCCCACCCTGATAAATCAACGCGCCA
ATCACGGCGGCAGCCAAAACCCCCGCTACCGCACCGGCCGGCATCTGCCACGTCGGGCTC
TGTTTGAATACGGCAAGCAGGAAAAAAACGCTCTCCAAACCTTCGCGCGCCACGGCAAGA
AACGCCATACCGACCAAGGCCCATCCTTGACCGCTGCCACGGTTCAAAGCCGCCTGCACA
GAATCCTGAAGCTGCCGCTTCATCGAACGGGCGGCTTTTTTCATCCATAAAATCATATAA
GTCAGCATCGCGACAGCAACCAAACCGATAATGCCGACGACGAACTCCTGCTGCTTCTGG
GGAATCTCGCCCGTTGCCGAATGGATTCCGTACCCCAGCCCCAAACACATCAAAGAAGCA
AGAACAACCCCGAACCAGACCTTAGGCATCAGTTTGGAATGTCCGGACTGTTTCAGAAAA
CCGGCAACGATGCCGACGATGAGCGCGGCTTCGATACCCTCGCGCAACATAATTAAAAAA
GCGACCAGCATAAACGCGAACGAACAAGGATGATGAATAATATATTATCGGAATATTTTC
ATTGCTTGTAAATACAAATGCAAGTTATTTTTATCTGCAGTACCGCGCGGCGGAAAGTTC
CGCAGCTGCAGCTGCGCCCTGTGTTAAAATCCCCTCTCCACGGCTGCCGCAACGCCGCCC
GAAACCATCTTTCTTATTACTGCCGGCAACATTGTCCATT

SEQ. ID NO:39
Nucleotide sequence of DNA region (1000 bp ) up-stream from the ompCD gene
from Moraxella catarrhalis
GCTGATTTGTGAGCAAGCGGGCGCATCAGGGATTACCTTGCATTTGCGAGAAGATCGTCG
ACATATTCAAGATGAAGATGTTTATGAATTGATTGGGCAATTGACAACACGCATGAATCT
TGAGATGGCAGTCACTGATGAGATGCTAAATATTGCCCTAAAGGTACGACCAGCATGGGT
GTGTTTAGTACCAGAAAAACGCCAAGAGCTGACTACAGAAGGTGGGCTTGATATCGCCAA
TTTATCAAATATTCAAGCATTTATACACAGTCTTCAGCAGGCGGATATTAAGGTTTCTTT
ATTCATCGATCCAGATCCGCATCAAATTGATGCTGCAATTGCTTTGGGTGCTGATGCGAT
TGAGCTGCATACGGGAGCTTATGCTCAAGCGACTTTACAAAATAATCAAAAGCTTGTTGA
TAAAGAGCTTGACCGTATTCAAAAAGCCGTTGCAATGGCACAAAAAAAATCATCATTATT
GATTAATGCAGGTCATGGTTTGACGCGTGATAATGTTGCAGCGATTGCCCAAATTGATGG
TATTCATGAGCTGAATATCGGGCATGCATTGATTTCAGATGCGATATTTATGGGGCTTGA
TAATGCAGTCAAGGCAATGAAAATGGCTTTTATTCAAGATAAAACGACCAATCATTGATG
CGTTAGAAAGAAAATCGTAAATAATGATGACTATTGTGTAATATTATGTATTTTTGTTCA
AAAAAAGGTTGTAAAAAAATTCATTTACCATTAAGCTAAGCCCACAAGCCACAATGAATA
CCTATTGGTTTGACTCATTAGTCACTAAGAATCTGCAAAATTTTGTAACAGATTATTGGC
AGGTCTTGGATCGCTATGCTAAAATAGGTGCGGTAATCTTGAAAAACCAACCATTCCTTG
GAGGAATTTATGAAAAAGGGATATAAACGCTCTTGCGGTCATCGCAGCCGTTGCAGCTCC
AGTTGCAGCTCCAGTTGCTGCTCAAGCTGGTGTGACAGTC

SEQ. ID NO:40
Nucleotide sequence of DNA region (1000 bp ) up-stream from the copB gene
from Moraxella catarrhalis
GATGCTGTTAAAGTGGGTATTGGTCCTGGTTCTATTTGTACAACCCGTATTGTTGCAGGC
ATTGGCGTCCCGCAGATAAGTGCCATTGATAGTGTGGCAAGTGCGTTAAAAGATCGCATT
CCTTTGATTGCCGATGGCGGTATTCGTTTTTCGGGTGATATCGCCAAAGCCATCGCAGCA
GGCGCTTCATGTATTATGGTGGGTAGCTTGTTGGCAGGTACCGAAGAAGCACCTGGTGAG
GTGGAATTATTCCAAGGTCGTTATTATAAGGCTTATCGTGGTATGGGCAGCTTGGGGGCA
ATGTCTGGTCAAAATGGCTCATCGGATCGTTATTTTCAAGATGCCAAAGATGGTGTTGAA
AAACTGGTTCCAGAGGGTATCGAAGGCCGTGTTCCTTATAAAGGCCCTGTGGCAGGCATC
ATCGGTCAATTGGCAGGTGGTCTAAGATCATCCATGGGTTATACAGGTTGCCAGACCATC
GAACAGATGCGTAAGAATACCAGCTTTGTCAAAGTGACTTCCGCAGGCATGAAGGAATCG
CATGTACACGATGTACAGATTACCAAAGAAGCACCCAATTATCGCCAAAATTAACTCTAT
TAATAGCAAATACAAGCACTCATTAGATAGGGTGGGTGCTTTTTAGAGCATAAAAAATAA
ACTGACACATGACTTATTGTCATATTTTTAAAATGCTTTTAATTTAGATTTTTAATTTAG
ATAATGGCTAAAAATAACAGAATATTAATTTAAAGTTTTCAAAATCAAGCGATTAGATGA
AATTATGAAAATAAATAACAATAATTCTGATTTATTTTAACCAATAATATCAATTATCAT
TTACAAGAAAAAATTTTTTTTGATAAAATTCTTACTTGTACCTTGCTATTTTTTCTTATTT
ATCATTTTTGGCGGTATTTTCGTTGATTTTAGTAAGTAGATGAGCAAGGGATAATTTGAC
AAAAACAAATTTGATTTCAAGCCTCATAATCGGAGTTATT

SEQ. ID NO:41
Nucleotide sequence of DNA region (1000 bp ) up-stream from the D15 gene
from Moraxella catarrhalis
AAAACTGGTGATGTCTTCACTGCTATTCATGGTGAACCAATCAATGATTGGCTAAGTGCC
ACCAAGATTATTCAGGCAAATCCAGAAACCATGCTTGATGTGACAGTCATGCGTCAAGGT
AAGCAGGTTGATTTAAAATTAATGCCCCGTGGTGTAAAGACACAAAACGGCGTAGTCGGT
CAACTGGGTATTCGCCCCCAGATTGATATCGATACGCTCATTCCTGATGAATATCGTATG
ACGATTCAATATGATGTCGGTGAGGCATTTACTCAAGCCATCCGACGAACTTATGATTTA
TCAATAATGACCTTAGATGCGATGGGTAAGATGATTACAGGATTGATTGGCATTGAAAAT
CTATCAGGTCCCATTGCCATTGCCGATGTTTCTAAGACCAGTTTTGAGTTGGGATTTCAA
GAAGTGTTATCGACAGCCGCAATCATCAGTTTAAGCTTGGCAGTACTGAATCTTTTACCC

ATTCCAGTGTTAGATGGCGGGCATTTGGTATTTTATACTTATGAATGGATTATGGGCAAA
TCTATGAATGAAGCGGTGCAGATGGCAGCATTTAAAGCGGGTGCGTTATTGCTTTTTTGT
TTCATGTTACTTGCAATCAGTAACGATATCATGCGATTTTTTGGCTAAGTTCTGATTTAT
CGTACCATTAACAAAATTTTTGGCTTTTTTAAGCTGAAATACTTGCCAAATTTAACTTTT
TGGCTTACCTTTACACAATATAAATTTGGGTGTAGAAAATTTTGGATACATTTTTATACC
TTATTTTTAGAAATTTTAAAAATTAAGTTTGGATAGACTTATGCGTAATTCATATTTTAA
AGGTTTTCAGGTCAGTGCAATGACAATGGCTGTCATGATGGTAATGTCAACTCATGCACA
AGCGGCGGATTTTATGGCAAATGACATTGCCATCACAGGACTACAGCGAGTGACCATTGA
AAGCTTACAAAGCGTGCTGCCGTTTCGCTTGGGTCAAGTG

SEQ. ID NO:42
Nucleotide sequence of DNA region (1000 bp ) up-stream from the omplA gene
from Moraxella catarrhalis
ACTTGGCGAAAATACCATTTATATCGATTGTGATGTTATACAGGCAGATGGCGGTACACG
CACAGCCAGTATCAGTGGTGCTGCGGTGGCACTTATTGATGCTTTAGAACACTTGCAGCG
TCGTAAAAAGCTTACCCAAGATCCGCTTTTGGGCTTGGTGGCAGCGGTTTCTGTGGGTGT
TAATCAAGGCCGTGTATTGCTTGATTTGGATTATGCTGAAGATTCAACTTGTGATACCGA
TTTAAATGTGGTCATGACGCAGGCAGGTGGGTTTATTGAGATTCAAGGCACAGCAGAAGA
AAAGCCATTTACTCGTGCTGAAGCTAATGCGATGCTTGATTTGGCAGAGCTGGGAATTGG
GCAGATTATCGAAGCCCAAAAGCAAGTATTAGGCTGGTGATATGCTAATCGTTGAAGATA
ATGGCGTGATCATCACATTAAATGGACAAGTAAAAGACCCATTATTTTGGTGGTCGATGA
TATTGCTGCTGCTGGGTGTCTTGGTGGCAATCATTTGTTTGATTGCACCCGTTTTTTATG
CAATCGGTGCGTTGGCTTTATTTGCAGTTGTGGTATTTGTGTTTAATATTCAAAGGCAAA
AAGCCAAAACTTGTCATATGTTTTCACAAGGTCGCTTGAAGATTACGTCCAAACGCTTTG
AGATTCATAACAAATCACTAACCTTATCAGCATCGGCAACAATATCTGCTAAAGATAACA
AAATGACAATTGTTGATCGGGGCATTGAATATCATTTTACAGGTTTTGCTGATGACCGTG
AAATTAATATAGCCAAACAGGTACTTTTGGGAAAGTCAATCAAAACCAATGCGGTGGCGG
TAACATTGGCTAAGTAGTTGTTGTGATACAGACAGGTTGGATGGTCTTTAACTCCACCCA
CCTAACTTTTTCTTTGTTTGGATTTAAGAGTATGTTATGATGGGCAGGATTTTATTTTAA
GTCATCATTTAATGCAATCAGTTGTCCAGAGTAGCCGTTC

SEQ. ID NO:43
Nucleotide sequence of DNA region (1000 bp ) up-stream from the hly3 gene
from Moraxella catarrhalis
GTGATCGGCAACACCCCACCATTCAGGAGCAACCAAAATTGCCCGTGCCTTGCCTGTCTT
GGTGGTATCATTTGGCAGGGCAATGTGGCTAAGTAGTGGTGTGCCATCAGGTGCGGTGGT
GGTGAGTGTACGATTCGTTATTGTCATAAAATTATCCTTTTGGGTTGGATGATATCAATG
AAATACCCTACGGTTGTATGGAATTTTATCCATTGTACCACGGTATTGGTCTTTTTAAAT
TAACAAGCAGCTTCTAGCAAGTCAAAGTTTTTATGCCTATTTTTTCAGATTTTAAGGTAC
AATAAAGCCAATTGTTAATAATATGGTATTGTCATGATTTATGATGAATTGCGACCAAAA
TTTTGGGAAAATTATCCCTTAGATGCGTTAACAGATGCTGAATGGGAAGCATTATGTGAC
GGATGTGGCGCGTGTTGTTTGGTGAAATTTCTTGATGATGACAATGTTAAATTGACCGAA
TATACCGATGTTGCCTGCCAGCTATTGGATTGCTCAACAGGATTTTGCCAAAACTATGCC
AAGCGTCAAACGATTGTGCCAGATTGTATTCGCTTAACACCTGATATGCTGCCTGATATG
CTGTGGTTGCCACGCCATTGTGCTTATAAGCGGTTGTATCTTGGGCAAAATCTGCCAGCA
TGGCACAGGCTCATTAAACATAGCCAAAACCATGGTGCAGGATTTGCGAAAGTTTCAACT
GCTGGGCGATGTGTGAGTGAGCTTGGTATGAGTGATGAAGACATAGAAAGGCGAGTGGTG
AAATGGGTTAAACCTTGACATGATTGTTGACATGATTGACAGACAATAAAAATTGGCAAA
TTTGATAAAATTGGTGTATGTGTGTGATTTTATCAAAAGCACTTGAATAAAACCGAGTGA
TACGCTAAATTGTAGCAAACCAATCAATTCATCATAATTTTAATGAACACGAGGTTAAAT
TATACTGTCTATGTCTGATGACAATTCAAGCACTTGGTCG

SEQ. ID NO:44
Nucleotide sequence of DNA region (1000 bp ) up-stream from the lbpA gene
from Moraxella catarrhalis
TAACAAAGGCAACCCAACACGCAGTTATTTTGTGCAAGGCGGTCAAGCGGATGTCAGTAC
TCAGCTGCCCAGTGCAGGTAAATTCACCTATAATGGTCTTTGGGCAGGCTACCTGACCCA
GAAAAAAGACAAAGGTTATAGCAAAGATGAGGATACCATCAAGCAAAAAGGTCTTAAAGA
TTATATATTGACCAAAGACTTTATCCCACAAGATGACGATGACGATGACGATGACGATAG
TTTGACCGCATCTGATGATTCACAAGATGATAATACACATGGCGATGATGATTTGATTGC
ATCTGATGATTCACAAGATGATGACGCAGATGGCGATGACGATTCAGATGATTTGGGTGA
TGGTGCAGATGATGACGCCGCAGGCAAAGTGTATCATGCAGGTAATATTCGCCCTGAATT
TGAAAACAAATACTTGCCCATTAATGAGCCTACTCATGAAAAAACCTTTGCCCTAGATGG
TAAAAATAAGGCTAAGTTTGATGTAAACTTTGACACCAACAGCCTAACTGGTAAATTAAA
CGATGAGAGAGGTGATATCGTCTTTGATATCAAAAATGGCAAAATTGATGGCACAGGATT
TACCGCCAAAGCCGATGTGCCAAACTATCGTGAAGAAGTGGGTAACAACCAAGGTGGCGG
TTTCTTATACAACATCAAAGATATTGATGTTAAGGGGCAATTTTTTGGCACAAATGGCGA
AGAGTTGGCAGGACGGTTACATCATGACAAAGGCGATGGCATCACTGACACCGCCGAAAA
AGCAGGGGCTGTCTTTGGGGCTGTTAAAGATAAATAAAGCCCCCCTCATCATCGTTTAGT
CGCTTGACCGACAGTTGATGACGCCCTTGGCAATGTCTTAAAACAGCACTTTGAAACAGT
GCCTTGGGCGAATTCTTGGATAAATGCACCAGATTTGCCTCGGGCTAATATCTTGATAAA
ACATCGCCATAAAATAGAAAATAAAGTTTAGGATTTTTTT

SEQ. ID NO:45
Nucleotide sequence of DNA region (1000 bp ) up-stream from the lbpB gene
from Moraxella catarrhalis
CAGCTTGTACCATTTGGTGAATATATACCATTTGGTGGTTTGTTGGATATTTTACCAGGG
CTTGAGGGTGTCGCTAGCCTAAGCCGTGGCGATGATAAGCAACCACCGCTCAAATTGGGC
GGCGGCGTGGGCGATACGATTGGTGCGGCAATTTGTTATGAGGTGGCATATCCTGAGACG
ACGCGTAAAAATGCACTTGGCAGTAATTTTTTATTAACCGTCTCAAACGATGCTTGGTTT
GGTACAACAGCAGGTCCTTTGCAGCATTTACAAATGGTGCAAATGCGAAGCTTGGAGACG
GGCGATGGTTTGTGCGTGCAACAAACAACGGAGTGACTGCATTAATTGACCATCAAGGA
CGGATTATCAAGCAGATACCGCAGTTTCAGCGAGATATTTTGCGAGGTGATGTACCCAGT
TATGTTGGACACACGCCTTATATGGTTTGGGGGCATTATCCCATGTTGGGGTTTTCTTTG
GTGCTGATTTTTCTTAGTATCATGGCAAAGAAAATGAAAAATACCACCGCCAAACGAGAA
AAATTTTATACCGCTGATGGTGTGGTAGACCGCTGAATTGTGCCACTTTGGGCGTTAGAG
CATGAGCAAGATTAGGCGTTGGGTGAGCTTTGGTTGTATTACTCATCAGCCTACCCGAAA
CCTGCCAAACATCACCGCCCAAAACCTAAACATACAATGGCTAAAAATATCAGAAAATAA
CTTGCTGTATTGTAAATTCTTATGTTATCATGTGATAATAATTATCATTAGTACCAAGAT
ATCCATTACTAAACTTCATCCCCCATCTTAACAGTTACCAAGCGGTGAGCGGATTATCCG
ATTGACAGCAAGCTTAGCATGATGGCATCGGCTGATTGTCTTTTTGCCTTGTTGTGTGTT
TGTGGGAGTTGATTGTACTTACCTTAGTGGTGGATGCTTGGGCTGATTTAATTAAATTTG
ATCAAAGCGGTCTTCACAACACACCAAACGAGATATCACC

SEQ. ID NO:46
Nucleotide sequence of DNA region (1000 bp ) up-stream from the tbpB gene
from Moraxella catarrhalis
AGTTTGCCCTGATTTTGAGAGCCACTGCCATCATGAATTTGTTGGCGTAAACACCACTCG
TATTCTTCTTCGGTTTCCCCTTTCCATGCAAACACAGGGATACCAGCGGCCGCCATGGCA
GCGGCGGCGTGGTCTTGGGTGCTAAAAATATTGCATGATGTCCAGCGAACTTCTGCACCC
AAGGCAACCAAAGTCTCAATCAGCACCGCTGTTTGAATGGTCATGTGGATACAGCCTAGG
ATTTTAGCACCCTTAAGTGGTTGCTGGTCTTGATAGCGTTTTCTTAACCCCATCAGGGCT
GGCATCTCAGCTTCTGCCAAGGCAATCTCACGGCGACCATAATCGGCTAAACGGATATCA
GCGACTTTATAATCGGTGAAGTTTTGGGTGGTACTTGGATTGATTGAGGTAGGCATATCT
TTATTCCTAAGCTATTTTAAAGTATTTTTAACAATAATTTTGATGAATTTGAGATAATTG
ATGCTAAAAGGTTGAATGACCAAACCATCGCTAACAATCAAGAAAAGACATTTTAAGCAT
AAAAAGCAAATGTGTCTTGATGGCTTATTATAACAGTTATTATGATAAATTTGGGTAGAA
AGTTAAATGGATCGTTGGGTAAGTTTGTTGGCTATCCTTAATTAATTATAATTTTTTAAT
AATGCTTTTACTTTATTTTAAAAATAGAGTAAAAAATGGTTGGCTTTGGGTTTTTATCTC
ACTATGGTAGATAAAATTGATACAAAATGGTTTGTATTATCACTTGTATTTGTATTATAA
TTTTACTTATTTTTACAAACTATACACTAAAATCAAAAATTAATCACTTTGGTTGGGTGG
TTTTAGCAAGCAAATGGTTATTTTGGTAAACAATTAAGTTCTTAAAAACGATACACGCTC
ATAAACAGATGGTTTTTGGCATCTGCAATTTGATGCCTGCCTTGTGATTGGTTGGGGTGT
ATCGGTGTATCAAAGTGCAAAAGCCAACAGGTGGTCATTG

SEQ. ID NO:47
Nucleotide sequence of DNA region (1000 bp ) up-stream from the tbpA gene
from Moraxella catarrhalis
TTGGGGGCGGATAAAAAGTGGTCTTTGCCCAAAGGGGCATATGTGGGAGCGAACACCCAA
ATCTATGGCAAACATCATCAAAATCACAAAAAATACAACGACCATTGGGGCAGACTGGGG
GCAAATTTGGGCTTTGCTGATGCCAAAAAAGACCTTAGCATTGAGACCTATGGTGAAAAA
AGATTTTATGGGCATGAGCGTTATACCGACACCATCGGCATACGCATGTCGGTTGATTAT
AGAATCAACCCAAAATTTCAAAGCCTAAACGCCATAGACATATCACGCCTAACCAACCAT
CGGACGCCCAGGGCTGACAGTAATAACACTTTATACAGCACATCATTGATTTATTACCCA
AATGCCACACGCTATTATCTTTTGGGGGCAGACTTTTATGATGAAAAAGTGCCACAAGAC
CCATCTGACAGCTATGAGCGTCGTGGCATACGCACAGCGTGGGGGCAAGAATGGGCGGGT
GGTCTTTCAAGCCGTGCCCAAATCAGCATCAACAAACGCCATTACCAAGGGGCAAACCTA
ACCAGTGGCGGACAAATTCGCCATGATAAACAGATGCAAGCGTCTTTATCGCTTTGGCAC
AGAGACATTCACAAATGGGGCATCACGCCACGGCTGACCATCAGTACAAACATCAATAAA
AGCAATGACATCAAGGCAAATTATCACAAAAATCAAATGTTTGTTGAGTTTAGTCGCATT
TTTTGATGGGATAAGCACGCCCTACTTTTGTTTTTGTAAAAAAATGTGCCATCATAGACA
ATATCAAGAAAAAAATCAAGAAAAAAAGATTACAAATTTAATGATAATTGTTATTGTTTAT
GTTATTATTTATCAATGTAAATTTGCCGTATTTTGTCCATCACAAACGCATTTATCATCA
ATGCCCAGACAAATACGCCAAATGCACATTGTCAACATGCCAAAATAGGCATTAACAGAC
TTTTTAGATAATACCATCAACCCATCAGAGGATTATTTT

SEQ. ID NO:48
Nucleotide sequence of DNA region (1000 bp ) up-stream from the ompE gene
from Moraxella catarrhalis
AAAGACATTACACATCATCATTCAAACGCCCAACCATGTACCTCTGCCCCGTGGTCGCAC
GCCAACGCTTTTTGATGCGGTGCGTTGGGTTCAGATGGCTTGTCAATCATTTGGTTTTAT
TAAAATTCATACCTTTGGTAGTTTGGCTTTACCTGATATGTCATTTGATTATCGAAACAA
TACGCAGTTGACCAAACATCAATTTTTAGCCATTTGCCAAGCACTCAATATTACCGCTCA
TACGACCATGCTTGGTATTAAATCATCACATAAAGATACTTTACATCCATTTGAATTGAC

ATTACCCAAATACGGCCATGCCTCAAATTATGATGATGAATTGGTGCAAAACAATCCATT
GGCTTATTTTCATCAACTGTCTGCCGTCTGCCGATATTTTTATACCCAAACGGTTTGTAT
TGTTGGCGGTGAAAGCTCAGGGAAAACTACCTTGGTGCAAAAACTTGCCAATTATTATGG
TGCCAGCATCGCACCTGAAATGGGTCGATTATACACACACTCCCATCTCGGCGGTAGCGA
ACTTGCCCTTCAATACAGCGACTACGCATCCATTGCCATCAATCACGCCAACGCTATCGA
AACCGCTCGTACCACTGCCAGCTCTGCTGTTACACTGATTGATACTGATTTTGCGACAAC
GCAAGCATTTTGTGAAATTTATGAAGGGCGAACGCATCCGCTTGTCGCAGAATTTGCTAA
ACAAATGCGATTGGATTTTACGATTTATTTAGATAATAATGTTGCTTGGGTCGCTGATGG
CATGCGTAGGCTTGGTGATGATCATCAACGCAGTTTGTTCGCCAATAAATTGCTTGAGAT
TTTGGCACGATATGATATTAGTTATCATATCATTAATGACACCGACTACCACAAACGCTA
TCTACAAGCATTAAGCTTGATAGACAATCATATTTTTAATCATTTTACAAAAATTCATGA
CAATTAATTAGGGAAAATCTGATGAAAATTGATATTTTAG

SEQ. ID NO:49
Nucleotide sequence of DNA region (1000 bp ) up-stream from the uspa1 gene
from Moraxella catarrhalis
GGATGTGGCATATCTGCCCATCGACCCAATACACATCGGTCGAGGCTATCAAGATGTGGT
ACGAATTAATAGCCAGTCAGGTAAGGGCGGTGCTGCGTATATCTTGCAGCGGCATTTTGG
TTTTAATTTACCACGCTGGACACAGATTGATTTTGCTCGTGTGGTACAGGCTTATGCAGA
AAGTATGGCGCGTGAACTAAAAACTGATGAGCTGCTTGAAATTTTTACCCAAGCGTATCT
TAAGCAAGATAAATTCCGCCTAAGTGACTATACCATCAGCAATAAAGGCGATGCTGTCAG
CTTCCAAGGCCAAGTAGCGACACCCAAAGCGGTGTTTGAGGTGATTGGTCAAGGCAATGG
TGCGTTATCTGCGTTCATTGATGGCTTGGTGAAATCCACAGGCAGACAGATTCATGTCAC
CAATTACGCCGAACACGCCATCGATAACAAAACCCATCAAAAAACCGATACGGATAACCA
AACCGATGCCGCCGTGCCGCTTATATCCAGCTGTCGGTAGAGGGGCAGATTTATTCAGGC
ATCGCCACTTGCCATAGCACCGTATCCGCCATGCTAAAAGGTGCATTATCCGCTTTGGCA
CAGGCGTGGTAATCTGACCCAATCAAAATCCTGCATGATGGCAGGATTTTATTATTTAGT
GGGCTGCCCAACAATGATGATCATCAGCATGTGAGCAAATGACTGGCGTAAATGACTGAT
GAGTGTCTATTTAATGAAAGATATCAATATATAAAAGTTGACTATAGCGATGCAATACAG
TAAAATTTGTTACGGCTAAACATAACGACGGTCCAAGATGGCGGATATCGCCATTTACCA
ACCTGATAATCAGTTTGATAGCCATTAGCGATGGCATCAAGTTGTGTTGTTGTATTGTCA
TATAAACGGTAAATTTGGTTTGGTGGATGCCCCATCTGATTTACCGTCCCCCTAATAAGT
GAGGGGGGGGGAGACCCCAGTCATTTATTAGGAGACTAAG

SEQ. ID NO:50
Nucleotide sequence of DNA region (1000 bp ) up-stream from the uspa2 gene
from Moraxella catarrhalis
CCCCAAGCTTTCCGTTTGTGTGCCTGCTGGTGTCGGGCGGTCATACCATGCTGGTGCGTG
CCGATGGTGTGGGCGTGTATCAGATATTGGGCGAGTCTATCGATGATGCGGTGGGTGAAT
GCTTTGATAAAACGGCAAAAATGCTCAAACTGCCCTATCCTGGTGGCCCAAATATCGAAA
AATTAGCCAAAAACGGCAACCCACACGCCTATGAGCTGCCAAGACCCATGCAGCATAAAG
GGCTGGATTTTTCGTTCAGTGGCATGAAAACCGCCATTCATAATCTCATCAAAGACACAC
CAAACGCCCAAAGCGACCCCGCCACACGAGCAGACATCGCCGCAAGCTTTGAGTATGCGG
TGGTGGATACTTTGGTCAAAAAATGCACCAAAGCACTACAGATGACAGGCATTCGCCAGC
TGGTGGTCGCAGGGGGCGTCTCTGCCAATCAGATGCTACGCCGCACCCTGACCGAGACGC
TCCGCCAAATCGATGCGTCGGTGTACTATGCCCCGACCGAGCTATGCACGGATAATGGTG
CGATGATCGCCTATGCTGGCTTTTGTCGGCTCAGCTGTGGACAGTCGGATGACTTGGCCG
TTCGCTGTATTCCCCGATGGGATATGACGACGCTTGGCGTATCGGCTCATAGATAGCCAC
ATCAATCATACCAACCAAATCGTACAAACGGTTGATACATGCCAAAAATACCATATTGAA
AGTAGGGTTTGGGTATTATTTATGTAACTTATATCTAATTTGGTGTTGATACTTTGATAA
AGCCTTGCTATACTGTAACCTAAATGGATATGATAGAGATTTTTCCATTTATGCCAGCAA
AAGAGATAGATAGATAGATAGATAGATAGAACTCTGTCTTTTATCTGTCCGCTGATGCTT
TCTGCCTGCCACCGATGATATCATTTATCTGCTTTTTAGGCATCAGTTATTTCACCGTGA
TGACTGATGTGATGACTTAACCACCAAAAGAGAGTGCTAA

SEQ. ID NO:51
Nucleotide sequence of DNA region (1000 bp ) up-stream from the omp21 gene
from Moraxella catarrhalis
GAGTGAACTTTATTGTAAAATATGATTCATTAAAGTATCAAAATCATCAAACGCAGCATC
AGGGTTTGCTAAATCAATTTTTTCACCATAATTATAGCCATAACGCACAGCAAGCGTAGT
TATGCCAGCGGCTTGCCCTGATAAAATATCATTTTTGGAATCACCAACCATAATGGCATC
AGTCGGTGCGATGCCCAGTGATTGACACAGGTATAATAAAGGCGTTGGGTCGGGCTTTTT
GACGCTGAGCGTATCACCGCCAATCACTTGGTCAAACAGTGTCAGCCATCCAAAATGTGA
TAAAATTTTAGGCAAATAACGCTCAGGCTTATTGGTACAAATTGCCAAATAAAACCCCGC
TGCTTTTAATCGTTCAAGCCCTTGTATAACCCCTGCATAGCTTTGCGTATTTTCAATTGT
TTTATGGGCATATTCTGCCAAAAATAACTCATGGGCATGGTGAATCATAGTCGTATCATA
GATATGATGTGCTTGCATTGCTCGCTCAACCAATTTTAGCGAACCATTGCCCACCCAGCT
TTTGATGATATCAATTGGCATAGGCGGTAAGTTAAGCTTGGCATACATGCCATTGACCGC
CGCCGCCAAATCAGGGGCACTATCGATAAGCGTACCATCCAAATCAAATATAATCAGTTT
TTTGCCAGTCATTGACAGTGTTTGCATGCTTTTTCCTTATTCTTAAAATTGGCGGCTGTT
TGGTATTTTTTAAATCAGTCAATTTTTACCATTTGTCATATAATGACAAAGTACAAATTT
AGCAATATTTTAGTGCATTTTTTGGCGAAGTTTTATGAAAACTGGTCATTGGTTGCAAAA
CTTTACACAGTACCTATAAAACTTGCACAGTTAATAAGAAATATTTTGTTACTATAGGGG

CGTCATTTGGAACAAGACAGTTATTTGTAAATAGTTATTTGCAAAAGACGGCTAAAAGAC
AGAACAGCGTTTGTTTCAGTGATTAACTAGGAGAAAAACA

SEQ. ID NO:52
Nucleotide sequence of DNA region (1000 bp ) up-stream from the omp106 gene
from Moraxella catarrhalis
TTGATCGGTTTTGCCCCACTGTTTCATGATTTACTCAAAACAGGCGGCTTGATCGTGCTG
GCAGGTCTGACCCAAAACCAAACCCAAGCGGTCATCGATGCCTACTCGCCTTATGTTACG
CTTGATACGCCATTTTGTTATGCAGATGCCCAAGACTGCCATTGGCAACGCCTAAGCGGC
ATCAAACCTACCAACCCATAAGCGATATGCCATGAGCCACAAACCTAAGCCAACACCGCT
ATATCAACAAGTTGAGCAGACCGCCAAGCGTTATTTTGAGACATTGGGCGATGCTCATAC
TCATGATGTCTATGCCACTTTTTTGGCCGAATTTGAAAAACCGCTGCTCATCGCCGCACT
CAATCACACGCACGGCAATCAGTCAAAAACCGCCCAAATCCTTGGTATCAATCGTGGCAC
ATTACGCACCAAAATGAAAACCCATCACTTACTTTAGACCGCCAGTTATCGCCATGGATA
TGGGCAGGTGTGCTCGCCTGCCGTATGATGGCGATGACACCCCATTTGCCCCATATCTGC
ACGATTTGACATGATTTAACATGTGATATGATTTAACATGTGACATGATTTAACATTGTT
TAATACTGTTGCCATCATTACCATAATTTAGTAACGCATTTGTAAAAATCATTGCCCCCT
TTTTTTATGTGTATCATATGAATAGAATATTATGATTGTATCTGATTATTGTATCAGAAT
GGTGATGCCTACGAGTTGATTTGGGTTAATCACTCTATTATTTGATATGTTTTGAAACTA
ATCTATTGACTTAAATCACCATATGGTTATAATTTAGCATAATGGTAGGCTTTTTGTAAA
AATCACATCGCAATATTGTTCTACTGTTACCACCATGCTTGAATGACGATCCAAATCACC
AGATTCATTCAAGTGATGTGTTTGTATACGCACCATTTACCCTAATTATTTCAATCAAAT
GCCTATGTCAGCATGTATCATTTTTTTAAGGTAAACCACC

SEQ. ID NO:53
Nucleotide sequence of DNA region (1000 bp ) up-stream from the HtrB gene
from Moraxella catarrhalis
ACTATTCTGCTTTTTGTTTTTCACGAATGCGAATGCCCAACTCACGCAACTGGCGATTAT
CAACTTCAGCAGGTGCTTCGGTCAATGGGCAATCTGCCGTCTTGGTTTTTGGGAAGGCGA
TCACATCACGGATTGAGCTGGCACCAACCATCAGCATAATCAGGCGATCTAGACCAAATG
CCAAACCACCGTGCGGCGGTGCACCAAAACGCAATGCATCCATCAAAAACTTAAACTTAA
GCTCTGCTTCTTCTTTAGAAATACCCAAGGCATCAAATACCGCCTCTTGCATGTCAACCG
TATTAATACGCAGCGAACCGCCACCAATTTCTGTGCCATTTAGTACCATGTCATAGGCAA
TGGATAGGGCGGTTTCGGGACTTTGTTTGAGTTCCTCAACCGAGCCTTTTGGGCGTGTAA
AAGGATGATGAACTGATGTCCACTTACCATCATCAGTTTCCTCAAACATTGGAAAATCAA
CGACCCAAAGCGGTGCCCATTCACAGGTAAATAAATTTAAATCAGTACCGATTTTAACAC
GCAATGCACCCATAGCATCATTGACGATTTTGGCTTTATCGGCACCAAAGAAAATGATAT
CGCCAGTTTGGGCATCGGTACGCTCAATCAGCTCAATCAAAACCTCATCGGTCATATTTT
TAATGATGGGTGATTGTAATCCTGATTCTTTTTCAACGCCATTATTGATATTGCTTGCGT
CATTGACCTTAATATATGCCAATCCACGAGCGCCATAAATACCAACAAATTTGGTGTACT
CATCAATCTGCTTGCGACTCATGTTACCGCCATTTGGAATGCGTAAGGCAACAACACGGC
CTTTAGGATCTTGGGCGGGCCCTGAAAATACTTTAAATTCAACATGTTGCATGATGTCAG
CAACATCAATAAGTTTTAAGGGAATGCGTAAATCAGGCTTATCTGAGGCATAATCACGCA
TGGCATCTGCGTAAGTCATGCGGGGGAAGGTATCAAACTCA

SEQ. ID NO:54
Nucleotide sequence of DNA region (1000 bp ) up-stream from the MsbB gene
from Moraxella catarrhalis
TGGATCATATTCTTTATTAATGGTACTGTTTAAACCTGTATTTTAAAGTTTATTGGGTCA
TATTTTCAAGCTCATCCCATCGCTCAAGCTTCATCATCAAAAGCTCATCAATCTCTACCA
ATCGCTCACCAGCCTTCGTTGCTGCCGCCAAATCGGTATTAAACCATGAACCATCTTCAA
TCTTTTTGGCAAGCTGTGCCTGCTCTTGTTCAAGTGCAGCAATTTCATTAGGCAAATCTT
CAAGTTCACGCTGCTCTTTATAGCTGAGTTTGCGTTTTTGGGCAACGCCTGATTGAGGTG
GTTTGATTTGGATGGGTTCAGCGGGTTTTGTCGCCTTAGGTTTATTGTCTGTGGCGTGAT
GAGCAAGCCATCTTTCATGCTGTTGTACATAGTCTTCATAACCGCCAACATATTCCAAAA
CGATACCGTCGCCGTACTTATCAGTATCAAATACCCAAGTTTGGGTAACAACATTATCCA
TAAAAGCACGGTCATGGCTGATGAGTAATACCGTGCCTTTAAAATTGACCACAAAATCTT
CTAAAAGCTCAAGTGTTGCCATATCCAAATCATTGGTAGGCTCATCAAGCACCAAAACAT
TGGCAGGTTTTAGCAATAATTTGGCCAATAAAACGCGTGCTTTTTCACCGCCTGATAGTG
CTTTAACAGGTGTGCGAGCACGATTTGGCGTGAATAAAAAATCTTGCAAATAGCTTAAAA
TGTGCGTAGTTTTTCCACCAACATCGACATGGTCAGAGCCTTCTGAAACATTATCTGCGA
TAGATTTTTCAGGGTCTAGGTCGTCTTTGAGTTGGTCAAAAAAAGCAATATTTAGATTGG
TGCCAAGCTTAACTGAACCTGACTGAATCGCTGAATCATCCAAACCCAAAATGCTTTTAA
TTAAGGTTGTTTTACCAACGCCATTTTTGCCAATGATACCAACTTTATCACCACGAACAA
GCAGCGTTGAAAAATCCTTAACTAAGGTTTTATTGTCGTAT

SEQ. ID NO:55
Nucleotide sequence of DNA region (1000 bp ) up-stream from the PilQ gene
from Moraxella catarrhalis
CAACTTGAAAATCAGCTCAATGCTCTGCCACGCACAGCACCGATGAGCGAGATTATCGGA

ATGATAAATACCAAAGCACAAGCGGTTAATGTGCAGGTGGTGAGTGCATCAGTTCAAGCA
GGTCGTGAACAGGATTATTATACCGAACGCCCTATCGCAGTGAGTGCGACAGGGGATTAT
CATGCTTTGGGTCGATGGTTACTTGAGTTGTCAGAGGCTAACCATTTGCTGACAGTGCAT
GATTTTGATCTGAAGGCTGGTTTGAACCATCAGCTGATGATGATTGTTCAGATGAAAACT
TATCAAGCGAACAAACGCCCAAAACCAGTTGCTCAGCAGGTGCCTGATGTTCAATGAATA
TTATCGGTGGGGCATTTTGGGTGCTTGGATTTGGGTTGGGATTGGATGTGCTGATAGCAC
CAGTCAAGTTGTTGATGATAAGCTTGCACATATTACCCATGAAGAGCGTATGGCGATCAG
TGAGCCTGTGCCGATACCCTTATCTGTGCCGATGATATATCAGCAAGGCAAAGATCCTTT
TATCAATCCTTATAGAAATGTTGAGGTTCTTGATACCAATCATGCCGCTGATCAGCAAGA
TGAGCCAAAAACCGAATCTACCAAAGCTTGGCCTATGGCAGACACTATGCCATCTCAGCC
ATCTGATACTCATCAGTCTGCCAAGGCTCAGGCACAAGTCTTCAAAGGCGATCCGATAGT
CATTGATACCAACCGTGTTCGAGAGCCTTTAGAAAGCTATGAGTTATCAAGCCTACGCTA
TCATGGTCGTATTTTTGATGATGTTAGACTTGTGGCACTCATTATGAGTCCTGATGGCAT
CGTTCATCGTGTGAGTACTGGACAATATCTTGGTAAAAATCACGGAAAAATTACCCATAT
TGACAGTCGTACGATACATCTGATTGAAGCGGTCGCTGATACACAAGGTGGCTATTATCG
CCGTGATGTAAACATTCATTTTATTCATAAGCAATGACAC

SEQ. ID NO:56
Nucleotide sequence of DNA region (1000 bp) up-stream from the lipo18 gene
from Moraxella catarrhalis
TTCATGCAACAAGCGACCATCTTGGCCGATGATACCATCCTGCTCACCTAAGAAAATCAG
TTTATCAGCTTGCAGGGCAATGGCTGTGGTCAGTGCTACATCTTCTGCCAATAGATTAAA
AATTTCGCCCGTAACCGAAAAACCTGTCGGTCCTAGTAGGACAATATGGTCATTATCCAA
ATTATGGCGAATGGCATCGACATCAATTGAGCGTACCTCACCTGTCATCTGATAATCCAT
ACCATCTCTGATGCCGTAAGGGCGAGCGGTGACAAAATTACCCGAAATGGCATCAATACG
AGATCCGTACATTGGGGAGTTAGCAAGCCCCATCGACAGCCGAGCTTCGATTTGTAGACG
AATTGAGCCGACTGCCTCCAAGATGGCAGGCATAGATTCATACGGTGTTACACGCACATT
CTCATGTAGGTTTGATATCAGCTTGCGATTTTGTAAATTTTTTTCCACTTGTGGGCGTAC
ACCATGCACAAGCACCAATTTGATGCCCAAGCTGTGTAGCAGTGCAAAATCATGAATCAG
CGTACTAAAATTGTCACGAGCGACCGCCTCATCACCAAACATAACCACAAAGGTTTTGCC
ACGATGGGTGTTAATGTACGGGGCAGAATTACGAAACCAATGCACAGGTGTGAGTGCAGG
AGTGTTCTGATAGGTGCTGACAGAATTCATGAATGCTCCAAAGAGTCAATGGCTGGTAAA
ATAAGAATGGCGAACAATATATGGCGAGAGCGTCTGATGTTGGTCAAATGTCCCATTAAT
AACTATCAAGATACCATCATACCATAGCAAAGTTTTGGGCAGATGCCAAGCGAATTTATC
AGCTTGATAAGGTTGGCATATGATAAAATCTACCATCATCGTCGCCAGTTTTGAGCATGT
GTAAGTAGTTACCATAATTAAACAGTCAAGAAATTCACACCGTCAATCAGCTGTGCTATG
CTTATGGGCACATAAAACTTGACCAACACAGGATAAATTTA

SEQ. ID NO:57
Nucleotide sequence of DNA region (1000 bp ) up-stream from the lipo11 gene
from Moraxella catarrhalis
GGCATACTTTTGCCATGCTTTATTTGGCATAACTGCTATAAGCCCATTGCTACTTTTTA
TCATTTATCCATATGTCCAATAATGTGCTTTATGTAATTTAGGCACACTATTAACTCGTG
CCACTGTTAACATTCAGCATAAAAATCTTAACAATGAATCAAAGCATCGTATTGGCTGTT
AAATGATAAGCTTATATTTATTTAAATTCAGACTAAATGATTGTAATATGGACATATCAA
GGTTGAAATCAAAAATTTTGGAGAGTTATGTACGATAATGATAAAAAATTGACCACCATC
GTAGGGGTGTTGTATACGGTGTCTTATATTGCCATATGGTTGGTCAGTGGCTATATTTTA
TGGGGCTGGATTGGTGTGACAGGATTTACTCGTGCGATACTTTGGCTGATCGCTTGGATG
ATTGTGGGTACGATTGCTGATAGAATTCTGATACCGATTATTTTGACCGTCGTGGTTGGG
TTATTTTCTATCTTTTTTGAAAAAAGGCGATAATTTGGTTATTTTTTCACAAAAAATCAT
GATTTTTTTTGTAAACTATCTAAAATATCAATTATGTTATATTATGTGATAAAAGATGGG
CATGCTTAAGTTTTGGATTGCAAAAATCCTAATATCATCACTGACCAAAGCTGTGATGAT
ATCAAAACTTTATCAAAGTTCTTAGGGTATTATCAAGATATCATACCAAATGAATACTTA
CCCAACTTACTATAAAAATCAAATGATATGACTGTGATTTTATTATCATAGATACAAAAA
TCAAAACGCATGAGCCAAAGGTATGATGAATGAATACAAAATTTCGCACACATTATGACA
ATCTAAATGTCGCCAGAAACGCTGACATTGCGGTGATTTGGTGGGATAGGGGTCAAGCCA
GTGCGATTAAGCTAAATTTTTATGTGGGCAATCGCTGACTTTATTTTATTTGTGCCAGTT
GGAACAATTCGTGGTCTAATGTATTTATTTTAAGGAGATAA

SEQ. ID NO:58
Nucleotide sequence of DNA region (1000 bp ) up-stream from the lipo10 gene
from Moraxella catarrhalis
TCTGGTCTACATCCCAAACTATTTACACAAGAAACACTAAAGACAGTGGAGCAGATGACG
CTCAAAAAGGCATCTTATAGTAATTTGACAGTTAATTTTCGTCAAGTGCTTGTACAAAAA
TACACCATCGTGCAAGAAGTTTGTACCAATTTAAGCACAATCATTTTGGCACACACTGTC
AAGCAATGCTTCAGGCAAATTAGCTGCTGGTAAAGATACTTGGGTCATCATGCAATCGCA
TCAACCCTTCTTGCTGCGTTGAAGCGATAAGTTTGCCATCTTGCCAAAATTGACCATGGT
TTAGACCCTTGGCGTGGCTTGTGGTATCGCTCCACATGTCGTAGAGTAGATATTCGGTCA
TATCAAAAGGGCGATGGAAATGTATGGAATGGTCAATACTAGCCATTTGTAGACCTTGTG
TCATCAGGCTTAGCCCATGACTCATTAAACCTGTGCTGACCAAATAATAATCAGACACAA
ACGCAAGTAGTGCTTGATGAATGGCAACTGGCTGCTCCCCAATATCAGCGATACGCACCC
AATTGGCTTGGCGTGGACGCTCAGGCTTGGGTGTCACAGGGTCTCGTGGTGTGACGGGGC
GGATTTCGACATGACGCTGACGCATAAATCTTGCTTTGAGTGGTTCGGGAATTTTATGTA
AATAATCCGCTTTGAGTTCTTGCTCGGTTTTTAGGCTTTCAGGGGGTGGATAATCAGGCA
TGGTTTCTTGGTAATCAAGCCCGCCTTCCATGGGTGAAAATGAGGCAATCATCGAAAAAA

TGACCTGTTCATTGGTCGTATGATTACCGTTTTTGTCGGTGGTTGGCACATATTGCACCG
CAATGACTTCTCGAGCTGATAAACTGCGTCCATCACGTAAGCGGCGTACTTGATAGATGA
CTGGTAGACGAATATCGCCACCTCGTAAAAAATAACCATGTAGGCTATGACAAGGTTTAT
CAATCGTTAATGTGTTAGCACCAGCAAGCAGCGCTTGGGCA

SEQ. ID NO:59
Nucleotide sequence of DNA region (1000 bp ) up-stream from the lipo2 gene
from Moraxella catarrhalis
TAAAATGACCTTACAAAATAAAATTATATGTTCAAAAATCGCTTAAGTATTGAAAAAAGC
TATAAAAACTTATCTATTAAAGCATAAAAGATATTAAAGCATAAAAGACGAGAAAAGAGC
AAGCGTCAATGATGATATTTCATATAAAAACTTATGAAATTTTTCAATTTTTTATCGATT
GATTCAGCTTGGCTATCGGTGGTCAACTTTGGCTGCCAAGACATCGCCGGCTTTTTGAAA
AATCATCACAATGGCAACAATGATGATGGTTGAAATCCACTTGACATATACCATGTTGCG
ATGCTCACCATAGTTAATCGCAAGGCTTCCCAAGCCACCACCGCCAACCACACCTGCCAT
TGCAGAATAACCAATCAAAGACACCAAGGTCAATGTGACCGCATTAATCAAAATGGGCAG
GCTTTCAGCAAAATAGTATTTGCTGACAACCTGCCAATGCGTTGCACCCATAGATTTGGC
AGCTTCGGTCAGTCCTGTGGGTACTTCTAATAAAGCATTGGCACTCAAGCGTGCAAAAAA
TGGAATTGCTGCCACACTCAAAGGGACGATGGCGGCTGTTGTGCCAAGGGTTGTTCCCAC
CAAAAATCGTGTGACTGGCATGAGAATAATGAGCAAAATAATAAAAGGAACGGAGCGACC
AATATTAATAATAACATCCAAAATTACAAATACACTGCGATTTTCAAGGATACGCCCTTT
ATCGGTTAAAAATGCCAAAAACCCTATCGGTAGCCCAACCAAAACAGCGATGGCAGTGGC
AGCAAGCCCCATATAGATGGTTTCCCAAGTGGATTGGGCAACCATCTCCCACATTCTTGG
GTGCATTTCACTGACAAATTTTGTGACGATTTCATTCCACATAGCCGATAATCTCAATAT
TGACCCGATGGGTGGTTAAAAATTCTATTGCTTGCATGACCGAGGTGCCTTCACCGATAA
GCTCAGCAATGGTAAAGCCAAATTTTATATCACCTGCATAA

SEQ. ID NO:60
Nucleotide sequence of DNA region (1000 bp ) up-stream from the lipo7 gene
from Moraxella catarrhalis
AGTAAACAATGGTAACAAATACAGCAGTGTCGCACAGTCCTCAGTACGATGATTCTGAAT
TTGAATATGCAGGATTTTGGATACGATTTGTGGCATGTCTTGTCGATAATTTAATTGTTA
TGATTATAATTGCACCGTATTGGTTTTATAATTATCAGCAAATGATGGCCATGCCTGCTG
ACCAAATACCGTTTTATAGTGTTGGGGATGCCATCCTTTATAGTGCTGGGGATGCTATCC
TAAACTTAGTGATGGCGGCGGCGGTTGTTTGGTTTTGGGTAAAAAAAGGTGCAACACCAG
GTAAAATGCTCTTTGGGCTGCAAGTCCGTGATGCCAAAACAGGGCAATTTATCAGTGTGC
CAAGGGCATTATTGCGATATTTTAGTTATCTGATTTCATCCGTGATTCTTTGTTTGGGAC
TTATTTGGGTTGGTTTTGATAAGAAAAAACAAGGCTGGCATGATAAAATTGCCAAAACTG
TTGTGGTAAAACGCATTCGCTGATGGGTCGCCAGTTAAACAATAAAACCATCAAACGCAA
GCAGGGCGATGTGTTTGAGCAGTTGGCGGTAGATAAGCTAAAACAAGCAGGCTATGAAAT
TATTTTAACCAACTTTACCACCCCATTTGTTGGTGAGATTGATATTATCGCCAGACAGCC
TTTGGAGCAATCGCACCGTTTGGTGCAGCCAAGATTTTGTACGGTATTTGTTGAAGTGCG
TAGCCGAACAAGTTCTGTGTATGGTACAGCGCTTGAGAGTGTTACCTCAAAAAAGCAGGC
AAAAATCTACCGAACAGCAGAACGATTTTTAATCAATTATCCCAAATATATTGATGATGC
ATACCGTTTTGATGTCATGGTTTTTGATTTGGTTGATGGATTGATTGAACATGAATGGAT
AAAAAATGCGTTTTGATTGGCTCAATGGTCGTGAATTAAAATCAATCAAGCAATCCGTAG
CTTTACTATAAGATATATCCCAGTAATATGGAAACATAGCA

SEQ. ID NO:61
Nucleotide sequence of DNA region (1000 bp ) up-stream from the lipo6 gene
from Moraxella catarrhalis
CGTTTAGCTTCATACGCAGACCTTGTGCACCTTCGGGCAACCGAAGCATCACGCCAGCAT
CACGCATCCGCACAAAACCCATCATGCCATCAATTTCGCTGCTGATATGATATACCCCCA
CCAAAGTAAACCGCTTAAATCGTGGAATAACGCCTGCTGCTGAGGGTGAGGCTTCAGGCA
AAACCAAGGTAACCTTATCCCCCAACTTAAGTCCCATGTCAGAGACAATGGACTCACCTA
ATATAATACCAAACTCGCCGATATGTAAATCATCCAAATTGCCTGCGGTCATATGCTCAT
CAATGATAGAAACTTGCTTTTCGTAATCAGGCTCAATGCCAGAAACCACGATTCCAGTCA
CCTGACCTTCAGCGGTTAACATACCTTGTAGTTGAATATAAGGGGCAACTGCTTGCACTT
CTGGATTTTGCATTTTGATTTTTTCGGCAAGTTCTTGCCAATTTGTCAAAATTTCTGTTG
AGGTAACTGAAGCTTGAGGCACCATGCCAAGAATGCGTGATTTAATTTCACGGTCAAAGC
CATTCATGACCGACAAAACCGTGATAAGCACTGCAACCCCAAGCGTAAGCCCAATGGTTG
AGATAAAAGAAATAAAGGAAATAAAGCCATTTTTACGCTTAGCTTTGGTATATCTAAGCC
CAATAAATAACGCCAAGGGACGAAACATAAGCTGTGTTCCAAACGACCCAACCGTGCTAG
TTTAGCACTTTTTTGGACAAATACCAAACATCACATAACAAATGAATCATCAGGTTGGTT
TTGTTGCGCTTGTGTATCTGTATGATAAGTTTCTTGCTAAAACAGCTTTTTTATGTCAGA
ATACAGAAAAGGTATATACTTATATTTTTAACTTTAAATAGATCTGCTTTTTTATACCGA
TGATTTGGCATGAAGTTTATCGGTCTGATATGCTGGATATAAGTTTATCGGCTTGATATA
AATTTTAATTAATCATCAAATTTTTAAGGAATTTATCATTA

SEQ. ID NO:62
Nucleotide sequence of DNA region (1000 bp ) up-stream from the P6 gene from
Moraxella catarrhalis
TAAGGATACCAGATTTTGGCTTGTCAATCGTTGTGTTAATCATTGTAACGGTTTATAGTG
ATTGTCAATTAATAAGGGTAAAAAAGTATTTATCAAGTAATAATCTTTCTTATATGTGAA
TATAATGACAAATTTATCACATTTTTACAAGGATTTTTTATCAAGATTAGGATATGTTCC

AGCTTAATTATTAGTGATGAGCGTGTGATTATTTGGCATCGTTAAATTTATGAGTGCTAA
AATTGCCAAATGATTAAAATTTTGCTAACATGATAGCCCCTTTGGTAGGCTTTATTTGGT
ATTGATGAGCAATAATAATATACCGAGTTAAATGGATTAACTTAACATACGCCAAAAACT
TAACAACGAAAAGTAGATGATTATGACAGATACAGTACAAAAAGATACAGCACAGTCCCC
CAAAAAAGTTTATCTAAAAGACTACACGCCGCCAGTATATGCAGTTAATAAAGTGGATTT
GGATATCCGCTTGTTTGATGATCATGCTGTCGTTGGTGCCAAACTTAAAATGACACGAGC
ACACGCAGGCGAGCTTCGGCTTCTTGGGCGAGATTTAAAGCTTAAAAGCATTCACCTAAA
TGGTCAGGAATTAGAGTCGCAGGCGTATCATCTTGATAAGGAAGGCTTAACAATTTTAGA
TGCACCAGATGTCGCAGTGATTGAGACATTGGTTGAGATTTCACCACAAACCAACACAAC
ACTTGAAGGGCTATATCAAGCAGGAACAGGTGATGATAAGATGTTTGTGACACAATGCGA
ACCTGAGGGTTTTCGCAAAATCACCTTTTTCCCTGACCGCCCTGATGTTTTGACAGAATA
CACCACACGCCTAGAAGCACCAAAGCATTTTAAAACCTTGCTTGCCAATGGTAATTTGGT
TGAGTCAGGAGATGTGGATGAAAATCGCCATTATACCATTTGGCATGATCCTACCAAAAA
ACCCAGCTATCTATTCGCCGCTGTCATTGCCAATCTAGAAG

SEQ. ID NO:63
Nucleotide sequence of DNA region (1000 bp ) up-stream from the MsbB gene
from Haemophilus influenzae (HiRd)
AAATCAAGCGCCTGTGCCTGCTGGTGATGGTTGTGGAGACGAATTATATTCTTGGTTTGA
ACCGCCAAAACCAGGCACTTCAGTGAGCAAACCTAAAGTTACACCGCCTGAGCCGTTTTT
GTGCCAACAGATTTTGAACTCACCGAATCGGAGAGAATGGTTAGAATAGCATTGAGGTAA
ATCAATATGGATATCGGCATTGATCTTTTAGCAATATTGTTTTGTGTTGGTTTTGTCGCA
TCATTTATCGATGCAATTGCTGGCGGTGGTGGATTAATCACCATTCCAGCGTTACTCATG
ACAGGTATGCCACCAGCAATGGCGTTAGGCACCAACAAATTGCAAGCTATGGGCGGTGCA
TTATCCGCAAGCCTTTATTTCTTGCGAAAAAGAGCGGTCAATTTACGCGATATTTGGTTT
ATTTTGATTTGGGTTTTCTTAGGTTCTGCCCTAGGTACATTATTAATTCAATCAATTGAC
GTGGCGATTTTCAAAAAAATGCTTCCTTTTTTGATTTTAGCCATTGGTCTATATTTTTTA
TTTACTCCTAAATTAGGTGATGAAGATCGAAAACAACGATTAAGTTATCTGTTATTTGGT
CTTTTAGTTAGCCCATTTTTAGGTTTTTATGATGGCTTCTTTGGGCCAGGGACTGGCTCA
ATCATGAGTTTAGCCTGTGTTACTTTGCTAGGATTTAATCTCCCGAAAGCGGCAGCACAT
GCAAAAGTGATGAACTTCACTTCGAACCTTGCTTCTTTTGCACTTTTCTTATTGGGCGGA
CAAATTCTTTGGAAAGTGGGTTTCGTGATGATGGCTGGGAGCATTTTAGGTGCAAATTTA
GGTGCCAAAATGGTGATGACGAAAGGTAAAACCTTGATTCGACCGATGGTTGTTATCATG
TCTTTTATGATGACGGCTAAAATGGTTTACGATCAGGGTTGGTTTCATTTTTAATTCGGA
AAGCGCGCAAAAGTGCGGTTAAAATTAATTACATTTTATTA

SEQ. ID NO:64
Nucleotide sequence of DNA region (1000 bp ) up-stream from the HtrB gene
from Haemophilus influenzae (HiRd)
TTGAAGTCCCCAATTTACCCACCACAATTCCTGCGGCAACATTGGCTAGGTAACAAGATT
CTTCGAAAGAACGTCCATCTGCTAATGTGGTTGCTAATACACTAATGACAGTGTCACCGG
CTCCCGTCACATCAAACACTTCTTTTGCAACGGTTGGCAAATGATAAGGCTCTTGATTTG
GGCGTAATAATGTCATGCCTTTTTCAGAACGCGTCACCAAAAGTGCGGTTAATTCAATAT
CAGAAATTAATTTTAAACCTTTCTTAATAATCTCTTCTTCTGTATTACATTTACCTACAA
CGGCTTCAAATTCAGACATATTGGGTGTCAATAATGTAGCCCCACGATAACGTTCAAAAT
CAGTTCCCTTTGGATCGATCAACACAGGCACATTCGCTTTGCGTGCAATTTGAATCATTT
TCTGAACATCTTTAAGCGTGCCTTTGCCGTAATCAGAAAGAATCAAAGCACCGTAATTTT
TCACCGCACTTTCTAACTTCGCTAATAAATCCTTGCAATCTACATTATTGAAATCTTCTT
CAAAATCAAGGCGGAGCAGCTGTTGATGACGAGATAAAATACGTAATTTAGTAATGGTTG
GATGGGTTTCTAATGCAACAAAATTACAATCAATCTTTTGTTTTTCTAATAAGTGGGAAA
GTGCAGAACCTGTCTCATCTTGTCCAATCAATCCCATTAACTGAACGGGTACATTGAGTG
AAGCAATATTCATCGCCACATTTGCAGCACCGCCCGCGCGTTCTTCATTTTCTTGTACGC
GAACTACTGGCACTGGTGCTTCTGGTGAAATACGGTTGGTTGCACCGAACCAATAACGAT
CAAGCATCACATCGCCTAATACAAGTACTTTTGCTTGCTTAAATTCTGCTGAATATTGAG
CCATTTTAAAATCTCTCTATTTGAATAACCAAAATTGTGGCGATTTTACCACAACTCAAA
TTTACGATAAACTACGCCCCTAACTTACGTGGAAAGAACAA

SEQ. ID NO:65
Nucleotide sequence of DNA region (1000 bp ) up-stream from the protein D
gene from Haemophilus influenzae (HiRd)
AGCAATAATTATAGCTGGAATATTCTTTAAAGATGAAAGAGATCGTATAAGACAAAAAGA
ATTTTATATTGGAGAATTATTAGCAATTATTGGTTCGCTAATATTCGTAATAAATAGTTC
AAATAATGATGGAAATACAGACTTTTTCTTGGGGCAATATTTCTTTTTACAGCTATTTT
TATTCAATCTGTACAGAATTTAATTGTAAAAAAAGTAGCCAAAAAGATAAATGCTGTTGT
AATAAGTGCATCGACAGCAACAATTTCAGGAGTATTATTTTTATGTTTAGCTTTTAATAC
TAAACAAATATATTTATTACAAGATGTTGGCATTGGAATGTTGATAGGTTTAGTTTGCGC
TGGCTTTTATGGGATGCTAACAGGGATGTTGATGGCTTTTTATATTGTTCAAAAACAGGG
AATCACTGTTTTTAACATTTTGCAATTATTAATTCCTCTTTCAACTGCGATAATAGGTTA
CTTAACATTAGATGAAAGAATAAATATCTATCAGGGAATTAGCGGTATTATTGTAATTAT
TGGTTGTGTATTGGCATTAAAAAGAAAAAACAAGGAGTGTTGATATATAAAGTAGATGAT
GTTGGTGGAATAGGTATAGTTAAATATCTGGTTCAATTGGTTTTATTAAGGGCGTTAGCA
ATTCTCCATTTAAGTTTATGTTTGAATTAGATATTTTGGGAAAAGATGGAAGAATAAAGC
TGTTAAATAATGCTGAAACATATGAACTATACCAATACTCAAATAAAAATAATTCTGCTG
GAAATGATTATAAATCTCTAATTCTAACTTGTAGAGAGGATAATGACTATCAATCAGAAA
GAATGATTAAAGCCATTAAAAATATTATTCATTGTATGACTAATAATCATCAACCTATTT

CAAGTGCTGAAACATCTTTAGAAACTATTAAAATTATTCACGGAATAATTAATTCTGTTA
AAATAGGTAATGATCCTAACAATATATAAGGAGAATAAGT

SEQ. ID NO:66
Nucleotide sequence of DNA region (1000 bp ) up-stream from the Hin47 gene
from Haemophilus influenzae (HiRd)
TAAATACTCCAAAATAAATTTCAGATAACGTGGTCTGTAAGACAAAAAAATAAAAAAAAT
GTTCAATAAGAGGAGAGCAAATTATCTTGTTTAAAAGGAAATCGGAGCAGTACAAAAACG
GTCTTACAAGTAGCAAATTCTATAAATTTATGTTCTAATACGCGCAATTTTCTAGTCAAT
AAAAAGGTCAAAAAATGAGCTGGATTAACCGAATTTTTAGTAAAAGTCCTTCTTCTTCCA
CTCGAAAAGCCAATGTGCCAGAAGGCGTATGGACAAAATGTACTGCTTGTGAACAAGTAC
TTTATAGTGAAGAACTCAAACGTAATCTGTATGTTTGCCCGAAATGTGGTCATCATATGC
GTATTGATGCTCGTGAGCGTTTATTAAATTTATTGGACGAAGATTCAAGCCAAGAAATTG
CGGCAGATTTAGAACCAAAAGATATTTTAAAATTCAAAGATTTAAAGAAATATAAAGATC
GTATCAATGCGGCGCAAAAAGAAACGGGCGAGAAAGATGCGCTAATTACTATGACAGGTA
CACTTTATAATATGCCAATCGTTGTGGCTGCATCGAACTTTGCTTTTATGGGCGGTTCAA
TGGGTTCTGTAGTTGGTGCAAAATTTGTTAAAGCGGCTGAAAAAGCGATGGAAATGAATT
GTCCATTTGTGTGTTTCTCTGCGAGTGGTGGTGCTCGTATGCAGGAAGCATTATTCTCTT
TAATGCAAATGGCAAAAACTAGTGCCGTACTTGCTCAAATGCGTGAAAAGGGTGTGCCAT
TTATTTCAGTATTAACGGATCCGACTTTAGGCGGCGTATCAGCCAGTTTTGCGATGTTAG
GGGATTTAAATATTGCCGAGCCAAAAGCCTTAATTGGTTTTGCAGGGCCACGCGTTATTG
AACAAACTGTGCGTGAAAAATTGCCAGAAGGTTTCCAACGTAGTGAGTTTCTACTTGAGA
AAGGGGCAATTGATATGATCGTGAAACGTTCAGAAATGCGT

SEQ. ID NO:67
Nucleotide sequence of DNA region (1000 bp ) up-stream from the P5 gene from
Haemophilus influenzae (HiRd)
TCACTTAATTCAAGCGCATCAATGTTTTCTAAAACATCAACAGAATTGACCGCACTTGTA
TCTAAAATTTCGCCATTTATTAAGACTGCGCGTAATGCCAAAACATGATTAGAGGTTTTA
CCATATTGCAATGAGCCTTGCCCAGAGGCATCGGTGTTAATCATTCCACCTAAAGTCGCT
CGATTGCTGGTGGACAGTTCTGGGGCAAAGAACAAACCATGTGGTTTTAAAAATTGATTA
AGTTGATCTTTTACTACGCCTGCTTGTACTCGAACCCAACGTTCTTTTACATTGAGTTCT
AAGATGGCTGTCATATGACGAGAAAGATCCACTATTATATTGTTATTGATGGATTGCCCA
TTTGTGCCAGTGCCTCCACCGCGAGGCGTAAAGCTGATTGATTGATATTCAGGTAAATTT
GCCAATTTTGTTATCCGCACTATATCAGCAACCGTTTTCGGAAAAAGAATTGCTTGTGGA
AGTTGTTGGTAAACGCTGTTATCCGTAGCCAGACTTAATCTATCTGCATAGTTTGTCGCA
ATATCCCCCTCAAAATGTTGGCATTGAAGATCATCAAGATAATCAAGTACATATTGTTCA
ACTTGAGGAATGCGATTTAGATTTGGCAACATAGTATTTGACCCATTTAAACATATCAGA
TGGAGGCTTTGATAATATCCTAAGGCTAGAATAATGTCGATTAGGAAAGAGAGAGGAGAA
AGTAAAAAGTCTGTTTAAGAAAGTGTTATTTTGGATAAAAACTAAACAAAAAAATTCAAAA
GAATTTGATCTTTTCAATTTTTATAGGATAATAAGCGCACTTTTGAACGTTCCTTTGGGG
TAAACATAAGCAAAGGAATTGAATTTGTCAAAAGGTAATAAAGTAGGGCAAATTCAAAAC
CCTAGTTAAGTGACTGTTTATAATGTAGCTTTAATTAAAAGTTCAGTATAAACAAGGACA
CTTTTTATTACTATTCGATCACTAAATAGAGGACATCAAAA

SEQ. ID NO:68
Nucleotide sequence of DNA region (1000 bp ) up-stream from the D15 gene
from Haemophilus influenzae (HiRd)
TCGATTGTATCCTATATAAATTATAGACGTAAAAAATCATTAAATAATGCAAACACCGTT
AAGCTTAATAACAGTGCTGCGCCAATTCGATAACAGATGCTTTGCACCCGCTCAGAAACA
GGTTTTCCTTTAACAGCTTCCATTGTTAAAAAAACTAAATGACCGCCATCTAATACTGGT
AATGGAAATAAATTCATAATCCCTAAATTTACACTAATCAATGCCATAAAACTTAAAAAA
TACACCAATCCAATATTTGCTGATGCGCCAGCACCTTTTGCAATAGAAATTGGCCCACTT
AAATTATTTAATGACAAATCGCCAGTAAGTAATTTCCCTAATATTTTCAAGGTTAAAAGG
GAAAGCTGTCCTGTTTTTTCAATGCCTTTTTGTAAAGATTCAAGAATACCATATTTTAAT
TCAGTACGGTATTCATCCGCTAATTTTGTTAAGGCTGGGCTAACCCCAACAAACCATTTG
CCATTTTGATTACGCACTGGAGTTAGGACTTTGTCAAATGTTTCTCCATTACGTTCAACT
TTAATAGAAAAAGATTCGCCTTGTTCGACCTGTTTTATAAAATCTTGCCAAGGAAGTGCG
GTTAAATTTTCTTTTAAAATTTTATCACCGATTTGTAAACCAGCTTTCTCAGCGGGAGAA
TTTTGAACAACTTTAGAAAGCACCATTTCAATTTTAGGACGCATAGGCATAATCCCTAAT
GCCTCAAAAGCACTTTCTTTTTCAGGATCGAATGTCCAATTTGTAAGATTTAAAGTCCGT
TGTTGTTCAATATTAGAATTGAAAGGAGAAAGGCTAATCTCAACATTAGGCTCCCCCATT
TTTGTGGCAAGTAGCATATTGATGGTTTCCCAATCTTGAGTTTCTTCGCCATCAATTGTA
AGAATTTGCGTATTGGGTTCAATGTGGGCTTGTGCTGCGATTGAGTTTGGTGTTATTGAT
TCAATCACTGGTTTAACCGTTGGCATTCCATAAAGGTAAAT

SEQ. ID NO:69
Nucleotide sequence of DNA region (1000 bp ) up-stream from the Omp26 gene
from Haemophilus influenzae (HiRd)
TTTGATAAATATCCTTAATTAAATGATGGGTTTAATATTTTCTCTGCCCAATTAAATTAG
GCAGAGAACGTTGTTTTTGAGTTCTGATGAAGAAAAAAGTTCAATTTATTAGAAAGAACC
TCCAATACTAAATTGGAACTGTTCGACATCATCATTTTCATATTTTTTAATTGGTTTGGC
ATAAGAGAATACCAATGGCCCAATAGGAGATTGCCATTGGAATCCGACACCTGTAGAGGC

GCGAATACGGCTTGATTTGCCATAATCGGGTAAGCTTTTTAATACATTGTTATCTAACCC
ACTCTTATCCGATTTCCACTTAGTATTCCAAACACTTGCCGCATCAACAAATAGGGAGGT
TCGGACTGTATTTTGGCTTTTATCACTCACAAACGGTGTTGGTACAATAAGTTCTGCACT
CGCAGTTGTGATTGCATTACCACCAATCACATCAGAACTTATCTTCTTAAAAGTACCATT
ACCATTACCATGTTCTGCATAAATTGCGTTAGGTCCAATACTACCATAAGCAAAACCACG
TAATGAACCGATGCCACCCGCTGTATAAGTTTGATAGAACGGTAAACGCTTGTTTCCAAA
ACCATTTGCATATCCTGCAGATGCTTTTGCAGATACAACCCAGAGGTGATCTCTGTCTAA
TGGGTAGAAACCCTGTACGTCTGCACTTAGTTTGTAGTATTTGTTATCAGAACCTGGAAT
AGTAACTCGTCCACCAAGACTTGCTTTAACCCCTTTAGTTGGGAAATAGCCTCTATTAAG
GCTGTTATAGTTCCAACCAAAAGAAAAATCAAAGTCATTTGTTTTAATGCCATTACCTTT
AAATTTCATTGATTGAATATATAAATTACGGTTATATTCTAGAGCAAAGTTACTAATTTT
ATTATAGGTATGGCCTAATCCTACATAATAGGAGTTATTTTCATTTACAGGGAAACCTAA
AGTAACATTACTTCCATAAGTCGTACGCTTATAGTTAGAGG

SEQ. ID NO:70
Nucleotide sequence of DNA region (1000 bp ) up-stream from the P6 gene from
Haemophilus influenzae (HiRd)
TTAGATTTCTCCTAAATGAGTTTTTATTTAGTTAAGTATGGAGACCAAGCTGGAAATTT
AACTTGACCATCACTTCCTGGAAGGCTCGCCTTAAAGCGACCATCTGCGGAAACCAATTG
TAGCACCTTTCCTAAGCCCTGTGTAGAACTATAAATAATCATAATTCCATTTGGAGAGAG
GCTTGGGCTTTCGCCTAGAAAAGATGTACTAAGTACCTCTGAAACGCCCGTTGTGAGATC
TTGTTTAACTACATTATTGTTACCATTAATCATCACAAGTGTTTTTCCATCTGCACTAAT
TTGTGCGCTACCGCGACCACCCACTGCTGTTGCACTACCACCGCTTGCATCCATTCGATA
AACTTGTGGCGAACCACTTCTATCGGATGTAAATAAAATTGAATTTCCGTCTGGCGACCA
CGCTGGTTCAGTATTATTACCCGCACCACTCGTCAATTGAGTAGGTGTACCGCCATTTGC
TCCCATAACGTAAATATTCAGAACACCATCACGAGAAGAAGCAAAAGCTAAACGAGAACC
ATCTGGCGAAAAGGCTGGTGCGCCATTATGCCCTTGAAAAGATGCCACTACTTTACGTGC
GCCAGAATTTAAATCCTGTACAACAAGTTGTGATTTTTTATTTTCAAACGATACATAAGC
CAAACGCTGGCCGTCTGGAGACCAAGCTGGAGACATAATTGGTTGGGCACTACGATTGAC
GATAAATTGATTATAGCCATCATAATCTGCTACACGAACTTCATAAGGTTGCGAACCGCC
ATTTTTTTGCACAACATAAGCGATACGAGTTCTAAAGGCACCACGGATCGCAGTTAATTT
TTCAAAAACTTCATCGCTCACAGTATGCGCGCCATAGCGTAACCATTTATTTGTTACTGT
ATAGCTATTTTGCATTAATACAGTCCCTGGCGTACCTGATGCACCAACCGTATCAATTAA
TTGATAAGTAATACTATAACCATTACCCGATGGAACCACTT

SEQ. ID NO:71
Nucleotide sequence of DNA region (1000 bp ) up-stream from the TbpA gene
from Haemophilus influenzae (non-typeable)
GGCGATAACCGAGTTTTTGGGGTATTTAGTGCCAAAGAAGACCCACAAAACCCAAAATTA
TCCAGAGAAACCTTAATTGATGGCAAGCTAACTACTTTTAAAAGAACTGATGCAAAAACC
AATACAACAGCCGATACAACAACCAATAAAACAACCAATGCAATAACCGATGAAAAAAAC
TTTAAGACGGAAGATATACTAAGTTTTGGTGAAGCTGATTATCTTTTAATTGACAATCAG
CCTGTTCCGCTTTTACCTGAAAAAAAATACTGATGATTTCATAAGTAGTAGGCATCATACT
GTAGGAAATAAACGCTATAAAGTGGAAGCATGTTGCAAGAATCTAAGCTATGTAAAATTT
GGTATGTATTATGAAGACCCACTTAAAGAAGAAGAAAAAGAAAAAGAAAAAGAAAAAGAC
CAAGAAAAAAAAAGAAAAAGAAAAACAAACGACGACAACATCTATCGAGACTTATTATCAA
TTCTTATTAGGTCACCGTACTGCCAAGGCCGACATACCTGCAACGGGAAACGTGAAATAT
CGCGGTAATTGGTTTGGTTATATTGGTGATGACACGACATCTTACTCCACTACTGGAGAT
AAAAATGCTCTCGCCGAGTTTGATGTAAATTTTGCCGATAAAAAGCTAACAGGCGAATTA
AAACGACACGATAATGGAAATACCGTATTTAAAATTACTGCAGACCTTCAAAGTGGTAAG
AATGACTTCACTGGTACAGCAACCGCAACAAATTTTGTAATAGATGGTAACAATAGTCAA
ACTGGAAATACCCAAATTAATATTAAAACTGAAGTAAATGGGGCATTTTATGGACCTAAG
GCTACAGAATTAGGCGGTTATTTCACCTATAACGGAAATTCTACAGCTAAAAATTCCTCA
ACCGTACCTTCACCACCCAATTCACCAAATGCAAGAGCTGCAGTTGTGTTTGGAGCTAAA
AAACAACAAGTAGAAACAACCAAGTAATGGAATACTAAAAA

SEQ. ID NO:72
Nucleotide sequence of DNA region (1000 bp ) up-stream from the TbpB gene
from Haemophilus influenzae (HiRd)
TAGAATTATATTCTTATACAAAATTGATAATTGTTCGCATTATCATTTTTTTTTTGTAAT
AATGTCAACTTATAATTTTTTAAGTTCATGGATAAAATATGAAAAATGGCGTAAAACAAC
TTTTTCTCTTATCATTAATAGGCTTATCATTAACGAATGTAGCTTGGGCAGAAGTTGCAC
GTCCTAAAAATGATACATTGACAAATACGATTCAAAGTGCGGAATTAAAAACCTCCTCTT
TTTCCTCTATGCCTAAGAAAGAAATACCAAATAGGCATATTATTTCTCTTTCCAAAAGCC
AATTAGCGCACCATCCAAGGCTTGTTTTGCGTGGGTTAATTCCTGCTTTATATCAAAATA
ACACTCAGGCAGTTCAACTGTTATTACCACTATATAAACAATTTCCTCAACAAGATAATT
TCTTACTAACTTGGGCAAAGGCTATTGAAGCTCGTGAACAAGGTGATTTAACTCAATCTA
TTGCTTATTATCGTGAATTATTCGCTCGAGACGCATCTTTACTACCTTTACGTTATTAAT
TAGCTCAAGCTCTATTTTTTAACTATGAAAATGAAGCTGCCAAAATTCAATTTGAAAAAT
TACGTACAGAGGTAGATGATGAAAAATTTTTAGGTGTTATTGATCAGTATCTTTTAACAC
TAAATCAGCGGAATCAATGGATATGGCAAGTAGGATTAAATTTTTTAAATGATGATAATT
TGAATAACGCTCCAAAAAGTGGCACAAAAATTGGTAGTTGGACCGCTTGGGAAAAAGAAA
GTGGGCAGGGGGTAGGGTATTCTTTATCAGTAGAAAAAAAATGGCCATGGGCAGATCATT
TTTTTAGTAAAACTATGTTTAATGGGAATGGAAAATATTATTGGGATAATAAAAAATACA
ATGAGGCTACTGTGCGTATAGGTGGTGGTTTAGGCTATCAAACTGCCTCAGTTGAAGTCT

CGTTGTTTCCTTTTCAAGAAAAACGCTGGTATGCAGGCGGT

SEQ. ID NO:73
Nucleotide sequence of DNA region (1000 bp ) up-stream from the HifA (pilin)
gene from Haemophilus influenzae (LKP serotype 1 genome)
TAATAAATTGCTCCATAAAGAGGTTTGTGCCTTATAAATAAGGCAATAAAGATTAATATA
AACCGTTTATTAAAATGCCAAAGGCTTAATAAACAGCAAACTTTGTTTTCCCAAAAAAAG
TAAAAAACTCTTCCATTATATATATATATATATATATAATTAAAGCCCTTTTTGAAAAATTT
CATATTTTTTTGAATTAATTCGCTGTAGGTTGGGTTTTTGCCCACATGGAGACATATAAA
AAAGATTTGTAGGGTGGGCGTAAGCCCACGCGGAACATCATCAAACAACTGTAATGTTGT
ATTAGGCACGGTGGGCTTATGCCTCGCCTACGGGGAAATGAATAAGGATAAATATGGGCT
TAGCCCAGTTTATGGATTTAATTATGTTGAAATGGGGAAAACAATGTTTAAAAAAACACT
TTTATTTTTTACCGCACTATTTTTTGCCGCACTTTGTGCATTTTCAGCCAATGCAGATGT
GATTATCACTGGCACCAGAGTGATTTATCCCGCTGGGCAAAAAAATGTTATCGTGAAGTT
AGAAAACAATGATGATTCGGCAGCATTGGTGCAAGCCTGGATTGATAATGGCAATCCAAA
TGCCGATCCAAAATACACCAAAACCCCTTTTGTGATTACCCCGCCTGTTGCTCGAGTGGA
AGCGAAATCAGGGCAAAGTTTGCGGATTACGTTCACAGGCAGCGAGCCTTTACCTGATGA
TCGCGAAAGCCTCTTTTATTTTAATTTGTTAGATATTCCGCCGAAACCTGATGCGGCATT
TCTGGCAAAACACGGCAGCTTTATGCAAATTGCCATTCGCTCACGTTTGAAGTTGTTTTA
TCGCCCTGCGAAACTCTCGATGGATTCTCGTGATGCAATGAAAAAAGTAGTGTTTAAAGC
CACACCTGAAGGGGTGTTGGTGGATAATCAAACCCCTTATTATATGAACTACATTGGTTT
GTTACATCAAAATAAACCTGCGAAAAATGTCAAAATGGTTG

SEQ. ID NO:73
Nucleotide sequence of DNA region (1000 bp ) up-stream from the HifE (tip
pilin) gene from Haemophilus influenzae (LKP serotype 1 genome)
TAGTAGATTTCCGCACGGGCAAAAATACAATGGTGTTATTTAACCTCACTTTGCCAAATG
GCGAGCCAGTGCCAATGGCATCCACCGCACAAGATAGCGAAGGGGCATTTGTGGGCGATG
TGGTGCAAGGTGGTGTGCTTTTCGCTAATAAACTTACCCAGCCAAAAGGCGAGTTAATCG
TCAAATGGGGTGAGCGAGAAAGCGAACAATGCCGTTTCCAATATCAAGTTGATTTGGATA
ACGCACAAATACAAAGTCACGATATTCAATGCAAAACCGCAAAATAAATAATTGAAGAGG
ATTTATGCAAAAAACACCCAAAAAATTAACCGCGCTTTTCCATCAAAAATCCACTGCTAC
TTGTAGTGGAGCAAATTATAGTGGAGCAAATTATAGTGGCTCAAAATGCTTTAGGTTTCA
TCGTCTGGCTCTGCTTGCTTGCGTGGCTCTGCTTGATTGCATTGTGGCACTGCCTGCTTA
TGCTTACGATGGCAGAGTGACCTTTCAAGGGGAGATTTTAAGTGATGGCACTTGTAAAAT
TGAAACAGACAGCCAAAATCGCACGGTTACCCTGCCAACAGTGGGAAAAGCTAATTTAAG
CCACGCAGGGCAAACCGCCGCCCCTGTGCCTTTTTCCATCACGTTAAAAGAATGCAATGC
AGATGATGCTATGAAAGCTAATCTGCTATTTAAAGGGGGAGACAACACAACAGGGCAATC
TTATCTTTCCAATAAGGCAGGCAACGGCAAAGCCACCAACGTGGGCATTCAAATTGTCAA
AGCCGATGGCATAGGCACGCCTATCAAGGTGGACGGCACCGAAGCCAACAGCGAAAAAGC
CCCCGACACAGGTAAAGCGCAAAACGGCACAGTTATTCAACCCCGTTTTGGCTACTTTGG
CTCGTTATTACGCCACAGGTGAAGCCACCGCAGGCGACGTTGAAGCCACTGCAACTTTTG
AAGTGCAGTATAACTAAAATATTTATTATCCAGTGAAAAAA

SEQ. ID NO:75
Nucleotide sequence of DNA region (1000 bp ) up-stream from the P2 gene from
Haemophilus influenzae (HiRd)
1 TTATCCGCTA ACATTTCATC AGTAATTCCA TGAACTTTAA TCGCATCAGG
51 ATCANCGGGG CGATCTGGCT TAATATAAAT ATGAYAATTA TTACCTGTGT
101 AACGACGATT TATTAATTCA ACTGCACCAA TTTCAATAAT GCAGTGTCCT
151 TCATAATGCG CGCCAAGCTG ATTCATACCT GTAGTTTCAG TATCTAATAC
201 AATTTGGCGA TTGGGATTAA TCATTTGTTC AACCTATCTC TTTCCATTAA
251 AATACTTGCC ATTCTACACA ACAACCTTTT TGTTATGCCK AAACAGATTG
301 AAATTTTTAC TGATGGATCT TGCTTAGGTA ATCCAGGGGC GGGCGGAATT
351 GGTGCCGTAT TGCGTTATAA ACAACATGAA AAAACACTCT CCAAAGGCTA
401 TTTCCAAACC ACCAATAATC GAATGGAATT ACGCGCTGTC ATTGAAGCAT
451 TAAATACATT AAAAGAACCT TGCTTGATCA CGCTTTATAG TGATAGCCAA
501 TATATGAAAA ATGGCATAAC CAAATGGATC TTTAACTGGA AAAAAAATAA
551 TTGGAAAGCA AGTTCTGGAA AGCCTGTAAA AAACCAAGAT TTATGGATAG
601 CCTTAGATGA ATCCATCCAA CGTCATAAAA TTAATTGGCA ATGGGTAAAA
651 GGCCATGCTG GACACAGAGA AAATGAAATT TGCGATGAAT TAGCAAAAAA
701 AGGGGCAGAA AATCCGACAT TGGAAGATAT GGGGTACATA GAAGAATAAT
751 ACAACTGATA TAACGTCATA TTTTTCGATA CCTAAAAATA TTTAATACTT
801 AAACCTAAAA CAGAATAAAA AATAATCAAA TTCATTTAAA AAATGTGATC
851 TCGATCAGAT TTCAAGAAAA TTAAAATTTT GGAGTATTGA CATCAAAAAT
901 TTTTTTTGTA AAGATGCAGC TCGTCCGTTT TGGCGATTGG ACAATTCTAT
951 TGGAGAAAAG TTCAATCATA GATAGTAAAC AACCATAAGG AATACAAATT
1001 A

SEQ. ID NO:76
Nucleotide sequence of DNA coding region (partial) of the Moraxella Catarrhalis
HtrB gene

1   TCAGTGCTTG GTTTTTTAAG ATATGTACCG CTGTCAGTCC TGCATGGATT
51  GGCGGCGTGT GCGTCTTATA TTTCCTATCA TTGCAGGCTT AGTATTTATC
101 GCAGCATCCA AGCCAATTTA ATCTTGGTTC ACCCCAAGAT GCCAGACGCA
151 CAGCGGCAAA AACTCGCCAA ACAAATCCTA AAAAATCAGC TCATCAGTGC
201 AGTCGACAGT CTTAAAACTT GGGCAATGCC ACCAAAATGG TCTATCGCAC
251 AAATTAAAAC GGTTCATCAT GAAGATATCC TAATCAAAGC ACTTGCCAAT
301 CCAAGTGGTA TGCTTGCCAT TGTGCCTCAT ATCGGCACTT GGGAGATGAT
351 GAATGCTTGG CTCAATACCT TTGGCTCCCC TACTATCATG TATAAGCCCA
401 TCAAAAATGC GGCGGTAGAT CGCTTTGTTT TACAGGGGCG TGAAAGACTA
451 AATGCCAGCC TTGTACCCAC AGATGCTAGT GGTGTTAAGG CAATTTTTAA
501 AACACTCAAA GCAGGTGGAT TTAGTATCAT ACTGCCCGAC CATGTACCTG
551 ATCCATCAGG TGGTGAGATT GCTCCTTTTT TTGGTATTAA AACCCTAACC
601 AGTACGCTGG CGTCAAAGCT TGCTGCAAAA ACTGGTTGTG CTCTTGTTGG
651 CTTAAGCTGT ATTCGGCGTG AAGATGGCGA TGGTTTTGAA ATTTTTTGTT
701 ATGAATTAAA TGATGAACAA CTTTATTCAA AAAATACCAA AATTGCAACC
751 ACTGCTTTAA ATGGTGCGAT GGAACAAATG ATTTATCCAC ATTTTTTGCA
801 TTATATGTGG AGCTATCGTC GGTTCAAGCA TACACCACTA TTAAATAATC
851 CTTATTTACT TAATGAAAAT GAGCTAAAAA AAATAGCCAT AAAGCTTCAA
901 GCCATGTCAA AGGATAGTTA TGAG

Protein Seq: 25% identity and 35% similarity with HtrB from E. coli
1   SVLGFLRYVP LSVLHGLAAC ASYISYHCRL SIYRSIQANL ILVHPKMPDA
51  QRQKLAKQIL KNQLISAVDS LKTWAMPPKW SIAQIKTVHH EDILIKALAN
101 PSGMLAIVPH IGTWEMMNAW LNTFGSPTIM YKPIKNAAVD RFVLQGRERL
151 NASLVPTDAS GVKAIFKTLK AGGFSIILPD HVPDPSGGEI APFFGIKTLT
201 STLASKLAAK TGCALVGLSC IRREDGDGFE IFCYELNDEQ LYSKNTKIAT
251 TALNGAMEQM IYPHFLHYMW SYRRFKHTPL LNNPYLLNEN ELKKIAIKLQ
301 AMSKDSYE

SEQ. ID NO:77
Nucleotide sequence of DNA coding region of the Neisseria (meningococcus B)
HtrB gene
1 ATGTTTCGTT TACAATTCGG GCTGTTTCCC CCTTTGCGAA CCGCCATGCA
51 CATCCTGTTG ACCGCCCTGC TCAAATGCCT CTCCCTGCTG CCACTTTCCT
101 GTCTGCACAC GCTGGGAAAC CGGCTCGGAC ATCTGGCGTT TTACCTTTTA
151 AAGGAAGACC GCGCGCGCAT CGTCGCCAAT ATGCGTCAGG CAGGCATGAA
201 TCCCGACCCC AAAACAGTCA AAGCCGTTTT TGCGGAAACG GCAAAAGGCG
251 GTTTGGAACT TGCCCCCGCG TTTTTCAGAA AACCGGAAGA CATAGAAACA
301 ATGTTCAAAG CGGTACACGG CTGGGAACAT GTGCAGCAGG CTTTGGACAA
351 ACACGAAGGG CTGCTATTCA TCACGCCGCA CATCGGCAGC TACGATTTGG
401 GCGGACGCTA CATCAGCCAG CAGCTTCCGT TCCCGCTGAC CGCCATGTAC
451 AAACCGCCGA AAATCAAAGC GATAGACAAA ATCATGCAGG CGGGCAGGGT
501 TCGCGGCAAA GGAAAAACCG CGCCTACCAG CATACAAGGG GTCAAACAAA
551 TCATCAAAGC CCTGCGTTCG GGCGAAGCAA CCATCGTCCT GCCCGACCAC
601 GTCCCCTCCC CTCAAGAAGG CGGGGAAGGC GTATGGGTGG ATTTCTTCGG
651 CAAACCTGCC TATACCATGA CGCTGGCGGC AAAATTGGCA CACGTCAAAG
701 GCGTGAAAAC CCTGTTTTTC TGCTGCGAAC GCCTGCCTGG CGGACAAGGT
751 TTCGATTTGC ACATCCGCCC CGTCCAAGGG GAATTGAACG GCGACAAAGC
801 CCATGATGCC GCCGTGTTCA ACCGCAATGC CGAATATTGG ATACGCCGTT
851 TTCCGACGCA GTATCTGTTT ATGTACAACC GCTACAAAAT GCCG

Protein Sequence - 30% identity and 38% similarity with Htrb E. coli
1 MFRLQFGLFP PLRTAMHILL TALLKCLSLL PLSCLHTLGN RLGHLAFYLL
51 KEDRARIVAN MRQAGMNPDP KTVKAVFAET AKGGLELAPA FFRKPEDIET
101 MFKAVHGWEH VQQALDKHEG LLFITPHIGS YDLGGRYISQ QLPFPLTAMY
151 KPPKIKAIDK IMQAGRVRGK GKTAPTSIQG VKQIIKALRS GEATIVLPDH
201 VPSPQEGGEG VWVDFFGKPA YTMTLAAKLA HVKGVKTLFF CCERLPGGQG
251 FDLHIRPVQG ELNGDKAHDA AVFNRNAEYW IRRFPTQYLF MYNRYKMP

SEQ. ID NO:78
Nucleotide sequence of DNA coding region of the Haemophilus influenzae (non-
typeable) HtrB gene
1 ATGAAAAACG AAAAACTCCC TCAATTTCAA CCGCACTTTT TAGCCCCAAA
51 ATACTGGCTT TTTTGGCTAG GCGTGGCAAT TTGGCGAAGT ATTTTATGTC
101 TTCCCTATCC TATTTTGCGC CATATTGGTC ATGGTTTCGG TTGGCTGTTT
151 TCACATTTAA AAGTGGGTAA ACGTCGAGCT GCCATTGCAC GCCGTAATCT
201 TGAACTTTGT TTCCCTGATA TGCCTGAAAA CGAACGTGAG ACGATTTTGC
251 AAGAAAATCT TCGTTCAGTA GGCATGGCAA TTATCGAAAC TGGCATGGCT
301 TGGTTTTGGT CGGATTCACG TATCAAAAAA TGGTCGAAAG TTGAAGGCTT
351 ACATTATCTA AAAGAAAATC AAAAAGATGG AATTGTTCTC GTCGGTGTTC
401 ATTTCTTAAC GCTAGAACTT GGCGCACGCA TCATTGGTTT ACATCATCCT
451 GGCATTGGTG TTTATCGTCC AAATGATAAT CCTTTGCTTG ATTGGCTACA
501 AACACAAGGC CGTTTACGCT CCAATAAAGA TATGCTTGAT CGTAAAGATT
551 TACGCGGAAT GATCAAAGCT TTACGCCACG AAGAAACCAT TGGTATGCG
601 CCTGATCACG ATTACGGCAG AAAAAATGCC GTTTTTGTTC CTTTTTTTGC

651 AGTACCTGAC ACTTGCACTA CTACTGGTAG TTATTATTTA TTGAAATCCT
701 CGCAAAACAG CAAAGTGATT CCATTTGCGC CATTACGCAA TAAAGATGGT
751 TCAGGCTATA CCGTGAGTAT TTCAGCGCCT GTTGATTTTA CGGATTTACA
801 AGATGAAACG GCGATTGCTG CGCGAATGAA TCAAATCGTA GAAAAGGAAA
851 TCATGAAGGG CATATCACAA TATATGTGGC TACATCGCCG TTTTAAAACA
901 CGTCCAGATG AAAATACGCC TAGTTTATAC GATTAA

Protein Sequence - 57% identity and 66% similarity with HtrB E. coli
1 MKNEKLPQFQ PHFLAPKYWL FWLGVAIWRS ILCLPYPILR HIGHGFGWLF
51 SHLKVGKRRA AIARRNLELC FPDMPENERE TILQENLRSV GMAIIETGMA
101 WFWSDSRIKK WSKVEGLHYL KENQKDGIVL VGVHFLTLEL GARIIGLHHP
151 GIGVYRPNDN PLLDWLQTQG RLRSNKDMLD RKDLRGMIKA LRHEETIWYA
201 PDHDYGRKNA VFVPFFAVPD TCTTTGSYYL LKSSQNSKVI PFAPLRNKDG
251 SGYTVSISAP VDFTDLQDET AIAARMNQIV EKEIMKGISQ YMWLHRRFKT
301 RPDENTPSLY D*

SEQ. ID NO:79
Nucleotide sequence of DNA coding region of the Haemophilus influenzae (non-
typeable) MsbB gene
1 ATGTCGGATA ATCAACAAAA TTTACGTTTG ACGGCGAGAG TGGGCTATGA
51 AGCGCACTTT TCATGGTCGT ATTTAAAGCC TCAATATTGG GGGATTTGGC
101 TTGGTATTTT CTTTTTATTG TTGTTAGCAT TTGTGCCTTT TCGTCTGCGC
151 GATAAATTGA CGGGAAAATT AGGTATTTGG ATTGGGCATA AAGCAAAGAA
201 ACAGCGTACG CGTGCACAAA CTAACTTGCA ATATTGTTTC CCTCATTGGA
251 CTGAACAACA ACGTGAGCAA GTGATTGATA AAATGTTTGC GGTTGTCGCT
301 CAGGTTATGT TTGGTATTGG TGAGATTGCC ATCCGTTCAA AGAAACATTT
351 GCAAAAACGC AGCGAATTTA TCGGTCTTGA ACATATCGAA CAGGCAAAAG
401 CTGAAGGAAA GAATATTATT CTTATGGTGC CACATGGCTG GGCGATTGAT
451 GCGTCTGGCA TTATTTTGCA CACTCAAGGC ATGCCAATGA CTTCTATGTA
501 TAATCCACAC CGTAATCCAT TGGTGGATTG GCTTTGGACG ATTACACGCC
551 AACGTTTCGG CGGAAAAATG CATGCACGCC AAAATGGTAT TAAACCTTTT
601 TTAAGTCATG TTCGTAAAGG CGAAATGGGT TATTACTTAC CCGATGAAGA
651 TTTTGGGGCG GAACAAAGCG TATTTGTTGA TTTCTTTGGG ACTTATAAAG
701 CGACATTACC AGGGTTAAAT AAAATGGCAA AACTTTCTAA AGCCGTTGTT
751 ATTCCAATGT TTCCTCGTTA TAACGCTGAA ACGGGCAAAT ATGAAATGGA
801 AATTCATCCT GCAATGAATT TAAGTGATGA TCCTGAACAA TCAGCCCGAG
851 CAATGAACGA AGAAATAGAA TCTTTTGTTA CGCCAGCGCC AGAGCAATAT
901 GTTTGGATTT TGCAATTATT GCGTACAAGG AAAGATGGCG AAGATCTTTA
951 TGATTAA

Protein Sequence - 45% identity and 56% similarity with MsbB E.coli
1 MSDNQQNLRL TARVGYEAHF SWSYLKPQYW GIWLGIFFLL LLAFVPFRLR
51 DKLTGKLGIW IGHKAKKQRT RAQTNLQYCF PHWTEQQREQ VIDKMFAVVA
101 QVMFGIGEIA IRSKKHLQKR SEFIGLEHIE QAKAEGKNII LMVPHGWAID
151 ASGIILHTQG MPMTSMYNPH RNPLVDWLWT ITRQRFGGKM HARQNGIKPF
201 LSHVRKGEMG YYLPDEDFGA EQSVFVDFFG TYKATLPGLN KMAKLSKAVV
251 IPMFPRYNAE TGKYEMEIHP AMNLSDDPEQ SARAMNEEIE SFVTPAPEQY
301 VWILQLLRTR KDGEDLYD*

SEQ. ID NO:80
Nucleotide sequence of DNA coding region of the Moraxella catarrhalis MsbB
gene
1 ATGAGTTGCC ATCATCAGCA TAAGCAGACA CCCAAACACG CCATATCCAT
51 TAAGCATATG CCAAGCTTGA CAGATACTCA TAAACAAAGT AGCCAAGCTG
101 AGCCAAAATC GTTTGAATGG GCGTTTTTAC ATCCCAAATA TTGGGGAGTT
151 TGGCTGGCTT TTGCGTTGAT TTTACCGCTG ATTTTTCTAC CGCTGCGTTG
201 GCAGTTTTGG ATCGGCAAGC GTCTTGGCAT TTTGGTACAT TACTTAGCTA
251 AAAGCCGAGT TCAAGACACT CTAACCAACC TGCAGCTTAC CTTCCCAAAT
301 CAACCAAAAT CAAAACACAA GGCCACCGCA CGGCAAGTAT TTATTAATCA
351 AGGTATTGGT ATTTTTGAAA GTTTATGTGC ATGGTTTCGC CCTAATGTCT
401 TTAAACGCAC TTTTAGCATT TCTGGTTTAC AGCATTTGAT TGATGCCCAA
451 AAACAAAATA AAGCGGTGAT TTTACTTGGT GGACATCGCA CGACGCTTGA
501 TTTGGGCGGT CGGTTATGTA CACAGTTTTT TGCGGCGGAC TGCGTGTATC
551 GCCCACAAAA CAACCCTTTG CTTGAATGGT TTATCTATAA TGCACGCCGC
601 TGTATCTTTG ATGAGCAAAT CTCAAATCGT GATATGAAAA AACTCATCAC
651 TCGGCTCAAA CAAGGTCGGA TAATTTGGTA TTCACCTGAT CAAGATTTTG
701 GTCTTGAGCA TGGCGTGATG GCGACCTTTT TTGGTGTGCC TGCAGCAACG
751 ATTACCGCTC AGCGTCGTCT TATTAAGCTG GGTGATAAAG CCAATCCTCC
801 TGTCATCATC ATGATGGATA TGCTCAGACA AACGCCCGAT TATATCGCAA
851 AAGGTCACCG TCCACATTAT CACATCAGCC TAAGCGCTGT GTTAAAAAAT
901 TATCCCAGCG ATGACGAAAC CGCCGATGCT GAACGCATCA ATCGACTGAT
951 TGAGCAAAAT ATTCAAAAAG ATTTAACCCA GTGGATGTGG TTTCATCGCC
1001 GCTTTAAAAC TCAAGCCGAT GACACCAATT ACTATCAACA TTAATG

Protein Sequence - 28% identity and 37 similarity with MsbB of E. coli
1 MSCHHQHKQT PKHAISIKHM PSLTDTHKQS SQAEPKSFEW AFLHPKYWGV

51 WLAFALILPL IFLPLRWQFW IGKRLGILVH YLAKSRVQDT LTNLQLTFPN
101 QPKSKHKATA RQVFINQGIG IFESLCAWFR PNVFKRTFSI SGLQHLIDAQ
151 KQNKAVILLG GHRTTLDLGG RLCTQFFAAD CVYRPQNNPL LEWFIYNARR
201 CIFDEQISNR DMKKLITRLK QGRIIWYSPD QDFGLEHGVM ATFFGVPAAT
251 ITAQRRLIKL GDKANPPVII MMDMLRQTPD YIAKGHRPHY HISLSAVLKN
301 YPSDDETADA ERINRLIEQN IQKDLTQWMW FHRRFKTQAD DTNYYQH*

SEQ. ID NO:81
Nucleotide sequence of DNA coding region of the Neisseria (meningococcus B)
MsbB gene
1 ATGAAATTTA TATTTTTTGT ACTGTATGTT TTGCAGTTTC TGCCGTTTGC
51 GCTGCTGCAC AAACTTGCCG ACCTGACGGG TTTGCTCGCC TACCTTTTGG
101 TCAAACCCCG CCGCCGTATC GGCGAAATCA ATTTGGCAAA ATGCTTTCCC
151 GAGTGGGACG GAAAAAAGCG CGAAACCGTA TTGAAGCAGC ATTTCAAACA
201 TATGGCGAAA CTGATGCTTG AATACGGCTT ATATTGGTAC GCGCCTGCCG
251 GGCGTTTGAA ATCGCTGGTG CGTTACCGCA ATAAGCATTA TTTGGACGAC
301 GCGCTGGCGG CGGGGGAAAA AGTCATCATT CTGTACCCGC ACTTCACCGC
351 GTTCGAGATG GCGGTGTACG CGCTTAATCA GGATGTACCG CTGATCAGTA
401 TGTATTCCCA CCAAAAAAAC AAGATATTGG ACGCACAGAT TTTGAAAGGC
451 CGCAACCGCT ACGACAATGT CTTCCTTATC GGGCGCACCG AAGGCGTGCG
501 CGCCCTCGTC AAACAGTTCC GCAAAAGCAG CGCGCCGTTT CTGTATCTGC
551 CCGATCAGGA TTTCGGACGC AACGATTCGG TTTTTGTGGA TTTTTTCGGT
601 ATTCAGACGG CAACGATTAC CGGCTTGAGC CGCATTGCCG CGCTTGCAAA
651 TGCAAAAGTG ATACCCGCCA TCCCCGTCCG CGAGGCGGAC AATACGGTTA
701 CATTGCATTT CTACCCGGCT TGGGAATCCT TTCCGAGTGA AGATGCGCAG
751 GCCGACGCGC AGCGCATGAA CCGTTTTATC GAGGAACCGT GCGCGAACAT
801 CCCGAGCAGT ATTTTTGGCT GCACAAGCGT TTCAAAACCC GTCCGGAAGG
851 CAGCCCCGAT TTTTACTGAT ACGTAA

Protein Sequence - 25% identity and 36% identity with MsbB E. coli
1 MKFIFFVLYV LQFLPFALLH KLADLTGLLA YLLVKPRRRI GEINLAKCFP
51 EWDGKKRETV LKQHFKHMAK LMLEYGLYWY APAGRLKSLV RYRNKHYLDD
101 ALAAGEKVII LYPHFTAFEM AVYALNQDVP LISMYSHQKN KILDAQILKG
151 RNRYDNVFLI GRTEGVRALV KQFRKSSAPF LYLPDQDFGR NDSVFVDFFG
201 IQTATITGLS RIAALANAKV IPAIPVREAD NTVTLHFYPA WESFPSEDAQ
251 ADAQRMNRFI EEPCANIPSS IFGCTSVSKP VRKAAPIFTD T*

### Embodiments of the invention are described in the subsequent lettered paragraphs:

A. A genetically-engineered bleb preparation from a Gram-negative bacterial strain characterized in that said preparation is obtainable by employing one or more processes selected from the following group:
   a) a process of reducing immunodominant variable or non-protective antigens within the bleb preparation comprising the steps of determining the identity of such antigen, engineering a bacterial strain to produce less or none of said antigen, and making blebs from said strain;
   b) a process of upregulating expression of protective OMP antigens within the bleb preparation comprising the steps of identifying such antigen, engineering a bacterial strain so as to introduce a stronger promoter sequence upstream of a gene encoding said antigen such that said gene is expressed at a level higher than in the non-modified bleb, and making blebs from said strain;
   c) a process of upregulating expression of conditionally-expressed, protective OMP antigens within the bleb preparation comprising the steps of identifying such an antigen, engineering a bacterial strain so as to remove the repressive control mechanisms of its expression, and making blebs from said strain;
   d) a process of modifying lipid A portion of bacterial LPS within the bleb preparation, comprising the steps of identifying a gene involved in rendering the lipid A portion of LPS toxic, engineering a bacterial strain so as to reduce or switch off expression of said gene, and making blebs from said strain;
   e) a process of modifying lipid A portion of bacterial LPS within the bleb preparation, comprising the steps of identifying a gene involved in rendering the lipid A portion of LPS less toxic, engineering a bacterial strain so as to introduce a stronger promoter sequence upstream of said gene such that said gene is expressed at a level higher than in the non-modified bleb, and making blebs from said strain;
   f) a process of reducing lipid A toxicity within the bleb preparation and increasing the levels of protective antigens, comprising the steps of engineering the chromosome of a bacterial strain to incorporate a gene encoding a Polymyxin A peptide, or a derivative or analogue thereof, fused to a protective antigen, and making blebs from said strain; or
   g) a process of creating conserved OMP antigens on the bleb preparation comprising the steps of identifying such antigen, engineering a bacterial strain so as to delete variable regions of a gene encoding said antigen, and making blebs from said strain.
B. The genetically-engineered bleb preparation of Paragraph A, wherein said preparation is obtainable by employing one or more further processes selected from the following group:
   h) a process of reducing expression within the bleb preparation of an antigen which shares a structural similarity with a human structure and may be capable of inducing an auto-immune response in humans, comprising the steps of identifying a gene involved in the biosynthesis of the antigen, engineering a bacterial strain so as to reduce or switch off expression of said gene, and making blebs from said strain; or
   i) a process of upregulating expression of protective OMP antigens within the bleb preparation comprising the steps of identifying such antigen, engineering a bacterial strain so as to introduce into the chromosome one or more further copies of a gene encoding said antigen controlled by a heterologous, stronger promoter sequence, and making blebs from said strain.
C. The bleb preparation of paragraph A or B, wherein the engineering steps of processes a), b), c), d), e), h) and i) are carried out by homologous recombination events between a sequence of at least 30 nucleotides on the bacterial chromosome, and a sequence of at least 30 nucleotides on a vector transformed within the strain.
D. The bleb preparation of paragraph C, wherein the engineering steps are carried out by double cross-over homologous recombination events between two sequences of at least 30 nucleotides on the bacterial chromosome separated by nucleotide sequence 'X', and two sequences of at least 30 nucleotides on a vector transformed within the strain separated by nucleotide sequence 'Y', wherein during the recombination event X and Y are interchanged.
E. The bleb preparation of paragraph D, wherein the two nucleotide sequences are of approximately the same length, and wherein the vector is a linear DNA molecule.
F. The bleb preparation of paragraph D or E, wherein the recombination events of processes a), b), c), d), e) and h) are carried out within the region of the chromosome 1000 bp upstream of the initiation codon of the gene of interest.
G. The bleb preparation of paragraph F, wherein for process a), d) or h) nucleotide sequence X comprises part of the promoter region of the gene, and nucleotide sequence Y comprises either a weak promoter region, or no promoter region.
H. The bleb preparation of paragraph F, wherein for process b) or e) nucleotide sequence Y comprises a strong promoter region for the bacterium.
I. The bleb preparation of paragraph H, wherein nucleotide sequence Y is inserted 200-600 bp upstream of the initiation codon of the gene of interest.
J. The bleb preparation of paragraph I, wherein nucleotide sequence Y is inserted approximately 400 bp upstream of the initiation codon of the gene of interest.
K. The bleb preparation of paragraph F, wherein for process c) nucleotide sequence X comprises part of the repressive control sequence of the promoter, and nucleotide sequence Y comprises no such sequence.
L. The bleb preparation of paragraph D or E, wherein the recombination events of processes a), d), and h) are carried out such that nucleotide sequence X comprises part of the coding sequence of the gene of interest.
M. The bleb preparation of paragraph D or E, wherein the recombination events of process i) is carried out such that nucleotide sequence Y comprises the further copy of the gene within an expression cassette.
N. The bleb preparation of paragraphs A-M isolated from a modified *Neisseria meningitidis* B strain.
O. The bleb preparation of paragraph N, obtainable by employing at least process b) and/or i), wherein one or more genes are upregulated from a list comprising: NspA, Hsf-like, Hap, PorA, PorB, OMP85, PilQ, P1dA, FrpB, TbpA, TbpB, FrpA, FrpC, LbpA, LbpB, FhaB, HasR, lipo02, Tbp2 (lipo28), MltA (lipo30), and ctrA.
P. The bleb preparation of paragraph N or O, obtainable by employing at least process a), wherein one or more genes are downregulated from a list comprising: PorA, PorB, PilC, TbpA, TbpB, LbpA, LbpB, Opa, and Opc.
Q. The bleb preparation of paragraphs N-P, obtainable by employing at least process d), wherein one or more genes are downregulated from a list comprising: htrB, msbB and lpxK.
R. The bleb preparation of paragraphs N-Q, obtainable by employing at least process e), wherein one or more genes are upregulated from a list comprising: pmrA, pmrB, pmrE, and pmrF.
S. The bleb preparation of paragraphs N-R, obtainable by employing at least process c), wherein the repressive control sequence of the promoter is the *fur* operator region of either or both of the TbpB or LbpB genes.
T. The bleb preparation of paragraphs N-S, obtainable by employing at least process h), wherein one or more genes are downregulated from a list comprising: galE, siaA, siaB, siaC, siaD, ctrA, ctrB, ctrC, and ctrD.
U. The bleb preparation of paragraphs A-M isolated from a modified *Moraxella catarrhalis* strain.
V. The bleb preparation of paragraph U, obtainable by employing at least process b) and/or i), wherein one or more genes are upregulated from a list comprising: OMP106, HasR, PilQ, OMP85, lipo06, lipo10, lipo11, lipo18, P6, ompCD, CopB, D15, OmplA1, Hly3, LbpA, LbpB, TbpA, TbpB, OmpE, UspA1, UspA2, and Omp21.
W. The bleb preparation of paragraph U or V, obtainable by employing at least process a), wherein one or more genes are downregulated from a list comprising: CopB, OMP106, OmpB1, TbpA, TbpB, LbpA, and LbpB.
X. The bleb preparation of paragraphs U-W, obtainable by employing at least process d), wherein one or more genes are downregulated from a list comprising: htrB, msbB and lpxK.
Y. The bleb preparation of paragraphs U-X, obtainable by employing at least process e), wherein one or more genes are upregulated from a list comprising: pmrA, pmrB, pmrE, and pmrF.
Z. The bleb preparation of paragraphs A-M isolated from a modified *Haemopltilus influenzae* strain.
AA. The bleb preparation of paragraph Z, obtainable by employing at least process b) and/or i), wherein one or more genes are upregulated from a list comprising: D 15, P6, TbpA, TbpB, P2, P5, OMP26, HMW1, HMW2, HMW3, HMW4, Hia, Hsf, Hap, Hin47, and Hif.
AB. The bleb preparation of paragraph Z or AA, obtainable by employing at least process a), wherein one or more genes are downregulated from a list comprising: P2, P5, Hif, IgA1-protease, HgpA, HgpB, HMW1, HMW2, Hxu, TbpA, and TbpB.
AC. The bleb preparation of paragraphs Z-AB, obtainable by employing at least process d), wherein one or more genes are downregulated from a list comprising: htrB, msbB and lpxK.
AD. The bleb preparation of paragraphs Z-AC, obtainable by employing at least process e), wherein one or more genes are upregulated from a list comprising: pmrA, pmrB, pmrE, and pmrF.
AE. A genetically-engineered bleb preparation from a Gram-negative bacterial strain characterized in that said preparation is obtainable by employing a process comprising the steps of: introducing a heterologous gene, optionally controlled by a strong promoter sequence, into the chromosome by homologous recombination, and making blebs from said strain.
AF. The bleb preparation of paragraph AE, wherein the Gram-negative strain is *Neisseria gonorrhoeae,* and the heterologous gene is a protective OMP from C. *trachomatis.*
AG. The bleb preparation of paragraph AE, wherein the Gram-negative strain is *Haemophilus influenzae,* and the heterologous gene is a protective OMP from *Moraxella catarrhalis.*
AH. The bleb preparation of paragraph AE, wherein the Gram-negative strain is *Moraxella catarrhalis,* and the heterologous gene is a protective OMP from *Haemophilus influenzae.*
AI. A vaccine comprising the bleb preparation of paragraphs A-AH and a pharmaceutically acceptable excipient.
AJ. A meningococcal vaccine comprising the bleb preparation of paragraphs N-T and one or more plain or conjugated meningococcal capsular polysaccharides selected from the serotypes A, C, Y or W.
AK. A meningitis vaccine comprising the preparation of paragraphs N-T or the bleb preparation of paragraph AF, a conjugated *H. influenzae* b capsular polysaccharide, and one or more plain or conjugated pneumococcal capsular polysaccharides.
AL. An otitis media vaccine comprising the bleb preparation of paragraphs U-Y and one or more plain or conjugated pneumococcal capsular polysaccharides, and one or more antigens that can protect a host against non-typeable *H. influenzae* infection.
AM. An otitis media vaccine comprising the bleb preparation of paragraphs Z-AD and one or more plain or conjugated pneumococcal capsular polysaccharides, and one or more antigens that can protect a host against *Moraxella catarrhalis* infection.
AN. The otitis media vaccine of paragraph AL or AM, wherein the vaccine additionally comprises one or more protein antigens that can protect a host against *Streptococcus pneumoniae* and/or one or more antigens that can protect a host against RSV and/or one or more antigens that can protect a host against influenza virus.
AO. A vector suitable for performing the recombination events described in paragraphs C-AH.
AP. A modified Gram-negative bacterial strain from which the bleb preparation of paragraphs A-AH is made.
AQ. An immuno-protective and non-toxic Gram-negative bleb, ghost, or killed whole cell vaccine suitable for paediatric use.
AR. An isolated polynucleotide sequence which hybridises under highly stringent conditions to at least a 30 nucleotide portion of the nucleotides in SEQ ID NO: 2-23, 25, 27-81 or a complementary strand thereof.
AS. The isolated polynucleotide sequence of paragraph AR comprising at least 30 nucleotides.
AT. The isolated polynucleotide sequence of paragraph AS comprising at least 30 nucleotides of SEQ ID NO: 2-23, 25, 27-81 or a complementary strand thereof.
AU. Use of the isolated polynucleotide sequence of paragraphs AR-AT in performing a homologous recombination event within 1000 bp upstream of a Gram-negative bacterial chromosomal gene in order to either increase or decrease expression of said gene.
AV. A process of genetically upregulating expression of a gene within a Gram-negative bacterial strain comprising the steps of: making a vector comprising a strong promoter sequence and a nucleotide sequence of at least 30 nucleotides which is capable of recombination with a sequence of at least 30 nucleotides in the 1000 bp upstream of the gene, transforming a bacterial strain with the vector, and performing a homologous recombination event between the chromosome and the vector to introduce the promoter within 1000 bp upstream of the initiation codon of the gene.
AW. The process of paragraph AV, wherein the promoter is introduced within 200-600 bp upstream of the initiation codon of the gene.
AX. The process of paragraph AW, wherein the promoter is introduced approximately 400 bp upstream of the initiation codon of the gene.
AY. The process of paragraph AV-AX, wherein the homologous recombination event is effected through a double cross-over between two sequences of at least 30 nucleotides on the bacterial chromosome separated by nucleotide sequence 'X', and two sequences of at least 30 nucleotides on the vector separated by nucleotide sequence Y' which comprises the strong promoter sequence, wherein the vector is a linear nucleotide sequence, and wherein during the recombination event X and Y are interchanged.
AZ. The process of paragraph AY, wherein the two nucleotide sequences are of approximately the same length.
BA. A modified bacterial strain obtainable by the processes of paragraphs AV-AZ.
BB. A method to produce a genetically-engineered bleb preparation from a Gram-negative bacterial strain by employing one or more processes selected from the following group:
   a) a process of reducing immunodominant variable or non-protective antigens within the bleb preparation comprising the steps of determining the identity of such antigen, engineering a bacterial strain to produce less or none of said antigen, and making blebs from said strain;
   b) a process of upregulating expression of protective OMP antigens within the bleb preparation comprising the steps of identifying such antigen, engineering a bacterial strain so as to introduce a stronger promoter sequence upstream of a gene encoding said antigen such that said gene is expressed at a level higher than in the non-modified bleb, and making blebs from said strain;
   c) a process of upregulating expression of conditionally-expressed, protective OMP antigens within the bleb preparation comprising the steps of identifying such an antigen, engineering a bacterial strain so as to remove the repressive control mechanisms of its expression, and making blebs from said strain;
   d) a process of modifying lipid A portion of bacterial LPS within the bleb preparation, comprising the steps of identifying a gene involved in rendering the lipid A portion of LPS toxic, engineering a bacterial strain so as to reduce or switch off expression of said gene, and making blebs from said strain;
   e) a process of modifying lipid A portion of bacterial LPS within the bleb preparation, comprising the steps of identifying a gene involved in rendering the lipid A portion of LPS less toxic, engineering a bacterial strain so as to introduce a stronger promoter sequence upstream of said gene such that said gene is expressed at a level higher than in the non-modified bleb, and making blebs from said strain;
   f) a process of reducing lipid A toxicity within the bleb preparation and increasing the levels of protective antigens, comprising the steps of engineering the chromosome of a bacterial strain to incorporate a gene encoding a Polymyxin A peptide, or a derivative or analogue thereof, fused to a protective antigen, and making blebs from said strain;
   g) a process of creating conserved OMP antigens on the bleb preparation comprising the steps of identifying such antigen, engineering a bacterial strain so as to delete variable regions of a gene encoding said antigen, and making blebs from said strain;
   h) a process of reducing expression within the bleb preparation of an antigen which shares a structural similarity with a human structure and may be capable of inducing an auto-immune response in humans, comprising the steps of identifying a gene involved in the biosynthesis of the antigen, engineering a bacterial strain so as to reduce or switch off expression of said gene, and making blebs from said strain; or
   i) a process of upregulating expression of protective OMP antigens within the bleb preparation comprising the steps of identifying such antigen, engineering a bacterial strain so as to introduce into the chromosome one or more further copies of a gene encoding said antigen controlled by a heterologous, stronger promoter sequence, and making blebs from said strain.
BC. A method of immunizing a human host against a disease caused by infection of one or more of the following: Neisseria meningitidis, Neisseria gonorrhoeae, Haemophilus influenza, Moraxella catarrhalis, Pseudomonas aeruginosa, Chlamydia trachomatis, Chlamydia pneumonia, which method comprises administering to the host an immunoprotective dose of the bleb preparation of paragraphs A-AH.
BD. Use of the bleb preparation of paragraphs A-AH in the manufacture of a medicamant for immunizing a human host against a disease caused by infection of one or more of the following: Neisseria meningitidis, Neisseria gonorrhoeae, Haemophilus influenza, Moraxella catarrhalis, Pseudomonas aeruginosa, Chlamydia trachomatis, Chlamydia pneumonia.

## Claims

1. A vaccine formulation containing wild-type meningococcus B bleb preparations from two or more strains belonging to several subtype/serotypes formulated with an other antigen.

2. The vaccine formulation of claim 1, wherein the subtypes are selected from a list consisting of P1.15, P1.7,16, P1.4 and P1.2.

3. The vaccine formulation of claim 1 or 2 formulated with 1, 2, 3, or all 4 of the following meningococcal capsular polysaccharides: A, C, Y or W.

4. The vaccine formulation of claim 3, wherein the meningococcal capsular polysaccharides are plain.

5. The vaccine formulation of claim 3, wherein the meningococcal capsular polysaccharides are conjugated to a protein carrier.

6. The vaccine formulation of any of claims 1-5, formulated with a conjugated *H*. *influenzae* b capsular polysaccharide, and one or more plain or conjugated pneumococcal capsular polysaccharides.

7. The vaccine formulation of claim 6, wherein the pneumococcal capsular polysaccharide is selected from a list of serotypes consisting of: 1, 2, 3, 4, 5, 6B, 7F, 8, 9N, 9V, 10A, 11A, 12F, 14, 15B, 17F, 18C, 19A, 19F, 20, 22F, 23F and 33F.

8. The vaccine formulation of claim 6, wherein the pneumococcal capsular polysaccharide is selected from a list of serotypes consisting of: 1, 3, 4, 5, 6B, 7F, 9V, 14, 18C, 19F and 23F.

9. The vaccine formulation of claims 6-8 comprising one or more protein antigens that can protect a host against *Streptococcus pneumoniae* infection.

10. The vaccine formulation of claim 9, comprising a pneumococcal protein antigen which is a toxin, adhesin, 2-component signal transducer, or lipoprotein of *Streptococcus pneumoniae,* or fragments thereof.


SEQUENCE LISTING

<110> SmithKline Beecham Biologicals s.a.

<120> Vaccine Composition

<130> B45189

<160> 156

<170> FastSEQ for Windows Version 3.0

<210> 1
<211> 5893
<212> DNA
<213> Artificial Sequence

<220>
<223> pCMK(+) vector

<400> 1
tcttccgctt cctcgctcac tgactcgctg cgctcggtcg ttcggctgcg gcgagcggta 60
tcagctcact caaaggcggt aatacggtta tccacagaat caggggataa cgcaggaaag 120
aacatgtgag caaaaggcca gcaaaaggcc aggaaccgta aaaaggccgc gttgctggcg 180
tttttccata ggctccgccc ccctgacgag catcacaaaa atcgacgctc aagtcagagg 240
tggcgaaacc cgacaggact ataaagatac caggcgtttc cccctggaag ctccctcgtg 300
cgctctcctg ttccgaccct gccgcttacc ggatacctgt ccgcctttct cccttcggga 360
agcgtggcgc tttctcatag ctcacgctgt aggtatctca gttcggtgta ggtcgttcgc 420
tccaagctgg gctgtgtgca cgaacccccc gttcagcccg accgctgcgc cttatccggt 480
aactatcgtc ttgagtccaa cccggtaaga cacgacttat cgccactggc agcagccact 540
ggtaacagga ttagcagagc gaggtatgta ggcggtgcta cagagttctt gaagtggtgg 600
cctaactacg gctacactag aagaacagta tttggtatct gcgctctgct gaagccagtt 660
accttcggaa aaagagttgg tagctcttga tccggcaaac aaaccaccgc tggtagcggt 720
ggtttttttg tttgcaagca gcagattacg cgcagaaaaa aaggatctca agaagatcct 780
ttgatctttt ctacggggtc tgacgctcag tggaacgaaa actcacgtta agggattttg 840
gtcatgagat tatcaaaaag gatcttcacc tagatccttt taaattaaaa atgaagtttt 900
aaatcaatct aaagtatata tgagtaaact tggtctgaca gttaccaatg cttaatcagt 960
gaggcaccta tctcagcgat ctgtctattt cgttcatcca tagttgcctg actccccgtc 1020
gtgtagataa ctacgatacg ggagggctta ccatctggcc ccagtgctgc aatgataccg 1080
cgagacccac gctcaccggc tccagattta tcagcaataa accagccagc cggaagggcc 1140
gagcgcagaa gtggtcctgc aactttatcc gcctccatcc agtctattaa ttgttgccgg 1200
gaagctagag taagtagttc gccagttaat agtttgcgca acgttgttgc cattgctaca 1260
ggcatcgtgg tgtcacgctc gtcgtttggt atggcttcat tcagctccgg ttcccaacga 1320
tcaaggcgag ttacatgatc ccccatgttg tgcaaaaaag cggttagctc cttcggtcct 1380
ccgatcgttg tcagaagtaa gttggccgca gtgttatcac tcatggttat ggcagcactg 1440
cataattctc ttactgtcat gccatccgta agatgctttt ctgtgactgg tgagtactca 1500
accaagtcat tctgagaata gtgtatgcgg cgaccgagtt gctcttgccc ggcgtcaata 1560
cgggataata ccgcgccaca tagcagaact ttaaaagtgc tcatcattgg aaaacgttct 1620
tcggggcgaa aactctcaag gatcttaccg ctgttgagat ccagttcgat gtaacccact 1680
cgtgcaccca actgatcttc agcatctttt actttcacca gcgtttctgg gtgagcaaaa 1740
acaggaaggc aaaatgccgc aaaaaaggga ataagggcga cacggaaatg ttgaatactc 1800
atactcttcc tttttcaata ttattgaagc atttatcagg gttattgtct catgagcgga 1860
tacatatttg aatgtattta gaaaaataaa caaatagggg ttccgcgcac atttccccga 1920
aaagtgccac ctgacgtcta agaaaccatt attatcatga cattaaccta taaaaatagg 1980
cgtatcacga ggccctttcg tctcgcgcgt ttcggtgatg acggtgaaaa cctctgacac 2040
atgcagctcc cggagacggt cacagcttgt ctgtaagcgg atgccgggag cagacaagcc 2100
cgtcagggcg cgtcagcggg tgttggcggg tgtcggggct ggcttaacta tgcggcatca 2160
gagcagattg tactgagagt gcaccataaa attgtaaacg ttaatatttt gttaaaattc 2220
gcgttaaatt tttgttaaat cagctcattt tttaaccaat aggccgaaat cggcaaaatc 2280

ccttataaat caaaagaata gcccgagata gggttgagtg ttgttccagt ttggaacaag 2340
agtccactat taaagaacgt ggactccaac gtcaaagggc gaaaaaccgt ctatcagggc 2400
gatggcccac tacgtgaacc atcacccaaa tcaagttttt tggggtcgag gtgccgtaaa 2460
gcactaaatc ggaaccctaa agggagcccc cgatttagag cttgacgggg aaagccggcg 2520
aacgtggcga gaaaggaagg gaagaaagcg aaaggagcgg gcgctagggc gctggcaagt 2580
gtagcggtca cgctgcgcgt aaccaccaca cccgccgcgc ttaatgcgcc gctacagggc 2640
gcgtactatg gttgctttga cgtatgcggt gtgaaatacc gcacagatgc gtaaggagaa 2700
aataccgcat caggcgccat tcgccattca ggctgcgcaa ctgttgggaa gggcgatcgg 2760
tgcgggcctc ttcgctatta cgccagctgg cgaaaggggg atgtgctgca aggcgattaa 2820
gttgggtaac gccagggttt tcccagtcac gacgttgtaa aacgacggcc agtgccaagc 2880
ttgccgtctg aatacatccc gtcattcctc aaaaacagaa aaccaaaatc agaaacctaa 2940
aatcccgtca ttcccgcgca ggcgggaatc cagtccgttc agtttcggtc atttccgata 3000
aattcctgct gcttttcatt tctagattcc cactttcgtg ggaatgacgg cggaagggtt 3060
ttggtttttt ccgataaatt cttgaggcat tgaaattcta gattcccgcc tgcgcgggaa 3120
tgacggctgt agatgcccga tggtctttat agcggattaa caaaaatcag gacaaggcga 3180
cgaagccgca gacagtacag atagtacgga accgattcac ttggtgcttc agcaccttag 3240
agaatcgttc tctttgagct aaggcgaggc aacgccgtac ttgtttttgt taatccacta 3300
taaagtgccg cgtgtgtttt tttatggcgt tttaaaaagc cgagactgca tccgggcagc 3360
agcgcatcgg cccgcacgag gtctctggag tcgcgagcat caagggcgaa ttctgcaggg 3420
ggggggggga aagccacgtt gtgtctcaaa atctctgatg ttacattgca caagataaaa 3480
atatatcatc atgaacaata aaactgtctg cttacataaa cagtaataca aggggtgtta 3540
tgagccatat tcaacgggaa acgtcttgct cgaggccgcg attaaattcc aacatggatg 3600
ctgatttata tgggtataaa tgggctcgcg ataatgtcgg gcaatcaggt gcgacaatct 3660
atcgattgta tgggaagccc gatgcgccag agttgtttct gaaacatggc aaaggtagcg 3720
ttgccaatga tgttacagat gagatggtca gactaaactg gctgacggaa tttatgcctc 3780
ttccgaccat caagcatttt atccgtactc ctgatgatgc atggttactc accactgcga 3840
tccccgggaa aacagcattc caggtattag aagaatatcc tgattcaggt gaaaatattg 3900
ttgatgcgct ggcagtgttc ctgcgccggt tgcattcgat tcctgtttgt aattgtcctt 3960
ttaacagcga tcgcgtattt cgtctcgctc aggcgcaatc acgaatgaat aacggtttgg 4020
ttgatgcgag tgattttgat gacgagcgta atggctggcc tgttgaacaa gtctggaaag 4080
aaatgcataa gcttttgcca ttctcaccgg attcagtcgt cactcatggt gatttctcac 4140
ttgataacct tatttttgac gaggggaaat taataggttg tattgatgtt ggacgagtcg 4200
gaatcgcaga ccgataccag gatcttgcca tcctatggaa ctgcctcggt gagttttctc 4260
cttcattaca gaaacggctt tttcaaaaat atggtattga taatcctgat atgaataaat 4320
tgcagtttca tttgatgctc gatgagtttt tctaatcaga attggttaat tggttgtaac 4380
actggcagag cattacgctg acttgacggg acggcggctt tgttgaataa atcgaacttt 4440
tgctgagttg aaggatcaga tcacgcatct tcccgacaac gcagaccgtt ccgtggcaaa 4500
gcaaaagttc aaaatcacca actggtccac ctacaacaaa gctctcatca accgtggctc 4560
cctcactttc tggctggatg atggggcgat tcaggcctgg tatgagtcag caacaccttc 4620
ttcacgaggc agacctcagc gccccccccc ccctgcagga ggtctgcgct tgaattgtgt 4680
tgtagaaaca caacgttttt gaaaaaataa gctattgttt tatatcaaaa tataatcatt 4740
tttaaaataa aggttgcggc atttatcaga tatttgttct gaaaaatggt tttttgcggg 4800
ggggggggta taattgaaga cgtatcgggt gtttgcccgg aattgtgagc ggataacaat 4860
tcgatgtttt taggttttta tcaaatttac aaaaggaagc ccatatgcat cctaggccta 4920
ttaatattcc ggagtatacg tagccggcta acgttaacaa ccggtacctc tagaactata 4980
gctagcatgc gcaaatttaa agcgctgata tcgatcgcgc gcagatctga ttaaataggc 5040
gaaaatacca gctacgatca aatcatcgcc ggcgttgatt atgatttttc caaacgcact 5100
tccgccatcg tgtctggcgc ttggctgaaa cgcaataccg gcatcggcaa ctacactcaa 5160
attaatgccg cctccgtcgg tttgcgccac aaattctaaa tatcggggcg gtgaagcgga 5220
tagctttgtt tttgacggct tcgccttcat tctttgattg caatctgact gccaatctgc 5280
ttcagcccca aacaaaaacc cggatacgga agaaaaacgg caataaagac agcaaatacc 5340
gtctgaaaga ttttcagacg gtatttcgca tttttggctt ggtttgcaca tatagtgaga 5400
ccttggcaaa aatagtctgt taacgaaatt tgacgcataa aaatgcgcca aaaaattttc 5460
aattgcctaa aaccttccta atattgagca aaaagtagga aaaatcagaa aagttttgca 5520
ttttgaaaat gagattgagc ataaaatttt agtaacctat gttattgcaa aggtctcgaa 5580
ttgtcattcc cacgcaggcg ggaatctagt ctgttcggtt tcagttattt ccgataaatt 5640
cctgctgcgc cgtctgaaga attcgtaatc atggtcatag ctgtttcctg tgtgaaattg 5700
ttatccgctc acaattccac acaacatacg agccggaagc ataaagtgta aagcctgggg 5760
tgcctaatga gtgagctaac tcacattaat tgcgttgcgc tcactgcccg ctttccagtc 5820
gggaaacctg tcgtgccagc tgcattaatg aatcggccaa cgcgcgggga gaggcggttt 5880
gcgtattggg cgc 5893

<210> 2
<211> 997
<212> DNA
<213> Neisseria meningitidis

<400> 2
ggaaccgaac acgccgttcg gtcatacgcc gccgaaaggt ttgccgcaag acgaagccgc 60
cctcgacatc gaagacgcgg tacacggcgc gctggaaagc gcgggttttg tccactacga 120
aacatcggct tttgcgaaac cagccatgca gtgccgccac aatttgaact actggcagtt 180
cggcgattat ttaggcatag gcgcgggcgc gcacggcaaa atttcctatc ccgaccgcat 240
cgagcgcacc gtccgccgcc gccaccccaa cgactacctc gccttaatgc aaaaccgacc 300
gagcgaagcc gtcgaacgca aaaccgtcgc cgccgaagat ttgccgttcg aattcatgat 360
gaacgccctg cgcctgaccg acggcgtacc caccgcgatg ttgcaggagc gcacgggcgt 420
accgagtgcc aaaatcatgg cgcaaatcga aacggcaagg caaaaaggcc tgctggaaac 480
cgaccccgcc gtattccgcc cgaccgaaaa aggacgcttg tttttaaacg atttgctgca 540
gtgtttttta tagtggatta acaaaaacca gtacggcgtt gcctcgcctt agctcaaaga 600
gaacgattct ctaaggtgct gaagcaccaa gtgaatcggt tccgtactat ctgtactgtc 660
tgcggcttcg tcgccttgtc ctgatttttg ttaatccact atataagcgc aaacaaatcg 720
gcggccgccc gggaaaaccc ccccgaacgc gtccggaaaa tatgcttatc gatggaaaac 780
gcagccgcat cccccgccgg gcgtttcaga cggcacagcc gccgccggaa atgtccgacg 840
cttaaggcac agacgcacac aaaaaaccgt atgcctgcac ctgcaacaat ccgacagata 900
ccgctgtttt ttccaaaccg tttgcaagtt tcacccatcc gccgcgtgat gccgccacca 960
ccatttaaag gcaacgcgcg ggttaacggc tttgccg 997

<210> 3
<211> 1000
<212> DNA
<213> Neisseria meningitidis

<400> 3
accattgccg cccgcgccgg cttccaaagc ggcgacaaaa tacaatccgt caacggcaca 60
cccgttgcag attggggcag cgcgcaaacc gaaatcgtcc tcaacctcga agccggcaaa 120
gtcgccgtcg ggttcagacg gcatcaggcg cgcaaaccgt ccgcaccatc gatgccgcag 180
gcacgccgga agccggtaaa atcgcaaaaa accaaggcta catcggactg atgcccttta 240
aaatcacaac cgttgccggt ggcgtggaaa aaggcagccc cgccgaaaaa gcaggcctga 300
aaccgggcga caggctgact gccgccgacg gcaaacccat tacctcatgg caagaatggg 360
caaacctgac ccgccaaagc cccggcaaaa aaatcaccct gaactacgaa cgcgccggac 420
aaacccatac cgccgacatc cgccccgata ctgtcgaaca gcccgaccac accctgatcg 480
ggcgcgtcgg cctccgtccg cagccggaca gggcgtggga cgcgcaaatc cgccgcagct 540
accgtccgtc tgttatccgc gcattcggca tgggctggga aaaaaccgtt tcccactcgt 600
ggacaaccct caaatttttc ggcaaactaa tcagcggcaa cgcctccgtc agccatattt 660
ccgggccgct gaccattgcc gacattgccg gacagtccgc cgaactcggc ttgcaaagtt 720
atttggaatt tttggcactg gtcagcatca gcctcggcgt gctgaacctg ctgcccgtcc 780
ccgttttgga cggcggccac ctcgtgtttt atactgccga atggatacgc ggcaaacctt 840
tgggcgaacg cgtccaaaac atcggtttgc gcttcgggct tgccctcatg atgctgatga 900
tggcggtcgc cttcttcaac gacgttaccc ggctgctcgg ttagatttta cgtttcggaa 960
tgccgtctga aaccgcattc cgcaccacaa ggaactgaca 1000

<210> 4
<211> 1036
<212> DNA
<213> Neisseria meningitidis

<400> 4
attcccgcgc aggcgggaat ccagaaacgc aacgcaacag gaatttatcg gaaaaaacag 60
aaacctcacc gccgtcattc ccgcaaaagc gggaatctag aaacacaacg cggcaggact 120
ttatcagaaa aaacagaaac cccaccgccg tcattcccgc aaaagcggga atccagaccc 180
gtcggcacgg aaacttaccg gataaaacag tttccttaga ttccacgtcc tagattcccg 240
ctttcgcggg aatgacgaga ttttagatta tgggaattta tcaggaatga ttgaatccat 300
agaaaaacca caggaatcta tcagaaaaaa cagaaacccc caccgcgtca ttcccgcgca 360

ggcgggaatc cagaaacaca acgcggcagg actttatcgg aaaaaaccga aaccccaccg 420
accgtcattc ccgcaaaagt tggaatccaa aaacgcaacg caacaggaat ttatcggaaa 480
aaacagaaac ccccaccgcg tcattcccgc gcaggcggga atccagaaac acaacgcaac 540
aggaatttat cggaaaaaac agaaacccca ccgaccgtca ttcccgcaaa agcgggaatc 600
cagcaaccga aaaaccacag gaatctatca gcaaaaacag aaacccccac cgaccgtcat 660
tcccgcgcag gcgggaatcc agaaacacaa cgcggcagga ctttatcgga aaaaacagaa 720
accccaccga ccgtcattcc cgcaaaagct ggaatccaaa aacgcaacgc aacaggaatt 780
tatcggaaaa aacagaaacc ccaccgccgt cattcccgca aaagcgggaa tccagacccg 840
tcggcacgga aacttaccgg ataaaacagt ttccttagat tccacgtccc agattcccgc 900
cttcgcggga atgacgagat tttaagttgg gggaatttat cagaaaaccc ccaaccccca 960
aaaaccgggc ggatgccgca ccatccgccc ccaaaccccg atttaaccat tcaaacaaac 1020
caaaagaaaa aacaaa 1036

<210> 5
<211> 772
<212> DNA
<213> Neisseria meningitidis

<400> 5
gcgatgtcgg gaagccttct cccgaatcat taccccttga gtcgctgaaa atcgcccaat 60
ctccggaaaa cggcggcaat catgacggca agagcagcat cctgaacctc agtgccattg 120
ccaccaccta ccaagcaaaa tccgtagaag agcttgccgc agaagcggca caaaatgccg 180
agcaaaaata acttacgtta gggaaaccat gaaacactat gccttactca tcagctttct 240
ggctctctcc gcgtgttccc aaggttctga ggacctaaac gaatggatgg cacaaacgcg 300
acgcgaagcc aaagcagaaa tcataccttt ccaagcacct accctgccgg ttgcgccggt 360
atacagcccg ccgcagctta cagggccgaa cgcattcgac ttccgccgca tggaaaccga 420
caaaaaaggg gaaaatgccc ccgacaccaa gcgtattaaa gaaacgctgg aaaaattcag 480
tttggaaaat atgcgttatg tcggcatttt gaagtctgga cagaaagtct ccggcttcat 540
cgaggctgaa ggttatgtct acactgtcgg tgtcggcaac tatttgggac aaaactacgg 600
tagaatcgaa agcattaccg acgacagcat cgtcctgaac gagctgatag aagacagcac 660
gggcaactgg gtttcccgta aagcagaact gctgttgaat tcttccgaca aaaacaccga 720
acaagcggca gcacctgccg cagaacaaaa ttaagaagag gattactcca tt 772

<210> 6
<211> 1057
<212> DNA
<213> Neisseria meningitidis

<400> 6
gtgcggcaaa aaacagcaaa agcccgctgt cgattgcctg accgtccgcg tccgtaaaat 60
cagcataggt tgccacgcgc ggcttgggcg ttttcccaca caaagcctct gccatcggca 120
gcaggttttt ccccgatatg cgtatcacgc ccacgccgcc gcgcccgggt gcggtagcga 180
ctgccgcaat cgttggaacg ttatccgaca taaaaccccc gaaaattcaa aacagccgcg 240
attatagcaa atgccgtctg aagtccgacg gtttggcttt cagacggcat aaaaccgcaa 300
aaatgcttga taaatccgtc cgcctgacct aatataacca tatggaaaaa cgaaacacat 360
acgccttcct gctcggtata ggctcgctgc tgggtctgtt ccatcccgca aaaaccgcca 420
tccgccccaa tcccgccgac gatctcaaaa acatcggcgg cgattttcaa cgcgccatag 480
agaaagcgcg aaaatgaccg aaaacgcaca ggacaaggcg cggcaggctg tcgaaaccgt 540
cgtcaaatcc ccggagcttg tcgagcaaat cctgtccgac gagtacgtgc aaataatgat 600
agcccggcgt ttccattcgg gatcgttgcc gccgccgtcc gacttggcgc aatacaacga 660
cattatcagc aacggggcag accgcattat ggcaatggcg gaaaaagaac aagccgtccg 720
gcacgaaacc atacggcaag accaaacctt caacaggcgc gggcaactgt acggcttcat 780
cagcgtcatc ctgatactgc tttttgccgt cttcctcgta tggagcggct accccgcaac 840
cgccgcctcc cttgccggcg gcacagtggt tgccttggcg ggtgctttcg tgattggaag 900
aagccgagac caaggcaaaa attaattgca aatcctaggg cgtgcttcat atccgcccga 960
acgccgaacc gcacatatag gcacatcccg cgcgccgccg gaagcggaag ccgcgccctc 1020
ccaaacaaac ccgaatcccg tcagataagg aaaaata 1057

<210> 7
<211> 924
<212> DNA

<213> Neisseria meningitidis

<400> 7
ggaaccgaac acgccgttcg gtcatacgcc gccgaaaggt ttgccgcaag acgaagccgc 60
cctcgacatc gaagacgcgg tacacggcgc gctggaaggc gcgggttttg tccactacga 120
aacatcggct tttgcgaaac cagccatgca gtgccgccac aatttgaact actggcagtt 180
cggcgattat ttaggcatag gcgcgggcgc tcacggcaaa atttcctatc ccgaccgcat 240
cgagcgcacc gtccgccgcc gccaccccaa cgactacctc gccttaatgc aaagccaacc 300
gagtgaagcc gtcgaacgca aaaccgttgc cgccgaagat ttgccgtttg agttcatgat 360
gaacgccctg cgcctgaccg acgcgtaccc gccgcgatgt tgcaggagcg cacgggcgta 420
ccgagtgcca aaatcatggc gcaaatcgaa acggcaaggc aaaaaggcct gctggaaacc 480
gaccccgccg tattccgccc gaccgaaaaa ggacgcttgt ttttaaacga tttgctgcag 540
tgttttttat agtggattaa caaaaaccag tacggcgttg cctcgcctta gctcaaagag 600
aacgattctc taaggtgctg aagcaccaag tgaatcggtt ccgtactatt tgtactgtct 660
gcggcttcgt cgccttgtcc tgatttttgt taatccacta tataagcgca aacaaatcgg 720
cggccgcccg ggaaaacccg ccccgaacgc gtccggaaaa tatgcttatc gatggaaaac 780
gcagccgcat cccccgccgg gcgtttcaga cggcacagcc gccgccggaa atgtccgacg 840
cttaaggcac agacgcacac aaaaccgtat gcctgcacct gcaacaatcc gacagatacc 900
gctgtttttt ccaaaccgtt tgca 924

<210> 8
<211> 1000
<212> DNA
<213> Neisseria meningitidis

<400> 8
aagtgggaat ctaaaaatga aaagcaacag gaatttatcg gaaatgaccg aaactgaacg 60
gactggattc ccgctttcgc gggaatgacg gcgacagggt tgctgttata gtggatgaac 120
aaaaaccagt acgtcgttgc ctcgccttag ctcaaagaga acgattctct aaggtgctga 180
agcaccaagt gaatcggttc cgtcctattt gtactgtctg cggcttcgtc gccttgtcct 240
gatttctgtt cgttttcggt tattcccgat aaattaccgc cgtttctcgt catttcttta 300
acccttcgtc attcccgcgc aggcgggaat ctagtttttt tgagttccag ttgtttctga 360
taaattcttg cagctttgag ttcctagatt cccactttcg tgggaatgac ggtggaaaag 420
ttgccgtgat ttcggataaa ttttcgtaac gcataatttc cgttttaccc gataaatgcc 480
cgcaatctca aatcccgtca ttccccaaaa acaaaaaatc aaaaacagaa atatcgtcat 540
tcccgcgcag gcgggaatct agaccttaga acaacagcaa tattcaaaga ttatctgaaa 600
gtccgagatt ctagattccc actttcgtgg gaatgacgaa ttttaggttt ctgtttttgg 660
ttttctgtcc ttgcgggaat gatgaaattt taagttttag gaatttatcg gaaaaaacag 720
aaaccgctcc gccgtcattc ccgcacaggc ttcgtcattc ccgcgcaggc ttcgtcattc 780
ccgcatttgt taatccacta tattcccgcc gttttttaca tttccgacaa aacctgtcaa 840
caaaaaacaa cacttcgcaa ataaaaacga taatcagctt tgcaaaaatc ccccccccct 900
gttaatataa ataaaaataa ttaattaatt atttttctta tcctgccaaa tcttaacggt 960
ttggatttac ttcccttcat acactcaaga ggacgattga 1000

<210> 9
<211> 1000
<212> DNA
<213> Neisseria meningitidis

<400> 9
ctataaagat gtaaataaaa atctcggtaa cggtaacact ttggctcagc aaggcagcta 60
caccaaaaca gacggtacaa ccgcaaaaat gggggattta cttttagcag ccgacaatct 120
gcacagccgc ttcacgaaca aaatgctatc cattagccat gttcgggaaa acacgatttc 180
cccgtttgtt ttaggctgtc taaacaaata accataaatg tatatcatta tttaaaataa 240
ataaaagtat ttaactatta ttgacgaaat tttagagaaa gagtagactg tcgattaaat 300
gacaaacaat agtgagaaag gaaatattta ctatccgagc acagagcata ttttaggtag 360
cctgtaactg ttcctgctgg cggaagagga tgaaggtgga cttacccgag aataaatgtc 420
ctgttgtgtg atatggatgc catgccgcga agcaattgat gcaatcacgg cagtcctact 480
tgaatgaaac ctgtcgttgc agaatttgaa aacgctattt ttaagaaagg ataaagggag 540
aaagaatttt tggtttttaa gctgcatgaa accgtgttgg aataaatgca cacctacgat 600
aattaataat tttcgttttt tattctacaa gctatttata tatgattgct aaaagtttat 660

tttttagatg ccaaaaaata tattttatat acttcatatt gtttatatgt ctttatttga 720
atatatctta cgatggggaa atatttatat attttataat aaattttact catttgctaa 780
tatgtcatgg aatattactt gtattttgta gaatttttcc atatgaaaat attccattta 840
ctatttttct gaactttatt agtttatttt taatattttt acctcttata tttaccataa 900
gagagctaat tgattcatat tatattgagt cgataattaa tttattctta attttaattc 960
ctcacgttat ttttttaatt tacttgaaag gaaagcagat 1000

<210> 10
<211> 1000
<212> DNA
<213> Neisseria meningitidis

<400> 10
ggaaacagag aaaaaagttt ctcttctatc ttggataaat atatttaccc tcagtttagt 60
taagtattgg aatttatacc taagtagtaa aagttagtaa attattttta actaaagagt 120
tagtatctac cataatatat tctttaacta atttctaggc ttgaaattat gagaccatat 180
gctactacca tttatcaact ttttattttg tttattggga gtgtttttac tatgacctca 240
tgtgaacctg tgaatgaaaa gacagatcaa aaagcagtaa gtgcgcaaca ggctaaagaa 300
caaaccagtt tcaacaatcc cgagccaatg acaggatttg aacatacggt tacatttgat 360
tttcagggca ccaaaatggt tatcccctat ggctatcttg cacggtatac gcaagacaat 420
gccacaaaat ggctttccga cacgcccggg caggatgctt actccattaa tttgatagag 480
attagcgtct attacaaaaa aaccgaccaa ggctgggttc ttgagccata caaccagcaa 540
aacaaagcac actttatcca atttctacgc gacggtttgg atagcgtgga cgatattgtt 600
atccgaaaag atgcgtgtag tttaagtacg actatgggag aaagattgct tacttacggg 660
gttaaaaaaa tgccatctgc ctatcctgaa tacgaggctt atgaagataa aagacatatt 720
cctgaaaatc catattttca tgaattttac tatattaaaa aaggagaaaa tccggcgatt 780
attactcatc ggaataatcg aataaaccaa actgaagaag atagttatag cactagcgta 840
ggttcctgta ttaacggttt cacggtacag tattacccgt ttattcggga aaagcagcag 900
ctcacacagc aggagttggt aggttatcac caacaagtag agcaattggt acagagtttt 960
gtaaacaatt caaataaaaa ataatttaaa ggatcttatt 1000

<210> 11
<211> 1000
<212> DNA
<213> Neisseria meningitidis

<400> 11
acgtccgaac cgtgattccg caacgccgcg cccaaaacca aagcccaagc caaaatgccg 60
atatagttgg cattggcaat cgcgttaatc gggttggcga ccaggttcat cagcagcgat 120
ttcaacactt ccacaatgcc ggaaggcggc gcggcggaca catcgcccgc gcccgccaaa 180
acaatgtgcg tcgggaaaac cataccggcg atgacggcgg tcagggctgc ggaaaacgta 240
ccaatgaggt aaaggatgat aatcggcctg atatgcgcct tgttgccttt ttggtgctgc 300
gcgattgtgg ccgccaccaa aataaatacc aaaaccggcg cgaccgcttt gagcgcgccg 360
acaaacaggc tgccgaacaa gcctgccgcc aagcccagtt gcggggaaac cgaaccgatt 420
acgatgccca acgccaaacc ggcggcaatc tgcctgacca ggctgacgcg gccgatcgca 480
tgaaataagg atttgccgaa cgccataatt cttccttatg ttgtgatatg ttaaaaaatg 540
ttgtatttta aaagaaaact cattctctgt gtttttttta tttttcggct gtgttttaag 600
gttgcgttga tttgccctat gcagtgccgg acaggctttg ctttatcatt cggcgcaacg 660
gtttaattta ttgaacgaaa ataaatttat ttaatcctgc ctattttccg gcactattcc 720
gaaacgcagc ctgttttcca tatgcggatt ggaaacaaaa taccttaaaa caagcagata 780
catttccggc gggccgcaac ctccgaaata ccggcggcag tatgccgtct gaagtgtccc 840
gccccgtccg aacaacacaa aaacagccgt tcgaaaccct gtccgaacag tgttagaatc 900
gaaatctgcc acaccgatgc acgacacccg taccatgatg atcaaaccga ccgccctgct 960
cctgccggct ttatttttct ttccgcacgc atacgcgcct 1000

<210> 12
<211> 772
<212> DNA
<213> Neisseria meningitidis

<400> 12

gcgatgtcgg gaagccttct cccgaatcat taccccttga gtcgctgaaa atcgcccaat 60
ctccggaaaa cggcggcaat catgacggca agagcagcat cctgaacctc agtgccattg 120
ccaccaccta ccaagcaaaa tccgtagaag agcttgccgc agaagcggca caaaatgccg 180
agcaaaaata acttacgtta gggaaaccat gaaacactat gccttactca tcagctttct 240
ggctctctcc gcgtgttccc aaggttctga ggacctaaac gaatggatgg cacaaacgcg 300
acgcgaagcc aaagcagaaa tcataccttt ccaagcacct accctgccgg ttgcgccggt 360
atacagcccg ccgcagctta cagggccgaa cgcattcgac ttccgccgca tggaaaccga 420
caaaaaaggg gaaaatgccc ccgacaccaa gcgtattaaa gaaacgctgg aaaaattcag 480
tttggaaaat atgcgttatg tcggcatttt gaagtctgga cagaaagtct ccggcttcat 540
cgaggctgaa ggttatgtct acactgtcgg tgtcggcaac tatttgggac aaaactacgg 600
tagaatcgaa agcattaccg acgacagcat cgtcctgaac gagctgatag aagacagcac 660
gggcaactgg gtttcccgta aagcagaact gctgttgaat tcttccgaca aaaacaccga 720
acaagcggca gcacctgccg cagaacaaaa ttaagaagag gattactcca tt 772

<210> 13
<211> 1000
<212> DNA
<213> Neisseria meningitidis

<400> 13
tttgtttttt cttttggttt gtttgaatgg ttaaatcggg gtttgggggc ggatggtgcg 60
gcatccgccc ggtttttggg ggttgggggt tttctgataa attcccccaa cttaaaatct 120
cgtcattccc gcgaaggcgg gaatctggga cgtggaatct aaggaaactg ttttatccgg 180
taagtttccg tgccgacggg tctggattcc cgcttttgcg ggaatgacgg cggtggggtt 240
tctgtttttt ccgataaatt cctgttgcgt tgcgtttttg gattccagct tttgcgggaa 300
tgacggtcgg tggggtttct gttttttccg ataaagtcct gccgcgttgt gtttctggat 360
tcccgcctgc gcgggaatga cggtcggtgg gggtttctgt ttttgctgat agattcctgt 420
ggtttttcgg ttgctggatt cccgcttttg cgggaatgac ggtcggtggg gtttctgttt 480
tttccgataa attcctgttg cgttgtgttt ctggattccc gcctgcgcgg gaatgacgcg 540
gtgggggttt ctgttttttc cgataaattc ctgttgcgtt gcgtttttgg attccaactt 600
ttgcgggaat gacggtcggt ggggtttcgg ttttttccga taaagtcctg ccgcgttgtg 660
tttctggatt cccgcctgcg cgggaatgac gcggtggggg tttctgtttt ttctgataga 720
ttcctgtggt ttttctatgg attcaatcat tcctgataaa ttcccataat ctaaaatctc 780
gtcattcccg cgaaagcggg aatctaggac gtggaatcta aggaaactgt tttatccggt 840
aagtttccgt gccgacgggt ctggattccc gcttttgcgg gaatgacggc ggtggggttt 900
ctgttttttc tgataaagtc ctgccgcgtt gtgtttctag attcccgctt ttgcgggaat 960
gacggcggtg aggtttctgt tttttccgat aaattcctgt 1000

<210> 14
<211> 1000
<212> DNA
<213> Neisseria meningitidis

<400> 14
aatcagcata ggttgccacg cgcggcttgg gcgttttccc acacaaagcc tctgccatcg 60
gcagcaggtt tttccccgat atgcgtatca cgcccacgcc gccgcgcccg ggtgcggtag 120
cgactgccgc aatcgttgga acgttatccg acataaaacc cccgaaaatt caaaacagcc 180
gcgattatag caaatgccgt ctgaagtccg acggtttggc tttcagacgg cataaaaccg 240
caaaaatgct tgataaatcc gtccgcctga cctaatataa ccatatggaa aaacgaaaca 300
catacgcctt cctgctcggt ataggctcgc tgctgggtct gttccatccc gcaaaaaccg 360
ccatccgccc caatcccgcc gacgatctca aaaacatcgg cggcgatttt caacgcgcca 420
tagagaaagc gcgaaaatga ccgaaaacgc acaggacaag gcgcggcagg ctgtcgaaac 480
cgtcgtcaaa tccccggagc ttgtcgagca aatcctgtcc gacgagtacg tgcaaataat 540
gatagcccgg cgtttccatt cgggatcgtt gccgccgccg tccgacttgg cgcaatacaa 600
cgacattatc agcaacgggg cagaccgcat tatggcaatg gcggaaaaag aacaagccgt 660
ccggcacgaa accatacggc aagaccaaac cttcaacagg cgcgggcaac tgtacggctt 720
catcagcgtc atcctgatac tgctttttgc cgtcttcctc gtatggagcg gctaccccgc 780
aaccgccgcc tcccttgccg gcggcacagt ggttgccttg gcgggtgctt tcgtgattgg 840
aagaagccga gaccaaggca aaaattaatt gcaaatccta gggcgtgctt catatccgcc 900
cgaacgccga accgcacata taggcacatc ccgcgcgccg ccggaagcgg aagccgcgcc 960
ctcccaaaca aacccgaatc ccgtcagata aggaaaaata 1000

<210> 15
<211> 1000
<212> DNA
<213> Neisseria meningitidis

<400> 15
gattttggtc atcccgacaa gcttcttgtc gaagggcgtg aaattccttt ggttagccaa 60
gagaaaacca tcaagcttgc cgatggcagg gaaatgaccg tccgtgcttg ttgcgacttt 120
ttgacctatg tgaaactcgg acggataaaa accgaacgcc cggcaagtaa accaaaggcg 180
gaagataaaa gggaggatga agagagtgca ggcgttggta acgtcgaaga aggcgaaggc 240
gaagtttccg aagatgaagg cgaagaagcc gaagaaatcg tcgaagaaga acccgaagaa 300
gaagctgaag aggaagaagc tgaacccaaa gaagttgaag aaaccgaaga aaaatcgccg 360
acagaagaaa gcggcagcgg ttcaaacgcc atcctgcctg cctcggaagc ctctaaaggc 420
agggacatcg accttttcct gaaaggtatc cgcacggcgg aagccgacat tccaagaacc 480
ggaaaagcac actataccgg cacttgggaa gcgcgtatcg gcacacccat tcaatgggac 540
aatcaggcgg ataaagaagc ggcaaaagca gaatttaccg ttaatttcgg cgagaaatcg 600
atttccggaa cgctgacgga gaaaaacggt gtacaacctg ctttctatat tgaaaacggc 660
aagattgagg gcaacggttt ccacgcaaca gcacgcactc gtgagaacgg catcaatctt 720
tcgggaaatg gttcgaccaa ccccagaacc ttccaagcta gtgatcttcg tgtagaagga 780
ggattttacg gcccgcagcg gaggaattgg gcggtattat tttcaataag gatgggaaat 840
ctcttggtat aactgaaggt actgaaaata aagttgaagt tgaagctgaa gttgaagttg 900
aagctgaaac tggtgttgtc gaacagttag aacctgatga agttaaaccc caattcggcg 960
tggtattcgg tgcgaagaaa gataataaag aggtggaaaa 1000

<210> 16
<211> 1000
<212> DNA
<213> Neisseria meningitidis

<400> 16
cggcgttaga gtttagggca gtaagggcgc gtccgccctt agatctgtaa gttacgattc 60
cgttaaataa cttttactga ctttgagttt tttgacctaa gggtgaaagc acccttactg 120
cttaaagtcc aacgacaaaa accaaaagac aaaaacactt ttattaccct aaaatcgaac 180
acccataaat gacctttttt gtctttggcg aggcggcagt aagggcgcgt ccgcccttag 240
atctgtaagt tatgattccg ttaaatagcc tttactgact ttgagttttt tgacctaagg 300
gcggacgcgc ccttactgct tcaccttcaa tgggctttga attttgttcg ctttggcttg 360
cttgacctaa gggtgaaagc acccttactg ccgcctcgcc aaagacgaaa agggttattt 420
acgggggttg gattttaggc agtaagggcg cgtccgccct tagatctgta agttatgatt 480
ccgttaaata gcctttactg actttgagtt ttttgaccta agggtgaaag cacccttact 540
gcttcacctt caatgggctt tgaattttgt tcgctttggc ttgcttgatc taagggtgaa 600
agcaccctta ctgccgtctc gccgaagaca acgagggcta tttacggcgt tagagtttag 660
ggcagtaagg gcgcgtccgc ccttagatcc agacagtcac gcctttgaat agtccatttt 720
gccaaagaac tctaaaacgc aggacctaag ggtgaaagca cccttactgc cttacatcca 780
agcaccctta ctgcaccacg tccacgcacc cttactgccc tacgtccacg cacccttact 840
gccctacatc caagcaccct tactgcctta catagacatg acagacgccg agcagcggaa 900
caggactaaa aacaattaag tgatattttt gcccaactat aatagacatg tataattata 960
ttactattaa taataattag tttatcctcc ttttcatccc 1000

<210> 17
<211> 731
<212> DNA
<213> Neisseria meningitidis

<400> 17
tatgaagtcg aagtctgctg ttccaccttc aattatctga attacggaat gttgacgcgc 60
aaaaacagca agtccgcgat gcaggcagga gaaagcagta gtcaagctga tgctaaaacg 120
gaacaagttg gacaaagtat gttcctccaa ggcgagcgca ccgatgaaaa agagattcca 180
aacgaccaaa acgtcgttta tcgggggtct tggtacgggc atattgccaa cggcacaagc 240
tggagcggca atgcttccga taaagagggc ggcaacaggg cggactttac tgtgaatttc 300
ggtacgaaaa aaattaacgg cacgttaacc gctgacaaca ggcaggcggc aacctttacc 360

attgtgggcg atattgaggg caacggtttt tccggtacgg cgaaaactgc tgactcaggt 420
tttgatctcg atcaaagcaa taacacccgc acgcctaagg catatatcac aaacgccaag 480
gtgcagggcg gtttttacgg gcccaaagcc gaagagttgg gcggatggtt tgcctattcg 540
gacgataaac aaacgaaaaa tgcaacagat gcatccggca atggaaattc agcaagcagt 600
gcaactgtcg tattcggtgc gaaacgccaa aagcctgtgc aataagcacg gttgccgaac 660
aatcaagaat aaggcctcag acggcaccgc tccttccgat accgtctgaa agcgaagagt 720
agggaaacac t 731

<210> 18
<211> 373
<212> DNA
<213> Neisseria meningitidis

<400> 18
cgtaccgcat tccgcactgc agtgaaaaaa gtattgaaag cagtcgaagc aggcgataaa 60
gctgccgcac aagcggttta ccaagagtcc gtcaaagtca tcgaccgcat cgccgacaag 120
ggcgtgttcc ataaaaacaa agcggctcgc cacaaaaccc gtttgtctca aaaagtaaaa 180
ccttggcttg attttgcaaa aacctgcaat ccggttttca tcgtcgattc cgaaaacccc 240
tgaagcccga cggtttcggg gttttctgta ttgcggggac aaaatcccga aatggcggaa 300
agggtgcggt tttttatccg aatccgctat aaaatgccgt ctgaaaacca atatgccgac 360
aatgggggtg gag 373

<210> 19
<211> 1000
<212> DNA
<213> Neisseria meningitidis

<400> 19
ttttggcttc cagcgtttca ttgttttcgt acaagtcgta agtcagcttc agattgttgg 60
cttttttaaa gtcttcgacc gtactctcat caacatagtt cgaccagttg tagatgttca 120
gagtatcggt ggcagcggct tcggcattgg cagcagacgc agcgtctgct tgaggttgca 180
cggcgttttt ttcgctgccg ccgcaggctg ccagagacag cgcggccaaa acggctaata 240
cggatttttt catacgggca gattcctgat gaaagaggtt ggaaaaaaag aaatccccgc 300
gccccatcgt taccccggcg caaggtttgg gcattgtaaa gtaaatttgt gcaaactcaa 360
agcgatattg gactgatttt cctaaaaaat tatcctgttt ccaaaagggg agaaaaacgt 420
ccgcccgatt ttgccgtttt tttgcgctgt cagggtgtcc gacgggcgga tagagagaaa 480
aggcttgcat ataatgtaaa ccccctttaa aattgcgcgt ttacagaatt tatttttctt 540
ccaggagatt ccaatatggc aaacagcgca caagcacgca aacgtgcccg ccagtccgtc 600
aaacaacgcg cccacaatgc tagcctgcgt accgcattcc gcaccgcagt gaaaaaagta 660
ttgaaagcag tcgaagcagg cgataaagct gccgcacaag cggtttacca agagtccgtc 720
aaagtcatcg accgcatcgc cgacaagggc gtgttccaca aaaacaaagc ggcacgccac 780
aaaagccgtc tgtctgcaaa agtaaaagcc ttggcttgat ttttgcaaaa ccgccaaggc 840
ggttgatacg cgataagcgg aaaaccctga agcccgacgg tttcggggtt ttctgtattg 900
cgggggcaaa atcccgaaat ggcggaaagg gtgcgatttt ttatccgaat ccgctataaa 960
atgccgtttg aaaaccaata tgccgacaat gggggcggag 1000

<210> 20
<211> 1000
<212> DNA
<213> Neisseria meningitidis

<400> 20
tacggaaact gcaagcggat ccagaagtta cagcgtgcat tattcggtgc ccgtaaaaaa 60
atggctgttt tcttttaatc acaatggaca tcgttaccac gaagcaaccg aaggctattc 120
cgtcaattac gattacaacg gcaaacaata tcagagcagc ctggccgccg agcgcatgct 180
ttggcgtaac agacttcata aaacttcagt cggaatgaaa ttatggacac gccaaaccta 240
taaatacatc gacgatgccg aaatcgaagt gcaacgccgc cgctctgcag gctgggaagc 300
cgaattgcgc caccgtgctt acctcaaccg ttggcagctt gacggcaagt tgtcttacaa 360
acgcgggacc ggcatgcgcc aaagtatgcc tgcaccggaa gaaaacggcg gcgatattct 420
tccaggtaca tctcgtatga aaatcattac tgccggtttg gacgcagccg ccccatttat 480
tttaggcaaa cagcagtttt tctacgcaac cgccattcaa gctcaatgga acaaaacgcc 540

gttggttgcc caagataaat tgtcaatcgg cagccgctac accgttcgcg gatttgatgg 600
ggagcagagt cttttcggag agcgaggttt ctactggcag aatactttaa cttggtattt 660
tcatccgaac catcagttct atctcggtgc ggactatggc cgcgtatttg gcgaaagtgc 720
acaatatgta tcgggcaagc agctgatggg tgcagtggtc ggcttcagag gagggcataa 780
agtaggcggt atgtttgctt atgatctgtt tgccggcaag ccgcttcata aacccaaagg 840
ctttcagacg accaacaccg tttacggctt caacttgaat tacagtttct aacctctgaa 900
ttttttactg atatttagac ggtctttcct tatcctcaga ccgtcaaact ttacctacgt 960
acttggcgcg cagtacgttc atcttcaaaa tggaatagac 1000

<210> 21
<211> 1000
<212> DNA
<213> Neisseria meningitidis

<400> 21
ttatcttggt gcaaaacttt gtcggggtcg gactggctac ggctttgggt ttggacccgc 60
tcatcggtct gattaccggt tcggtgtcgc tgacgggcgg acacggtacg tcaggtgcgt 120
ggggacctaa ttttgaaacg caatacggct tggtcggcgc aaccggtttg ggtattgcat 180
cggctacttt cgggctggtg ttcggcggcc tgatcggcgg gccggttgcg cgccgcctga 240
tcaacaaaat gggccgcaaa ccggttgaaa acaaaaaaca ggatcaggac gacaacgcgg 300
acgacgtgtt cgagcaggca aaacgcaccc gcctgattac ggcggaatct gccgttgaaa 360
cgcttgccat gtttgccgcg tgtttggcgt ttgccgagat tatggacggc ttcgacaaag 420
aatatctgtt cgacctgccc aaattcgtgt ggtgtctgtt tggcggcgtg gtcatccgca 480
acatcctcac tgccgcattc aaggtcaata tgttcgaccg cgccatcgat gtgttcggca 540
atgcttcgct ttcgcttttc ttggcaatgg cgttgctgaa tttgaaactg tgggagctga 600
ccggtttggc ggggcctgta accgtgattc ttgccgtaca aaccgtggtg atggttttgt 660
acgcgacttt tgttacctat gtctttatgg ggcgcgacta tgatgcggca gtattggctg 720
ccggccattg cggtttcggc ttgggtgcaa cgccgacggc ggtggcaaat atgcagtccg 780
tcacgcatac tttcggcgcg tcgcataagg cgtttttgat tgtgcctatg gtcggcgcgt 840
tcttcgtcga tttgattaat gccgcgattc tcaccggttt tgtgaatttc tttaaaggct 900
gattttccgc ctttccgaca aagcacctgc aaggtttacc gcctgcaggt gcttttgcta 960
tgatagccgc tatcggtctg caccgtttgg aaggaacatc 1000

<210> 22
<211> 1000
<212> DNA
<213> Neisseria meningitidis

<400> 22
cctactccac cgattccaat atgctcggcg cgacccacga agccaaagac ttggaatttt 60
tgaactcggg catcaaaatc gtcaaaccca ttatgggcgt tgccttttgg gacgaaaacg 120
ttgaagtcag ccccgaagaa gtcagcgtgc gctttgaaga aggcgtgccg gttgcactga 180
acggcaaaga atacgccgac cccgtcgaac tcttcctcga agccaaccgc atcggcggcc 240
gccacggctt gggtatgagc gaccaaatcg aaaaccgcat catcgaagcc aaatcgcgcg 300
gcatctacga agccccgggt atggcgttgt tccacatcgc ctacgaacgc ttggtgaccg 360
gcatccacaa cgaagacacc atcgaacaat accgcatcaa cggcctgcgc ctcggccgtt 420
tgctctacca aggccgctgg ttcgacagcc aagccttgat gttgcgcgaa accgcccaac 480
gctgggtcgc caaagccgtt accggcgaag ttaccctcga actgcggcgc ggcaacgact 540
actcgattct gaacaccgaa tcgcccaacc tgacctacca acccgaacgc ctgagtatgg 600
aaaaagtcga aggtgcggcg tttaccccgc tcgaccgcat cggacagctc acgatgcgca 660
acctcgacat caccgacacc cgcgccaaac tgggcatcta ctcgcaaagc ggtttgctgt 720
cgctgggcga aggctcggta ttaccgcagt tgggcaataa gaaataaggt ttgctgtttt 780
gcatcattag caacttaagg ggtcgtctga aaagatgatc ccttatgtta aaaggaatcc 840
tatgaaagaa tacaaagtcg tcatttatca ggaaagccag ttgtccagcc tgtttttcgg 900
cgcggcaaag gtcaaccccg tcaatttcag cgcgttcctc aacaaacaaa ccccccgaag 960
gctggcgggt cgagaccttt gcaataacat aggttactaa 1000

<210> 23
<211> 1000
<212> DNA
<213> Neisseria meningitidis

<400> 23
gaatgacaat tcataagttt cccgaaattc caacataacc gaaacctgac aataaccgta 60
gcaactgaac cgtcattccc gcaaaagcgg gaatccagtc cgttcagttt cggtcatttc 120
cgataaatgc ctgttgcttt tcatttctag attcccactt tcgtgggaat gacggcggaa 180
gggttttggt tttttccgat aaattcttga ggcattgaaa ttccaaattc ccgcctgcgc 240
gggaatgacg gctgcagatg cccgacggtc tttatagtgg attaacaaaa atcaggacaa 300
ggcgacgagc tgcagacagt acagatagta cggaaccgat tcacttagtg cttcagtatc 360
ttagagaatc gttctctttg agctaaggcg aggcaacgtc gtactggttt ttgttcatcc 420
actatatatg acacggaaaa cgccgccgtc caaaccatgc cgtctgaaga aaactacaca 480
gataccgccg cttatattac aatcgccgcc ccgtggttcg aaaacctccc acactaaaaa 540
actaaggaaa ccctatgtcc cgcaacaacg aagagctgca aggtatctcg cttttgggta 600
atcaaaaaac ccaatatccg gccgaatacg cgcccgaaat tttggaagcg ttcgacaaca 660
aacatcccga caacgactat ttcgtcaaat tcgtctgccc agagttcacc agcctctgcc 720
ccatgaccgg gcagcccgac ttcgccacca tcgtcatccg ctacattccg cacatcaaaa 780
tggtggaaag caaatccctg aaactctacc tcttcagctt ccgcaaccac ggcgattttc 840
atgaagactg cgtcaacatc atcatgaaag acctcattgc cctgatggat ccgaaataca 900
tcgaagtatt cggcgagttc acaccgcgcg gcggcatcgc cattcatcct ttcgccaatt 960
acggcaaagc aggcaccgag tttgaagcat tggcgcgtaa 1000

<210> 24
<211> 228
<212> DNA
<213> Neisseria meningitidis

<400> 24
gatatcgagg tctgcgcttg aattgtgttg tagaaacaca acgtttttga aaaaataagc 60
tattgtttta tatcaaaata taatcatttt taaaataaag gttgcggcat ttatcagata 120
tttgttctga aaatggtttt tttgcggggg gggggtata attgaagacg tatcgggtgt 180
ttgcccgatg tttttaggtt tttatcaaat ttacaaaagg aagcccat 228

<210> 25
<211> 1000
<212> DNA
<213> Neisseria meningitidis

<400> 25
gttttctgtt tttgagggaa tgacgggatg taggttcgta agaatgacgg gatataggtt 60
tccgtgcgga tggattcgtc attcccgcgc aggcgggaat ctagaacgtg gaatctaaga 120
aaccgtttta tccgataagt ttccgtgcgg acaagtttgg attcccgcct gcgcgggaat 180
gacgggattt taggtttcta attttggttt tctgtttttg agggaatgac gggatgtagg 240
ttcgtaggaa tgacgggata taggtttccg tgcggatgga ttcgtcattc ccgcgcaggc 300
gggaatctag accttagaac aacagcaata ttcaaagatt atctgaaagt ccgagattct 360
agattcccgc ctgagcggga atgacgaaaa gtggcgggaa tgacggttag cgttgcctcg 420
ccttagctca aagagaacga ttctctaagg tgctgaagca ccaagtgaat cggttccgta 480
ctatttgtac tgtctgcggc ttcgtcgcct tgtcctgatt tttgttaatc cactatctcc 540
tgccgcaggg gcgggttttg catccgcccg ttccgaaaga aaccgcgtgt gcgttttttg 600
ccgtctttat aacccccggt ttgcaatgcc ctccaatacc ctcccgagta agtgttgtaa 660
aaatgcaaat cttaaaaaat ttaaataacc atatgttata aaacaaaaaa tacccataat 720
atctctatcc gtccttcaaa atgcacatcg aattccacac aaaaacaggc agaagtttgt 780
tttttcagac aggaacatct atagtttcag acatgtaatc gccgagcccc tcggcggtaa 840
atgcaaagct aagcggcttg gaaagcccgg cctgcttaaa tttcttaacc aaaaaaggaa 900
tacagcaatg aaaaaatccc tgattgccct gactttggca gcccttcctg ttgcagcaat 960
ggctgacgtt accctgtacg gcaccatcaa aaccggcgta 1000

<210> 26
<211> 537
<212> DNA
<213> Neisseria meningitidis

<400> 26

gttttctgtt tttgagggaa tgacgggatg taggttcgta agaatgacgg gatataggtt 60
tccgtgcgga tggattcgtc attcccgcgc aggcgggaat ctagaacgtg gaatctaaga 120
aaccgtttta tccgataagt tttccgtgcg gacaagtttg gattcccgcc tgcgcgggaa 180
tgacgggatt ttaggtttct aattttggtt ttctgttttt gagggaatga cgggatgtag 240
gttcgtagga atgacgggat ataggtttcc gtgcggatgg attcgtcatt cccgcgcagg 300
cgggaatcca gaccttagaa caacagcaat attcaaagat tatctgaaag tccgagattc 360
tagattcccg cctgagcggg aatgacgaaa agtggcggga atgacggtta gcgttgcctc 420
gccttagctc aaagagaacg attctctaag gtgctgaagc actaagtgaa tcggttccgt 480
actatttgta ctgtctgcgg cttcgtcgcc ttgtcctgat ttttgttaat ccactat 537

<210> 27
<211> 1000
<212> DNA
<213> Neisseria meningitidis

<400> 27
atacggccaa tggcttcaga aagcgataag cctctggctg aaaaaccgat ttcttgtgtt 60
ctccccaccg cacccataga cgtaaaggta tagggattgg taatcatggt aaccacatca 120
ccgcgacgca gcaaaatatt ttgtcgcgga tttgcaacta aatcttccaa ggcaacagtt 180
cgtactacat tgccacgtgt cagctgcaca ttcgtatcct gcacatttgc cgttgaacca 240
cctaccgcag ccaccgcatc caacacacgc tcaccggctg ccgtcagcgg catacgcaca 300
ctattcccag cacgaatcac cgacacattc gccgcattat tctgcaccaa acgcaccatc 360
acttgtggct gattggccat ttttttcagg cggcctttaa taatttcctg aacctgacca 420
ggcgttttac cgaccaccga aatatcgcca acaaacggca cagaaaccgt accacgtgcc 480
gtgaccaact gctctggcaa cttagtttga tgcgcactac ccgagcccat cgaagaaagg 540
ccaccaccaa acaatactgc cggcggcgct tcccaaatca taatatccaa tacatcacca 600
atatttagcg taccagccga agcataacca tcgccaaact gagtgaatga ctgatttatc 660
tgagccttat ataataactg agcaaccgta tgattcacat caatcagctc cacttcagga 720
atttgaactt cagattgttg ccctaaagag acaatttttt ttgcgctggg gcctgatgaa 780
ggaatcgcag agcatcctac aattaaactt ccacacaata ataatactgc gtgacgaata 840
taaaatttca ctttaaacac aagccaaatc ctaatataat tataaatggc ctaattatag 900
cacttaatcg aaataaattt atgagtacgt agagtataat tagtattctt ctttccaact 960
tccttatact tatatatata tacttataga ttctaaaatc 1000

<210> 28
<211> 1000
<212> DNA
<213> Neisseria meningitidis

<400> 28
gccaaagcat tgggcgcgga tgccgccgct gccgaacgcg ccgcgcgtct tgccaaagcc 60
gacttggtaa ccgaaatggt cggcgagttc cccgaactgc aaggcacgat gggcaaatac 120
tatgcctgtt tggacggcga aaccgaagaa attgccgaag ccgtcgagca gcactatcag 180
ccgcgttttg ccggcgacaa gctgcccgaa agcaaaattg ccgccgccgt ggcactggcc 240
gacaaactag aaaccttggt cggcatttgg ggcatcggtc tgattccgac cggcgacaaa 300
gacccctacg ccctgcgccg cgctgccttg ggtattttgc gtatgctgat gcagtatggt 360
ttggacgtga acgaactgat tcagacggca ttcgacagct tccccaaagg tttgctcaac 420
gaaaaaacgc cgtctgaaac cgccgacttt atgcaggcgc gccttgccgt gttgctgcaa 480
aacgattatc cgcaagacat cgttgccgcc gtactcgcca aacagccgcg ccgtttggac 540
gatttgaccg ccaaactgca ggccgttgcc gcgttcaaac aactgcccga agccgccgcg 600
ctcgccgccg ccaacaaacg cgtgcaaaac ctgctgaaaa agccgatgcc cgagttgggc 660
gcggttaacg aaagcctgtt gcaacaggac gaagaaaaag ccctctttgc cgccgcgcaa 720
ggcttgcagc cgaaaatcgc cgccgccgtc gccgaaggca atttccaaac cgccttgtcc 780
gaactggctt ccgtcaaacc gcaagtcgat gcattctttg acggcgtgat ggtaatggcg 840
gaagatgccg ccgtaaaaca aaaccgcctg aacctgctga accgcttggc agagcaaatg 900
aacgcggtag ccgacatcgc gcttttgggc gagtaaccgt tgtacagtcc aaatgccgtc 960
tgaagccttc agacggcatc gtgcctatcg ggagaataaa 1000

<210> 29
<211> 1000
<212> DNA

<213> Neisseria meningitidis

<400> 29
gaacgaaccg gattcccact ttcgtgggaa tgacgaattt caggttactg tttttggttt 60
tctgtttttg tgaaaataat gggatttcag cttgtgggta tttaccggaa aaaacagaaa 120
ccgctccgcc gtcattcccg cgcaggcggg aatctaggtc tgtcggtgcg gaaacttatc 180
ggataaaacg gtttcttgag atttttcgtc ctggattccc actttcgtgg gaatgacgcg 240
aacagaaacc gctccgccgt cattcccgcg caggcgggaa tctagacatt caatgctaag 300
gcaatttatc gggaatgact gaaactcaaa aaactggatt cccactttcg tgggaatgac 360
gtggtgcagg tttccgtatg gatggattcg tcattcccgc gcaggcggga atctagacct 420
tcaatactaa ggcaatttat cggaaatgac tgaaactcga aaaactggat tcccactttt 480
gtgggaatga cgcgattaga gtttcaaaat ttattctaaa tagctgaaac tcaacacact 540
ggattcccgc ctgcgcggga atgacgaagt ggaagttacc cgaaacttaa aacaagcgaa 600
accgaacgaa ctggattccc actttcgtgg gaatgacgga atgtaggttc gtgggaatga 660
cggcggagcg gtttctgctt tttccaataa atgaccccaa cttaaaatcc cgtcattccc 720
gcgcaggcgg gaatctaggt ctgtcggtgc ggaaacttat cgggtaaaac ggtttcttga 780
gattttgcgt cctggattcc cactttcgtg ggaatgacgg aatgtaggtt cgtgggaatg 840
acgggatata ggtttccgtg cggacgcgtt cggattcatg actgcgcggg aatgacggga 900
ttttggtgta ttccctaaaa aaataaaaaa gtatttgcaa atttgttaaa aataaataaa 960
ataataatcc ttatcattct ttaattgaat tggatttatt 1000

<210> 30
<211> 1000
<212> DNA
<213> Neisseria meningitidis

<400> 30
caaaggctac gacagtgcgg aaaaccggca acatctggaa gaacatcagt tgttggacgg 60
cattatgcgc aaagcctgcc gcaaccgtcc gctgtcggaa acgcaaacca aacgcaaccg 120
gtatttgtcg aagacccgtt atagtggatt aaatttaaat caggacaagg cgacgaagcc 180
gcagacagta caaatagtac ggcaaggcga ggcaacgccg tactggttta aatttaatcc 240
actatatgtg gtcgaacaga gcttcggtac gctgcaccgt aaattccgct atgcgcgggc 300
agcctatttc ggactgatta aagtgagtgc gcaaagccat ctgaaggcga tgtgtttgaa 360
cctgttgaaa gccgccaaca agctaagtgc gcccgctgcc gcctaaaagg agaccggatg 420
cctgattatc gggtatccgg ggagggttaa gggggtattt gggtaaaatt aggaggtatt 480
tggggcgaaa atagacgaaa acctgtgttt gggtttcggc tgtcgggagg gaaaggaatt 540
ttgcaaagat ctcatcctgt tattttcaca aaaacagaaa accaaaaaca gcaacctgaa 600
attcgtcatt cccgcgcagg cgggaatcca gacccccaac gcggcaggaa tctatcggaa 660
ataaccgaaa ccggacgaac ctagattccc gctttcgcgg gaatgacggc agagtggttt 720
cagttgctcc cgataaatgc cgccatctca agtctcgtca ttcccttaaa acagaaaacc 780
gaaatcagaa acctaaaatt tcgtcattcc cataaaaaac agaaaaccaa gtgagaataa 840
caattcgttg taaacaaata actatttgtt aatttttatt aatatatgta aaatcccccc 900
cccccccccc cgaaagctta agaatataat tgtaagcgta acgattattt acgttatgtt 960
accatatccg actacaatcc aaattttgga gattttaact 1000

<210> 31
<211> 1000
<212> DNA
<213> Neisseria meningitidis

<400> 31
ataatgcagg cgctgaagtt gttaaacatc aaacacacat cgttgaagac gaaatgtctg 60
atgaggccaa acaagtcatt ccaggcaatg cagatgtctc tatttatgaa attatggaac 120
gttgcgccct gaatgaagaa gatgagatta aattaaaaga atacgtagag agtaagggta 180
tgatttttat cagtactcct ttctctcgtg cagctgcttt acgattacaa cgtatggata 240
ttccagcata taaaatcggc tctggcgaat gtaataacta cccattaatt aaactggtgg 300
cctcttttgg taagcctatt attctctcta ccggcatgaa ttctattgaa agcatcaaaa 360
agtcggtaga aattattcga gaagcagggg taccttatgc tttgcttcac tgtaccaaca 420
tctacccaac cccttacgaa gatgttcgat tgggtggtat gaacgattta tctgaagcct 480
ttccagacgc aatcattggc ctgtctgacc ataccttaga taactatgct tgcttaggag 540
cagtagcttt aggcggttcg attttagagc gtcactttac tgaccgcatg gatcgcccag 600

gtccggatat tgtatgctct atgaatccgg atacttttaa agagctcaag caaggcgctc 660
atgctttaaa attggcacgc ggcggcaaaa aagacacgat tatcgcggga gaaaagccaa 720
ctaaagattt cgcctttgca tctgtcgtag cagataaaga cattaaaaaa ggagaactgt 780
tgtccggaga taacctatgg gttaaacgcc caggcaatgg agacttcagc gtcaacgaat 840
atgaaacatt atttggtaag gtcgctgctt gcaatattcg caaaggtgct caaatcaaaa 900
aaactgatat tgaataatgc ttattaactt agttacttta ttaacagagg attggctatt 960
acatatagct aattctcatt aatttttaag agatacaata 1000

<210> 32
<211> 1000
<212> DNA
<213> Neisseria meningitidis

<400> 32
atacctgcac ttgagttgcc gaccataaat ttagcatgtt tcaataagac taaaaaatat 60
tcaaatcgaa tggaaggaaa tgcaataaat ttatcagatt gatattttaa taattcttgc 120
agaatacttt cagtgccagt gtcattatta gggtagatgc taatgatatt ttggccactt 180
aattctaatg ctttgaaata ttgggccgca tattgtggca ttaaatgtgc ttctgtagtc 240
acggggtgaa acatagaaat accataattt tcgtatggta aaccgtaata ttctttgact 300
tcttctaagg atgggagggt ggaagaggcc ataacatcta aatcggggga gccgatgatg 360
tgaatatgct ttcttttttc tcccatttgc actaggcgag tgacagcttg ttcatttgct 420
accaagtgga tatgagaaag tttactaata gaatgacgaa tggagtcatc tactgtacca 480
gatagttcac caccttcgat atggcaaact aaacggctgc ttaatgcacc tacagctgcg 540
cctgctagtg cttctaaacg gtcgccgtga atcatgacca tatcaggttc aatttcatca 600
gatagacgag agataaacgt aatggtattg cctaaaacgg cacccattgg ttcaccttgg 660
atttgatttg aaaacagata tgtatgttga tagttttctc gagttacttc cttgtaggtt 720
ctgccatatg ttttcatcat atgcatacca gttacaatca aatgcaattc aaggtctggg 780
tgattttcaa tataggctaa taaaggtttt agcttgccga agtcggctct ggtacctgta 840
atgcaaagaa ttcttttcat gattttagaa tctataagta tatatatata agtataagga 900
agttggaaag aagaatacta attatactct acgtactcat aaatttattt cgattaagtg 960
ctataattag gccatttata attatattag gatttggctt 1000

<210> 33
<211> 1000
<212> DNA
<213> Neisseria meningitidis

<400> 33
   tctttttcgg actgaaagga cgcatcatcc cgacatcgag cgcgtgttcg tccggcagcc 60
   aaggcatagg ttatgcctac gaagccatca aatacggtct gaccgatatg atgctggcgg 120
   gcggaggcga agaatttttc ccgtccgaag tgtatgtttt cgactcgctt tatgccgcca 180
   gccgccgcaa cggcgaaccg gaaaaaaccc cgcgcccata cgacgcgaac cgcgacgggc 240
   tggtcatcgg cgaaggcgcg gggattttcg tgctggaaga attggaacac gccaaacggc 300
   gcggtgcgat aatttacgcc gaactcgtcg gctacggagc caacagcgat gcctaccata 360
   tttccacgcc ccgccccgac gcgcaaggcg caatccttgc ctttcagacg gcattgcaac 420
   acgcagacct tgcgcccgaa gacatcggct ggattaatct gcacggcacc gggacgcacc 480
   acaacgacag tatggaaagc cgcgccgttg cagcggtttt cggcaacaat acgccctgca 540
   cgtccaccaa gccgcaaacc ggacacacgc tgggcgcggc gggcgcaatc gaagccgcgt 600
   tcgcgtgggg cattgctgac cggaaaagca atcccgaagg gaaacttccg ccccagcttt 660
   gggacgggca gaacgatccc gaccttcccg ccatcaacct gaccggcagc ggcagccgct 720
   gggaaaccga aaaacgcatt gccgccagct cgtcgtttgc cttcggagga agcaactgcg 780
   ttttactcat cggatgaaat aagtttgtca atcccaccgc tatgctatac aatacgcgcc 840
   tactcttgat gggtctgtag ctcaggggtt agagcagggg actcataatc ccttggtcgt 900
   gggttcgagc cccaccggac ccaccaattc ccaagcccgg acgtatgttt gggctttttt 960
   gccgccctgt gaaaccaaaa tgctttgaga aaccttgata 1000

<210> 34
<211> 1000
<212> DNA
<213> Neisseria meningitidis

<400> 34
tagaaaaata tttcgcccaa tcattagccg ccgtcgtgaa tcagacttgg cgcaacttgg 60
agattttgat tgtcgatgac ggctcgacag acggtacgct tgccattgcc aaggattttc 120
aaaagcggga cagccgtatc aaaatccttg cacaagctca aaattccggc ctgattccct 180
ctttaaacat cgggctggac gaattggcaa agtcaggaat gggggaatat attgcacgca 240
ccgatgccga cgatattgcc gcccccgact ggattgagaa aatcgtgggc gagatggaaa 300
aagaccgcag catcatcgcg atgggcgcgt ggctggaagt tttgtcggaa gaaaaggacg 360
gcaaccggct ggcgcggcat cacaggcacg gcaaaatttg gaaaaagccg acccggcacg 420
aagatattgc cgactttttc cctttcggca accccataca caacaacacg atgattatga 480
ggcgcagcgt cattgacggc ggtttgcgtt acaacaccga gcgggattgg gcggaagatt 540
accaattttg gtacgatgtc agcaaattgg gcaggctggc ttattatccc gaagccttgg 600
tcaaataccg ccttcacgcc aatcaggttt catccaaata cagcatccgc caacacgaaa 660
tcgcgcaagg catccaaaaa accgccagaa acgatttttt gcagtctatg ggttttaaaa 720
cccggttcga cagccttgaa taccgccaaa taaaagcagt agcgtatgaa ttgctggaga 780
aacatttgcc ggaagaagat tttgaacgcg cccgccggtt tttgtaccaa tgcttcaaac 840
ggacggacac gctgcccgcc ggcgcgtggc tggattttgc ggcagacggc aggatgcggc 900
ggctgtttac cttgaggcaa tacttcggca ttttgcaccg attgctgaaa aaccgttgaa 960
aaacgccgct ttatccaaca gacaaaaaac aggataaatt 1000

<210> 35
<211> 806
<212> DNA
<213> Neisseria meningitidis

<400> 35
gcgcacggct ttttcttcat cggtttgagg gtcggcagga taatcgggga cggcaaagcc 60
tttagactgc aattctttaa tcgcggcggt cagttgaggt acggatgcgc tgatgttcgg 120
cagtttgatt acgtttgcat cgggctgttt caccagttcg cccaattcgg caagcgcgtc 180
gggtacgcgc tgcgcttcgg tcagatattc ggggaatgcc gccaaaatac ggccggacag 240
ggaaatgtcg gcagttttga catcaatatc ggcgtggcgg gcaaacgcct gcacaatcgg 300
cagcagcgat tgggtcgcca gcgcgggggc ttcgtcggta tgggtataaa caatggtgga 360
tttttgagtc ataggattat tctcttgtag gttggttttt tcttttggaa cacattgcgc 420
ggggaatgtg cgcggctatt atggcatatt ttggcggctt tgttcgcgct ttgttcgatc 480
ttggcgtgtt tgaacgcggc agcgtgaaag gaagggggaa atggttttcc cgcgtttggc 540
ggcggtgtcg gaggtgctgt gcctgatgtg cggcggcata ttttcggtga aattgatttt 600
atagtggttt aaatttaaac cagtacagcg ttgcctcgcc ttgtcgtact atctgtactg 660
tctgcggctt cgttgccttg tcctgattta aatttaaacc actataatat tcggtaactg 720
tcggaatatc tgctaaaatt ccgcattttt ccgcctcggg acactcgggg cgtatgttta 780
atttgtcgga atggagtttt agggat 806

<210> 36
<211> 1000
<212> DNA
<213> Neisseria meningitidis

<400> 36
gcccgacggc gaacagacac gtcgtgaaat caaccgcttg gacagtacgg cggcgcaata 60
cgacatgctt gcaggttatc ttgaaagact tgccggaaaa accgaccgtt gggcgtgcgc 120
ctaccgccaa aatgccgtct gaacacccga ttatcctttt gaaagcgcga ttatgcccca 180
tacccttccc gatatttccc aatgtatcag acaaaatttg gaacaatatt tcaaagacct 240
gaacggtacc gaaccttgcg gcgtgtacga tatggtcttg catcaggtgg aaaaaccgct 300
gctggtgtgc gtgatggaac aatgcggcgg caaccagtcc aaagcctccg tcatgttggg 360
actgaaccgc aatactttgc gtaaaaaact gattcaacac ggtttgctgt gaatatgtcg 420
gcaaccgtcc gtatcttggg tattgacccg ggcagtcgcg taacgggttt cggtgtcatc 480
gatgtcaggg ggcgcgatca tttttacgtc gcctccggct gcatcaaaac gcctgccgat 540
gcgcctctgg cagacaggat tgccgtgatt gtgcggcata tcggcgaagt cgttaccgtt 600
tacaagcctc aacaggcggc agtggaacag gtgttcgtca acgtcaatcc ggcatcgacg 660
ctgatgctcg gtcaggctag ggcgcggcca ttggcggcat tggtcagcca taagctgccc 720
gtttcggaat acacggcctt gcaggtcaaa caggcggtag tcggcaaggg caaggcggca 780
aaagaacagg tgcagcatat ggtggtgcag atgctggggc tttcgggaac gccgcaggan 840
tggcggcgga cggtcttgcc gtcgcgctga cccacgcctt acgcaaccac gggcttgccg 900

ccaaactcaa tccttcgggg atgcaggtca agcgcggcag gtttcaatag tttcagacgg 960
catttgtatt ttgccgtctg aaaagaaaat gtgtatcgag 1000

<210> 37
<211> 1000
<212> DNA
<213> Neisseria meningitidis

<400> 37
ccgccaagcg tttccccctt tgtcgggctt aacatttgct ttgtacggca gactttttcc 60
cttcataacg ccgcctttcc gaaaagacga tggtaggcgc gacgtaattc tcaaccctta 120
aggtacggtt ggacgaaaag ttttcctttt cattccacct gccaactttt cggctacacc 180
gagtggtctc gttaggtttg ggcgaactac gcccttaaaa aaacggacat tctttgcatg 240
cccgtctcta aggtttcacg gtaagtttac ccttataaag agttgactta ccatacttat 300
ccctttaaaa cgatataaag ggcgacagct gtaatacaag tatgttgtac ggcagacttc 360
ttctaccaaa caaaaagttc cttttagagt tactcgctta tagacaaatg aaggcttagc 420
cataggcttc cggtaggcct atttcaacgg ctggttcaca ggctacgcta aaacctacgg 480
tagaaccgcg ttctggggtt tcgcgcacag cggcgtcttt ggaaccagtt gtgtccgaac 540
acgcataacc gcccgcttta atggtggtgg cgggttcacc tgatgtagtt tcagcgtgcg 600
ctttggtagt ttgcgtagcc gatgttgagg aggctcgacc cgaaactacg gttgccgacg 660
cgccagccgc acatgatgct ggtcgttaga ggcctgtagc gggttccgca cttgcttccg 720
cttccgtaac tgaacttggt tccgcgaccg ctggttccaa actacaagcc gatacggacg 780
ctgctttggg gctgggacta cggcaaacgg tagataatgt cggtggcgga ctacgtcgca 840
gtttcgctta atgcgtttct gccggaggac ggaaccgacg cagggctgcg ttttcgggtt 900
gactggcacc aaatgctatc gcttaggccg tttcattttg cgtaactatg gcagcaggag 960
agatacgttg tgctgggcct ttagccaata cttctcaact 1000

<210> 38
<211> 1000
<212> DNA
<213> Neisseria meningitidis

<400> 38
cacaaaaacc aagttatgac gggaataagg tacagcagcc aaaccaaggc ctcgccctgc 60
gtcggatggt cggtatagcc gaaaaatccg ccgagcagca cgcccaacgg gctgtcttcg 120
tgcaaatatt ttgatgagtc gaacacaatg tcctgaagcg cgttccaaat gcctgcttcg 180
tgcagcgcac gcagcgaacc ggcaagcaga ccagcggcaa cgataatcag aaacgcccct 240
gtccaacgga aaaacttcgc cagattcagg cgcatcccac cctgataaat caacgcgcca 300
atcacggcgg cagccaaaac cccgctaccg gcaccggccg gcatctgcca cgtcgggctc 360
tgtttgaata cggcaagcag gaaaaaaacg ctctccaaac cttcgcgcgc cacggcaaga 420
aacgccatac cgaccaaggc ccatccttga ccgctgccac ggttcaaagc cgcctgcaca 480
gaatcctgaa gctgccgctt catcgaacgg gcggcttttt tcatccataa aatcatataa 540
gtcagcatcg cgacagcaac caaaccgata atgccgacga cgaactcctg ctgcttctgg 600
ggaatctcgc ccgttgccga atggattccg taccccagcc ccaaacacat caaagaagca 660
agaacaaccc cgaaccagac cttaggcatc agtttggaat gtccggactg tttcagaaaa 720
ccggcaacga tgccgacgat gagcgcggct tcgataccct cgcgcaacat aattaaaaaa 780
gcgaccagca taaacgcgaa cgaacaagga tgatgaataa tatattatcg gaatattttc 840
attgcttgta aatacaaatg caagttattt ttatctgcag taccgcgcgg cggaaagttc 900
cgcagctgca gctgcgccct gtgttaaaat cccctctcca cggctgccgc aacgccgccc 960
gaaaccatct ttcttattac tgccggcaac attgtccatt 1000

<210> 39
<211> 1000
<212> DNA
<213> Moraxella catarrhalis

<400> 39
gctgatttgt gagcaagcgg gcgcatcagg gattaccttg catttgcgag aagatcgtcg 60
acatattcaa gatgaagatg tttatgaatt gattgggcaa ttgacaacac gcatgaatct 120
tgagatggca gtcactgatg agatgctaaa tattgcccta aaggtacgac cagcatgggt 180
gtgtttagta ccagaaaaac gccaagagct gactacagaa ggtgggcttg atatcgccaa 240

tttatcaaat attcaagcat ttatacacag tcttcagcag gcggatatta aggtttcttt 300
attcatcgat ccagatccgc atcaaattga tgctgcaatt gctttgggtg ctgatgcgat 360
tgagctgcat acgggagctt atgctcaagc gactttacaa aataatcaaa agcttgttga 420
taaagagctt gaccgtattc aaaaagccgt tgcaatggca caaaaaaaat catcattatt 480
gattaatgca ggtcatggtt tgacgcgtga taatgttgca gcgattgccc aaattgatgg 540
tattcatgag ctgaatatcg ggcatgcatt gatttcagat gcgatattta tggggcttga 600
taatgcagtc aaggcaatga aaatggcttt tattcaagat aaaacgacca atcattgatg 660
cgttagaaag aaaatcgtaa ataatgatga ctattgtgta atattatgta tttttgttca 720
aaaaaaggtt gtaaaaaaat tcatttacca ttaagctaag cccacaagcc acaatgaata 780
cctattggtt tgactcatta gtcactaaga atctgcaaaa ttttgtaaca gattattggc 840
aggtcttgga tcgctatgct aaaataggtg cggtaatctt gaaaaaccaa ccattccttg 900
gaggaattta tgaaaaaggg atataaacgc tcttgcggtc atcgcagccg ttgcagctcc 960
agttgcagct ccagttgctg ctcaagctgg tgtgacagtc 1000

<210> 40
<211> 1000
<212> DNA
<213> Moraxella catarrhalis

<400> 40
gatgctgtta aagtgggtat tggtcctggt tctatttgta caacccgtat tgttgcaggc 60
attggcgtcc cgcagataag tgccattgat agtgtggcaa gtgcgttaaa agatcgcatt 120
cctttgattg ccgatggcgg tattcgtttt tcgggtgata tcgccaaagc catcgcagca 180
ggcgcttcat gtattatggt gggtagcttg ttggcaggta ccgaagaagc acctggtgag 240
gtggaattat tccaaggtcg ttattataag gcttatcgtg gtatgggcag cttgggggca 300
atgtctggtc aaaatggctc atcggatcgt tattttcaag atgccaaaga tggtgttgaa 360
aaactggttc cagagggtat cgaaggccgt gttccttata aaggccctgt ggcaggcatc 420
atcggtcaat tggcaggtgg tctaagatca tccatgggtt atacaggttg ccagaccatc 480
gaacagatgc gtaagaatac cagctttgtc aaagtgactt ccgcaggcat gaaggaatcg 540
catgtacacg atgtacagat taccaaagaa gcacccaatt atcgccaaaa ttaactctat 600
taatagcaaa tacaagcact cattagatag ggtgggtgct ttttagagca taaaaaataa 660
actgacacat gacttattgt catattttta aaatgctttt aatttagatt tttaatttag 720
ataatggcta aaaataacag aatattaatt taaagttttc aaaatcaagc gattagatga 780
aattatgaaa ataaataaca ataattctga tttattttaa ccaataatat caattatcat 840
ttacaagaaa aatttttttt gataaaattc ttacttgtac cttgctattt tttcttattt 900
atcatttttg gcggtatttt cgttgatttt agtaagtaga tgagcaaggg ataatttgac 960
aaaaacaaat ttgatttcaa gcctcataat cggagttatt 1000

<210> 41
<211> 1000
<212> DNA
<213> Moraxella catarrhalis

<400> 41
aaaactggtg atgtcttcac tgctattcat ggtgaaccaa tcaatgattg gctaagtgcc 60
accaagatta ttcaggcaaa tccagaaacc atgcttgatg tgacagtcat gcgtcaaggt 120
aagcaggttg atttaaaatt aatgccccgt ggtgtaaaga cacaaaacgg cgtagtcggt 180
caactgggta ttcgccccca gattgatatc gatacgctca ttcctgatga atatcgtatg 240
acgattcaat atgatgtcgg tgaggcattt actcaagcca tccgacgaac ttatgattta 300
tcaataatga ccttagatgc gatgggtaag atgattacag gattgattgg cattgaaaat 360
ctatcaggtc ccattgccat tgccgatgtt tctaagacca gttttgagtt gggatttcaa 420
gaagtgttat cgacagccgc aatcatcagt ttaagcttgg cagtactgaa tcttttaccc 480
attccagtgt tagatggcgg gcatttggta ttttatactt atgaatggat tatgggcaaa 540
tctatgaatg aagcggtgca gatggcagca tttaaagcgg gtgcgttatt gcttttttgt 600
ttcatgttac ttgcaatcag taacgatatc atgcgatttt ttggctaagt tctgatttat 660
cgtaccatta acaaaatttt tggctttttt aagctgaaat acttgccaaa tttaactttt 720
tggcttacct ttacacaata taaatttggg tgtagaaaat tttggataca tttttatacc 780
ttatttttag aaattttaaa aattaagttt ggatagactt atgcgtaatt catattttaa 840
aggttttcag gtcagtgcaa tgacaatggc tgtcatgatg gtaatgtcaa ctcatgcaca 900
agcggcggat tttatggcaa atgacattgc catcacagga ctacagcgag tgaccattga 960
aagcttacaa agcgtgctgc cgtttcgctt gggtcaagtg 1000

<210> 42
<211> 1000
<212> DNA
<213> Moraxella catarrhalis

<400> 42
   acttggcgaa aataccattt atatcgattg tgatgttata caggcagatg gcggtacacg 60
   cacagccagt atcagtggtg ctgcggtggc acttattgat gctttagaac acttgcagcg 120
   tcgtaaaaag cttacccaag atccgctttt gggcttggtg gcagcggttt ctgtgggtgt 180
   taatcaaggc cgtgtattgc ttgatttgga ttatgctgaa gattcaactt gtgataccga 240
   tttaaatgtg gtcatgacgc aggcaggtgg gtttattgag attcaaggca cagcagaaga 300
   aaagccattt actcgtgctg aagctaatgc gatgcttgat ttggcagagc tgggaattgg 360
   gcagattatc gaagcccaaa agcaagtatt aggctggtga tatgctaatc gttgaagata 420
   atggcgtgat catcacatta aatggacaag taaaagaccc attattttgg tggtcgatga 480
   tattgctgct gctgggtgtc ttggtggcaa tcatttgttt gattgcaccc gttttttatg 540
   caatcggtgc gttggcttta tttgcagttg tggtatttgt gtttaatatt caaaggcaaa 600
   aagccaaaac ttgtcatatg ttttcacaag gtcgcttgaa gattacgtcc aaacgctttg 660
   agattcataa caaatcacta accttatcag catcggcaac aatatctgct aaagataaca 720
   aaatgacaat tgttgatcgg ggcattgaat atcattttac aggttttgct gatgaccgtg 780
   aaattaatat agccaaacag gtacttttgg gaaagtcaat caaaaccaat gcggtggcgg 840
   taacattggc taagtagttg ttgtgataca gacaggttgg atggtcttta actccaccca 900
   cctaactttt tctttgtttg gatttaagag tatgttatga tgggcaggat tttattttaa 960
   gtcatcattt aatgcaatca gttgtccaga gtagccgttc 1000

<210> 43
<211> 1000
<212> DNA
<213> Moraxella catarrhalis

<400> 43
   gtgatcggca acaccccacc attcaggagc aaccaaaatt gcccgtgcct tgcctgtctt 60
   ggtggtatca tttggcaggg caatgtggct aagtagtggt gtgccatcag gtgcggtggt 120
   ggtgagtgta cgattcgtta ttgtcataaa attatccttt tgggttggat gatatcaatg 180
   aaatacccta cggttgtatg gaattttatc cattgtacca cggtattggt ctttttaaat 240
   taacaagcag cttctagcaa gtcaaagttt ttatgcctat tttttcagat tttaaggtac 300
   aataaagcca attgttaata atatggtatt gtcatgattt atgatgaatt gcgaccaaaa 360
   ttttgggaaa attatccctt agatgcgtta acagatgctg aatgggaagc attatgtgac 420
   ggatgtggcg cgtgttgttt ggtgaaattt cttgatgatg acaatgttaa attgaccgaa 480
   tataccgatg ttgcctgcca gctattggat tgctcaacag gattttgcca aaactatgcc 540
   aagcgtcaaa cgattgtgcc agattgtatt cgcttaacac ctgatatgct gcctgatatg 600
   ctgtggttgc cacgccattg tgcttataag cggttgtatc ttgggcaaaa tctgccagca 660
   tggcacaggc tcattaaaca tagccaaaac catggtgcag gatttgcgaa agtttcaact 720
   gctgggcgat gtgtgagtga gcttggtatg agtgatgaag acatagaaag gcgagtggtg 780
   aaatgggtta aaccttgaca tgattgttga catgattgac agacaataaa aattggcaaa 840
   tttgataaaa ttggtgtatg tgtgtgattt tatcaaaagc acttgaataa aaccgagtga 900
   tacgctaaat tgtagcaaac caatcaattc atcataattt taatgaacac gaggttaaat 960
   tatactgtct atgtctgatg acaattcaag cacttggtcg 1000

<210> 44
<211> 1000
<212> DNA
<213> Moraxella catarrhalis

<400> 44
taacaaaggc aacccaacac gcagttattt tgtgcaaggc ggtcaagcgg atgtcagtac 60
tcagctgccc agtgcaggta aattcaccta taatggtctt tgggcaggct acctgaccca 120
gaaaaaagac aaaggttata gcaaagatga ggataccatc aagcaaaaag gtcttaaaga 180
ttatatattg accaaagact ttatcccaca agatgacgat gacgatgacg atgacgatag 240
tttgaccgca tctgatgatt cacaagatga taatacacat ggcgatgatg atttgattgc 300
atctgatgat tcacaagatg atgacgcaga tggcgatgac gattcagatg atttgggtga 360

tggtgcagat gatgacgccg caggcaaagt gtatcatgca ggtaatattc gccctgaatt 420
tgaaaacaaa tacttgccca ttaatgagcc tactcatgaa aaaacctttg ccctagatgg 480
taaaaataag gctaagtttg atgtaaactt tgacaccaac agcctaactg gtaaattaaa 540
cgatgagaga ggtgatatcg tctttgatat caaaaatggc aaaattgatg gcacaggatt 600
taccgccaaa gccgatgtgc caaactatcg tgaagaagtg ggtaacaacc aaggtggcgg 660
tttcttatac aacatcaaag atattgatgt taaggggcaa ttttttggca caaatggcga 720
agagttggca ggacggttac atcatgacaa aggcgatggc atcactgaca ccgccgaaaa 780
agcaggggct gtctttgggg ctgttaaaga taaataaagc ccccctcatc atcgtttagt 840
cgcttgaccg acagttgatg acgcccttgg caatgtctta aaacagcact ttgaaacagt 900
gccttgggcg aattcttgga taaatgcacc agatttgcct cgggctaata tcttgataaa 960
acatcgccat aaaatagaaa ataaagttta ggattttttt 1000

<210> 45
<211> 1000
<212> DNA
<213> Moraxella catarrhalis

<400> 45
cagcttgtac catttggtga atatatacca tttggtggtt tgttggatat tttaccaggg 60
cttgagggtg tcgctagcct aagccgtggc gatgataagc aaccaccgct caaattgggc 120
ggcggcgtgg gcgatacgat tggtgcggca atttgttatg aggtggcata tcctgagacg 180
acgcgtaaaa atgcacttgg cagtaatttt ttattaaccg tctcaaacga tgcttggttt 240
ggtacaacag caggtccttt gcagcattta caaatggtgc aaatgcgaag cttggagacg 300
gggcgatggt ttgtgcgtgc aacaaacaac ggagtgactg cattaattga ccatcaagga 360
cggattatca agcagatacc gcagtttcag cgagatattt tgcgaggtga tgtacccagt 420
tatgttggac acacgcctta tatggtttgg gggcattatc ccatgttggg gttttctttg 480
gtgctgattt ttcttagtat catggcaaag aaaatgaaaa ataccaccgc caaacgagaa 540
aaattttata ccgctgatgg tgtggtagac cgctgaattg tgccactttg ggcgttagag 600
catgagcaag attaggcgtt gggtgagctt tggttgtatt actcatcagc ctacccgaaa 660
cctgccaaac atcaccgccc aaaacctaaa catacaatgg ctaaaaatat cagaaaataa 720
cttgctgtat tgtaaattct tatgttatca tgtgataata attatcatta gtaccaagat 780
atccattact aaacttcatc ccccatctta acagttacca agcggtgagc ggattatccg 840
attgacagca agcttagcat gatggcatcg gctgattgtc tttttgcctt gttgtgtgtt 900
tgtgggagtt gattgtactt accttagtgg tggatgcttg ggctgattta attaaatttg 960
atcaaagcgg tcttcacaac acaccaaacg agatatcacc 1000

<210> 46
<211> 1000
<212> DNA
<213> Moraxella catarrhalis

<400> 46
agtttgccct gattttgaga gccactgcca tcatgaattt gttggcgtaa acaccactcg 60
tattcttctt cggtttcccc tttccatgca aacacaggga taccagcggc cgccatggca 120
gcggcggcgt ggtcttgggt gctaaaaata ttgcatgatg tccagcgaac ttctgcaccc 180
aaggcaacca aagtctcaat cagcaccgct gtttgaatgg tcatgtggat acagcctagg 240
attttagcac ccttaagtgg ttgctggtct tgatagcgtt ttcttaaccc catcagggct 300
ggcatctcag cttctgccaa ggcaatctca cggcgaccat aatcggctaa acggatatca 360
gcgactttat aatcggtgaa gttttgggtg gtacttggat tgattgaggt aggcatatct 420
ttattcctaa gctattttaa agtattttta acaataattt tgatgaattt gagataattg 480
atgctaaaag gttgaatgac caaaccatcg ctaacaatca agaaaagaca ttttaagcat 540
aaaaagcaaa tgtgtcttga tggcttatta taacagttat tatgataaat ttgggtagaa 600
agttaaatgg atcgttgggt aagtttgttg gctatcctta attaattata attttttaat 660
aatgctttta ctttatttta aaaatagagt aaaaaatggt tggctttggg tttttatctc 720
actatggtag ataaaattga tacaaaatgg tttgtattat cacttgtatt tgtattataa 780
ttttacttat ttttacaaac tatacactaa aatcaaaaat taatcacttt ggttgggtgg 840
ttttagcaag caaatggtta ttttggtaaa caattaagtt cttaaaaacg atacacgctc 900
ataaacagat ggtttttggc atctgcaatt tgatgcctgc cttgtgattg gttggggtgt 960
atcggtgtat caaagtgcaa aagccaacag gtggtcattg 1000

<210> 47

<211> 1000
<212> DNA
<213> Moraxella catarrhalis

<400> 47
   ttgggggcgg ataaaaagtg gtctttgccc aaaggggcat atgtgggagc gaacacccaa 60
   atctatggca aacatcatca aaatcacaaa aaatacaacg accattgggg cagactgggg 120
   gcaaatttgg gctttgctga tgccaaaaaa gaccttagca ttgagaccta tggtgaaaaa 180
   agattttatg ggcatgagcg ttataccgac accatcggca tacgcatgtc ggttgattat 240
   agaatcaacc caaaatttca aagcctaaac gccatagaca tatcacgcct aaccaaccat 300
   cggacgccca gggctgacag taataacact ttatacagca catcattgat ttattaccca 360
   aatgccacac gctattatct tttgggggca gacttttatg atgaaaaagt gccacaagac 420
   ccatctgaca gctatgagcg tcgtggcata cgcacagcgt gggggcaaga atgggcgggt 480
   ggtctttcaa gccgtgccca aatcagcatc aacaaacgcc attaccaagg ggcaaaccta 540
   accagtggcg gacaaattcg ccatgataaa cagatgcaag cgtctttatc gctttggcac 600
   agagacattc acaaatgggg catcacgcca cggctgacca tcagtacaaa catcaataaa 660
   agcaatgaca tcaaggcaaa ttatcacaaa aatcaaatgt ttgttgagtt tagtcgcatt 720
   ttttgatggg ataagcacgc cctacttttg tttttgtaaa aaaatgtgcc atcatagaca 780
   atatcaagaa aaaatcaaga aaaaaagatt acaaatttaa tgataattgt tattgtttat 840
   gttattattt atcaatgtaa atttgccgta ttttgtccat cacaaacgca tttatcatca 900
   atgcccagac aaatacgcca aatgcacatt gtcaacatgc caaaataggc attaacagac 960
   ttttttagat aataccatca acccatcaga ggattatttt 1000

<210> 48
<211> 1000
<212> DNA
<213> Moraxella catarrhalis

<400> 48
aaagacatta cacatcatca ttcaaacgcc caaccatgta cctctgcccc gtggtcgcac 60
gccaacgctt tttgatgcgg tgcgttgggt tcagatggct tgtcaatcat ttggttttat 120
taaaattcat acctttggta gtttggcttt acctgatatg tcatttgatt atcgaaacaa 180
tacgcagttg accaaacatc aatttttagc catttgccaa gcactcaata ttaccgctca 240
tacgaccatg cttggtatta aatcatcaca taaagatact ttacatccat ttgaattgac 300
attacccaaa tacggccatg cctcaaatta tgatgatgaa ttggtgcaaa acaatccatt 360
ggcttatttt catcaactgt ctgccgtctg ccgatatttt tatacccaaa cggtttgtat 420
tgttggcggt gaaagctcag ggaaaactac cttggtgcaa aaacttgcca attattatgg 480
tgccagcatc gcacctgaaa tgggtcgatt atacacacac tcccatctcg gcggtagcga 540
acttgccctt caatacagcg actacgcatc cattgccatc aatcacgcca acgctatcga 600
aaccgctcgt accactgcca gctctgctgt tacactgatt gatactgatt ttgcgacaac 660
gcaagcattt tgtgaaattt atgaagggcg aacgcatccg cttgtcgcag aatttgctaa 720
acaaatgcga ttggatttta cgatttattt agataataat gttgcttggg tcgctgatgg 780
catgcgtagg cttggtgatg atcatcaacg cagtttgttc gccaataaat tgcttgagat 840
tttggcacga tatgatatta gttatcatat cattaatgac accgactacc acaaacgcta 900
tctacaagca ttaagcttga tagacaatca tatttttaat cattttacaa aaattcatga 960
caattaatta gggaaaatct gatgaaaatt gatattttag 1000

<210> 49
<211> 1000
<212> DNA
<213> Moraxella catarrhalis

<400> 49
ggatgtggca tatctgccca tcgacccaat acacatcggt cgaggctatc aagatgtggt 60
acgaattaat agccagtcag gtaagggcgg tgctgcgtat atcttgcagc ggcattttgg 120
ttttaattta ccacgctgga cacagattga ttttgctcgt gtggtacagg cttatgcaga 180
aagtatggcg cgtgaactaa aaactgatga gctgcttgaa atttttaccc aagcgtatct 240
taagcaagat aaattccgcc taagtgacta taccatcagc aataaaggcg atgctgtcag 300
cttccaaggc caagtagcga cacccaaagc ggtgtttgag gtgattggtc aaggcaatgg 360
tgcgttatct gcgttcattg atggcttggt gaaatccaca ggcagacaga ttcatgtcac 420
caattacgcc gaacacgcca tcgataacaa aacccatcaa aaaaccgata cggataacca 480

aaccgatgcc gccgtgccgc ttatatccag ctgtcggtag aggggcagat ttattcaggc 540
atcgccactt gccatagcac cgtatccgcc atgctaaaag gtgcattatc cgctttggca 600
caggcgtggt aatctgaccc aatcaaaatc ctgcatgatg gcaggatttt attatttagt 660
gggctgccca acaatgatga tcatcagcat gtgagcaaat gactggcgta aatgactgat 720
gagtgtctat ttaatgaaag atatcaatat ataaaagttg actatagcga tgcaatacag 780
taaaatttgt tacggctaaa cataacgacg gtccaagatg gcggatatcg ccatttacca 840
acctgataat cagtttgata gccattagcg atggcatcaa gttgtgttgt tgtattgtca 900
tataaacggt aaatttggtt tggtggatgc cccatctgat ttaccgtccc cctaataagt 960
gagggggggg gagaccccag tcatttatta ggagactaag 1000

<210> 50
<211> 1000
<212> DNA
<213> Moraxella catarrhalis

<400> 50
ccccaagctt tccgtttgtg tgcctgctgg tgtcgggcgg tcataccatg ctggtgcgtg 60
ccgatggtgt gggcgtgtat cagatattgg gcgagtctat cgatgatgcg gtgggtgaat 120
gctttgataa aacggcaaaa atgctcaaac tgccctatcc tggtggccca aatatcgaaa 180
aattagccaa aaacggcaac ccacacgcct atgagctgcc aagacccatg cagcataaag 240
ggctggattt ttcgttcagt ggcatgaaaa ccgccattca taatctcatc aaagacacac 300
caaacgccca aagcgacccc gccacacgag cagacatcgc cgcaagcttt gagtatgcgg 360
tggtggatac tttggtcaaa aaatgcacca aagcactaca gatgacaggc attcgccagc 420
tggtggtcgc agggggcgtc tctgccaatc agatgctacg ccgcaccctg accgagacgc 480
tccgccaaat cgatgcgtcg gtgtactatg ccccgaccga gctatgcacg gataatggtg 540
cgatgatcgc ctatgctggc ttttgtcggc tcagctgtgg acagtcggat gacttggcgg 600
ttcgctgtat tccccgatgg gatatgacga cgcttggcgt atcggctcat agatagccac 660
atcaatcata ccaaccaaat cgtacaaacg gttgatacat gccaaaaata ccatattgaa 720
agtagggttt gggtattatt tatgtaactt atatctaatt tggtgttgat actttgataa 780
agccttgcta tactgtaacc taaatggata tgatagagat ttttccattt atgccagcaa 840
aagagataga tagatagata gatagataga actctgtctt ttatctgtcc gctgatgctt 900
tctgcctgcc accgatgata tcatttatct gctttttagg catcagttat ttcaccgtga 960
tgactgatgt gatgacttaa ccaccaaaag agagtgctaa 1000

<210> 51
<211> 1000
<212> DNA
<213> Moraxella catarrhalis

<400> 51
gagtgaactt tattgtaaaa tatgattcat taaagtatca aaatcatcaa acgcagcatc 60
agggtttgct aaatcaattt tttcaccata attatagcca taacgcacag caagcgtagt 120
tatgccagcg gcttgccctg ataaaatatc atttttggaa tcaccaacca taatggcatc 180
agtcggtgcg atgcccagtg attgacacag gtataataaa ggcgttgggt cgggcttttt 240
gacgctgagc gtatcaccgc caatcacttg gtcaaacagt gtcagccatc caaaatgtga 300
taaaatttta ggcaaataac gctcaggctt attggtacaa attgccaaat aaaaccccgc 360
tgcttttaat cgttcaagcc cttgtataac ccctgcatag ctttgcgtat tttcaattgt 420
tttatgggca tattctgcca aaaataactc atgggcatgg tgaatcatag tcgtatcata 480
gatatgatgt gcttgcattg ctcgctcaac caattttagc gaaccattgc ccacccagct 540
tttgatgata tcaattggca taggcggtaa gttaagcttg gcatacatgc cattgaccgc 600
cgccgccaaa tcaggggcac tatcgataag cgtaccatcc aaatcaaata taatcagttt 660
tttgccagtc attgacagtg tttgcatgct ttttccttat tcttaaaatt ggcggctgtt 720
tggtattttt taaatcagtc aatttttacc atttgtcata taatgacaaa gtacaaattt 780
agcaatattt tagtgcattt tttggcgaag ttttatgaaa actggtcatt ggttgcaaaa 840
ctttacacag tacctataaa acttgcacag ttaataagaa atattttgtt actatagggg 900
cgtcatttgg aacaagacag ttatttgtaa atagttattt gcaaaagacg gctaaaagac 960
agaacagcgt ttgtttcagt gattaactag gagaaaaaca 1000

<210> 52
<211> 1000
<212> DNA

<213> Moraxella catarrhalis

<400> 52
ttgatcggtt ttgccccact gtttcatgat ttactcaaaa caggcggctt gatcgtgctg 60
gcaggtctga cccaaaacca aacccaagcg gtcatcgatg cctactcgcc ttatgttacg 120
cttgatacgc cattttgtta tgcagatgcc caagactgcc attggcaacg cctaagcggc 180
atcaaaccta ccaacccata agcgatatgc catgagccac aaacctaagc caacaccgct 240
atatcaacaa gttgagcaga ccgccaagcg ttattttgag acattgggcg atgctcatac 300
tcatgatgtc tatgccactt ttttggccga atttgaaaaa ccgctgctca tcgccgcact 360
caatcacacg cacggcaatc agtcaaaaac cgcccaaatc cttggtatca atcgtggcac 420
attacgcacc aaaatgaaaa cccatcactt actttagacc gccagttatc gccatggata 480
tgggcaggtg tgctcgcctg ccgtatgatg gcgatgacac cccatttgcc ccatatctgc 540
acgatttgac atgatttaac atgtgatatg atttaacatg tgacatgatt taacattgtt 600
taatactgtt gccatcatta ccataattta gtaacgcatt tgtaaaaatc attgccccct 660
ttttttatgt gtatcatatg aatagaatat tatgattgta tctgattatt gtatcagaat 720
ggtgatgcct acgagttgat ttgggttaat cactctatta tttgatatgt tttgaaacta 780
atctattgac ttaaatcacc atatggttat aatttagcat aatggtaggc tttttgtaaa 840
aatcacatcg caatattgtt ctactgttac caccatgctt gaatgacgat ccaaatcacc 900
agattcattc aagtgatgtg tttgtatacg caccatttac cctaattatt tcaatcaaat 960
gcctatgtca gcatgtatca tttttttaag gtaaaccacc 1000

<210> 53
<211> 1001
<212> DNA
<213> Moraxella catarrhalis

<400> 53
actattctgc tttttgtttt tcacgaatgc gaatgcccaa ctcacgcaac tggcgattat 60
caacttcagc aggtgcttcg gtcaatgggc aatctgccgt cttggttttt gggaaggcga 120
tcacatcacg gattgagctg gcaccaacca tcagcataat caggcgatct agaccaaatg 180
ccaaaccacc gtgcggcggt gcaccaaaac gcaatgcatc catcaaaaac ttaaacttaa 240
gctctgcttc ttctttagaa atacccaagg catcaaatac cgcctcttgc atgtcaaccg 300
tattaatacg cagcgaaccg ccaccaattt ctgtgccatt tagtaccatg tcataggcaa 360
tggatagggc ggtttcggga ctttgtttga gttcctcaac cgagcctttt gggcgtgtaa 420
aaggatgatg aactgatgtc cacttaccat catcagtttc ctcaaacatt ggaaaatcaa 480
cgacccaaag cggtgcccat tcacaggtaa ataaatttaa atcagtaccg attttaacac 540
gcaatgcacc catagcatca ttgacgattt tggctttatc ggcaccaaag aaaatgatat 600
cgccagtttg ggcatcggta cgctcaatca gctcaatcaa aacctcatcg gtcatatttt 660
taatgatggg tgattgtaat cctgattctt tttcaacgcc attattgata ttgcttgcgt 720
cattgacctt aatatatgcc aatccacgag cgccataaat accaacaaat ttggtgtact 780
catcaatctg cttgcgactc atgttaccgc catttggaat gcgtaaggca acaacacggc 840
ctttaggatc ttgggcgggc cctgaaaata ctttaaattc aacatgttgc atgatgtcag 900
caacatcaat aagttttaag ggaatgcgta aatcaggctt atctgaggca taatcacgca 960
tggcatctgc gtaagtcatg cgggggaagg tatcaaactc a 1001

<210> 54
<211> 1001
<212> DNA
<213> Moraxella catarrhalis

<400> 54
tggatcatat tctttattaa tggtactgtt taaacctgta ttttaaagtt tattgggtca 60
tattttcaag ctcatcccat cgctcaagct tcatcatcaa aagctcatca atctctacca 120
atcgctcacc agccttcgtt gctgccgcca aatcggtatt aaaccatgaa ccatcttcaa 180
tctttttggc aagctgtgcc tgctcttgtt caagtgcagc aatttcatta ggcaaatctt 240
caagttcacg ctgctcttta tagctgagtt tgcgtttttg ggcaacgcct gattgaggtg 300
gtttgatttg gatgggttca gcgggttttg tcgccttagg tttattgtct gtggcgtgat 360
gagcaagcca tctttcatgc tgttgtacat agtcttcata accgccaaca tattccaaaa 420
cgataccgtc gccgtactta tcagtatcaa atacccaagt ttgggtaaca acattatcca 480
taaaagcacg gtcatggctg atgagtaata ccgtgccttt aaaattgacc acaaaatctt 540
ctaaaagctc aagtgttgcc atatccaaat cattggtagg ctcatcaagc accaaaacat 600

tggcaggttt tagcaataat ttggccaata aaacgcgtgc tttttcaccg cctgatagtg 660
ctttaacagg tgtgcgagca cgatttggcg tgaataaaaa atcttgcaaa tagcttaaaa 720
tgtgcgtagt ttttccacca acatcgacat ggtcagagcc ttctgaaaca ttatctgcga 780
tagatttttc agggtctagg tcgtctttga gttggtcaaa aaaagcaata tttagattgg 840
tgccaagctt aactgaacct gactgaatcg ctgaatcatc caaacccaaa atgcttttaa 900
ttaaggttgt tttaccaacg ccatttttgc caatgatacc aactttatca ccacgaacaa 960
gcagcgttga aaaatcctta actaaggttt tattgtcgta t 1001

<210> 55
<211> 1000
<212> DNA
<213> Moraxella catarrhalis

<400> 55
caacttgaaa atcagctcaa tgctctgcca cgcacagcac cgatgagcga gattatcgga 60
atgataaata ccaaagcaca agcggttaat gtgcaggtgg tgagtgcatc agttcaagca 120
ggtcgtgaac aggattatta taccgaacgc cctatcgcag tgagtgcgac aggggattat 180
catgctttgg gtcgatggtt acttgagttg tcagaggcta accatttgct gacagtgcat 240
gattttgatc tgaaggctgg tttgaaccat cagctgatga tgattgttca gatgaaaact 300
tatcaagcga acaaacgccc aaaaccagtt gctcagcagg tgcctgatgt tcaatgaata 360
ttatcggtgg ggcattttgg gtgcttggat ttgggttggg attggatgtg ctgatagcac 420
cagtcaagtt gttgatgata agcttgcaca tattacccat gaagagcgta tggcgatcag 480
tgagcctgtg ccgataccct tatctgtgcc gatgatatat cagcaaggca aagatccttt 540
tatcaatcct tatagaaatg ttgaggttct tgataccaat catgccgctg atcagcaaga 600
tgagccaaaa accgaatcta ccaaagcttg gcctatggca gacactatgc catctcagcc 660
atctgatact catcagtctg ccaaggctca ggcacaagtc ttcaaaggcg atccgatagt 720
cattgatacc aaccgtgttc gagagccttt agaaagctat gagttatcaa gcctacgcta 780
tcatggtcgt atttttgatg atgttagact tgtggcactc attatgagtc ctgatggcat 840
cgttcatcgt gtgagtactg gacaatatct tggtaaaaat cacggaaaaa ttacccatat 900
tgacagtcgt acgatacatc tgattgaagc ggtcgctgat acacaaggtg gctattatcg 960
ccgtgatgta aacattcatt ttattcataa gcaatgacac 1000

<210> 56
<211> 1001
<212> DNA
<213> Moraxella catarrhalis

<400> 56
ttcatgcaac aagcgaccat cttggccgat gataccatcc tgctcaccta agaaaatcag 60
tttatcagct tgcagggcaa tggctgtggt cagtgctaca tcttctgcca atagattaaa 120
aatttcgccc gtaaccgaaa aacctgtcgg tcctagtagg acaatatggt cattatccaa 180
attatggcga atggcatcga catcaattga gcgtacctca cctgtcatct gataatccat 240
accatctctg atgccgtaag ggcgagcggt gacaaaatta cccgaaatgg catcaatacg 300
agatccgtac attggggagt tagcaagccc catcgacagc cgagcttcga tttgtagacg 360
aattgagccg actgcctcca agatggcagg catagattca tacggtgtta cacgcacatt 420
ctcatgtagg tttgatatca gcttgcgatt ttgtaaattt ttttccactt gtgggcgtac 480
accatgcaca agcaccaatt tgatgcccaa gctgtgtagc agtgcaaaat catgaatcag 540
cgtactaaaa ttgtcacgag cgaccgcctc atcaccaaac ataaccacaa aggttttgcc 600
acgatgggtg ttaatgtacg gggcagaatt acgaaaccaa tgcacaggtg tgagtgcagg 660
agtgttctga taggtgctga cagaattcat gaatgctcca aagagtcaat ggctggtaaa 720
ataagaatgg cgaacaatat atggcgagag cgtctgatgt tggtcaaatg tcccattaat 780
aactatcaag ataccatcat accatagcaa agttttgggc agatgccaag cgaatttatc 840
agcttgataa ggttggcata tgataaaatc taccatcatc gtcgccagtt ttgagcatgt 900
gtaagtagtt accataatta aacagtcaag aaattcacac cgtcaatcag ctgtgctatg 960
cttatgggca cataaaactt gaccaacaca ggataaattt a 1001

<210> 57
<211> 1001
<212> DNA
<213> Moraxella catarrhalis

<400> 57
ggcatacttt tgccatgctt tattttggca taactgctat aagcccattg ctacttttta 60
tcatttatcc atatgtccaa taatgtgctt tatgtaattt aggcacacta ttaactcgtg 120
ccactgttaa cattcagcat aaaaatctta acaatgaatc aaagcatcgt attggctgtt 180
aaatgataag cttatattta tttaaattca gactaaatga ttgtaatatg gacatatcaa 240
ggttgaaatc aaaaattttg gagagttatg tacgataatg ataaaaaatt gaccaccatc 300
gtaggggtgt tgtatacggt gtcttatatt gccatatggt tggtcagtgg ctatatttta 360
tggggctgga ttggtgtgac aggatttact cgtgcgatac tttggctgat cgcttggatg 420
attgtgggta cgattgctga tagaattctg ataccgatta ttttgaccgt cgtggttggg 480
ttattttcta tcttttttga aaaaaggcga taatttggtt attttttcac aaaaaatcat 540
gatttttttt gtaaactatc taaaatatca attatgttat attatgtgat aaaagatggg 600
catgcttaag ttttggattg caaaaatcct aatatcatca ctgaccaaag ctgtgatgat 660
atcaaaactt tatcaaagtt cttagggtat tatcaagata tcataccaaa tgaatactta 720
cccaacttac tataaaaatc aaatgatatg actgtgattt tattatcata gatacaaaaa 780
tcaaaacgca tgagccaaag gtatgatgaa tgaatacaaa atttcgcaca cattatgaca 840
atctaaatgt cgccagaaac gctgacattg cggtgatttg gtgggatagg ggtcaagcca 900
gtgcgattaa gctaaatttt tatgtgggca atcgctgact ttattttatt tgtgccagtt 960
ggaacaattc gtggtctaat gtatttattt taaggagata a 1001

<210> 58
<211> 1001
<212> DNA
<213> Moraxella catarrhalis

<400> 58
tctggtctac atcccaaact atttacacaa gaaacactaa agacagtgga gcagatgacg 60
ctcaaaaagg catcttatag taatttgaca gttaattttc gtcaagtgct tgtacaaaaa 120
tacaccatcg tgcaagaagt ttgtaccaat ttaagcacaa tcattttggc acacactgtc 180
aagcaatgct tcaggcaaat tagctgctgg taaagatact tgggtcatca tgcaatcgca 240
tcaacccttc ttgctgcgtt gaagcgataa gtttgccatc ttgccaaaat tgaccatggt 300
ttagaccctt ggcgtggctt gtggtatcgc tccacatgtc gtagagtaga tattcggtca 360
tatcaaaagg gcgatggaaa tgtatggaat ggtcaatact agccatttgt agaccttgtg 420
tcatcaggct tagcccatga ctcattaaac ctgtgctgac caaataataa tcagacacaa 480
acgcaagtag tgcttgatga atggcaactg gctgctcccc aatatcagcg atacgcaccc 540
aattggcttg gcgtggacgc tcaggcttgg gtgtcacagg gtctcgtggt gtgacggggc 600
ggatttcgac atgacgctga cgcataaatc ttgctttgag tggttcggga attttatgta 660
aataatccgc tttgagttct tgctcggttt ttaggctttc agggggtgga taatcaggca 720
tggtttcttg gtaatcaagc ccgccttcca tgggtgaaaa tgaggcaatc atcgaaaaaa 780
tgacctgttc attggtcgta tgattaccgt ttttgtcggt ggttggcaca tattgcaccg 840
caatgacttc tcgagctgat aaactgcgtc catcacgtaa gcggcgtact tgatagatga 900
ctggtagacg aatatcgcca cctcgtaaaa aataaccatg taggctatga caaggtttat 960
caatcgttaa tgtgttagca ccagcaagca gcgcttgggc a 1001

<210> 59
<211> 1001
<212> DNA
<213> Moraxella catarrhalis

<400> 59
taaaatgacc ttacaaaata aaattatatg ttcaaaaatc gcttaagtat tgaaaaaagc 60
tataaaaact tatctattaa agcataaaag atattaaagc ataaaagacg agaaaagagc 120
aagcgtcaat gatgatattt catataaaaa cttatgaaat ttttcaattt tttatcgatt 180
gattcagctt ggctatcggt ggtcaacttt ggctgccaag acatcgccgg ctttttgaaa 240
aatcatcaca atggcaacaa tgatgatggt tgaaatccac ttgacatata ccatgttgcg 300
atgctcacca tagttaatcg caaggcttcc caagccacca ccgccaacca cacctgccat 360
tgcagaataa ccaatcaaag acaccaaggt caatgtgacc gcattaatca aaatgggcag 420
gctttcagca aaatagtatt tgctgacaac ctgccaatgc gttgcaccca tagatttggc 480
agcttcggtc agtcctgtgg gtacttctaa taaagcattg gcactcaagc gtgcaaaaaa 540
tggaattgct gccacactca aagggacgat ggcggctgtt gtgccaaggg ttgttcccac 600
caaaaatcgt gtgactggca tgagaataat gagcaaaata ataaaaggaa cggagcgacc 660
aatattaata ataacatcca aaattacaaa tacactgcga ttttcaagga tacgcccttt 720

atcggttaaa aatgccaaaa accctatcgg tagcccaacc aaaacagcga tggcagtggc 780
agcaagcccc atatagatgg tttcccaagt ggattgggca accatctccc acattcttgg 840
gtgcatttca ctgacaattt ttgtgacgat ttcattccac atagccgata atctcaatat 900
tgacccgatg ggtggttaaa aattctattg cttgcatgac cgaggtgcct tcaccgataa 960
gctcagcaat ggtaaagcca aattttatat cacctgcata a 1001

<210> 60
<211> 1001
<212> DNA
<213> Moraxella catarrhalis

<400> 60
agtaaacaat ggtaacaaat acagcagtgt cgcacagtcc tcagtacgat gattctgaat 60
ttgaatatgc aggattttgg atacgatttg tggcatgtct tgtcgataat ttaattgtta 120
tgattataat tgcaccgtat tggttttata attatcagca aatgatggcc atgcctgctg 180
accaaatacc gttttatagt gttggggatg ccatccttta tagtgctggg gatgctatcc 240
taaacttagt gatggcggcg gcggttgttt ggttttgggt aaaaaaaggt gcaacaccag 300
gtaaaatgct ctttgggctg caagtccgtg atgccaaaac agggcaattt atcagtgtgc 360
caagggcatt attgcgatat tttagttatc tgatttcatc cgtgattctt tgtttgggac 420
ttatttgggt tggttttgat aagaaaaaac aaggctggca tgataaaatt gccaaaactg 480
ttgtggtaaa acgcattcgc tgatgggtcg ccagttaaac aataaaacca tcaaacgcaa 540
gcagggcgat gtgtttgagc agttggcggt agataagcta aaacaagcag gctatgaaat 600
tattttaacc aactttacca ccccatttgt tggtgagatt gatattatcg ccagacagcc 660
tttggagcaa tcgcaccgtt tggtgcagcc aagattttgt acggtatttg ttgaagtgcg 720
tagccgaaca agttctgtgt atggtacagc gcttgagagt gttacctcaa aaaagcaggc 780
aaaaatctac cgaacagcag aacgattttt aatcaattat cccaaatata ttgatgatgc 840
ataccgtttt gatgtcatgg tttttgattt ggttgatgga ttgattgaac atgaatggat 900
aaaaaatgcg ttttgattgg ctcaatggtc gtgaattaaa atcaatcaag caatccgtag 960
ctttactata agatatatcc cagtaatatg gaaacatagc a 1001

<210> 61
<211> 1001
<212> DNA
<213> Moraxella catarrhalis

<400> 61
cgtttagctt catacgcaga ccttgtgcac cttcgggcaa ccgaagcatc acgccagcat 60
cacgcatccg cacaaaaccc atcatgccat caatttcgct gctgatatga tataccccca 120
ccaaagtaaa ccgcttaaat cgtggaataa cgcctgctgc tgagggtgag gcttcaggca 180
aaaccaaggt aaccttatcc cccaacttaa gtcccatgtc agagacaatg gactcaccta 240
atataatacc aaactcgccg atatgtaaat catccaaatt gcctgcggtc atatgctcat 300
caatgataga aacttgcttt tcgtaatcag gctcaatgcc agaaaccacg attccagtca 360
cctgaccttc agcggttaac ataccttgta gttgaatata aggggcaact gcttgcactt 420
ctggattttg cattttgatt ttttcggcaa gttcttgcca atttgtcaaa atttctgttg 480
aggtaactga agcttgaggc accatgccaa gaatgcgtga tttaatttca cggtcaaagc 540
cattcatgac cgacaaaacc gtgataagca ctgcaacccc aagcgtaagc ccaatggttg 600
agataaaaga aataaaggaa ataaagccat ttttacgctt agctttggta tatctaagcc 660
caataaataa cgccaaggga cgaaacataa gctgtgttcc aaacgaccca accgtgctag 720
tttagcactt ttttggacaa ataccaaaca tcacataaca aatgaatcat caggttggtt 780
ttgttgcgct tgtgtatctg tatgataagt ttcttgctaa aacagctttt ttatgtcaga 840
atacagaaaa ggtatatact tatattttta actttaaata gatctgcttt tttataccga 900
tgatttggca tgaagtttat cggtctgata tgctggatat aagtttatcg gcttgatata 960
aattttaatt aatcatcaaa tttttaagga atttatcatt a 1001

<210> 62
<211> 1001
<212> DNA
<213> Moraxella catarrhalis

<400> 62
   taaggatacc agattttggc ttgtcaatcg ttgtgttaat cattgtaacg gtttatagtg 60
   attgtcaatt aataagggta aaaaagtatt tatcaagtaa taatctttct tatatgtgaa 120
   tataatgaca aatttatcac atttttacaa ggatttttta tcaagattag gatatgttcc 180
   agcttaatta ttagtgatga gcgtgtgatt atttggcatc gttaaattta tgagtgctaa 240
   aattgccaaa tgattaaaat tttgctaaca tgatagcccc tttggtaggc tttatttggt 300
   attgatgagc aataataata taccgagtta aatggattaa cttaacatac gccaaaaact 360
   taacaacgaa aagtagatga ttatgacaga tacagtacaa aaagatacag cacagtcccc 420
   caaaaaagtt tatctaaaag actacacgcc gccagtatat gcagttaata aagtggattt 480
   ggatatccgc ttgtttgatg atcatgctgt cgttggtgcc aaacttaaaa tgacacgagc 540
   acacgcaggc gagcttcggc ttcttgggcg agatttaaag cttaaaagca ttcacctaaa 600
   tggtcaggaa ttagagtcgc aggcgtatca tcttgataag gaaggcttaa caattttaga 660
   tgcaccagat gtcgcagtga ttgagacatt ggttgagatt tcaccacaaa ccaacacaac 720
   acttgaaggg ctatatcaag caggaacagg tgatgataag atgtttgtga cacaatgcga 780
   acctgagggt tttcgcaaaa tcaccttttt ccctgaccgc cctgatgttt tgacagaata 840
   caccacacgc ctagaagcac caaagcattt taaaaccttg cttgccaatg gtaatttggt 900
   tgagtcagga gatgtggatg aaaatcgcca ttataccatt tggcatgatc ctaccaaaaa 960
   acccagctat ctattcgccg ctgtcattgc caatctagaa g 1001

<210> 63
<211> 1001
<212> DNA
<213> Haemophilus influenzae

<400> 63
aaatcaagcg cctgtgcctg ctggtgatgg ttgtggagac gaattatatt cttggtttga 60
accgccaaaa ccaggcactt cagtgagcaa acctaaagtt acaccgcctg agccgttttt 120
gtgccaacag attttgaact caccgaatcg gagagaatgg ttagaatagc attgaggtaa 180
atcaatatgg atatcggcat tgatctttta gcaatattgt tttgtgttgg ttttgtcgca 240
tcatttatcg atgcaattgc tggcggtggt ggattaatca ccattccagc gttactcatg 300
acaggtatgc caccagcaat ggcgttaggc accaacaaat tgcaagctat gggcggtgca 360
ttatccgcaa gcctttattt cttgcgaaaa agagcggtca atttacgcga tatttggttt 420
attttgattt gggtttcttt aggttctgcc ctaggtacat tattaattca atcaattgac 480
gtggcgattt tcaaaaaaat gcttcctttt ttgattttag ccattggtct atatttttta 540
tttactccta aattaggtga tgaagatcga aaacaacgat taagttatct gttatttggt 600
cttttagtta gcccattttt aggtttttat gatggcttct ttgggccagg gactggctca 660
atcatgagtt tagcctgtgt tactttgcta ggatttaatc tcccgaaagc ggcagcacat 720
gcaaaagtga tgaacttcac ttcgaacctt gcttcttttg cacttttctt attgggcgga 780
caaattcttt ggaaagtggg tttcgtgatg atggctggga gcattttagg tgcaaattta 840
ggtgccaaaa tggtgatgac gaaaggtaaa accttgattc gaccgatggt tgttatcatg 900
tcttttatga tgacggctaa aatggtttac gatcagggtt ggtttcattt ttaattcgga 960
aagcgcgcaa aagtgcggtt aaaattaatt acattttatt a 1001

<210> 64
<211> 1001
<212> DNA
<213> Haemophilus influenzae

<400> 64
ttgaagtccc caatttaccc accacaattc ctgcggcaac attggctagg taacaagatt 60
cttcgaaaga acgtccatct gctaatgtgg ttgctaatac actaatgaca gtgtcaccgg 120
ctcccgtcac atcaaacact tcttttgcaa cggttggcaa atgataaggc tcttgatttg 180
ggcgtaataa tgtcatgcct ttttcagaac gcgtcaccaa aagtgcggtt aattcaatat 240
cagaaattaa ttttaaacct ttcttaataa tctcttcttc tgtattacat ttacctacaa 300
cggcttcaaa ttcagacata ttgggtgtca ataatgtagc cccacgataa cgttcaaaat 360
cagttccctt tggatcgatc aacacaggca cattcgcttt gcgtgcaatt tgaatcattt 420
tctgaacatc tttaagcgtg cctttgccgt aatcagaaag aatcaaagca ccgtaatttt 480
tcaccgcact ttctaacttc gctaataaat ccttgcaatc tacattattg aaatcttctt 540
caaaatcaag gcggagcagc tgttgatgac gagataaaat acgtaattta gtaatggttg 600
gatgggtttc taatgcaaca aaattacaat caatcttttg tttttctaat aagtgggaaa 660
gtgcagaacc tgtctcatct tgtccaatca atcccattaa ctgaacgggt acattgagtg 720
aagcaatatt catcgccaca tttgcagcac cgcccgcgcg ttcttcattt tcttgtacgc 780
gaactactgg cactggtgct tctggtgaaa tacggttggt tgcaccgaac caataacgat 840

caagcatcac atcgcctaat acaagtactt ttgcttgctt aaattctgct gaatattgag 900
ccattttaaa atctctctat ttgaataacc aaaattgtgg cgattttacc acaactcaaa 960
tttacgataa actacgcccc taacttacgt ggaaagaaca a 1001

<210> 65
<211> 1000
<212> DNA
<213> Haemophilus influenzae

<400> 65
agcaataatt atagctggaa tattctttaa agatgaaaga gatcgtataa gacaaaaaga 60
atttatattt ggagaattat tagcaattat tggttcgcta atattcgtaa taaatagttc 120
aaataatgat ggaaatacag actttttctt tggggcaata tttcttttta cagctatttt 180
tattcaatct gtacagaatt taattgtaaa aaaagtagcc aaaaagataa atgctgttgt 240
aataagtgca tcgacagcaa caatttcagg agtattattt ttatgtttag cttttaatac 300
taaacaaata tatttattac aagatgttgg cattggaatg ttgataggtt tagtttgcgc 360
tggcttttat gggatgctaa cagggatgtt gatggctttt tatattgttc aaaaacaggg 420
aatcactgtt tttaacattt tgcaattatt aattcctctt tcaactgcga taataggtta 480
cttaacatta gatgaaagaa taaatatcta tcagggaatt agcggtatta ttgtaattat 540
tggttgtgta ttggcattaa aaagaaaaaa caaggagtgt tgatatataa agtagatgat 600
gttggtggaa taggtatagt taaatatctg gttcaattgg ttttattaag ggcgttagca 660
attctccatt taagtttatg tttgaattag atattttggg aaaagatgga agaataaagc 720
tgttaaataa tgctgaaaca tatgaactat accaatactc aaataaaaat aattctgctg 780
gaaatgatta taaatctcta attctaactt gtagagagga taatgactat caatcagaaa 840
gaatgattaa agccattaaa aatattattc attgtatgac taataatcat caacctattt 900
caagtgctga aacatcttta gaaactatta aaattattca cggaataatt aattctgtta 960
aaataggtaa tgatcctaac aatatataag gagaataagt 1000

<210> 66
<211> 1001
<212> DNA
<213> Haemophilus influenzae

<400> 66
taaatactcc aaaataaatt tcagataacg tggtctgtaa gacaaaaaaa taaaaaaaat 60
gttcaataag aggagagcaa attatcttgt ttaaaaggaa atcggagcag tacaaaaacg 120
gtcttacaag tagcaaattc tataaattta tgttctaata cgcgcaattt tctagtcaat 180
aaaaaggtca aaaaatgagc tggattaacc gaatttttag taaaagtcct tcttcttcca 240
ctcgaaaagc caatgtgcca gaaggcgtat ggacaaaatg tactgcttgt gaacaagtac 300
tttatagtga agaactcaaa cgtaatctgt atgtttgccc gaaatgtggt catcatatgc 360
gtattgatgc tcgtgagcgt ttattaaatt tattggacga agattcaagc caagaaattg 420
cggcagattt agaaccaaaa gatattttaa aattcaaaga tttaaagaaa tataaagatc 480
gtatcaatgc ggcgcaaaaa gaaacgggcg agaaagatgc gctaattact atgacaggta 540
cactttataa tatgccaatc gttgtggctg catcgaactt tgcttttatg ggcggttcaa 600
tgggttctgt agttggtgca aaatttgtta aagcggctga aaaagcgatg gaaatgaatt 660
gtccatttgt gtgtttctct gcgagtggtg gtgctcgtat gcaggaagca ttattctctt 720
taatgcaaat ggcaaaaact agtgccgtac ttgctcaaat gcgtgaaaag ggtgtgccat 780
ttatttcagt attaacggat ccgactttag gcggcgtatc agccagtttt gcgatgttag 840
gggatttaaa tattgccgag ccaaaagcct taattggttt tgcagggcca cgcgttattg 900
aacaaactgt gcgtgaaaaa ttgccagaag gtttccaacg tagtgagttt ctacttgaga 960
aaggggcaat tgatatgatc gtgaaacgtt cagaaatgcg t 1001

<210> 67
<211> 1001
<212> DNA
<213> Haemophilus influenzae

<400> 67
tcacttaatt caagcgcatc aatgttttct aaaacatcaa cagaattgac cgcacttgta 60
tctaaaattt cgccatttat taagactgcg cgtaatgcca aaacatgatt agaggtttta 120
ccatattgca atgagccttg cccagaggca tcggtgttaa tcattccacc taaagtcgct 180

cgattgctgg tggacagttc tggggcaaag aacaaaccat gtggttttaa aaattgatta 240
agttgatctt ttactacgcc tgcttgtact cgaacccaac gttcttttac attgagttct 300
aagatggctg tcatatgacg agaaagatcc actattatat tgttattgat ggattgccca 360
tttgtgccag tgcctccacc gcgaggcgta aagctgattg attgatattc aggtaaattt 420
gccaattttg ttatccgcac tatatcagca accgttttcg gaaaaagaat tgcttgtgga 480
agttgttggt aaacgctgtt atccgtagcc agacttaatc tatctgcata gtttgtcgca 540
atatccccct caaaatgttg gcattgaaga tcatcaagat aatcaagtac atattgttca 600
acttgaggaa tgcgatttag atttggcaac atagtatttg acccatttaa acatatcaga 660
tggaggcttt gataatatcc taaggctaga ataatgtcga ttaggaaaga gagaggagaa 720
agtaaaaagt ctgtttaaga aagtgttatt ttggataaaa actaaacaaa aaattcaaaa 780
gaatttgatc ttttcaattt ttataggata ataagcgcac ttttgaacgt tcctttgggg 840
taaacataag caaaggaatt gaatttgtca aaaggtaata aagtagggca aattcaaaac 900
cctagttaag tgactgttta taatgtagct ttaattaaaa gttcagtata aacaaggaca 960
ctttttatta ctattcgatc actaaataga ggacatcaaa a 1001

<210> 68
<211> 1001
<212> DNA
<213> Haemophilus influenzae

<400> 68
tcgattgtat cctatataaa ttatagacgt aaaaaatcat taaataatgc aaacaccgtt 60
aagcttaata acagtgctgc gccaattcga taacagatgc tttgcacccg ctcagaaaca 120
ggttttcctt taacagcttc cattgttaaa aaaactaaat gaccgccatc taatactggt 180
aatggaaata aattcataat ccctaaattt acactaatca atgccataaa acttaaaaaa 240
tacaccaatc caatatttgc tgatgcgcca gcaccttttg caatagaaat tggcccactt 300
aaattattta atgacaaatc gccagtaagt aatttcccta atattttcaa ggttaaaagg 360
gaaagctgtc ctgttttttc aatgcctttt tgtaaagatt caagaatacc atattttaat 420
tcagtacggt attcatccgc taattttgtt aaggctgggc taaccccaac aaaccatttg 480
ccattttgat tacgcactgg agttaggact ttgtcaaatg tttctccatt acgttcaact 540
ttaatagaaa aagattcgcc ttgttcgacc tgttttataa aatcttgcca aggaagtgcg 600
gttaaatttt cttttaaaat tttatcaccg atttgtaaac cagctttctc agcgggagaa 660
ttttgaacaa ctttagaaag caccatttca attttaggac gcataggcat aatccctaat 720
gcctcaaaag cactttcttt ttcaggatcg aatgtccaat ttgtaagatt taaagtccgt 780
tgttgttcaa tattagaatt gaaaggagaa aggctaatct caacattagg ctcccccatt 840
tttgtggcaa gtagcatatt gatggtttcc caatcttgag tttcttcgcc atcaattgta 900
agaatttgcg tattgggttc aatgtgggct tgtgctgcga ttgagtttgg tgttattgat 960
tcaatcactg gtttaaccgt tggcattcca taaaggtaaa t 1001

<210> 69
<211> 1001
<212> DNA
<213> Haemophilus influenzae

<400> 69
tttgataaat atccttaatt aaatgatggg tttaatattt tctctgccca attaaattag 60
gcagagaacg ttgtttttga gttctgatga agaaaaaagt tcaatttatt agaaagaacc 120
tccaatacta aattggaact gttcgacatc atcattttca tattttttaa ttggtttggc 180
ataagagaat accaatggcc caataggaga ttgccattgg aatccgacac ctgtagaggc 240
gcgaatacgg cttgatttgc cataatcggg taagcttttt aatacattgt tatctaaccc 300
actcttatcc gatttccact tagtattcca aacacttgcc gcatcaacaa atagggaggt 360
tcggactgta ttttggcttt tatcactcac aaacggtgtt ggtacaataa gttctgcact 420
cgcagttgtg attgcattac caccaatcac atcagaactt atcttcttaa aagtaccatt 480
accattacca tgttctgcat aaattgcgtt aggtccaata ctaccataag caaaaccacg 540
taatgaaccg atgccacccg ctgtataagt ttgatagaac ggtaaacgct tgtttccaaa 600
accatttgca tatcctgcag atgcttttgc agatacaacc cagaggtgat ctctgtctaa 660
tgggtagaaa ccctgtacgt ctgcacttag tttgtagtat ttgttatcag aacctggaat 720
agtaactcgt ccaccaagac ttgctttaac ccctttagtt gggaaatagc ctctattaag 780
gctgttatag ttccaaccaa aagaaaaatc aaagtcattt gttttaatgc cattaccttt 840
aaatttcatt gattgaatat ataaattacg gttatattct agagcaaagt tactaatttt 900
attataggta tggcctaatc ctacataata ggagttattt tcatttacag ggaaacctaa 960

agtaacatta cttccataag tcgtacgctt atagttagag g 1001

<210> 70
<211> 1001
<212> DNA
<213> Haemophilus influenzae

<400> 70
ttagatttct cctaaatgag ttttttattt agttaagtat ggagaccaag ctggaaattt 60
aacttgacca tcacttcctg gaaggctcgc cttaaagcga ccatctgcgg aaaccaattg 120
tagcaccttt cctaagccct gtgtagaact ataaataatc ataattccat ttggagagag 180
gcttgggctt tcgcctagaa aagatgtact aagtacctct gaaacgcccg ttgtgagatc 240
ttgtttaact acattattgt taccattaat catcacaagt gtttttccat ctgcactaat 300
ttgtgcgcta ccgcgaccac ccactgctgt tgcactacca ccgcttgcat ccattcgata 360
aacttgtggc gaaccacttc tatcggatgt aaataaaatt gaatttccgt ctggcgacca 420
cgctggttca gtattattac ccgcaccact cgtcaattga gtaggtgtac cgccatttgc 480
tcccataacg taaatattca gaacaccatc acgagaagaa gcaaaagcta aacgagaacc 540
atctggcgaa aaggctggtg cgccattatg cccttgaaaa gatgccacta ctttacgtgc 600
gccagaattt aaatcctgta caacaagttg tgatttttta ttttcaaacg atacataagc 660
caaacgctgg ccgtctggag accaagctgg agacataatt ggttgggcac tacgattgac 720
gataaattga ttatagccat cataatctgc tacacgaact tcataaggtt gcgaaccgcc 780
atttttttgc acaacataag cgatacgagt tctaaaggca ccacggatcg cagttaattt 840
ttcaaaaact tcatcgctca cagtatgcgc gccatagcgt aaccatttat ttgttactgt 900
atagctattt tgcattaata cagtccctgg cgtacctgat gcaccaaccg tatcaattaa 960
ttgataagta atactataac cattacccga tggaaccact t 1001

<210> 71
<211> 1001
<212> DNA
<213> Haemophilus influenzae

<400> 71
ggcgataacc gagtttttgg ggtatttagt gccaaagaag acccacaaaa cccaaaatta 60
tccagagaaa ccttaattga tggcaagcta actactttta aaagaactga tgcaaaaacc 120
aatacaacag ccgatacaac aaccaataaa acaaccaatg caataaccga tgaaaaaaac 180
tttaagacgg aagatatact aagttttggt gaagctgatt atcttttaat tgacaatcag 240
cctgttccgc ttttacctga aaaaaatact gatgatttca taagtagtag gcatcatact 300
gtaggaaata aacgctataa agtggaagca tgttgcaaga atctaagcta tgtaaaattt 360
ggtatgtatt atgaagaccc acttaaagaa gaagaaaaag aaaaagaaaa agaaaaagac 420
caagaaaaaa aagaaaaaga aaaacaaacg acgacaacat ctatcgagac ttattatcaa 480
ttcttattag gtcaccgtac tgccaaggcc gacatacctg caacgggaaa cgtgaaatat 540
cgcggtaatt ggtttggtta tattggtgat gacacgacat cttactccac tactggagat 600
aaaaatgctc tcgccgagtt tgatgtaaat tttgccgata aaaagctaac aggcgaatta 660
aaacgacacg ataatggaaa taccgtattt aaaattactg cagaccttca aagtggtaag 720
aatgacttca ctggtacagc aaccgcaaca aattttgtaa tagatggtaa caatagtcaa 780
actggaaata cccaaattaa tattaaaact gaagtaaatg gggcatttta tggacctaag 840
gctacagaat taggcggtta tttcacctat aacggaaatt ctacagctaa aaattcctca 900
accgtacctt caccacccaa ttcaccaaat gcaagagctg cagttgtgtt tggagctaaa 960
aaacaacaag tagaaacaac caagtaatgg aatactaaaa a 1001

<210> 72
<211> 1001
<212> DNA
<213> Haemophilus influenzae

<400> 72
tagaattata ttcttataca aaattgataa ttgttcgcat tatcattttt tttttgtaat 60
aatgtcaact tataattttt taagttcatg gataaaatat gaaaaatggc gtaaaacaac 120
tttttctctt atcattaata ggcttatcat taacgaatgt agcttgggca gaagttgcac 180
gtcctaaaaa tgatacattg acaaatacga ttcaaagtgc ggaattaaaa acctcctctt 240
tttcctctat gcctaagaaa gaaataccaa ataggcatat tatttctctt tccaaaagcc 300

aattagcgca ccatccaagg cttgttttgc gtgggttaat tcctgcttta tatcaaaata 360
acactcaggc agttcaactg ttattaccac tatataaaca atttcctcaa caagataatt 420
tcttactaac ttgggcaaag gctattgaag ctcgtgaaca aggtgattta actcaatcta 480
ttgcttatta tcgtgaatta ttcgctcgag acgcatcttt actaccttta cgttattaat 540
tagctcaagc tctatttttt aactatgaaa atgaagctgc caaaattcaa tttgaaaaat 600
tacgtacaga ggtagatgat gaaaaatttt taggtgttat tgatcagtat cttttaacac 660
taaatcagcg gaatcaatgg atatggcaag taggattaaa ttttttaaat gatgataatt 720
tgaataacgc tccaaaaagt ggcacaaaaa ttggtagttg gaccgcttgg gaaaaagaaa 780
gtgggcaggg ggtagggtat tctttatcag tagaaaaaaa atggccatgg gcagatcatt 840
tttttagtaa aactatgttt aatgggaatg gaaaatatta ttgggataat aaaaaataca 900
atgaggctac tgtgcgtata ggtggtggtt taggctatca aactgcctca gttgaagtct 960
cgttgtttcc ttttcaagaa aaacgctggt atgcaggcgg t 1001

<210> 73
<211> 1001
<212> DNA
<213> Haemophilus influenzae

<400> 73
taataaattg ctccataaag aggtttgtgc cttataaata aggcaataaa gattaatata 60
aaccgtttat taaaatgcca aaggcttaat aaacagcaaa ctttgttttc ccaaaaaaag 120
taaaaaactc ttccattata tatatatata tatataatta aagccctttt tgaaaaattt 180
catatttttt tgaattaatt cgctgtaggt tgggtttttg cccacatgga gacatataaa 240
aaagatttgt agggtgggcg taagcccacg cggaacatca tcaaacaact gtaatgttgt 300
attaggcacg gtgggcttat gcctcgccta cggggaaatg aataaggata aatatgggct 360
tagcccagtt tatggattta attatgttga aatggggaaa acaatgttta aaaaaacact 420
tttatttttt accgcactat tttttgccgc actttgtgca ttttcagcca atgcagatgt 480
gattatcact ggcaccagag tgatttatcc cgctgggcaa aaaaatgtta tcgtgaagtt 540
agaaaacaat gatgattcgg cagcattggt gcaagcctgg attgataatg gcaatccaaa 600
tgccgatcca aaatacacca aaaccccttt tgtgattacc ccgcctgttg ctcgagtgga 660
agcgaaatca gggcaaagtt tgcggattac gttcacaggc agcgagcctt tacctgatga 720
tcgcgaaagc ctcttttatt ttaatttgtt agatattccg ccgaaacctg atgcggcatt 780
tctggcaaaa cacggcagct ttatgcaaat tgccattcgc tcacgtttga agttgtttta 840
tcgccctgcg aaactctcga tggattctcg tgatgcaatg aaaaaagtag tgtttaaagc 900
cacacctgaa ggggtgttgg tggataatca aaccccttat tatatgaact acattggttt 960
gttacatcaa aataaacctg cgaaaaatgt caaaatggtt g 1001

<210> 74
<211> 1001
<212> DNA
<213> Haemophilus influenzae

<400> 74
tagtagattt ccgcacgggc aaaaatacaa tggtgttatt taacctcact ttgccaaatg 60
gcgagccagt gccaatggca tccaccgcac aagatagcga aggggcattt gtgggcgatg 120
tggtgcaagg tggtgtgctt ttcgctaata aacttaccca gccaaaaggc gagttaatcg 180
tcaaatgggg tgagcgagaa agcgaacaat gccgtttcca atatcaagtt gatttggata 240
acgcacaaat acaaagtcac gatattcaat gcaaaaccgc aaaataaata attgaagagg 300
atttatgcaa aaaacaccca aaaaattaac cgcgcttttc catcaaaaat ccactgctac 360
ttgtagtgga gcaaattata gtggagcaaa ttatagtggc tcaaaatgct ttaggtttca 420
tcgtctggct ctgcttgctt gcgtggctct gcttgattgc attgtggcac tgcctgctta 480
tgcttacgat ggcagagtga cctttcaagg ggagatttta agtgatggca cttgtaaaat 540
tgaaacagac agccaaaatc gcacggttac cctgccaaca gtgggaaaag ctaatttaag 600
ccacgcaggg caaaccgccg cccctgtgcc tttttccatc acgttaaaag aatgcaatgc 660
agatgatgct atgaaagcta atctgctatt taaaggggga gacaacacaa cagggcaatc 720
ttatctttcc aataaggcag gcaacggcaa agccaccaac gtgggcattc aaattgtcaa 780
agccgatggc ataggcacgc ctatcaaggt ggacggcacc gaagccaaca gcgaaaaagc 840
cccgacacaa ggtaaagcgc aaaacggcac agttattcaa ccccgttttg gctactttgg 900
ctcgttatta cgccacaggt gaagccaccg caggcgacgt tgaagccact gcaacttttg 960
aagtgcagta taactaaaat atttattatc cagtgaaaaa a 1001

<210> 75
<211> 1001
<212> DNA
<213> Haemophilus influenzae

<400> 75
ttatccgcta acatttcatc agtaattcca tgaactttaa tcgcatcagg atcancgggg 60
cgatctggct taatataaat atgayaatta ttacctgtgt aacgacgatt tattaattca 120
actgcaccaa tttcaataat gcagtgtcct tcataatgcg cgccaagctg attcatacct 180
gtagtttcag tatctaatac aatttggcga ttgggattaa tcatttgttc aacctatctc 240
tttccattaa aatacttgcc attctacaca acaacctttt tgttatgcck aaacagattg 300
aaatttttac tgatggatct tgcttaggta atccaggggc gggcggaatt ggtgccgtat 360
tgcgttataa acaacatgaa aaaacactct ccaaaggcta tttccaaacc accaataatc 420
gaatggaatt acgcgctgtc attgaagcat taaatacatt aaaagaacct tgcttgatca 480
cgctttatag tgatagccaa tatatgaaaa atggcataac caaatggatc tttaactgga 540
aaaaaaataa ttggaaagca agttctggaa agcctgtaaa aaaccaagat ttatggatag 600
ccttagatga atccatccaa cgtcataaaa ttaattggca atgggtaaaa ggccatgctg 660
gacacagaga aaatgaaatt tgcgatgaat tagcaaaaaa aggggcagaa aatccgacat 720
tggaagatat ggggtacata gaagaataat acaactgata taacgtcata tttttcgata 780
cctaaaaata tttaatactt aaacctaaaa cagaataaaa aataatcaaa ttcatttaaa 840
aaatgtgatc tcgatcagat ttcaagaaaa ttaaaatttt ggagtattga catcaaaaat 900
tttttttgta aagatgcagc tcgtccgttt tggcgattgg acaattctat tggagaaaag 960
ttcaatcata gatagtaaac aaccataagg aatacaaatt a 1001

<210> 76
<211> 924
<212> DNA
<213> Moraxella catarrhalis

<220>
<221> CDS
<222> (1)...(924)

<400> 76
tca gtg ctt ggt ttt tta aga tat gta ccg ctg tca gtc ctg cat gga 48
Ser Val Leu Gly Phe Leu Arg Tyr Val Pro Leu Ser Val Leu His Gly
1               5                   10                  15

ttg gcg gcg tgt gcg tct tat att tcc tat cat tgc agg ctt agt att 96
Leu Ala Ala Cys Ala Ser Tyr Ile Ser Tyr His Cys Arg Leu Ser Ile
            20                  25                  30

tat cgc agc atc caa gcc aat tta atc ttg gtt cac ccc aag atg cca 144
Tyr Arg Ser Ile Gln Ala Asn Leu Ile Leu Val His Pro Lys Met Pro
        35                  40                  45

gac gca cag cgg caa aaa ctc gcc aaa caa atc cta aaa aat cag ctc 192
Asp Ala Gln Arg Gln Lys Leu Ala Lys Gln Ile Leu Lys Asn Gln Leu
    50                  55                  60

atc agt gca gtc gac agt ctt aaa act tgg gca atg cca cca aaa tgg 240
Ile Ser Ala Val Asp Ser Leu Lys Thr Trp Ala Met Pro Pro Lys Trp
65                  70                  75                  80

tct atc gca caa att aaa acg gtt cat cat gaa gat atc cta atc aaa 288
Ser Ile Ala Gln Ile Lys Thr Val His His Glu Asp Ile Leu Ile Lys
                85                  90                  95

gca ctt gcc aat cca agt ggt atg ctt gcc att gtg cct cat atc ggc 336
Ala Leu Ala Asn Pro Ser Gly Met Leu Ala Ile Val Pro His Ile Gly
            100                 105                 110

act tgg gag atg atg aat gct tgg ctc aat acc ttt ggc tcc cct act 384
Thr Trp Glu Met Met Asn Ala Trp Leu Asn Thr Phe Gly Ser Pro Thr
        115                 120                 125

atc atg tat aag ccc atc aaa aat gcg gcg gta gat cgc ttt gtt tta 432
Ile Met Tyr Lys Pro Ile Lys Asn Ala Ala Val Asp Arg Phe Val Leu
    130                 135                 140

cag ggg cgt gaa aga cta aat gcc agc ctt gta ccc aca gat gct agt 480
Gln Gly Arg Glu Arg Leu Asn Ala Ser Leu Val Pro Thr Asp Ala Ser
145                 150                 155                 160

ggt gtt aag gca att ttt aaa aca ctc aaa gca ggt gga ttt agt atc 528
Gly Val Lys Ala Ile Phe Lys Thr Leu Lys Ala Gly Gly Phe Ser Ile
                165                 170                 175

ata ctg ccc gac cat gta cct gat cca tca ggt ggt gag att gct cct 576
Ile Leu Pro Asp His Val Pro Asp Pro Ser Gly Gly Glu Ile Ala Pro
            180                 185                 190

ttt ttt ggt att aaa acc cta acc agt acg ctg gcg tca aag ctt gct 624
Phe Phe Gly Ile Lys Thr Leu Thr Ser Thr Leu Ala Ser Lys Leu Ala
        195                 200                 205

gca aaa act ggt tgt gct ctt gtt ggc tta agc tgt att cgg cgt gaa 672
Ala Lys Thr Gly Cys Ala Leu Val Gly Leu Ser Cys Ile Arg Arg Glu
    210                 215                 220

gat ggc gat ggt ttt gaa att ttt tgt tat gaa tta aat gat gaa caa 720
Asp Gly Asp Gly Phe Glu Ile Phe Cys Tyr Glu Leu Asn Asp Glu Gln
225                 230                 235                 240

ctt tat tca aaa aat acc aaa att gca acc act gct tta aat ggt gcg 768
Leu Tyr Ser Lys Asn Thr Lys Ile Ala Thr Thr Ala Leu Asn Gly Ala
                245                 250                 255

atg gaa caa atg att tat cca cat ttt ttg cat tat atg tgg agc tat 816
Met Glu Gln Met Ile Tyr Pro His Phe Leu His Tyr Met Trp Ser Tyr
            260                 265                 270

cgt cgg ttc aag cat aca cca cta tta aat aat cct tat tta ctt aat 864
Arg Arg Phe Lys His Thr Pro Leu Leu Asn Asn Pro Tyr Leu Leu Asn
        275                 280                 285

gaa aat gag cta aaa aaa ata gcc ata aag ctt caa gcc atg tca aag 912
Glu Asn Glu Leu Lys Lys Ile Ala Ile Lys Leu Gln Ala Met Ser Lys
    290                 295                 300

gat agt tat gag 924
Asp Ser Tyr Glu
305

<210> 77
<211> 894
<212> DNA
<213> Neisseria meningitidis

<220>
<221> CDS

<222> (1)...(894)

<400> 77
atg ttt cgt tta caa ttc ggg ctg ttt ccc cct ttg cga acc gcc atg 48
Met Phe Arg Leu Gln Phe Gly Leu Phe Pro Pro Leu Arg Thr Ala Met
1               5                   10                  15

cac atc ctg ttg acc gcc ctg ctc aaa tgc ctc tcc ctg ctg cca ctt 96
His Ile Leu Leu Thr Ala Leu Leu Lys Cys Leu Ser Leu Leu Pro Leu
            20                  25                  30

tcc tgt ctg cac acg ctg gga aac cgg ctc gga cat ctg gcg ttt tac 144
Ser Cys Leu His Thr Leu Gly Asn Arg Leu Gly His Leu Ala Phe Tyr
        35                  40                  45

ctt tta aag gaa gac cgc gcg cgc atc gtc gcc aat atg cgt cag gca 192
Leu Leu Lys Glu Asp Arg Ala Arg Ile Val Ala Asn Met Arg Gln Ala
    50                  55                  60

ggc atg aat ccc gac ccc aaa aca gtc aaa gcc gtt ttt gcg gaa acg 240
Gly Met Asn Pro Asp Pro Lys Thr Val Lys Ala Val Phe Ala Glu Thr
65                  70                  75                  80

gca aaa ggc ggt ttg gaa ctt gcc ccc gcg ttt ttc aga aaa ccg gaa 288
Ala Lys Gly Gly Leu Glu Leu Ala Pro Ala Phe Phe Arg Lys Pro Glu
                85                  90                  95

gac ata gaa aca atg ttc aaa gcg gta cac ggc tgg gaa cat gtg cag 336
Asp Ile Glu Thr Met Phe Lys Ala Val His Gly Trp Glu His Val Gln
            100                 105                 110

cag gct ttg gac aaa cac gaa ggg ctg cta ttc atc acg ccg cac atc 384
Gln Ala Leu Asp Lys His Glu Gly Leu Leu Phe Ile Thr Pro His Ile
        115                 120                 125

ggc agc tac gat ttg ggc gga cgc tac atc agc cag cag ctt ccg ttc 432
Gly Ser Tyr Asp Leu Gly Gly Arg Tyr Ile Ser Gln Gln Leu Pro Phe
    130                 135                 140

ccg ctg acc gcc atg tac aaa ccg ccg aaa atc aaa gcg ata gac aaa 480
Pro Leu Thr Ala Met Tyr Lys Pro Pro Lys Ile Lys Ala Ile Asp Lys
145                 150                 155                 160

atc atg cag gcg ggc agg gtt cgc ggc aaa gga aaa acc gcg cct acc 528
Ile Met Gln Ala Gly Arg Val Arg Gly Lys Gly Lys Thr Ala Pro Thr
                165                 170                 175

agc ata caa ggg gtc aaa caa atc atc aaa gcc ctg cgt tcg ggc gaa 576
Ser Ile Gln Gly Val Lys Gln Ile Ile Lys Ala Leu Arg Ser Gly Glu
            180                 185                 190

gca acc atc gtc ctg ccc gac cac gtc ccc tcc cct caa gaa ggc ggg 624
Ala Thr Ile Val Leu Pro Asp His Val Pro Ser Pro Gln Glu Gly Gly
        195                 200                 205

gaa ggc gta tgg gtg gat ttc ttc ggc aaa cct gcc tat acc atg acg 672
Glu Gly Val Trp Val Asp Phe Phe Gly Lys Pro Ala Tyr Thr Met Thr
    210                 215                 220

ctg gcg gca aaa ttg gca cac gtc aaa ggc gtg aaa acc ctg ttt ttc 720
Leu Ala Ala Lys Leu Ala His Val Lys Gly Val Lys Thr Leu Phe Phe

225                 230                 235                 240

tgc tgc gaa cgc ctg cct ggc gga caa ggt ttc gat ttg cac atc cgc       768
Cys Cys Glu Arg Leu Pro Gly Gly Gln Gly Phe Asp Leu His Ile Arg
                245                 250                 255

ccc gtc caa ggg gaa ttg aac ggc gac aaa gcc cat gat gcc gcc gtg       816
Pro Val Gln Gly Glu Leu Asn Gly Asp Lys Ala His Asp Ala Ala Val
            260                 265                 270

ttc aac cgc aat gcc gaa tat tgg ata cgc cgt ttt ccg acg cag tat       864
Phe Asn Arg Asn Ala Glu Tyr Trp Ile Arg Arg Phe Pro Thr Gln Tyr
        275                 280                 285

ctg ttt atg tac aac cgc tac aaa atg ccg                               894
Leu Phe Met Tyr Asn Arg Tyr Lys Met Pro
    290                 295


<210> 78
<211> 936
<212> DNA
<213> Haemophilus influenzae

<220>
<221> CDS
<222> (1)...(936)

<400> 78
atg aaa aac gaa aaa ctc cct caa ttt caa ccg cac ttt tta gcc cca        48
Met Lys Asn Glu Lys Leu Pro Gln Phe Gln Pro His Phe Leu Ala Pro
 1               5                  10                  15

aaa tac tgg ctt ttt tgg cta ggc gtg gca att tgg cga agt att tta        96
Lys Tyr Trp Leu Phe Trp Leu Gly Val Ala Ile Trp Arg Ser Ile Leu
            20                  25                  30

tgt ctt ccc tat cct att ttg cgc cat att ggt cat ggt ttc ggt tgg       144
Cys Leu Pro Tyr Pro Ile Leu Arg His Ile Gly His Gly Phe Gly Trp
        35                  40                  45

ctg ttt tca cat tta aaa gtg ggt aaa cgt cga gct gcc att gca cgc       192
Leu Phe Ser His Leu Lys Val Gly Lys Arg Arg Ala Ala Ile Ala Arg
    50                  55                  60

cgt aat ctt gaa ctt tgt ttc cct gat atg cct gaa aac gaa cgt gag       240
Arg Asn Leu Glu Leu Cys Phe Pro Asp Met Pro Glu Asn Glu Arg Glu
65                  70                  75                  80

acg att ttg caa gaa aat ctt cgt tca gta ggc atg gca att atc gaa       288
Thr Ile Leu Gln Glu Asn Leu Arg Ser Val Gly Met Ala Ile Ile Glu
                85                  90                  95

act ggc atg gct tgg ttt tgg tcg gat tca cgt atc aaa aaa tgg tcg       336
Thr Gly Met Ala Trp Phe Trp Ser Asp Ser Arg Ile Lys Lys Trp Ser
            100                 105                 110

aaa gtt gaa ggc tta cat tat cta aaa gaa aat caa aaa gat gga att       384
Lys Val Glu Gly Leu His Tyr Leu Lys Glu Asn Gln Lys Asp Gly Ile
        115                 120                 125

gtt ctc gtc ggt gtt cat ttc tta acg cta gaa ctt ggc gca cgc atc     432
Val Leu Val Gly Val His Phe Leu Thr Leu Glu Leu Gly Ala Arg Ile
    130                 135                 140

att ggt tta cat cat cct ggc att ggt gtt tat cgt cca aat gat aat     480
Ile Gly Leu His His Pro Gly Ile Gly Val Tyr Arg Pro Asn Asp Asn
145                 150                 155                 160

cct ttg ctt gat tgg cta caa aca caa ggc cgt tta cgc tcc aat aaa     528
Pro Leu Leu Asp Trp Leu Gln Thr Gln Gly Arg Leu Arg Ser Asn Lys
                165                 170                 175

gat atg ctt gat cgt aaa gat tta cgc gga atg atc aaa gct tta cgc     576
Asp Met Leu Asp Arg Lys Asp Leu Arg Gly Met Ile Lys Ala Leu Arg
            180                 185                 190

cac gaa gaa acc att tgg tat gcg cct gat cac gat tac ggc aga aaa     624
His Glu Glu Thr Ile Trp Tyr Ala Pro Asp His Asp Tyr Gly Arg Lys
        195                 200                 205

aat gcc gtt ttt gtt cct ttt ttt gca gta cct gac act tgc act act     672
Asn Ala Val Phe Val Pro Phe Phe Ala Val Pro Asp Thr Cys Thr Thr
    210                 215                 220

act ggt agt tat tat tta ttg aaa tcc tcg caa aac agc aaa gtg att     720
Thr Gly Ser Tyr Tyr Leu Leu Lys Ser Ser Gln Asn Ser Lys Val Ile
225                 230                 235                 240

cca ttt gcg cca tta cgc aat aaa gat ggt tca ggc tat acc gtg agt     768
Pro Phe Ala Pro Leu Arg Asn Lys Asp Gly Ser Gly Tyr Thr Val Ser
                245                 250                 255

att tca gcg cct gtt gat ttt acg gat tta caa gat gaa acg gcg att     816
Ile Ser Ala Pro Val Asp Phe Thr Asp Leu Gln Asp Glu Thr Ala Ile
            260                 265                 270

gct gcg cga atg aat caa atc gta gaa aag gaa atc atg aag ggc ata     864
Ala Ala Arg Met Asn Gln Ile Val Glu Lys Glu Ile Met Lys Gly Ile
        275                 280                 285

tca caa tat atg tgg cta cat cgc cgt ttt aaa aca cgt cca gat gaa     912
Ser Gln Tyr Met Trp Leu His Arg Arg Phe Lys Thr Arg Pro Asp Glu
    290                 295                 300

aat acg cct agt tta tac gat taa                                     936
Asn Thr Pro Ser Leu Tyr Asp  *
305                 310

<210> 79
<211> 957
<212> DNA
<213> Haemophilus influenzae

<220>
<221> CDS
<222> (1)...(957)

<400> 79
atg tcg gat aat caa caa aat tta cgt ttg acg gcg aga gtg ggc tat      48
Met Ser Asp Asn Gln Gln Asn Leu Arg Leu Thr Ala Arg Val Gly Tyr

1                 5                   10                  15

gaa gcg cac ttt tca tgg tcg tat tta aag cct caa tat tgg ggg att      96
Glu Ala His Phe Ser Trp Ser Tyr Leu Lys Pro Gln Tyr Trp Gly Ile
                20                  25                  30

tgg ctt ggt att ttc ttt tta ttg ttg tta gca ttt gtg cct ttt cgt      144
Trp Leu Gly Ile Phe Phe Leu Leu Leu Leu Ala Phe Val Pro Phe Arg
            35                  40                  45

ctg cgc gat aaa ttg acg gga aaa tta ggt att tgg att ggg cat aaa      192
Leu Arg Asp Lys Leu Thr Gly Lys Leu Gly Ile Trp Ile Gly His Lys
        50                  55                  60

gca aag aaa cag cgt acg cgt gca caa act aac ttg caa tat tgt ttc      240
Ala Lys Lys Gln Arg Thr Arg Ala Gln Thr Asn Leu Gln Tyr Cys Phe
 65                  70                  75                  80

cct cat tgg act gaa caa caa cgt gag caa gtg att gat aaa atg ttt      288
Pro His Trp Thr Glu Gln Gln Arg Glu Gln Val Ile Asp Lys Met Phe
                 85                  90                  95

gcg gtt gtc gct cag gtt atg ttt ggt att ggt gag att gcc atc cgt      336
Ala Val Val Ala Gln Val Met Phe Gly Ile Gly Glu Ile Ala Ile Arg
            100                 105                 110

tca aag aaa cat ttg caa aaa cgc agc gaa ttt atc ggt ctt gaa cat      384
Ser Lys Lys His Leu Gln Lys Arg Ser Glu Phe Ile Gly Leu Glu His
        115                 120                 125

atc gaa cag gca aaa gct gaa gga aag aat att att ctt atg gtg cca      432
Ile Glu Gln Ala Lys Ala Glu Gly Lys Asn Ile Ile Leu Met Val Pro
    130                 135                 140

cat ggc tgg gcg att gat gcg tct ggc att att ttg cac act caa ggc      480
His Gly Trp Ala Ile Asp Ala Ser Gly Ile Ile Leu His Thr Gln Gly
145                 150                 155                 160

atg cca atg act tct atg tat aat cca cac cgt aat cca ttg gtg gat      528
Met Pro Met Thr Ser Met Tyr Asn Pro His Arg Asn Pro Leu Val Asp
                165                 170                 175

tgg ctt tgg acg att aca cgc caa cgt ttc ggc gga aaa atg cat gca      576
Trp Leu Trp Thr Ile Thr Arg Gln Arg Phe Gly Gly Lys Met His Ala
            180                 185                 190

cgc caa aat ggt att aaa cct ttt tta agt cat gtt cgt aaa ggc gaa      624
Arg Gln Asn Gly Ile Lys Pro Phe Leu Ser His Val Arg Lys Gly Glu
        195                 200                 205

atg ggt tat tac tta ccc gat gaa gat ttt ggg gcg gaa caa agc gta      672
Met Gly Tyr Tyr Leu Pro Asp Glu Asp Phe Gly Ala Glu Gln Ser Val
    210                 215                 220

ttt gtt gat ttc ttt ggg act tat aaa gcg aca tta cca ggg tta aat      720
Phe Val Asp Phe Phe Gly Thr Tyr Lys Ala Thr Leu Pro Gly Leu Asn
225                 230                 235                 240

aaa atg gca aaa ctt tct aaa gcc gtt gtt att cca atg ttt cct cgt      768
Lys Met Ala Lys Leu Ser Lys Ala Val Val Ile Pro Met Phe Pro Arg
                245                 250                 255

tat aac gct gaa acg ggc aaa tat gaa atg gaa att cat cct gca atg 816
Tyr Asn Ala Glu Thr Gly Lys Tyr Glu Met Glu Ile His Pro Ala Met
            260                 265                 270

aat tta agt gat gat cct gaa caa tca gcc cga gca atg aac gaa gaa 864
Asn Leu Ser Asp Asp Pro Glu Gln Ser Ala Arg Ala Met Asn Glu Glu
        275                 280                 285

ata gaa tct ttt gtt acg cca gcg cca gag caa tat gtt tgg att ttg 912
Ile Glu Ser Phe Val Thr Pro Ala Pro Glu Gln Tyr Val Trp Ile Leu
    290                 295                 300

caa tta ttg cgt aca agg aaa gat ggc gaa gat ctt tat gat taa 957
Gln Leu Leu Arg Thr Arg Lys Asp Gly Glu Asp Leu Tyr Asp *
305                 310                 315

<210> 80
<211> 1046
<212> DNA
<213> Moraxella catarrhalis

<220>
<221> CDS
<222> (1)...(1044)

<400> 80
atg agt tgc cat cat cag cat aag cag aca ccc aaa cac gcc ata tcc 48
Met Ser Cys His His Gln His Lys Gln Thr Pro Lys His Ala Ile Ser
1               5                   10                  15

att aag cat atg cca agc ttg aca gat act cat aaa caa agt agc caa 96
Ile Lys His Met Pro Ser Leu Thr Asp Thr His Lys Gln Ser Ser Gln
            20                  25                  30

gct gag cca aaa tcg ttt gaa tgg gcg ttt tta cat ccc aaa tat tgg 144
Ala Glu Pro Lys Ser Phe Glu Trp Ala Phe Leu His Pro Lys Tyr Trp
        35                  40                  45

gga gtt tgg ctg gct ttt gcg ttg att tta ccg ctg att ttt cta ccg 192
Gly Val Trp Leu Ala Phe Ala Leu Ile Leu Pro Leu Ile Phe Leu Pro
    50                  55                  60

ctg cgt tgg cag ttt tgg atc ggc aag cgt ctt ggc att ttg gta cat 240
Leu Arg Trp Gln Phe Trp Ile Gly Lys Arg Leu Gly Ile Leu Val His
65                  70                  75                  80

tac tta gct aaa agc cga gtt caa gac act cta acc aac ctg cag ctt 288
Tyr Leu Ala Lys Ser Arg Val Gln Asp Thr Leu Thr Asn Leu Gln Leu
                85                  90                  95

acc ttc cca aat caa cca aaa tca aaa cac aag gcc acc gca cgg caa 336
Thr Phe Pro Asn Gln Pro Lys Ser Lys His Lys Ala Thr Ala Arg Gln
            100                 105                 110

gta ttt att aat caa ggt att ggt att ttt gaa agt tta tgt gca tgg 384
Val Phe Ile Asn Gln Gly Ile Gly Ile Phe Glu Ser Leu Cys Ala Trp
        115                 120                 125

ttt cgc cct aat gtc ttt aaa cgc act ttt agc att tct ggt tta cag 432

Phe Arg Pro Asn Val Phe Lys Arg Thr Phe Ser Ile Ser Gly Leu Gln
    130                 135                 140

cat ttg att gat gcc caa aaa caa aat aaa gcg gtg att tta ctt ggt      480
His Leu Ile Asp Ala Gln Lys Gln Asn Lys Ala Val Ile Leu Leu Gly
145                 150                 155                 160

gga cat cgc acg acg ctt gat ttg ggc ggt cgg tta tgt aca cag ttt      528
Gly His Arg Thr Thr Leu Asp Leu Gly Gly Arg Leu Cys Thr Gln Phe
                165                 170                 175

ttt gcg gcg gac tgc gtg tat cgc cca caa aac aac cct ttg ctt gaa      576
Phe Ala Ala Asp Cys Val Tyr Arg Pro Gln Asn Asn Pro Leu Leu Glu
            180                 185                 190

tgg ttt atc tat aat gca cgc cgc tgt atc ttt gat gag caa atc tca      624
Trp Phe Ile Tyr Asn Ala Arg Arg Cys Ile Phe Asp Glu Gln Ile Ser
        195                 200                 205

aat cgt gat atg aaa aaa ctc atc act cgg ctc aaa caa ggt cgg ata      672
Asn Arg Asp Met Lys Lys Leu Ile Thr Arg Leu Lys Gln Gly Arg Ile
    210                 215                 220

att tgg tat tca cct gat caa gat ttt ggt ctt gag cat ggc gtg atg      720
Ile Trp Tyr Ser Pro Asp Gln Asp Phe Gly Leu Glu His Gly Val Met
225                 230                 235                 240

gcg acc ttt ttt ggt gtg cct gca gca acg att acc gct cag cgt cgt      768
Ala Thr Phe Phe Gly Val Pro Ala Ala Thr Ile Thr Ala Gln Arg Arg
                245                 250                 255

ctt att aag ctg ggt gat aaa gcc aat cct cct gtc atc atc atg atg      816
Leu Ile Lys Leu Gly Asp Lys Ala Asn Pro Pro Val Ile Ile Met Met
            260                 265                 270

gat atg ctc aga caa acg ccc gat tat atc gca aaa ggt cac cgt cca      864
Asp Met Leu Arg Gln Thr Pro Asp Tyr Ile Ala Lys Gly His Arg Pro
        275                 280                 285

cat tat cac atc agc cta agc gct gtg tta aaa aat tat ccc agc gat      912
His Tyr His Ile Ser Leu Ser Ala Val Leu Lys Asn Tyr Pro Ser Asp
    290                 295                 300

gac gaa acc gcc gat gct gaa cgc atc aat cga ctg att gag caa aat      960
Asp Glu Thr Ala Asp Ala Glu Arg Ile Asn Arg Leu Ile Glu Gln Asn
305                 310                 315                 320

att caa aaa gat tta acc cag tgg atg tgg ttt cat cgc cgc ttt aaa     1008
Ile Gln Lys Asp Leu Thr Gln Trp Met Trp Phe His Arg Arg Phe Lys
                325                 330                 335

act caa gcc gat gac acc aat tac tat caa cat taa tg                  1046
Thr Gln Ala Asp Asp Thr Asn Tyr Tyr Gln His  *
            340                 345

<210> 81
<211> 876
<212> DNA
<213> Neisseria meningitidis

<220>
<221> CDS
<222> (1)...(876)

<400> 81
atg aaa ttt ata ttt ttt gta ctg tat gtt ttg cag ttt ctg ccg ttt      48
Met Lys Phe Ile Phe Phe Val Leu Tyr Val Leu Gln Phe Leu Pro Phe
1               5                   10                  15

gcg ctg ctg cac aaa ctt gcc gac ctg acg ggt ttg ctc gcc tac ctt      96
Ala Leu Leu His Lys Leu Ala Asp Leu Thr Gly Leu Leu Ala Tyr Leu
            20                  25                  30

ttg gtc aaa ccc cgc cgc cgt atc ggc gaa atc aat ttg gca aaa tgc      144
Leu Val Lys Pro Arg Arg Arg Ile Gly Glu Ile Asn Leu Ala Lys Cys
        35                  40                  45

ttt ccc gag tgg gac gga aaa aag cgc gaa acc gta ttg aag cag cat      192
Phe Pro Glu Trp Asp Gly Lys Lys Arg Glu Thr Val Leu Lys Gln His
    50                  55                  60

ttc aaa cat atg gcg aaa ctg atg ctt gaa tac ggc tta tat tgg tac      240
Phe Lys His Met Ala Lys Leu Met Leu Glu Tyr Gly Leu Tyr Trp Tyr
65                  70                  75                  80

gcg cct gcc ggg cgt ttg aaa tcg ctg gtg cgt tac cgc aat aag cat      288
Ala Pro Ala Gly Arg Leu Lys Ser Leu Val Arg Tyr Arg Asn Lys His
                85                  90                  95

tat ttg gac gac gcg ctg gcg gcg ggg gaa aaa gtc atc att ctg tac      336
Tyr Leu Asp Asp Ala Leu Ala Ala Gly Glu Lys Val Ile Ile Leu Tyr
            100                 105                 110

ccg cac ttc acc gcg ttc gag atg gcg gtg tac gcg ctt aat cag gat      384
Pro His Phe Thr Ala Phe Glu Met Ala Val Tyr Ala Leu Asn Gln Asp
        115                 120                 125

gta ccg ctg atc agt atg tat tcc cac caa aaa aac aag ata ttg gac      432
Val Pro Leu Ile Ser Met Tyr Ser His Gln Lys Asn Lys Ile Leu Asp
    130                 135                 140

gca cag att ttg aaa ggc cgc aac cgc tac gac aat gtc ttc ctt atc      480
Ala Gln Ile Leu Lys Gly Arg Asn Arg Tyr Asp Asn Val Phe Leu Ile
145                 150                 155                 160

ggg cgc acc gaa ggc gtg cgc gcc ctc gtc aaa cag ttc cgc aaa agc      528
Gly Arg Thr Glu Gly Val Arg Ala Leu Val Lys Gln Phe Arg Lys Ser
                165                 170                 175

agc gcg ccg ttt ctg tat ctg ccc gat cag gat ttc gga cgc aac gat      576
Ser Ala Pro Phe Leu Tyr Leu Pro Asp Gln Asp Phe Gly Arg Asn Asp
            180                 185                 190

tcg gtt ttt gtg gat ttt ttc ggt att cag acg gca acg att acc ggc      624
Ser Val Phe Val Asp Phe Phe Gly Ile Gln Thr Ala Thr Ile Thr Gly
        195                 200                 205

ttg agc cgc att gcc gcg ctt gca aat gca aaa gtg ata ccc gcc atc      672
Leu Ser Arg Ile Ala Ala Leu Ala Asn Ala Lys Val Ile Pro Ala Ile
    210                 215                 220

ccc gtc cgc gag gcg gac aat acg gtt aca ttg cat ttc tac ccg gct      720
Pro Val Arg Glu Ala Asp Asn Thr Val Thr Leu His Phe Tyr Pro Ala
225                 230                 235                 240

tgg gaa tcc ttt ccg agt gaa gat gcg cag gcc gac gcg cag cgc atg      768
Trp Glu Ser Phe Pro Ser Glu Asp Ala Gln Ala Asp Ala Gln Arg Met
                245                 250                 255

aac cgt ttt atc gag gaa ccg tgc gcg aac atc ccg agc agt att ttt      816
Asn Arg Phe Ile Glu Glu Pro Cys Ala Asn Ile Pro Ser Ser Ile Phe
            260                 265                 270

ggc tgc aca agc gtt tca aaa ccc gtc cgg aag gca gcc ccg att ttt      864
Gly Cys Thr Ser Val Ser Lys Pro Val Arg Lys Ala Ala Pro Ile Phe
        275                 280                 285

act gat acg taa                                                      876
Thr Asp Thr  *
    290

<210> 82
<211> 38
<212> DNA
<213> Artificial Sequence

<220>
<223> PorA5' Fwd primer

<400> 82
cccaagcttg ccgtctgaat acatcccgtc attcctca                              38

<210> 83
<211> 34
<212> DNA
<213> Artificial Sequence

<220>
<223> PorA5'Rev primer

<400> 83
cgatgctcgc gactccagag acctcgtgcg ggcc                                  34

<210> 84
<211> 38
<212> DNA
<213> Artificial Sequence

<220>
<223> PorA3' Fwd  primer

<400> 84
ggaagatctg attaaatagg cgaaaatacc agctacga                              38

<210> 85
<211> 37
<212> DNA
<213> Artificial Sequence

<220>
<223> PorA3'Rev  primer

<400> 85
gccgaattct tcagacggcg cagcaggaat ttatcgg                                37

<210> 86
<211> 41
<212> DNA
<213> Artificial Sequence

<220>
<223> PoLa Rev1 primer

<400> 86
gaattgttat ccgctcacaa ttccgggcaa acacccgata c                           41

<210> 87
<211> 70
<212> DNA
<213> Artificial Sequence

<220>
<223> PoLa Rev2 primer

<400> 87
gaattccata tgatcggctt ccttttgtaa atttgataaa aacctaaaaa catcgaattg        60
ttatccgctc                                                              70

<210> 88
<211> 30
<212> DNA
<213> Artificial Sequence

<220>
<223> PorAlacO Fwd primer

<400> 88
aagctctgca ggaggtctgc gcttgaattg                                        30

<210> 89
<211> 28
<212> DNA
<213> Artificial Sequence

<220>
<223> PorAlacO Rev primer

<400> 89
cttaaggcat atgggcttcc ttttgtaa                                          28

<210> 90
<211> 22
<212> DNA
<213> Artificial Sequence

<220>
<223> PPA1  primer

<400> 90
gcggccgttg ccgatgtcag cc                                                22

<210> 91

<211> 24
<212> DNA
<213> Artificial Sequence

<220>
<223> PPA2  primer

<400> 91
ggcatagctg atgcgtggaa ctgc                                              24

<210> 92
<211> 33
<212> DNA
<213> Artificial Sequence

<220>
<223> N-full-01 primer

<400> 92
gggaattcca tatgaaaaaa gcacttgcca cac                                    33

<210> 93
<211> 31
<212> DNA
<213> Artificial Sequence

<220>
<223> Nde-NspA-3  primer

<400> 93
ggaattccat atgtcagaat ttgacgcgca c                                      31

<210> 94
<211> 30
<212> DNA
<213> Artificial Sequence

<220>
<223> PNS1 primer

<400> 94
ccgcgaattc ggaaccgaac acgccgttcg                                        30

<210> 95
<211> 27
<212> DNA
<213> Artificial Sequence

<220>
<223> PNS1 primer

<400> 95
cgtctagacg tagcggtatc cggctgc                                           27

<210> 96
<211> 38
<212> DNA
<213> Artificial Sequence

<220>
<223> PromD15-51X primer

<400> 96
gggcgaattc gcggccgccg tcaacggcac acccgttg                                38

<210> 97
<211> 28
<212> DNA
<213> Artificial Sequence

<220>
<223> ProD15-52 primer

<400> 97
gctctagagc ggaatgcggt ttcagacg                                          28

<210> 98
<211> 47
<212> DNA
<213> Artificial Sequence

<220>
<223> PNS4 primer

<400> 98
agctttattt aaatccttaa ttaacgcgtc cggaaaatat gcttatc                     47

<210> 99
<211> 33
<212> DNA
<213> Artificial Sequence

<220>
<223> PNS5 primer

<400> 99
agctttgttt aaaccctgtt ccgctgcttc ggc                                    33

<210> 100
<211> 43
<212> DNA
<213> Artificial Sequence

<220>
<223> D15-S4 primer

<400> 100
gtccgcattt aaatccttaa ttaagcagcc ggacagggcg tgg                         43

<210> 101
<211> 33
<212> DNA
<213> Artificial Sequence

<220>
<223> D15-S5 primer

<400> 101
agctttgttt aaaggatcag ggtgtggtcg ggc                                    33

<210> 102
<211> 28

<212> DNA
<213> Artificial Sequence

<220>
<223> DT88 primer

<400> 102
gaagagaagg tggaaatggc gttttggc                                          28

<210> 103
<211> 27
<212> DNA
<213> Artificial Sequence

<220>
<223> DT89 primer

<400> 103
ccaaaacgcc atttccacct tctcttc                                           27

<210> 104
<211> 25
<212> DNA
<213> Artificial Sequence

<220>
<223> PorA3 primer

<400> 104
ccaaatcctc gctcccctta aagcc                                             25

<210> 105
<211> 24
<212> DNA
<213> Artificial Sequence

<220>
<223> p1-2 primer

<400> 105
cgctgatttt cgtcctgatg cggc                                              24

<210> 106
<211> 25
<212> DNA
<213> Artificial Sequence

<220>
<223> p1-1 primer

<400> 106
ggtcaattgc gcctggatgt tcctg                                             25

<210> 107
<211> 26
<212> DNA
<213> Artificial Sequence

<220>
<223> porB1 primer

<400> 107
ggtagcggtt gtaacttcag taactt                                          26

<210> 108
<211> 25
<212> DNA
<213> Artificial Sequence

<220>
<223> porB2 primer

<400> 108
gtcttcttgg cctttgaagc cgatt                                           25

<210> 109
<211> 25
<212> DNA
<213> Artificial Sequence

<220>
<223> porB3 primer

<400> 109
ggagtcagta ccggcgatag atgct                                           25

<210> 110
<211> 37
<212> DNA
<213> Artificial Sequence

<220>
<223> ProD15-51X primer

<400> 110
gggcgaattc gcggccgccg tcaacggcac accgttg                              37

<210> 111
<211> 43
<212> DNA
<213> Artificial Sequence

<220>
<223> TnRD15-KpnI/XbaI + US   primer

<400> 111
cgccggtacc tctagagccg tctgaaccac tcgtggacaa ccc                       43

<210> 112
<211> 29
<212> DNA
<213> Artificial Sequence

<220>
<223> TnR03Cam(KpnI) primer

<400> 112
cgccggtacc gccgctaact ataacggtc                                       29

<210> 113
<211> 31
<212> DNA

<213> Artificial Sequence

<220>
<223> PorA-01 primer

<400> 113
cgccggtacc gaggtctgcg cttgaattgt g                                    31

<210> 114
<211> 33
<212> DNA
<213> Artificial Sequence

<220>
<223> PorA02 primer

<400> 114
cgccggtacc tctagacatc gggcaaacac ccg                                  33

<210> 115
<211> 20
<212> DNA
<213> Artificial Sequence

<220>
<223> Cam-05 primer

<400> 115
gtactgcgat gagtggcagg                                                 20

<210> 116
<211> 31
<212> DNA
<213> Artificial Sequence

<220>
<223> Hsf 01-Nde primer

<400> 116
ggaattccat atgatgaaca aaatataccg c                                    31

<210> 117
<211> 31
<212> DNA
<213> Artificial Sequence

<220>
<223> Hsf 02-Nhe primer

<400> 117
gtagctagct agcttaccac tgataaccga c                                    31

<210> 118
<211> 36
<212> DNA
<213> Artificial Sequence

<220>
<223> GFP-mut-Asn primer

<400> 118

aactgcagaa ttaatatgaa aggagaagaa cttttc 36

<210> 119
<211> 33
<212> DNA
<213> Artificial Sequence

<220>
<223> GFP-Spe primer

<400> 119
gacatactag tttatttgta gagctcatcc atg 33

<210> 120
<211> 30
<212> DNA
<213> Artificial Sequence

<220>
<223> RP1 (SacII) primer

<400> 120
tccccgcggg ccgtctgaat acatcccgtc 30

<210> 121
<211> 51
<212> DNA
<213> Artificial Sequence

<220>
<223> RP2 primer

<400> 121
   catatgggct tccttttgta aatttgaggg caaacacccg atacgtcttc a 51

<210> 122
<211> 48
<212> DNA
<213> Artificial Sequence

<220>
<223> RP3 primer

<400> 122
agacgtatcg ggtgtttgcc ctcaaattta caaaaggaag cccatatg 48

<210> 123
<211> 33
<212> DNA
<213> Artificial Sequence

<220>
<223> RP4 (ApaI) primer

<400> 123
gggtattccg ggcccttcag acggcgcagc agg 33

<210> 124
<211> 28
<212> DNA
<213> Artificial Sequence

<220>
<223> PNS1' primer

<400> 124
ccgcgaattc gacgaagccg ccctcgac                                          28

<210> 125
<211> 37
<212> DNA
<213> Artificial Sequence

<220>
<223> BAD01-2 primer

<400> 125
ggcgcccggg ctcgagctta tcgatggaaa acgcagc                                37

<210> 126
<211> 47
<212> DNA
<213> Artificial Sequence

<220>
<223> BAD02-2  primer

<400> 126
ggcgcccggg ctcgagttca gacggcgcgc ttatatagtg gattaac                     47

<210> 127
<211> 39
<212> DNA
<213> Artificial Sequence

<220>
<223> BAD 15-2 primer

<400> 127
ggcgcccggg ctcgagtcta gacatcgggc aaacacccg                              39

<210> 128
<211> 39
<212> DNA
<213> Artificial Sequence

<220>
<223> BAD 03-2  primer

<400> 128
ggcgcccggg ctcgagcact agtattaccc tgttatccc                              39

<210> 129
<211> 19
<212> DNA
<213> Artificial Sequence

<220>
<223> BAD 25   primer

<400> 129
gagcgaagcc gtcgaacgc                                                    19

<210> 130
<211> 20
<212> DNA
<213> Artificial Sequence

<220>
<223> BAD08 primer

<400> 130
cttaagcgtc ggacatttcc                                                    20

<210> 131
<211> 31
<212> DNA
<213> Artificial Sequence

<220>
<223> PLA1 Amo5   primer

<400> 131
gccgtctgaa tttaaaattg cgcgtttaca g                                       31

<210> 132
<211> 38
<212> DNA
<213> Artificial Sequence

<220>
<223> PLA1 Amo3   primer

<400> 132
gtagtctaga ttcagacggc gcaatttggt ttccgcac                                38

<210> 133
<211> 27
<212> DNA
<213> Artificial Sequence

<220>
<223> CIRC1-Bgl  primer

<400> 133
cctagatctc tccgcccccc attgtcg                                            27

<210> 134
<211> 46
<212> DNA
<213> Artificial Sequence

<220>
<223> CIRC1-XH-RBS/2  primer

<400> 134
ccgctcgagt acaaaaggaa gccgatatga atatacggaa tatgcg                       46

<210> 135
<211> 24
<212> DNA
<213> Artificial Sequence

<220>
<223> CIRC2-XHO/2 primer

<400> 135
ccgctcgaga tgaatatacg gaat 24

<210> 136
<211> 38
<212> DNA
<213> Artificial Sequence

<220>
<223> BAD20 primer

<400> 136
tcccccggga gatctcacta gtattaccct gttatccc 38

<210> 137
<211> 32
<212> DNA
<213> Artificial Sequence

<220>
<223> CM-PORA-3 primer

<400> 137
ccgctcgaga taaaaaccta aaaacatcgg gc 32

<210> 138
<211> 28
<212> DNA
<213> Artificial Sequence

<220>
<223> CM-PORA-D15/3 primer

<400> 138
cggctcgagt gtcagttcct tgtggtgc 28

<210> 139
<211> 45
<212> DNA
<213> Artificial Sequence

<220>
<223> BAD16 primer

<400> 139
ggcctagcta gccgtctgaa gcgattagag tttcaaaatt tattc 45

<210> 140
<211> 42
<212> DNA
<213> Artificial Sequence

<220>
<223> BAD17 primer

<400> 140
ggccaagctt cagacggcgt tcgaccgagt ttgagccttt gc 42

<210> 141
<211> 39
<212> DNA
<213> Artificial Sequence

<220>
<223> BAD18  primer

<400> 141
tcccccggga agatctggac gaaaaatctc aagaaaccg                              39

<210> 142
<211> 64
<212> DNA
<213> Artificial Sequence

<220>
<223> BAD19 primer

<400> 142
ggaagatctc cgctcgagca aatttacaaa aggaagccga tatgcaacag caacatttgt       60
tccg                                                                    64

<210> 143
<211> 36
<212> DNA
<213> Artificial Sequence

<220>
<223> BAD21 primer

<400> 143
ggaagatctc cgctcgagac atcgggcaaa cacccg                                 36

<210> 144
<211> 36
<212> DNA
<213> Artificial Sequence

<220>
<223> PQ-rec5-Nhe   primer

<400> 144
ctagctagcg ccgtctgaac gacgcgaagc caaagc                                 36

<210> 145
<211> 37
<212> DNA
<213> Artificial Sequence

<220>
<223> PQ-rec3-Hin  primer

<400> 145
gccaagcttt tcagacggca cggtatcgtc cgattcg                                37

<210> 146
<211> 30
<212> DNA
<213> Artificial Sequence

<220>
<223> CIRC1-PQ-Bgl primer

<400> 146
ggaagatcta atggagtaat cctcttctta 30

<210> 147
<211> 50
<212> DNA
<213> Artificial Sequence

<220>
<223> CIRC1-PQ-XHO primer

<400> 147
ccgctcgagt acaaaaggaa gccgatatga ttaccaaact gacaaaaatc 50

<210> 148
<211> 33
<212> DNA
<213> Artificial Sequence

<220>
<223> CIRC2-PQ-X primer

<400> 148
ccgctcgaga tgaataccaa actgacaaaa atc 33

<210> 149
<211> 40
<212> DNA
<213> Artificial Sequence

<220>
<223> CM-PORA-3 primer

<400> 149
ccgctcgaga taaaaaccta aaaacatcgg gcaaacaccc 40

<210> 150
<211> 28
<212> DNA
<213> Artificial Sequence

<220>
<223> CM-PORA-D153 primer

<400> 150
gggctcgagt gtcagttcct tgtggtgc 28

<210> 151
<211> 32
<212> DNA
<213> Artificial Sequence

<220>
<223> CIRC-Kan-Nco primer

<400> 151
catgccatgg ttagaaaaac tcatcgagca tc 32

<210> 152
<211> 31
<212> DNA
<213> Artificial Sequence

<220>
<223> CIRC-Kan-Xba primer

<400> 152
ctagtctaga tcagaattgg ttaattggtt g 31

<210> 153
<211> 43
<212> DNA
<213> Artificial Sequence

<220>
<223> SAC/NCO/NEW5 primer

<400> 153
catgccatgg gaggatgaac gatgaacatc aaaaagtttg caa 43

<210> 154
<211> 33
<212> DNA
<213> Artificial Sequence

<220>
<223> SAC/NCO/NEW3 primer

<400> 154
gatcccatgg ttatttgtta actgttaatt gtc 33

<210> 155
<211> 72
<212> DNA
<213> Artificial Sequence

<220>
<223> Kan-PorA-5 primer

<400> 155
gccgtctgaa cccgtcattc ccgcgcaggc gggaatccag tccgttcagt ttcgggaaag 60
ccacgttgtg tc 72

<210> 156
<211> 69
<212> DNA
<213> Artificial Sequence

<220>
<223> Kan-PorA-3 primer

<400> 156
ttcagacggc gcagcaggaa tttatcggaa ataactgaaa ccgaacagac taggctgagg 60
tctgcctcg 69